# EUROPEAN PATENT APPLICATION

(11) **EP 4 563 146 A1**
(43) Date of publication of application: **04.06.2025**
(21) Application number: 23212518.7
(22) Date of filing: 28.11.2023
(51) Int. Cl.: A61K 31/4245, A61P 31/12

(54) **FLAVIVIRUS INHIBITORS**

(71) Applicant: IRBM S.P.A., 00071 Pomezia (RM) (IT); C.N.C.C.S. S.c.a.r.l. Collezione Nazionale Dei Composti Chimici e Centro Screening, 00071 Pomezia (RM) (IT)
(72) Inventor: ONTORIA ONTORIA, Jesus Maria, 00071 Pomezia (RM) (IT); MONTALBETTI, Christian, 00071 Pomezia (RM) (IT); AMAUDRUT, Jerome, 21100 Dijon (FR); DI FABIO, Romano, 37138 Verona (VR) (IT); BENCHEVA, Leda Ivanova, 26839 Zelo Buon Persico (LO) (IT); QUOTADAMO, Antonio, 00071 Pomezia (RM) (IT); TORRENTE DE HARO, Esther, 00071 Pomezia (RM) (IT); CORIO, Alessandra, 00071 Pomezia (RM) (IT); IEVOLI, Giovanni, 00071 Pomezia (RM) (IT)
(74) Representative: Pace Napoleone, Maria

(57) **Abstract**

The invention relates to compounds that are inhibitors of Flavivirus NS2B-NS3 proteases and inhibit replication of flavivirus in cells. Compounds of this invention are useful alone or in combination with other agents for use in the treatment of infections caused by Flaviviruses, in particular by Dengue, West Nile, Zika, Japan Encephalitis viruses.

## Description

### SUMMARY OF THE INVENTION

The present invention relates to compounds that are inhibitors of Flavivirus NS2B-NS3 proteases and inhibit replication of flavivirus in cells. Compounds of this invention are useful alone or in combination with other agents for use in the treatment of infections caused by Flaviviruses, in particular by Dengue, West Nile, Zika, Japan Encephalitis viruses. The present invention also relates to pharmaceutical compositions containing said compounds.

### BACKGROUND

The Flavivirus genus of the Flaviviridae family includes more than 70 related arthropodborne viruses. The most common and representative members are the dengue virus (DENV) with four serotypes (DENV-1, -2, -3, and -4), Zika (ZIKV), West Nile (WNV), Japanese-encephalitis (JEV), Yellow Fever (YFV), and tick-borne encephalitis (TBEV) viruses. These are the causative agents for viral haemorrhagic fever and encephalitis in human beings. These viruses are transmitted primarily by *Aedes* mosquitos. Infections with flaviviruses are a continuing public health threat The mosquito-borne dengue virus serotypes 1-4 (DENV 1-4) are included in the National Institute of Allergy and Infectious Disease (NIAID) list of Category A, B, or C emerging human pathogens along with yellow fever virus (YFV), West Nile virus (WNV), and Japanese encephalitis virus (JEV). DENVs cause serious illnesses associated with considerable morbidity and mortality. According to World Health Organization estimates, globally over 50 million people suffer from the symptoms of dengue fever annually, and of these at least 250,000 cases develop into dengue hemorrhagic fever (DHF) or dengue shock syndrome (DSS) resulting in considerable mortality, particularly among children in South Asian Countries. The World Health Organization estimates that 2.5 billion people (~40% of the world's population) live in areas that have an increased risk of contracting dengue virus infections. Frequent outbreaks occur in the Americas including the continental U.S., mainly in the Southern states as well as in Puerto Rico and Hawaii.

Zika virus (ZIKV) has caused three major outbreaks in Pacific Ocean islands, Brazil, and other American countries, in which more than 1 million infections were reported and a large number of patients sought medical treatment. More seriously, Zika infection has been correlated with a 20-fold increased incidence of serious neurological disorders, including Guillain-Barre syndrome and more than 4,000 cases of microcephaly in newborns. Zika has quickly spread to 48 Pan-American countries and has recently been found to be transmitted through sex or body fluids.

Inhibitors of two major steps of flaviviral entry have been reported: (i) molecules that block virus-receptor interaction; (ii) compounds that prevent conformational change of viral envelope protein during virus-host membrane fusion (ACS Infect. Dis. 2015, 1, 9, 428-434).

As such, a need exists for more effective compositions and methods for treating viral infections caused by flaviviruses. In addition to vaccine development and vector control, the search for antiviral agents that alleviate symptoms in patients are of considerable interest. Flaviviruses share epidemiological, structural, and ecologic features and often different viruses can co-infect the same host. Therefore, the identification of broad-spectrum inhibitors is highly desirable either for known flaviviruses or for viruses that likely will emerge in the future. Strategies targeting both virus and host factors have been pursued to identify broad-spectrum antiflaviviral agents (https://doi.org/10.1002/cmdc.202000464).

The Flavivirus genome consists of a positive-sense single-stranded RNA which is ~11 kb in length, consisting of a single long open reading frame (ORF). The single ORF encodes a polyprotein that is further processed by proteases from the host and the viral NS2B-NS3 complex. The flavivirus protease is highly conserved and essential for virus replication. The viral polyprotein is processed to three structural proteins (Capsid, pr-Membrane, and Envelope) and seven non-structural (NS) proteins (NS1, NS2A, NS2B, NS3, NS4A, NS4B, and NS5). Three structural proteins form the virus shell, whereas seven NS proteins participate in the membrane-bound replication complex. Among these NS proteins, only NS3 and NS5 bear enzymatic function. (Pathogens 2022, 11 293). Flaviviral replication depends on the NS3 protease, which is a 69 kDa protein with two domains that have different enzymatic functions: a trypsin-like serine protease domain located within the *N*-terminal region; while the C-terminal region has the activities of an RNA-helicase and an RNA-stimulated NTPase. Moreover, it is only entirely activated when associated with its cofactor, namely NS2B; forming an enzymatic complex, NS2B-NS3. Considering this, NS2B-NS3 proteases represent a valuable target for the development of new antiviral compounds, which act inhibiting their replication complex and leading to the flaviviral death.

Flaviviral proteases share a high degree of structural similarity and substrate-recognition profile, which may facilitate a strategy towards development of pan-flaviviral protease inhibitors. However, the success of various drug discovery attempts during the last decade has been limited by the nature of the viral enzyme as well as a lack of robust structural templates. Small-molecular, structurally diverse protease inhibitors have been reported to reach affinities in the lower micromolar range. Peptide-based, substrate-derived compounds are often nanomolar inhibitors, however, with highly compromised drug-likeness. With some exceptions, the antiviral cellular activity of most of the reported compounds has been patchy and insufficient for further development. Recent progress has been made in the elucidation of inhibitor binding using different structural methods. This will hopefully lead to more rational attempts for the identification of various lead compounds that may be successful in cellular assays, animal models and ultimately translated to patients.

WO2021/175437 and WO2021/175670 provide non-peptidic (4-phenoxy)-phenylglycine and (4-benzyloxy)-phenylglycine derivatives as inhibitors of the NS2B-NS3 serine protease for use in the treatment and prevention of flaviviruses infection.

The present invention yields a novel series of drug-like, broadly active inhibitors of flavivirus proteases.

### SUMMARY OF THE INVENTION

The present invention provides a series of compounds targeting the NS2B-NS3 protease. The compounds of the invention are sydnone imine derivatives thus having a mesoionic heterocyclic core properly substituted with a nitrogen derivative substituent at the imino nitrogen-and with an alkyl-aromatic or heteroaromatic substituent. The chemotype discovered in the present invention results in extremely potent ZIKV NS2B-NS3 protease inhibitors, active also on the JEV, Dengue and WNV proteases. The compounds of the invention are capable of inhibiting replication of the above viruses in cells.

It is therefore an object of the invention a compound of general Formula (I): 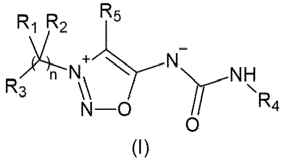 wherein:
n is 0, 1 or 2;
R₁ and R₂ are independently selected from Hand C₁₋₆alkyl optionally substituted with one or more substituents selected from: hydroxy, OC₁₋₃alkyl, halogen, NH₂, NHCOC₁₋₆alkyl, NHCOOC₁₋₆alkyl, NHC(=O)NHC₁₋₆alkyl, NHSO₂C₁₋₆alkyl, NHC₁₋₃alkyl, N(C₁₋₃alkyl)₂, or a cyclic amine selected from aziridine, azetidine, pyrrolidine, pyperidine, morpholine, piperazine, *N-*methylpyperazine;
or R₁ and R₂ are linked together to form a C₃₋₁₀-cycloalkyl ring or a C₄₋₁₂-heterocycloalkyl ring;
R₃ is selected from:
   - saturated or unsaturated C₃₋₁₀cycloalkyl ring, saturated or unsaturated C₃₋₁₀cycloheteroalkyl ring, each of said ring being optionally substituted with one or more substituents independently selected from halogen, hydroxy, NH₂, NHC₁₋₃alkyl, N(C₁₋₃alkyl)₂, C₁₋₆alkylNH₂, C₁₋₆alkylNHC₁₋₃alkyl, C₁₋₆alkylN(C₁₋₃alkyl)₂, C₁₋₆alkylNHC₁₋₃haloalkyl, C₁₋₆alkylC(O)OC₁₋₃alkyl,NHCOC₁₋₆alkyl, NHCOOC₁₋₆alkyl, NHC(=O)NHC₁₋₆alkyl, NHSO₂C₁₋₆alkyl, C₁₋₆alkyl, C₁₋₆haloalkyl, C₁₋₆alkylOH, C(O)OH, C(O)OC₁₋₃alkyl, C(O)NH₂, C(O)NH(C₁₋₃alkyl), C(O)N(C₁₋₃alkyl)₂, optionally substituted aziridine, optionally substituted azetidine, optionally substituted pyrrolidine, optionally substituted pyperidine, optionally substituted morpholine, optionally substituted piperazine, optionally substituted *N*-methylpyperazine, optionally substituted aryl, optionally substituted heteroaryl; or
   - aromatic or heteroaromatic ring optionally substituted with one or more substituents independently selected from halogen, hydroxy, NH₂, NHCOC₁₋₆alkyl, NHCOOC₁₋₆alkyl, NHC(=O)NHC₁₋₆alkyl, NHSO₂C₁₋₆alkyl, NHC₁₋₃alkyl, N(C₁₋₃alkyl)₂, C₁₋₆alkyl, C₁₋₆alkylC(O)OH, C₁₋₆alkylC(O)OC₁₋₃alkyl, hydroxyC₁₋₆alkyl C₃₋₆cycloalkyl, haloC₁₋₆alkyl, C₁₋₆alkoxy, cyano, aryl, heteroaryl wherein said aryl or heteroaryl ring are optionally substituted with one or more substituents each independently selected from halogen, hydroxy, cyano, NH₂, NHCOC₁₋₆alkyl, NHCOOC₁₋₆alkyl, NHC₁₋₃alkyl, N(C₁₋₃alkyl)₂, C₃₋₆cycloalkyl, aziridine, azetidine, pyrrolidine, pyperidine, morpholine, piperazine, *N-*methylpyperazine, C₁₋₆alkyl, C₁₋₆alkoxy, wherein said C₁₋₆alkyl, C₁₋₆alkoxy are optionally substituted with one or more halogen, hydroxy, NH₂, NHCOCH₃, NHCOOC₁₋₆alkyl, NHC₁₋₃alkyl, N(C₁₋₃alkyl)₂, aziridine, azetidine, pyrrolidine, pyperidine, morpholine, piperazine and *N*-methylpyperazine;
R₄ is selected from:
   a) a ring of formula (IIa) or (IIb): 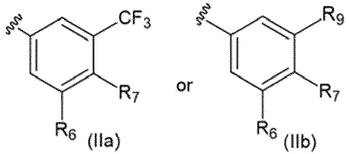 wherein
      - R₆ is selected from NHC(O)Rs, N(C₁₋₆alkyl)C(O)R₈, NHSO₂R₈, NH(C₁₋₆alkyl), NH(C₁₋₆haloalkyl), hydroxy-C₁₋₆alkyl, CN, C(O)OC₁₋₆alkyl, C(O)NHRs, C₂₋₆alkynyl, C₁₋₆alkylNHC(O)R₈, C₁₋₆alkylN(C₁₋₆alkyl)C(O)R₈, and 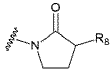 and R₇ is H; or
      - R₆ is H and R₇ is selected from NHC(O)Rs, N(C₁₋₆alkyl)C(O)R₈, NHSO₂R₈, NH(C₁₋₆alkyl), NH(C₁₋₆haloalkyl), hydroxy-C₁₋₆alkyl, CN, C(O)OC₁₋₆alkyl, C(O)NHRs C₂₋₆alkynyl, C₁₋₆alkylNHC(O)R₈, C₁₋₆alkylN(C₁₋₆alkyl)C(O)R₈, and 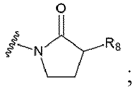 or
      - R₆ and R₇ are H, with the proviso that when R₆ and R₇ are H, R₃ is selected from saturated or unsaturated C₃₋₁₀cycloalkyl ring, saturated or unsaturated C₃₋₁₀heterocycloalkyl ring, each of said ring being optionally substituted with halogen, C₁₋₆alkyl, haloC₁₋₆alkyl, halogen, CN, OH, C₁₋₆alkoxy, amine, optionally substituted aryl, optionally substituted heteroaryl; or when R₆ and R₇ are H, R₃ is (4,6-dimethylpyrimidin-5-yl)pyridazin-3-yl; or
      - R₆ is OCH₃ or OH and R₇ is H, or R₆ is H and R₇ is OCH₃ or C(O)OCH₃, or R₆ and R₇ are joined to form together with the ring of formula (IIa) a 8-(trifluoromethyl)quinoline-6-yl ring, with the proviso that R₃ is 4-(4,6-dimethylpyrimidin-5-yl)phenyl;
      - R₈ is C₁₋₆alkyl, C₁₋₆haloalkyl, C₁₋₆alkyl-aryl, C₁₋₆alkyl-heteroaryl, C₁₋₆alkyl-C₃₋₁₀cycloalkyl, C₁₋₆alkyl-C₃₋₁₀heterocycloalkyl, C₁₋₆haloalkyl-aryl, C₁₋₆haloalkyl-heteroaryl, C₁₋₆haloalkyl-C₃₋₁₀cycloalkyl, C₁₋₆haloalkyl-C₃₋₁₀heterocycloalkyl, C₁₋₆alkylSO₂aryl, C₁₋₆alkylSO₂heterocycloalkyl, C₂₋₆alkynyl-aryl, aryl, heteroaryl, C₃₋₁₀cycloalkyl ring, C₃₋₁₀heterocycloalkyl ring, wherein each of said cycloalkyl and heterocycloalkyl ring is saturated or unsaturated and wherein each of said aryl, heteroaryl, cycloalkyl and herocycloalkyl ring is optionally substituted with one or more substituents independently selected from C₁₋₆alkyl, C₁₋₆alkoxy, C₃₋₁₀cycloalkyl, C₃₋₁₀heteroycloalkyl, amine, CN, C₁₋₃alkyl-aryl, C₁₋₃alkyl-heteroaryl, C₁₋₃alkyl-C₃₋₁₀cycloalkyl, C₁₋₃alkyl-C₃₋₁₀heterocycloalkyl, C₁₋₆alkylCOOH, C₁₋₆alkylCOOCH₃, C₁₋₆alkylCONH₂ COOH, C(O)OC₁₋₃alkyl, NHC(O)C₁₋₆alkyl, NHC(O)C₃₋₆cycloalkyl, C₁₋₆haloalkyl, halogen, C₁₋₆alkylamine, OH, optionally substituted C₁₋₃alkylaryl, optionally substituted aryl, optionally substituted heteroaryl, optionally substituted heteroaryloxy, optionally substituted aryloxy optionally substituted heteroaryl-alkoxy, heterocycloalkoxy, C₁₋₆alkyl-SO₂NH₂, C₁₋₆alkyl-OC₁₋₃alkylheteroaryl wherein said heteroaryl is optionally substituted with CH₃, halogen or OH;
      - R₉ is C₁₋₆alkyl, halogen, C₃₋₆cycloalkyl or C₃₋₆heterocycloalkyl each of said cycloalkyl or heterocycloalkyl being optionally substituted with C₁₋₃alkyl, haloC₁₋₃alkyl, halogen;
   b) saturated or unsaturated C₃₋₁₀cycloalkyl ring, saturated or unsaturated C₃₋₁₀cycloheteroalkyl ring, each of said ring being optionally substituted with one or more substituents each independently selected from C₁₋₆alkyl, haloC₁₋₆alkyl, halogen, CN, OH, C₁₋₆alkoxy, NHC(O)Rs, N(C₁₋₆alkyl)C(O)R₈, NHSO₂R₈, NH(C₁₋₆alkyl), NH(C₁₋₆haloalkyl), hydroxy-C₁₋₆alkyl, C(O)OC₁₋₆alkyl, C(O)NHRs, C₂₋₆alkynyl, C₁₋₆alkylNHC(O)R₈, C₁₋₆alkylN(C₁₋₆alkyl)C(O)R₈, and;
   c) a ring of formula (III): 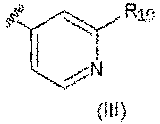 wherein R₁₀ is CF₃, OCH₃, t-butyl; and wherein when R₄ is a ring of formula (III), R₃ is selected from:
      - C₃₋₁₀cycloalkyl ring, C₃₋₁₀cycloalkenyl ring, C₃₋₁₀cycloheteroalkyl ring, each of said ring being optionally substituted with halogen, C₁₋₆alkyl, haloC₁₋₆alkyl, halogen, CN, OH, C₁₋₆alkoxy, amine, optionally substituted aryl, optionally substituted heteroaryl; or
      - (1-methyl-1,2,3,4-tetrahydroquinolin-2-yl), 2-methyl-1,2,3,4-tetrahydroisoquinolin-1-yl, (3-methylpyridazin-4-yl)phenyl, 4-(4-(trifluoromethyl)pyrimidin-5-yl)phenyl, 4-(4-methoxy-6-methylpyrimidin-5-yl)phenyl, 4-(1,4-dimethyl-1*H*-pyrazol-5-yl)phenyl, (2-methylthiazol-4-yl)phenyl, (3,5-dimethylisothiazol-4-yl)phenyl, (4-methylpyrimidin-5-yl)phenyl, (1,3-dimethyl-1*H*-pyrazol-4-yl)phenyl, 4-(4-methyl-1-(oxetan-3-yl)-1*H-*pyrazol-5yl)phenyl, 4-(4-methyl-1-(oxetan-3-yl)-1*H*-pyrazol-3-yl)phenyl, 4-(2-methylpyridin-3-yl)phenyl, 4-(2,5-dimethylthiazol-4-yl)phenyl, 4-(2-amino-4-methylpyrimidin-5-yl)phenyl, 4-(2,4-dimethylpyridin-3-yl)phenyl, 4-(pyrimidin-5-yl)phenyl, 4-(1,3,5-trimethyl-1*H*-pyrazol-4-yl)phenyl,4-(4,6-dimethylpyrimidin-5-yl)phenyl, 4-(4-Methyl-2-(oxetan-3-yl)pyrazol-3-yl]phenyl);

      and R₅ is H, C₁₋₆alkyl, C₁₋₆haloalkyl or halogen;
      or a pharmaceutically acceptable salt, tautomer, solvate, or stereoisomer thereof.

In a preferred embodiment, in the compound of formula (I)
- n is 0 or 1;
- R₁ and R₂ are H; and/or
- R₅ is H; and/or
- R₉ is cyclopropyl, methyl-cyclopropyl, trifluoromethyl-cyclopropyl, fluorocyclopropyl, difluorocyclopropyl, oxetane.

Still preferably R₃ is phenyl, pyridinyl, pyrazinyl, trifluoromethylphenyl, cyclobutyl, cyclohexyl, pyperidine, oxabicyclo[3.3.1]nonanyl, oxazepanyl, bicyclo[1.1.1]pentanyl, tetrahydroquinolinyl, bicyclo[2.2.1]heptanyl, tetrahydronaphtalenyl, azabicyclo[3.2.1]octane, 2-azaspiro[3.5]nonane, 3-azaspiro[5.5]undecane, wherein each of said ring is optionally substituted with one or more substituents independently selected from optionally substituted heteroaryl, optionally substituted aryl, C₁₋₃alkyl, halogen, hydroxy, isoindoline, NH₂, NHCH₃, N(CH₃)₂, N(C₁₋₃alkyl)(haloC₁₋₃alkyl), C₁₋₃alkyl-NH₂, C₁₋₃alkyl-N(CH₃)₂, (1,3-dioxoisoindolin-2-yl)methyl, C₁₋₃alkyl-NHCH₃, C₁₋₃alkyl-N(CH₃)₂, C₁₋₃alkyl-NHC(O)CH₃, COOH, C(O)NH₂, C(O)NH(C₁₋₃alkyl), C(O)N(C₁₋₃alkyl)₂, cycloamino-C(O) C₁₋₃alkyl-C(O)O(C₁₋₃alkyl), C₁₋₃alkyl-OH, C(O)OC₁₋₃alkyl, NHCH₂CF₃, CH₂NHCH₂CF₃, C₁₋₃alkyl-OH, haloC₁₋₃alkyl, NH(CO)CF₃.

Still preferably, it is an object of the invention a compound of formula (I) wherein R₄ is a ring of formula (IIa) or (IIb): 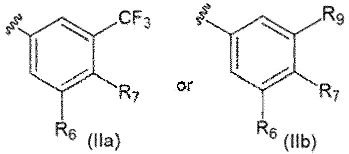 wherein
- R₆ is selected from NHC(O)Rs, N(C₁₋₃alkyl)C(O)R₈, NHSO₂R₈, NH(C₁₋₃alkyl), NH(C₁₋₃haloalkyl), hydroxy-C₁₋₃alkyl, CN, C(O)OC₁₋₃alkyl, C(O)NHRs, C₂₋₆alkynyl, C₁₋₃alkylNHC(O)R₈, C₁₋₃alkylN(C₁₋₃alkyl)C(O)R₈, and 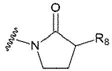 and R₇ is H; or
- R₆ is H and R₇ is selected from NHC(O)Rs, N(C₁₋₃alkyl)C(O)R₈, NHSO₂R₈, NH(C₁₋₃alkyl), NH(C₁₋₃haloalkyl), hydroxy-C₁₋₃alkyl, CN, C(O)OC₁₋₃alkyl, C(O)NHRs C₂₋₆alkynyl, C₁₋₃alkylNHC(O)R₈, C₁₋₃alkylN(C₁₋₃alkyl)C(O)R₈, and 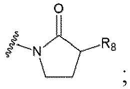 or
- R₆ and R₇ are H, with the proviso that when R₆ and R₇ are H, R₃ is selected from saturated or unsaturated C₃₋₁₀cycloalkyl ring, saturated or unsaturated C₃₋₁₀heterocycloalkyl ring, each of said ring being optionally substituted with halogen, C₁₋₆alkyl, haloC₁₋₆alkyl, halogen, CN, OH, C₁₋₆alkoxy, amine, optionally substituted aryl, optionally substituted heteroaryl; or when R₆ and R₇ are H, R₃ is (4,6-dimethylpyrimidin-5-yl)pyridazin-3-yl; or
- R₆ is OCH₃ or OH and R₇ is H, or R₆ is H and R₇ is OCH₃ or C(O)OCH₃, or R₆ and R₇ are joined to form together with the ring of formula (IIa) a 8-(trifluoromethyl)quinoline-6-yl ring, with the proviso that R₃ is 4-(4,6-dimethylpyrimidin-5-yl)phenyl;
R₈ is C₁₋₆alkyl, C₁₋₆haloalkyl, C₁₋₆alkyl-aryl, C₁₋₃alkyl-heteroaryl, C₁₋₃alkyl-C₃₋₆cycloalkyl, C₁₋₃alkyl-C₃₋₈heterocycloalkyl, C₁₋₃haloalkyl-aryl, C₁₋₃haloalkyl-heteroaryl, C₁₋₃haloalkyl-C₃₋₆cycloalkyl, C₁₋₃haloalkyl-C₃₋₈heterocycloalkyl, C₁₋₃alkylSO₂aryl, C₁₋₃alkylSO₂heterocycloalkyl, C₂₋₆alkynyl-aryl, aryl, heteroaryl, C₃₋₆cycloalkyl ring, C₃₋₁₀heterocycloalkyl ring, wherein each of said cycloalkyl and heterocycloalkyl ring is saturated or unsaturated and wherein each of said aryl, heteroaryl, cycloalkyl and herocycloalkyl ring is optionally substituted with one or more substituents independently selected from C₁₋₃alkyl, C₁₋₃alkoxy, C₃₋₆cycloalkyl, C₃₋sheteroycloalkyl, amine, CN, C₁₋₃alkyl-aryl, C₁₋₃alkyl-heteroaryl, C₁₋₃alkyl-C₃₋₆cycloalkyl, C₁₋₃alkyl-C₃₋₈heterocycloalkyl, C₁₋₃alkylCOOH, C₁₋₃alkylCOOCH₃, C₁₋₃alkylCONH₂ COOH, C(O)OC₁₋₃alkyl, NHC(O)C₁₋₃alkyl, NHC(O)C₃₋₆cycloalkyl, C₁₋₃haloalkyl, halogen, C₁₋₃alkylamine, OH, optionally substituted C₁₋₃alkylaryl, optionally substituted aryl, optionally substituted heteroaryl, optionally substituted heteroaryloxy, optionally substituted aryloxy optionally substituted heteroaryl-alkoxy, heterocycloalkoxy, C₁₋₃alkyl-SO₂NH₂, C₁₋₃alkyl-OC₁₋₃alkylheteroaryl wherein said heteroaryl is optionally substituted with CH₃, halogen or OH; and wherein in said compound R₉ is C₃₋₆cycloalkyl or C₃₋₆heterocycloalkyl each of said cycloalkyl or heterocycloalkyl being optionally substituted with C₁₋₃alkyl, haloC₁₋₃alkyl, halogen; preferably R₉ is cyclopropyl, methyl-cyclopropyl, trifluoromethyl-cyclopropyl, fluorocyclopropyl, difluorocyclopropyl, oxetane.

Still preferably, it is an object of the invention a compound of formula (I) wherein R₄ is saturated or unsaturated C₃₋₁₀cycloalkyl ring, saturated or unsaturated C₃₋₁₀cycloheteroalkyl ring, each of said ring being optionally substituted with one or more substituents each independently selected from C₁₋₆alkyl, haloC₁₋₆alkyl, halogen, CN, OH, C₁₋₆alkoxy, NHC(O)Rs, N(C₁₋₆alkyl)C(O)R₈, NHSO₂R₈, NH(C₁₋₆alkyl), NH(C₁₋₆haloalkyl), hydroxy-C₁₋₆alkyl, C(O)OC₁₋₆alkyl, C(O)NHRs, C₂₋₆alkynyl, C₁₋₆alkylNHC(O)R₈, C₁₋₆alkylN(C₁₋₆alkyl)C(O)R₈; and wherein R₉ is C₃₋₆cycloalkyl or C₃₋₆heterocycloalkyl each of said cycloalkyl or heterocycloalkyl being optionally substituted with C₁₋₃alkyl, haloC₁₋₃alkyl, halogen; preferably R₉ is cyclopropyl, methyl-cyclopropyl, trifluoromethyl-cyclopropyl, fluorocyclopropyl, difluorocyclopropyl, oxetane.

Still preferably R₃ is selected from: 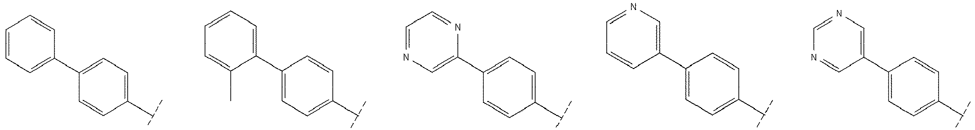 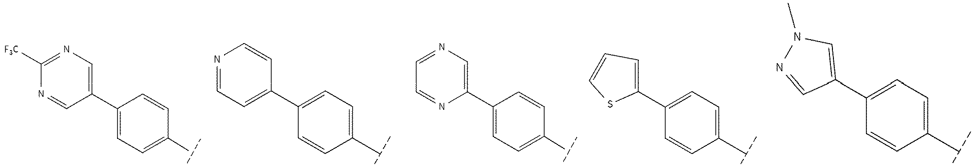 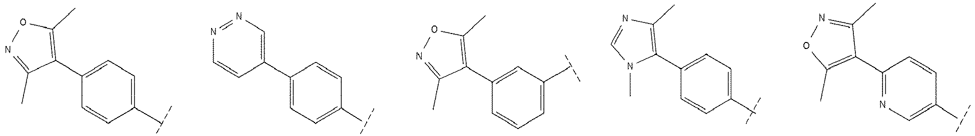 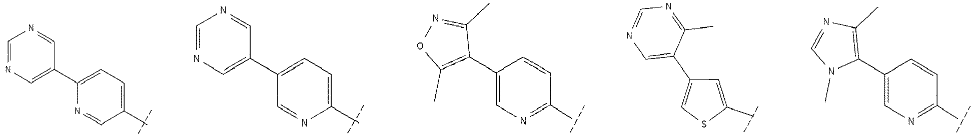 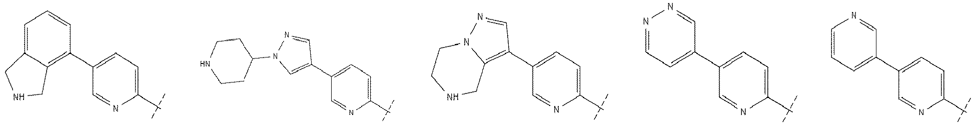 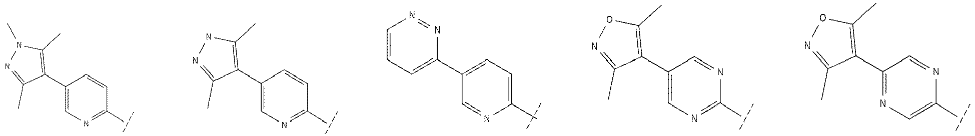 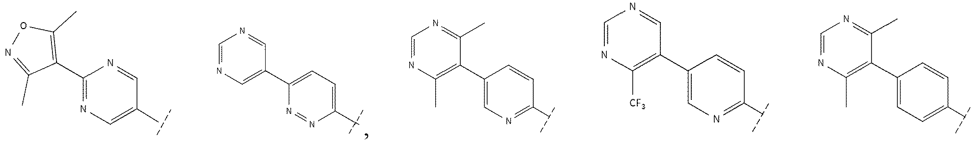 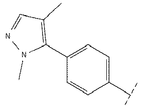 wherein each of the phenyl or heteroaromatic ring can be optionally further substituted by one or two substituents independently selected from methyl, CF₃, halogen, cyclopropyl, methoxy, cyano. Even more preferably R₃ is selected from 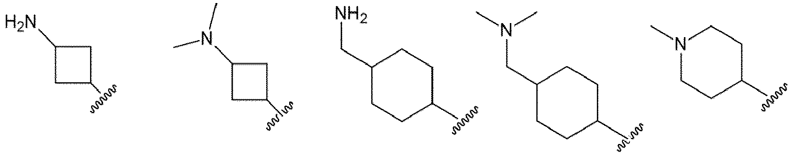 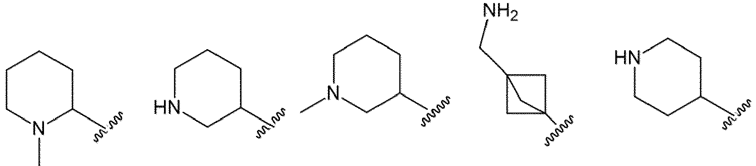 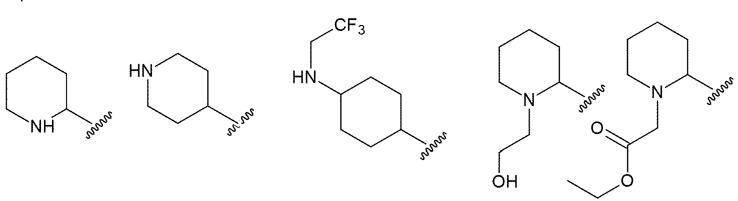 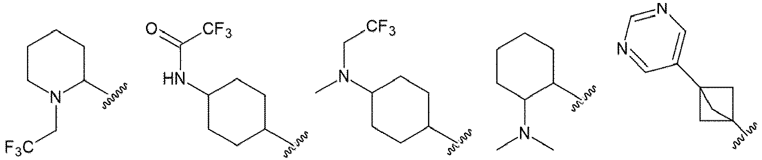 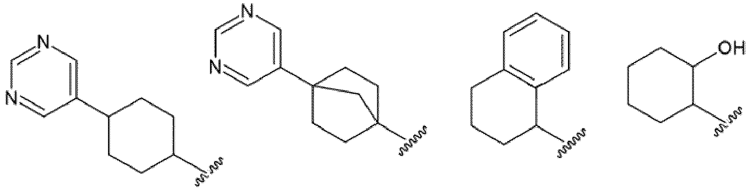 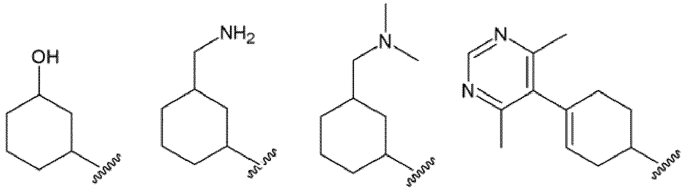 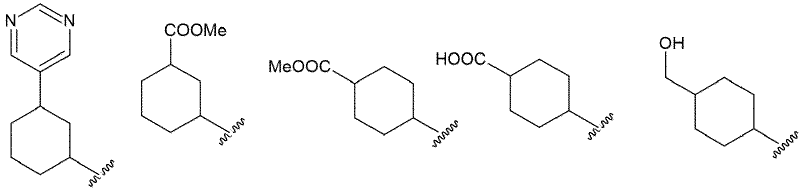 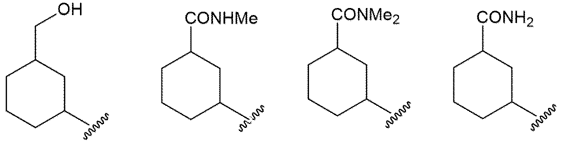 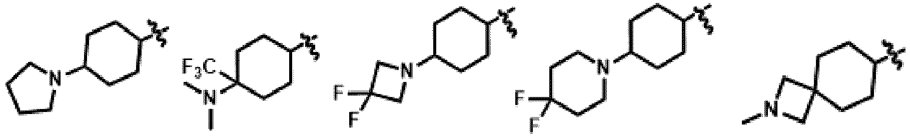 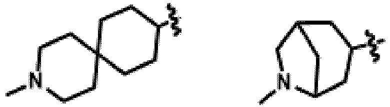 and any stereoisomer thereof.

Still preferably R₄ is selected from: 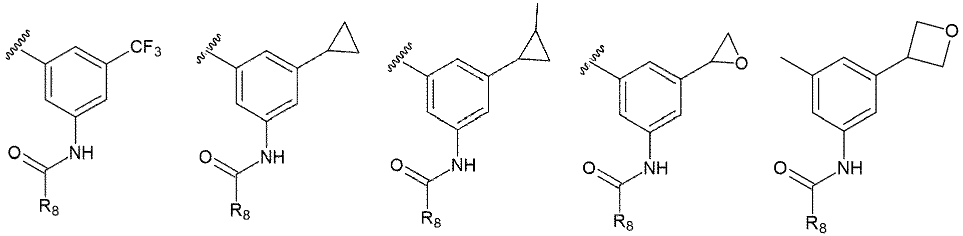 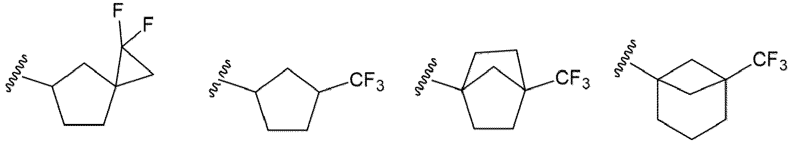 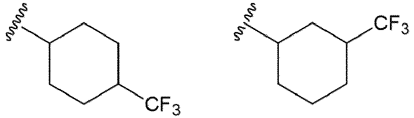 and stereoisomers thereof.

In a further preferred embodiment, the compound of formula (I) has formula (Ia) or formula (Ib) or formula (Ic) or formula (Id):

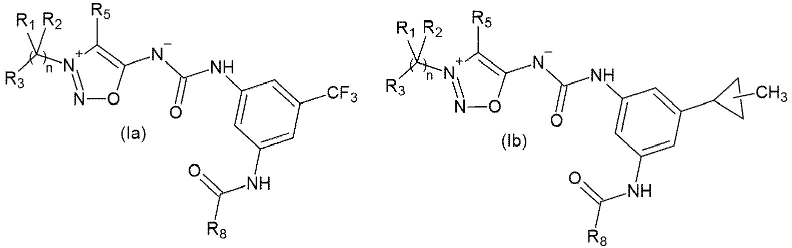

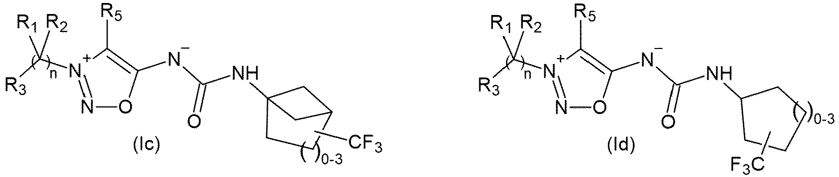

Preferably the compound of general formula (I) is selected from the following list:
- (3-(4-(2-Methoxy-4-methylpyrimidin-5-yl)benzyl)-1,2,3-oxadiazol-3-ium-5-yl)((3-(2-phenylacetamido)-5-(trifluoromethyl)phenyl)carbamoyl)amide
- ((3-((Cyclohexylmethyl)sulfonamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-Amino-5-(trifluoromethyl)phenyl)-carbamoyl)(3-(4-(2-methoxy-4-methylpyrimidin-5-yl)benzyl)-1,2,3-oxadiazol-3-ium-5-yl)amide
- (3-((5-(2-(Aminomethyl)phenyl)pyridin-2-yl)methyl)-1,2,3-oxadiazol-3-ium-5-yl)((3-(2-phenylacetamido)-5-(trifluoromethyl)phenyl-)carbamoyl)amide
- (3-((5-(1-Methyl-1H-imidazol-5-yl)pyridin-2-yl)methyl)-1,2,3-oxadiazol-3-ium-5-yl)((3-(2-phenylacetamido)-5-(trifluoromethyl)-phenyl)carbamoyl)amide
- ((3-(2-Phenylacetamido)-5-(trifluoromethyl)-phenyl)carbamoyl)(3-(pyridin-2-ylmethyl)-1,2,3-oxadiazol-3-ium-5-yl)amide
- ((3-(2-Cyclohexylacetamido)-5-(trifluoro-methyl)phenyl)carbamoyl)(3-(4-(2-methoxy-4-methylpyrimidin-5-yl)benzyl)-1,2,3-oxadiazol-3-ium-5-yl)amide
- (3-(4-(2-Methoxy-4-methylpyrimidin-5-yl)benzyl)-1,2,3-oxadiazol-3-ium-5-yl)((3-(3-phenylpropanamido)-5-(trifluoro-methyl)phenyl)carbamoyl)amide
- (3-(4-(2-Methoxy-4-methylpyrimidin-5-yl)benzyl)-1,2,3-oxadiazol-3-ium-5-yl)((3-((phenylmethyl)sulfonamido)-5-(trifluoro-methyl)phenyl)carbamoyl)amide
- ((3-Acetamido-5-(trifluoromethyl)phenyl)-carbamoyl)(3-(4-(2-methoxy-4-methylpyrimidin-5-yl)benzyl)-1,2,3-oxadiazol-3-ium-5-yl)amide
- (3-((5-Bromopyridin-2-yl)methyl)-1,2,3-oxadiazol-3-ium-5-yl)((3-(2-phenylacetamido)-5-(trifluoromethyl)phenyl)carbamoyl)amide
- (3-((5-(2-Methoxy-4-methylpyrimidin-5-yl)-pyridin-2-yl)methyl)-1,2,3-oxadiazol-3-ium-5-yl)((3-(2-phenylacetamido)-5-(trifluoromethyl)-phenyl)carbamoyl)amide
- (3-((5-(Isoindolin-4-yl)pyridin-2-yl)methyl)-1,2,3-oxadiazol-3-ium-5-yl)((3-(2-phenyla-cetamido)-5-(trifluoromethyl)phenyl)carbamoyl)-amide
- ((3-Benzamido-5-(trifluoromethyl)phenyl)-carbamoyl)(3-(4-(2-methoxy-4-methylpyrimidin-5-yl)benzyl)-1,2,3-oxadiazol-3-ium-5-yl)amide
- (3-(4-(2-Methoxy-4-methylpyrimidin-5-yl)benzyl)-1,2,3-oxadiazol-3-ium-5-yl)((3-(2-(4-methoxyphenyl)acetamido)-5-(trifluoromethyl)-phenyl)carbamoyl)amide
- ((3-(2-(4-Chlorophenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-(Cyclopentanecarboxamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- (3-(4-(2-Methoxy-4-methylpyrimidin-5-yl)benzyl)-1,2,3-oxadiazol-3-ium-5-yl)((3-(2-(pyridin-3-yl)acetamido)-5-(trifluoromethyl)phenyl)carbamoyl)amide
- ((3-(2-(Benzo[d]isoxazol-3-yl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-(2-(2-Chlorophenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-(2-(4-(Aminomethyl)phenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- (R)-(3-(2-Amino-1-(4-(3,5-dimethylisoxazol-4-yl)phenyl)ethyl)-1,2,3-oxadiazol-3-ium-5-yl)((3-(2-phenylacetamido)-5-(trifluoromethyl)phenyl)-carbamoyl)amide
- (S)-(3-(2-Amino-1-(4-(3,5-dimethylisoxazol-4-yl)phenyl)ethyl)-1,2,3-oxadiazol-3-ium-5-yl)((3-(2-phenylacetamido)-5-(trifluoromethyl)phenyl)carbamoyl)amide
- (3-((5-(2-Amino-4-methylpyrimidin-5-yl)pyridin-2-yl)methyl)-1,2,3-oxadiazol-3-ium-5-yl)((3-(2-phenylacetamido)-5-(trifluoromethyl)-phenyl)carbamoyl)amide
- (3-((1R,3R)-3-Aminocyclobutyl)-1,2,3-oxadiazol-3-ium-5-yl)((3-(2-phenylacetamido)-5-(trifluoromethyl)phenyl)carbamoyl)amide
- ((3-(2-(4-(Aminomethyl)phenyl)acetamido)-5-(trifluoromethyl)phenyl)carbamoyl)(3-(pyridin-2-ylmethyl)-1,2,3-oxadiazol-3-ium-5-yl)amide
- (3-Benzyl-1,2,3-oxadiazol-3-ium-5-yl)((3-(2-phenylacetamido)-5-(trifluoromethyl)phenyl)-carbamoyl)amide
- ((3-(1H-Benzo[d]imidazole-5-carboxamido)-5-(trifluoromethyl)phenyl)carbamoyl)(3-(pyridin-2-ylmethyl)-1,2,3-oxadiazol-3-ium-5-yl)amide
- 5-(3-(3-(5-(Morpholinomethyl)furan-2-carboxamido)-5-(trifluoromethyl)phenyl)ureido)-3 -(pyridin-2-ylmethyl)-1,2,3 -oxadiazol-3 -ium
- ((3-(3-Phenoxybenzamido)-5-(trifluoromethyl)-phenyl)carbamoyl)(3-(pyridin-2-ylmethyl)-1,2,3-oxadiazol-3-ium-5-yl)amide
- ((3-(2-Phenylpropanamido)-5-(trifluoromethyl)-phenyl)carbamoyl)(3-(pyridin-2-ylmethyl)-1,2,3-oxadiazol-3-ium-5-yl)amide
- ((3-(2-(5-Methyl-2-phenyloxazol-4-yl)acetamido)-5-(trifluoromethyl)phenyl)-carbamoyl)(3-(pyridin-2-ylmethyl)-1,2,3-oxadiazol-3-ium-5-yl)amide
- ((3-(1-Phenyl-1H-pyrazole-5-carboxamido)-5-(trifluoromethyl)phenyl)carbamoyl)(3-(pyridin-2-ylmethyl)-1,2,3-oxadiazol-3-ium-5-yl)amide
- ((3-(1-Benzyl-1H-pyrazole-4-carboxamido)-5-(trifluoromethyl)phenyl)carbamoyl)(3-(pyridin-2-ylmethyl)-1,2,3-oxadiazol-3-ium-5-yl)amide
- ((3-(2-(1H-Pyrrolo[3,2-b]pyridin-3-yl)acetamido)-5-(trifluoromethyl)phenyl)-carbamoyl)(3-(pyridin-2-ylmethyl)-1,2,3-oxadiazol-3-ium-5-yl)amide
- ((3-(6-Cyanoimidazo[1,2-a]pyridine-2-carboxamido)-5-(trifluoromethyl)-phenyl)carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(4,5,6,7-tetrahydro-1H-indazole-7-carboxamido)-5-(trifluoromethyl)phenyl)-carbamoyl)amide
- ((3-(5-Methyl-5,6-dihydro-4H-thieno[2,3-c]pyrrole-2-carboxamido)-5-(trifluoromethyl)phenyl)carbamoyl)(3-(pyridin-2-ylmethyl)-1,2,3-oxadiazol-3-ium-5-yl)amide
- ((3-(2-(1-Methyl-1H-pyrazol-3-yl)acetamido)-5-(trifluoromethyl)phenyl)carbamoyl)(3-(pyridin-2-ylmethyl)-1,2,3-oxadiazol-3-ium-5-yl)amide
- ((3-(2-(2-Methylthiazol-4-yl)acetamido)-5-(trifluoromethyl)phenyl)-carbamoyl)(3-(pyridin-2-ylmethyl)-1,2,3-oxadiazol-3-ium-5-yl)amide
- ((3-(2-(6-Aminopyridin-3-yl)acetamido)-5-(trifluoromethyl)phenyl)carbamoyl)(3-(pyridin-2-ylmethyl)-1,2,3-oxadiazol-3-ium-5-yl)amide
- ((3-(2-(Phenylsulfonyl)acetamido)-5-(trifluoromethyl)phenyl)carbamoyl)(3-(pyridin-2-ylmethyl)-1,2,3-oxadiazol-3-ium-5-yl)amide
- ((3-(Isoxazole-3-carboxamido)-5-(trifluoromethyl)-phenyl)carbamoyl)(3-(pyridin-2-ylmethyl)-1,2,3-oxadiazol-3-ium-5-yl)amide
- ((3-(Pyrazine-2-carboxamido)-5-(trifluoromethyl)-phenyl)carbamoyl)(3-(pyridin-2-ylmethyl)-1,2,3-oxadiazol-3-ium-5-yl)amide
- ((3-(3-Phenylpropiolamido)-5-(trifluoromethyl)-phenyl)carbamoyl)(3-(pyridin-2-ylmethyl)-1,2,3-oxadiazol-3-ium-5-yl)amide
- ((3-(Bicyclo[4.2.0]octa-1(6),2,4-triene-7-carboxamido)-5-(trifluoromethyl)phenyl)-carbamoyl)(3-(pyridin-2-ylmethyl)-1,2,3-oxadiazol-3-ium-5-yl)amide
- ((3-(1-Isopropyl-1H-pyrazole-3-carboxamido)-5-(trifluoromethyl)phenyl)carbamoyl)(3-(pyridin-2-ylmethyl)-1,2,3-oxadiazol-3-ium-5-yl)amide
- ((3-(1-Methylazetidine-3-carboxamido)-5-(trifluoromethyl)phenyl)carbamoyl)(3-(pyridin-2-ylmethyl)-1,2,3-oxadiazol-3-ium-5-yl)amide
- ((3-(2-Phenylcyclopropane-1-carboxamido)-5-(trifluoromethyl)phenyl)carbamoyl)(3-(pyridin-2-ylmethyl)-1,2,3-oxadiazol-3-ium-5-yl)amide
- ((3-(3-(Methoxycarbonyl)bicyclo[1.1.1]pentane-1-carboxamido)-5-(trifluoromethyl)phenyl)-carbamoyl)(3-(pyridin-2-ylmethyl)-1,2,3-oxadiazol-3-ium-5-yl)amide
- ((3-(3-Carboxybicyclo[1.1.1]pentane-1-carboxamido)-5-(trifluoromethyl)phenyl)-carbamoyl)(3-(pyridin-2-ylmethyl)-1,2,3-oxadiazol-3-ium-5-yl)amide
- ((3-(Phenethylamino)-5-(trifluoromethyl)-phenyl)carbamoyl)(3-(pyridin-2-ylmethyl)-1,2,3-oxadiazol-3-ium-5-yl)amide
- ((3-(2-Cyclobutylacetamido)-5-(trifluoromethyl)phenyl)carbamoyl)(3-(pyridin-2-ylmethyl)-1,2,3-oxadiazol-3-ium-5-yl)amide
- (3-((1R3R)-3-(Dimethylamino)cyclobutyl)-1,2,3-oxadiazol-3-ium-5-yl)((3-(2-phenyl-acetamido)-5-(trifluoromethyl)phenyl)-carbamoyl)amide
- (3-(4-(Aminomethyl)benzyl)-1,2,3-oxadiazol-3-ium-5-yl)((3-(2-phenylacetamido)-5-(trifluoromethyl)phenyl)carbamoyl)amide
- ((3-Methyl-5-(2-phenylacetamido)phenyl)-carbamoyl)(3-(pyridin-2-ylmethyl)-1,2,3-oxadiazol-3-ium-5-yl)amide
- ((3-Chloro-5-(2-phenylacetamido)phenyl)-carbamoyl)(3-(pyridin-2-ylmethyl)-1,2,3-oxadiazol-3-ium-5-yl)amide
- (3-((1S,4S)-4-(Aminomethyl)cyclohexyl)-1,2,3-oxadiazol-3-ium-5-yl)((3-(2-phenyl-acetamido)-5-(trifluoromethyl)phenyl)-carbamoyl)amide
- (3-((1S,4S)-4-((1,3-Dioxoisoindolin-2-yl)methyl)cyclohexyl)-1,2,3-oxadiazol-3-ium-5-yl)((3-(2-phenylacetamido)-5-(trifluoromethyl)-phenyl)carbamoyl)amide
- (3-((1S,4S)- -4-((Dimethylamino)methyl)cyclohexyl)-1,2,3-oxadiazol-3-ium-5-yl)((3-(2-phenylacetamido)-5-(trifluoromethyl)phenyl)carbamoyl)amide
- (3-((1S,4S)-4-(Acetamidomethyl)cyclohexyl)-1,2,3-oxadiazol-3-ium-5-yl)((3-(2-phenylacetamido)-5-(trifluoromethyl)phenyl)carbamoyl)amide
- ((3-(2-(Bicyclo[2. 1. 1]hexan-2-yl)acetamido)-5-(trifluoromethyl)phenyl)carbamoyl)(3-(pyridin-2-ylmethyl)-1,2,3-oxadiazol-3-ium-5-yl)amide
- ((3-(5,5-Dioxido-6,7-dihydro-4H-pyrazolo[5,1-c][1,4]thiazine-2-carboxamido)-5-(trifluoromethyl)phenyl)carbamoyl)(3-(pyridin-2-ylmethyl)-1,2,3-oxadiazol-3-ium-5-yl)amide
- ((3-(2-(2-(Difluoromethyl)-1H-benzo[d]imidazol-1-yl)acetamido)-5-(trifluoromethyl)phenyl)carbamoyl)(3-(pyridin-2-ylmethyl)-1,2,3-oxadiazol-3-ium-5-yl)amide
- ((3-(4-Hydroxybicyclo[2.2. 1]heptane-1-carboxamido)-5-(trifluoromethyl)phenyl)-carbamoyl)(3-(pyridin-2-ylmethyl)-1,2,3-oxadiazol-3-ium-5-yl)amide
- ((3-(7-Methyl-2,3-dihydrobenzofuran-3-carboxamido)-5-(trifluoromethyl)phenyl)-carbamoyl)(3-(pyridin-2-ylmethyl)-1,2,3-oxadiazol-3-ium-5-yl)amide
- ((3-(3-Benzylcyclobutane-1-carboxamido)-5-(trifluoromethyl)phenyl)carbamoyl)(3-(pyridin-2-ylmethyl)-1,2,3-oxadiazol-3-ium-5-yl)amide
- ((3-((1S,3S)-3-hydroxy-3-(pyridin-2-yl)cyclobutane-1-carboxamido)-5-(trifluoromethyl)phenyl)carbamoyl)(3-(pyridin-2-ylmethyl)-1,2,3-oxadiazol-3-ium-5-yl)amide
- ((3-(1-((5-Methylfuran-2-yl)methyl)piperidine-4-carboxamido)-5-(trifluoromethyl)phenyl)-carbamoyl)(3-(pyridin-2-ylmethyl)-1,2,3-oxadiazol-3-ium-5-yl)amide
- ((3-(1-(3-Cyano-1H-pyrazol-1-yl)cyclopropane-1-carboxamido)-5-(trifluoromethyl)phenyl)carbamoyl)(3-(pyridin-2-ylmethyl)-1,2,3-oxadiazol-3-ium-5-yl)amide
- ((3-(2-((4-Methyl-4H-1,2,4-triazol-3-yl)methoxy)acetamido)-5-(trifluoromethyl)-phenyl)carbamoyl)(3-(pyridin-2-ylmethyl)-1,2,3-oxadiazol-3-ium-5-yl)amide
- ((3-(3-(Cyclopentyloxy)propanamido)-5-(trifluoromethyl)phenyl)carbamoyl)(3-(pyridin-2-ylmethyl)-1,2,3-oxadiazol-3-ium-5-yl)amide
- ((3-((1S,4R,5R)-4-(3-Methoxyphenyl)-2-oxabicyclo[2.1.1]hexane-5-carboxamido)-5-(trifluoromethyl)phenyl)carbamoyl)(3-(pyridin-2-ylmethyl)-1,2,3-oxadiazol-3-ium-5-yl)amide
- ((3-(1-Cyclopropyl-1H-imidazole-5-carboxamido)-5-(trifluoromethyl)phenyl)-carbamoyl)(3-(pyridin-2-ylmethyl)-1,2,3-oxadiazol-3-ium-5-yl)amide
- ((3-(2,2-Difluoro-3-(pyrrolidin-1-yl)propanamido)-5-(trifluoromethyl)phenyl)-carbamoyl)(3-(pyridin-2-ylmethyl)-1,2,3-oxadiazol-3-ium-5-yl)amide
- ((3-(1-Cyclopropylazetidine-3-carboxamido)-5-(trifluoromethyl)phenyl)carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(4-(1-sulfamoylethyl)benzamido)-5-(trifluoromethyl)phenyl)carbamoyl)amide
- ((3-(2,3-Dihydrofuro[3,2-b]pyridine-5-carboxamido)-5-(trifluoromethyl)phenyl)-carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(5,6,7,8-tetrahydroimidazo[1,5-a]pyridine-7-carboxamido)-5-(trifluoromethyl)
- phenyl)carbamoyl)amide
- ((3-(3,4-Dihydro-1H-pyrrolo[2,1-c][1,4]oxazine-8-carboxamido)-5-(trifluoromethyl)-phenyl)carbamoyl)(3-(pyridin-2-ylmethyl)-1,2,3-oxadiazol-3-ium-5-yl)amide
- ((3-(5-(Piperidin-1-ylsulfonyl)pentanamido)-5-(trifluoromethyl)phenyl)carbamoyl)(3-(pyridin-2-ylmethyl)-1,2,3-oxadiazol-3-ium-5-yl)amide
- ((3-(3-(4-Chlorophenoxy)oxetane-3-carboxamido)-5-(trifluoromethyl)phenyl)-carbamoyl)(3-(pyridin-2-ylmethyl)-1,2,3-oxadiazol-3-ium-5-yl)amide
- ((3-(2,6-Dioxaspiro[4.5]decane-7-carboxamido)-5-(trifluoromethyl)phenyl)carbamoyl)(3-(pyridin-2-ylmethyl)-1,2,3-oxadiazol-3-ium-5-yl)amide
- ((3-(3,3-Difluorospiro[3.3]heptane-1-carboxamido)-5-(trifluoromethyl)phenyl)-carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(2-(3-(pyrrolidin-1-yl)oxetan-3-yl)acetamido)-5-(trifluoromethyl)phenyl)
- carbamoyl)amide
- (3-(Pyridin-2-ylmethyl)-1,2,3-oxadiazol-3-ium-5-yl)((3-(2,4,5,6-tetrahydrocyclopenta[c]pyrrole-1-carboxamido)-5-(trifluoromethyl)phenyl)-carbamoyl)amide
- ((3-(4,4-Dioxido-1,4-oxathiane-2-carboxamido)-5-(trifluoromethyl)phenyl)carbamoyl)(3-(pyridin-2-ylmethyl)-1,2,3-oxadiazol-3-ium-5-yl)amide
- ((3-Cyclopropyl-5-(2-phenylacetamido)phenyl)-carbamoyl)(3-(pyridin-2-ylmethyl)-1,2,3-oxadiazol-3-ium-5-yl)amide
- ((3-(2-(Benzylamino)-2-oxoethyl)-5-(trifluoromethyl)phenyl)carbamoyl)(3-(pyridin-2-ylmethyl)-1,2,3-oxadiazol-3-ium-5-yl)amide
- (3-((1S,4S)-4-Aminocyclohexyl)-1,2,3-oxadiazol-3-ium-5-yl)((3-(2-phenylacetamido)-5-(trifluoromethyl)phenyl)carbamoyl)amide
- (3-((1R,4R)-4-Aminocyclohexyl)-1,2,3-oxadiazol-3-ium-5-yl)((3-(2-phenylacetamido)-5-(trifluoromethyl)phenyl)carbamoyl)amide
- (3-((1R,4R)-4-(Aminomethyl)cyclohexyl)-1,2,3-oxadiazol-3-ium-5-yl)((3-(2-phenyl-acetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(2-phenylacetamido)-5-(trifluoromethyl)phenyl)-carbamoyl)amide
- (3-((1S,4S)-4-(Dimethylamino)cyclohexyl)-1,2,3-oxadiazol-3-ium-5-yl)((3-(2-phenylacetamido)-5-(trifluoromethyl)phenyl)-carbamoyl)amide
- (3-((1R,4R)-4-(dimethylamino)cyclohexyl)-1,2,3-oxadiazol-3-ium-5-yl)((3-(2-phenylacetamido)-5-(trifluoromethyl)phenyl)-carbamoyl)amide
- (3-(4-(Methoxycarbonyl)benzyl)-1,2,3-oxadiazol-3-ium-5-yl)((3-(2-phenylacetamido)-5-(trifluoromethyl)phenyl)carbamoyl)amide
- ((3-(2-Phenylacetamido)-5-(trifluoromethyl)-phenyl)carbamoyl)(3-(piperidin-4-yl)-1,2,3-oxadiazol-3-ium-5-yl)amide
- (3-(1-Methylpiperidin-4-yl)-1,2,3-oxadiazol-3-ium-5-yl)((3-(2-phenylacetamido)-5-(trifluoromethyl)phenyl)carbamoyl)amide
- (3-(4-Carboxybenzyl)-1,2,3-oxadiazol-3-ium-5-yl)((3-(2-phenylacetamido)-5-(trifluoromethyl)-phenyl)carbamoyl)amide
- (3-(4-(Hydroxymethyl)benzyl)-1,2,3-oxadiazol-3-ium-5-yl)((3-(2-phenylacetamido)-5-(trifluoromethyl)phenyl)carbamoyl)amide
- (3-(4-(Aminomethyl)benzyl)-1,2,3-oxadiazol-3-ium-5-yl)((3-(2-phenylacetamido)-5-(trifluoromethyl)phenyl)carbamoyl)amide
- (3-(4-Carbamoylbenzyl)-1,2,3-oxadiazol-3-ium-5-yl)((3-(2-phenylacetamido)-5-(trifluoromethyl)phenyl)carbamoyl)amide
- (3-((1-Methylpiperidin-2-yl)methyl)-1,2,3-oxadiazol-3-ium-5-yl)((3-(2-phenylacetamido)-5-(trifluoromethyl)phenyl)carbamoyl)amide
- (3-((1-Methylpiperidin-4-yl)methyl)-1,2,3-oxadiazol-3-ium-5-yl)((3-(2-phenylacetamido)-5-(trifluoromethyl)phenyl)carbamoyl)amide
- (3-(3-((1,3-Dioxoisoindolin-2-yl)methyl)benzyl)-1,2,3-oxadiazol-3-ium-5-yl)((3-(2-phenylacetamido)-5-(trifluoromethyl)phenyl)carbamoyl)amide
- (3-(3-(Aminomethyl)benzyl)-1,2,3-oxadiazol-3-ium-5-yl)((3-(2-phenylacetamido)-5-(trifluoromethyl)phenyl)carbamoyl)amide
- (3-(4-(2-Methoxy-2-oxoethyl)benzyl)-1,2,3-oxadiazol-3-ium-5-yl)((3-(2-phenylacetamido)-5-(trifluoromethyl)phenyl)carbamoyl)amide
- ((3-(2-Phenylacetamido)-5-(trifluoromethyl)-phenyl)carbamoyl)(3-(piperidin-3-ylmethyl)-1,2,3-oxadiazol-3-ium-5-yl)amide
- (3-(4-(2-Hydroxyethyl)benzyl)-1,2,3-oxadiazol-3-ium-5-yl)((3-(2-phenylacetamido)-5-(trifluoromethyl)phenyl)carbamoyl)amide
- (3-(9-Amino-3-oxabicyclo[3.3.1]nonan-7-yl)-1,2,3-oxadiazol-3-ium-5-yl)((3-(2-phenylacetamido)-5-(trifluoromethyl)phenyl)-carbamoyl)amide
- (3-((5-(Methoxycarbonyl)pyridin-2-yl)methyl)-1,2,3-oxadiazol-3-ium-5-yl)((3-(2-phenylacetamido)-5-(trifluoromethyl)phenyl)carbamoyl)amide
- (3-(2-(Aminomethyl)benzyl)-1,2,3-oxadiazol-3-ium-5-yl)((3-(2-phenylacetamido)-5-(trifluoromethyl)phenyl)carbamoyl)amide
- (3-((1,4-Oxazepan-2-yl)methyl)-1,2,3-oxadiazol-3-ium-5-yl)((3-(2-phenylacetamido)-5-(trifluoromethyl)phenyl)carbamoyl)amide
- (3-((5-(Hydroxymethyl)pyridin-2-yl)methyl)-1,2,3-oxadiazol-3-ium-5-yl)((3-(2-phenylacetamido)-5-(trifluoromethyl)phenyl)-carbamoyl)amide
- (3-((1 -Methylpiperidin-3 -yl)methyl)-1,2,3 -oxadiazol-3 -ium-5 -yl)((3 -(2-phenylacetamido)-5-(trifluoromethyl)phenyl)carbamoyl)amide
- (3-((3-Aminocyclobutyl)methyl)-1,2,3-oxadiazol-3-ium-5-yl)((3-(2-phenylacetamido)-5-(trifluoromethyl)phenyl)carbamoyl)amide
- (3-((1S,4S)-4-(Aminomethyl)cyclohexyl)-1,2,3-oxadiazol-3-ium-5-yl)((3-(2-oxo-3-phenyl-pyrrolidin-1-yl)-5-(trifluoromethyl)phenyl)-carbamoyl)amide
- ((3-(2-Phenylacetamido)-5-(trifluoromethyl)-phenyl)carbamoyl)(3-(piperidin-4-ylmethyl)-1,2,3-oxadiazol-3-ium-5-yl)amide
- (3-((5-(Isopropoxycarbonyl)pyridin-2-yl)methyl)-1,2,3-oxadiazol-3-ium-5-yl)((3-(2-phenylacetamido)-5-(trifluoromethyl)phenyl)-carbamoyl)amide
- ((3-(2-Oxo-4-phenylpyrrolidin-1-yl)-5-(trifluoromethyl)phenyl)carbamoyl)(3-(pyridin-2-ylmethyl)-1,2,3-oxadiazol-3-ium-5-yl)amide
- ((3-(2-(6-Oxo-1,6-dihydropyridin-3-yl)acetamido)-5-(trifluoromethyl)phenyl)-carbamoyl)(3-(pyridin-2-ylmethyl)-1,2,3-oxadiazol-3-ium-5-yl)amide
- ((3-(2-(1H-Benzo[d]imidazol-6-yl)acetamido)-5-(trifluoromethyl)phenyl)carbamoyl)(3-(pyridin-2-ylmethyl)-1,2,3-oxadiazol-3-ium-5-yl)amide
- ((3-(2-Phenylacetamido)-5-(trifluoromethyl)-phenyl)carbamoyl)(3-(piperidin-2-ylmethyl)-1,2,3-oxadiazol-3-ium-5-yl)amide
- ((3-(2-(1H-Indazol-7-yl)acetamido)-5-(trifluoromethyl)phenyl)carbamoyl)(3-(pyridin-2-ylmethyl)-1,2,3-oxadiazol-3-ium-5-yl)amide
- ((3-Cyclopropyl-5-(2-phenylacetamido)phenyl)-carbamoyl)(3-((1-methylpiperidin-2-yl)methyl)-1,2,3-oxadiazol-3-ium-5-yl)amide
- ((3-Cyclopropyl-5-(2-phenylacetamido)phenyl)-carbamoyl)(3-((5-(methoxycarbonyl)pyridin-2-yl)methyl)-1,2,3-oxadiazol-3-ium-5-yl)amide
- ((3-Cyclopropyl-5-(2-phenylacetamido)phenyl)-carbamoyl)(3-((5-(hydroxymethyl)pyridin-2-yl)methyl)-1,2,3-oxadiazol-3-ium-5-yl)amide
- ((3-((1R,2R)-2-Methylcyclopropyl)-5-(2-phenylacetamido)phenyl)carbamoyl)(3-(pyridin-2-ylmethyl)-1,2,3-oxadiazol-3-ium-5-yl)amide
- (3-(((1S,4S)-4-Aminocyclohexyl)methyl)-1,2,3-oxadiazol-3-ium-5-yl)((3-(2-oxo-3-phenylpyrrolidin-1-yl)-5-(trifluoromethyl)-phenyl)carbamoyl)amide
- (3-(((1R,4R)-4-Aminocyclohexyl)methyl)-1,2,3-oxadiazol-3-ium-5-yl)((3-(2-oxo-3-phenylpyrrolidin-1-yl)-5-(trifluoromethyl)-phenyl)carbamoyl)amide
- (3-(((1S,4S)-4-(Aminomethyl)cyclohexyl)-methyl)-1,2,3-oxadiazol-3-ium-5-yl)((3-(2-oxo-3-phenylpyrrolidin-1-yl)-5-(trifluoromethyl)-phenyl)carbamoyl)amide
- (3-((3-(((5-Hydroxypyridin-2-yl)methyl)amino)-cyclobutyl)methyl)-1,2,3-oxadiazol-3-ium-5-yl)((3-(2-phenylacetamido)-5-(trifluoromethyl)-phenyl)carbamoyl)amide
- ((3-(2-Oxo-3-phenylpyrrolidin-1-yl)-5-(trifluoromethyl)phenyl)carbamoyl)(3-((1S,4S)-4-(((2,2,2-trifluoroethyl)amino)methyl)-cyclohexyl)-1,2,3-oxadiazol-3-ium-5-yl)amide
- ((3-(2-Oxo-3-phenylpyrrolidin-1-yl)-5-(trifluoromethyl)phenyl)carbamoyl)(3-(((1s,4s)-4-((2,2,2-trifluoroethyl)amino)cyclohexyl)methyl)-1,2,3-oxadiazol-3-ium-5-yl)amide
- ((3-(2-Oxo-3-phenylpyrrolidin-1-yl)-5-(trifluoromethyl)phenyl)carbamoyl)(3-(((1R,4R)-4-((2,2,2-trifluoroethyl)amino)-cyclohexyl)methyl)-1,2,3-oxadiazol-3-ium-5-yl)amide
- (3-((1S,4S)-4-((Dimethylamino)methyl)-cyclohexyl)-1,2,3-oxadiazol-3-ium-5-yl)((3-(2-oxo-3-phenylpyrrolidin-1-yl)-5-(trifluoromethyl)-phenyl)carbamoyl)amide
- ((3-(2-Oxo-3-phenylpyrrolidin-1-yl)-5-(trifluoromethyl)phenyl)carbamoyl)(3-(((1s,4s)-4-(((2,2,2-trifluoroethyl)amino)methyl)cyclohexyl)-methyl)-1,2,3-oxadiazol-3-ium-5-yl)amide
- (3-((1-(2-Ethoxy-2-oxoethyl)piperidin-4-yl)methyl)-1,2,3-oxadiazol-3-ium-5-yl)((3-(2-phenylacetamido)-5-(trifluoromethyl)phenyl)-carbamoyl)amide
- (3-((1-(2-Hydroxyethyl)piperidin-4-yl)methyl)-1,2,3-oxadiazol-3-ium-5-yl)((3-(2-phenylacetamido)-5-(trifluoromethyl)phenyl)-carbamoyl)amide
- ((3-(2-Phenylacetamido)-5-(trifluoromethyl)-phenyl)carbamoyl)(3-((1-(2,2,2-trifluoroethyl)-piperidin-2-yl)methyl)-1,2,3-oxadiazol-3-ium-5-yl)amide
- (3-(((1R,4R)-4-Aminocyclohexyl)methyl)-1,2,3-oxadiazol-3-ium-5-yl)((3-((R)-2-oxo-3-phenylpyrrolidin-1-yl)-5-(trifluoromethyl)-phenyl)carbamoyl)amide
- ((3-((R)-2-Oxo-3-phenylpyrrolidin-1-yl)-5-(trifluoromethyl)phenyl)carbamoyl)(3-(((1r,4r)-4-((2,2,2-trifluoroethyl)amino)cyclohexyl)-methyl)-1,2,3-oxadiazol-3-ium-5-yl)amide
- ((3-((R)-2-Oxo-3-phenylpyrrolidin-1-yl)-5-(trifluoromethyl)phenyl)carbamoyl)(3-(((1R,4R)-4-(2,2,2-trifluoroacetamido)cyclohexyl)methyl)-1,2,3-oxadiazol-3-ium-5-yl)amide
- (3-(((1R,4R)-4-(Methyl(2,2,2-trifluoroethyl)-amino)cyclohexyl)methyl)-1,2,3-oxadiazol-3-ium-5-yl)((3-((R)-2-oxo-3-phenylpyrrolidin-1-yl)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(2-phenylacetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(2-oxo-3-phenylpyrrolidin-1-yl)-5-(trifluoromethyl)-phenyl)carbamoyl)amide
- (3-((5-(4,6-Dimethylpyrimidin-5-yl)pyridin-2-yl)methyl)-1,2,3-oxadiazol-3-ium-5-yl)((3-(2-phenylacetamido)-5-(trifluoromethyl)phenyl)-carbamoyl)amide
- ((3-Cyclopropyl-5-(2-(phenylsulfonyl)-acetamido)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-(phenylsulfonyl)acetamido)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(2-(3-methoxyphenyl)acetamido)-5-(trifluoromethyl)-phenyl)carbamoyl)amide
- (3-((1R,4R)-4-((Dimethylamino)methyl)-cyclohexyl)-1,2,3-oxadiazol-3-ium-5-yl)((3-(2-(2-methoxyphenyl)acetamido)-5-(trifluoromethyl)-phenyl)carbamoyl)amide
- ((3-(2-(Benzo[d][1,3]dioxol-5-yl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2-(3-(Carboxymethyl)phenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2-(4-Carboxyphenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(3-(1-methyl-1H-indol-3-yl)propanamido)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(3-(pyrazin-2-yl)propanamido)-5-(trifluoromethyl-)phenyl)carbamoyl)amide
- ((3-(2-(2-Carboxyphenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2,2-Difluoro-3-(pyrrolidin-1-yl)propanamido)-5-(trifluoromethyl)phenyl)-carbamoyl)(3-((1R,4R)-4-((dimethylamino)-methyl)cyclohexyl)-1,2,3-oxadiazol-3-ium-5-yl)amide
- (3-((1R,4R)-4-((dimethylamino)methyl)-cyclohexyl)-1,2,3-oxadiazol-3-ium-5-yl)((3-(2-(1-methyl-1H-indol-3-yl)acetamido)-5-(trifluoro-methyl)phenyl)carbamoyl)amide
- ((3-(2-(3-(2-Amino-2-oxoethyl)phenyl)-acetamido)-5-(trifluoromethyl)phenyl)-carbamoyl)(3-((1R,4R)-4-((dimethylamino)-methyl)cyclohexyl)-1,2,3-oxadiazol-3-ium-5-yl)amide
- ((3-(2-(3-(Aminomethyl)phenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-phenylpropanamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(2-phenylbutanamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(trifluoromethyl)-5-(2-(2-(trifluoromethyl)-phenyl)acetamido)phenyl)carbamoyl)amide
- (3-((1R,4R)-4-((Dimethylamino)methyl)-cyclohexyl)-1,2,3-oxadiazol-3-ium-5-yl)((3-(N-methyl-2-phenylpropanamido)-5-(trifluoromethyl)phenyl)carbamoyl)amide
- ((3-(2-(2-Chlorophenyl)acetamido)-5-(trifluoro-methyl)phenyl)carbamoyl)(3-((1R,4R)-4-((dimethylamino)methyl)cyclohexyl)-1,2,3-oxadiazol-3-ium-5-yl)amide
- ((3-(2-(2,3-Difluorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4S)-4-((Dimethylamino)methyl)-cyclohexyl)-1,2,3-oxadiazol-3-ium-5-yl)((3-((S)-2-phenylpropanamido)-5-(trifluoromethyl)-phenyl)carbamoyl)amide
- (3-((1R,4R)-4-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(N-methyl-2-phenylacetamido)-5-(trifluoromethyl)-phenyl)carbamoyl)amide
- (3-((1R,3R)-3-(Dimethylamino)cyclohexyl)-1,2,3-oxadiazol-3-ium-5-yl)((3-(2-phenylacetamido)-5-(trifluoromethyl)phenyl)-carbamoyl)amide
- (3-((1R,3S)-3-(Dimethylamino)cyclohexyl)-1,2,3-oxadiazol-3-ium-5-yl)((3-(2-phenyl-acetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-((R)-2-phenylpropanamido)-5-(trifluoromethyl)-phenyl)carbamoyl)amide
- ((3-(2-(3-Chlorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(1,2,3,4-tetrahydronaphthalene-1-carboxamido)-5-(trifluoromethyl)phenyl)carbamoyl)amide
- rac-(3-((1-Methyl-1,2,3,4-tetrahydroquinolin-2-yl)methyl)-1,2,3-oxadiazol-3-ium-5-yl)((2-(trifluoromethyl)pyridin-4-yl)carbamoyl)amide
- (3-(((1S,4S)-4-(Pyrimidin-5-yl)cyclohexyl)-methyl)-1,2,3-oxadiazol-3-ium-5-yl)((3-(trifluoromethyl)phenyl)carbamoyl)amide
- rac-(((3R,5S)-1,1-Difluorospiro[2.4]heptan-5-yl)carbamoyl)(3-((5-(4,6-dimethylpyrimidin-5-yl)pyridin-2-yl)methyl)-1,2,3-oxadiazol-3-ium-5-yl)amide
- rac-(3-((2-Methyl-1,2,3,4-tetrahydroisoquinolin -1-yl)methyl)-1,2,3-oxadiazol-3-ium-5-yl)((2-(trifluoromethyl)pyridin-4-yl)carbamoyl)amide
- (3-(((1S,4S)-4-(Pyrimidin-5-yl)cyclohexyl) methyl)-1,2,3-oxadiazol-3-ium-5-yl)((2-(trifluoro methyl)pyridin-4-yl)carbamoyl)amide
- (3-((3-(Pyrimidin-5-yl)bicyclo[1.1.1]pentan-1-yl)methyl)-1,2,3-oxadiazol-3-ium-5-yl)((3-(trifluoromethyl)phenyl)carbamoyl)amide
- (3-((6-(4,6-Dimethylpyrimidin-5-yl)pyridazin-3-yl)methyl)-1,2,3-oxadiazol-3-ium-5-yl)((3-(trifluoromethyl)phenyl)carbamoyl)amide
- (3-((4-(Pyrimidin-5-yl)bicyclo[2.2. 1]heptan-1-yl)methyl)-1,2,3-oxadiazol-3-ium-5-yl)((2-(trifluoromethyl)pyridin-4-yl)carbamoyl)amide
- (3-(((1R,4R)-4-(Pyrimidin-5-yl)cyclohexyl) methyl)-1,2,3-oxadiazol-3-ium-5-yl)((3-(trifluoro methyl)phenyl)carbamoyl)amide
- (3-(((1R,4R)-4-(Pyrimidin-5-yl)cyclohexyl)-methyl)-1,2,3-oxadiazol-3-ium-5-yl)((2-(trifluoromethyl)pyridin-4-yl)carbamoyl)amide
- (3-(4-(3-Methylpyridazin-4-yl)benzyl)-1,2,3-oxadiazol-3-ium-5-yl)((2-(trifluoromethyl)-pyridin-4-yl)carbamoyl)amide
- ((2-(Trifluoromethyl)pyridin-4-yl)carbamoyl)(3-(4-(4-(trifluoromethyl)pyrimidin-5-yl)benzyl)-1,2,3-oxadiazol-3-ium-5-yl)amide
- (3-(4-(4-Methoxy-6-methylpyrimidin-5-yl)benzyl)-1,2,3-oxadiazol-3-ium-5-yl)((2-(trifluoromethyl)pyridin-4-yl)carbamoyl)amide
- (3-((1R,4R)-4-((Dimethylamino)methyl)-cyclohexyl)-1,2,3-oxadiazol-3-ium-5-yl)((3-(2-(2-hydroxyphenyl)acetamido)-5-(trifluoromethyl)-phenyl)carbamoyl)amide
- rac-(3-((2-Methyl-1,2,3,4-tetrahydro isoquinolin-1-yl)methyl)-1,2,3-oxadiazol-3-ium-5-yl)((3-(2-phenylacetamido)-5-(trifluoromethyl) phenyl)carbamoyl)amide
- ((2-(2-(2-Chlorophenyl)acetamido)-6-(trifluoromethyl)pyridin-4-yl)carbamoyl)(3-((1R,4R)-4-((dimethylamino)methyl)-cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-fluoro-2-(2-fluorophenyl)acetamido)-5-(trifluoromethyl)phenyl)carbamoyl)amide
- ((3-(Bicyclo[4.2.0]octa-1(6),2,4-triene-7-carboxamido)-5-(trifluoromethyl)phenyl)-carbamoyl)(3-((1R,4R)-4-((dimethylamino-methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-(6-hydroxypyridin-2-yl)acetamido)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(2-(3,5-dimethylisoxazol-4-yl)acetamido)-5-(trifluoromethyl)phenyl)carbamoyl)amide
- ((3-(2-(2-Chloro-6-fluorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2-(2,6-Difluorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- Rac-(3-((1,2,3,4-Tetrahydronaphthalen-1-yl)methyl)-1,2,3-oxadiazol-3-ium-5-yl)((2-(trifluoromethyl)pyridin-4-yl)carbamoyl)amide
- (3-(4-(1,4-Dimethyl-1H-pyrazol-5-yl)benzyl)-1,2,3-oxadiazol-3-ium-5-yl)((2-(trifluoromethyl)-pyridin-4-yl)carbamoyl)amide
- (3-((1R,3S)-3-(Dimethylamino)cyclohexyl)-1,2,3-oxadiazol-3-ium-5-yl)((3-(2-(o-tolyl)acetamido)-5-(trifluoromethyl)phenyl)-carbamoyl)amide
- (3-((1R,2R)-2-Hydroxycyclohexyl)-1,2,3-oxadiazol-3-ium-5-yl)((3-(2-(o-tolyl)acetamido)-5-(trifluoromethyl)phenyl)carbamoyl)amide
- (3-((1R,3S)-3-(Dimethylamino)cyclohexyl)-1,2,3-oxadiazol-3-ium-5-yl)((2-(trifluoromethyl)-pyridin-4-yl)carbamoyl)amide
- ((3-(2-(2-Chlorophenyl)propanamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-(2-Hydroxypropan-2-yl)cyclohexyl)-1,2,3-oxadiazol-3-ium-5-yl)((3-(2-(o-tolyl)acetamido)-5-(trifluoromethyl)phenyl)-carbamoyl)amide
- ((4-Cyano-3-(trifluoromethyl)phenyl)-carbamoyl)(3-(4-(4,6-dimethylpyrimidin-5-yl)benzyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-methyl-2-phenylpropanamido)-5-(trifluoromethyl)phenyl)carbamoyl)amide
- (3-(4-(4,6-Dimethylpyrimidin-5-yl)benzyl)-1,2,3-oxadiazol-3-ium-5-yl)((2-methoxypyridin-4-yl)carbamoyl)amide
- (3-(4-(2-Methylthiazol-4-yl)benzyl)-1,2,3-oxadiazol-3-ium-5-yl)((2-(trifluoromethyl)pyridin -4-yl)carbamoyl)amide
- (3-(4-(3,5-Dimethylisothiazol-4-yl)benzyl)-1,2,3-oxadiazol-3-ium-5-yl)((2-(trifluoromethyl)-pyridin-4-yl)carbamoyl)amide
- (3-((1R,4R)-4-((Dimethylamino)methyl)-cyclohexyl)-1,2,3-oxadiazol-3-ium-5-yl)((3-(2-(2,3-dimethylphenyl)acetamido)-5-(trifluoromethyl)phenyl)carbamoyl)amide
- (3-((1S,3R)-3-((Dimethylamino)methyl)-cyclohexyl)-1,2,3-oxadiazol-3-ium-5-yl)((3-(2-(o-tolyl)acetamido)-5-(trifluoromethyl)phenyl) carbamoyl)amide
- (3-((1R,3S)-3-Hydroxycyclohexyl)-1,2,3-oxadiazol-3-ium-5-yl)((3-(2-(o-tolyl)acetamido)-5-(trifluoromethyl)phenyl)carbamoyl)amide
- (3-((1R,3R)-3-Hydroxycyclohexyl)-1,2,3-oxadiazol-3-ium-5-yl)((3-(2-(o-tolyl)acetamido)-5-(trifluoromethyl)phenyl)carbamoyl)amide
- (3-((4-(4,6-Dimethylpyrimidin-5-yl)cyclohex-3-en-1-yl)methyl)-1,2,3-oxadiazol-3-ium-5-yl)((2-(trifluoromethyl)pyridin-4-yl)carbamoyl)amide
- (3-(((1R,2R)-2Hydroxycyclohexyl)methyl)-1,2,3-oxadiazol-3-ium-5-yl)((3-(2-(o-tolyl)acetamido)-5-(trifluoromethyl)phenyl)-carbamoyl)amide
- (3-(((1S,2R)-2-Hydroxycyclohexyl)methyl)-1,2,3-oxadiazol-3-ium-5-yl)((3-(2-(o-tolyl)acetamido)-5-(trifluoromethyl)phenyl)-carbamoyl)amide
- (3-((1S,3S)-3-(Pyrimidin-5-yl)cyclohexyl)-1,2,3-oxadiazol-3-ium-5-yl)((2-(trifluoromethyl)pyridin -4-yl)carbamoyl)amide
- (3-((1S,3R)-3-(Pyrimidin-5-yl)cyclohexyl)-1,2,3-oxadiazol-3-ium-5-yl)((2-(trifluoromethyl)-pyridin-4-yl)carbamoyl)amide
- (3-((1S,3S)-3-(Pyrimidin-5-yl)cyclohexyl)-1,2,3-oxadiazol-3-ium-5-yl)((3-(2-(o-tolyl)acetamido)-5-(trifluoromethyl)phenyl)carbamoyl)amide
- (3 -((1S,3R)-3-(Pyrimidin-5-yl)cyclohexyl)-1,2,3 -oxadiazol-3 -ium-5-yl)((3 -(2-(o-tolyl)acetamido)-5-(trifluoromethyl)phenyl)-carbamoyl)amide
- (3-((1R,3S)-3-(Methoxycarbonyl)cyclohexyl)-1,2,3-oxadiazol-3-ium-5-yl)((3-(2-(o-tolyl)acetamido)-5-(trifluoromethyl)phenyl)-carbamoyl)amide
- (3-((1R,3R)-3-(Methoxycarbonyl)cyclohexyl)-1,2,3-oxadiazol-3-ium-5-yl)((3-(2-(o-tolyl)-acetamido)-5-(trifluoromethyl)phenyl)-carbamoyl)amide
- (3-(4-(4-Methylpyrimidin-5-yl)benzyl)-1,2,3-oxadiazol-3-ium-5-yl)((2-(trifluoromethyl)pyridin -4-yl)carbamoyl)amide
- ((2-(tert-Butyl)pyridin-4-yl)carbamoyl)(3-(4-(4,6-dimethylpyrimidin-5-yl)benzyl)-1,2,3-oxadiazol-3-ium-5-yl)amide
- (3-((1R,4R)-4-(Methoxycarbonyl)cyclohexyl)-1,2,3-oxadiazol-3-ium-5-yl)((3-(2-(o-tolyl)-acetamido)-5-(trifluoromethyl)phenyl)carbamoyl)
- amide
- (3-((1R,4R)-4-(Hydroxymethyl)cyclohexyl)-1,2,3-oxadiazol-3-ium-5-yl)((3-(2-(o-tolyl)acetamido)-5-(trifluoromethyl)phenyl) carbamoyl)amide
- (3-((1R,4R)-4-Carboxycyclohexyl)-1,2,3-oxadiazol-3-ium-5-yl)((3-(2-(o-tolyl)acetamido)-5-(trifluoromethyl)phenyl)carbamoyl)amide
- (3-((1R)-3-(Hydroxymethyl)cyclohexyl)-1,2,3-oxadiazol-3-ium-5-yl)((3-(2-(o-tolyl)acetamido)-5-(trifluoromethyl)phenyl)carbamoyl)amide
- (3-((1R,3R)-3-(Methylcarbamoyl)cyclohexyl)-1,2,3-oxadiazol-3-ium-5-yl)((3-(2-(o-tolyl)acetamido)-5-(trifluoromethyl)phenyl)-carbamoyl)amide
- (3-((1R,3S)-3-(Methylcarbamoyl)cyclohexyl)-1,2,3-oxadiazol-3-ium-5-yl)((3-(2-(o-tolyl)acetamido)-5-(trifluoromethyl)phenyl)-carbamoyl)amide
- (3-(4-(4,6-Dimethylpyrimidin-5-yl)benzyl)-1,2,3-oxadiazol-3-ium-5-yl)(((1S,3S)-3-(trifluoromethyl)cyclopentyl)carbamoyl)amide
- (3-((1R,3S)-3-(Dimethylcarbamoyl)cyclohexyl)-1,2,3-oxadiazol-3-ium-5-yl)((3-(2-(o-tolyl)-acetamido)-5-(trifluoromethyl)phenyl)-carbamoyl)amide
- (3-((1R,3R)-3-Carbamoylcyclohexyl)-1,2,3-oxadiazol-3-ium-5-yl)((3-(2-(o-tolyl)acetamido)-5-(trifluoromethyl)phenyl)carbamoyl)amide
- (3-(4-(Pyrimidin-5-yl)benzyl)-1,2,3-oxadiazol-3-ium-5-yl)((2-(trifluoromethyl)pyridin-4-yl)-carbamoyl)amide
- (3-(4-(1,3-Dimethyl-1H-pyrazol-4-yl)benzyl)-1,2,3-oxadiazol-3-ium-5-yl)((2-(trifluoromethyl)-pyridin-4-yl)carbamoyl)amide
- ((4-Cyano-3-(trifluoromethyl)phenyl)-carbamoyl)(3-(4-(1,4-dimethyl-1H-pyrazol-5-yl)benzyl)-1,2,3-oxadiazol-3-ium-5-yl)amide
- (3-((4-(4-Methyl-2-(oxetan-3-yl)pyrazol-3-yl]phenyl)methyl)oxadiazol-3-ium-5-yl)-((2-(trifluoromethyl)pyridin-4-yl)carbamoyl)-azanide
- (3-((1S,2R)-2-Hydroxycyclohexyl)-1,2,3-oxadiazol-3-ium-5-yl)((3-(2-(o-tolyl)acetamido)-5-(trifluoromethyl)phenyl)carbamoyl)amide
- (3-(((1R,4S)-4-(4-Methylpyrimidin-5-yl)cyclohexyl)methyl)-1,2,3-oxadiazol-3-ium-5-yl)((2-(trifluoromethyl)pyridin-4-yl)carbamoyl)-amide
- (3-(4-(1,4-Dimethyl-1H-pyrazol-5-yl)benzyl)-1,2,3-oxadiazol-3-ium-5-yl)((2-(trifluoromethyl)-pyridin-4-yl)carbamoyl)amide
- (3-((1R,4S)-4-((Dimethylamino)methyl)-cyclohexyl)-1,2,3-oxadiazol-3-ium-5-yl)((3-((S)-2-fluoro-2-(2-fluorophenyl)acetamido)-5-(trifluoromethyl)phenyl)carbamoyl)amide
- rac-(3-((1R,2S)-2-(Dimethylamino)cyclohexyl)-1,2,3-oxadiazol-3-ium-5-yl)((3-(2-(o-tolyl)acetamido)-5-(trifluoromethyl)phenyl)-carbamoyl)amide
- rac-(3-((1R,3S)-3-(Methylcarbamoyl)cyclohexyl)-1,2,3-oxadiazol-3-ium-5-yl)((3-(2-(o-tolyl)acetamido)-5-(trifluoromethyl)phenyl)carbamoyl)amide
- rac-(3-((1S,3R)-3-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide
- rac-(3-((1R,3S)-3-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide
- (3-((1R,4R)-4-(Dimethylcarbamoyl)-cyclohexyl)-1,2,3-oxadiazol-3-ium-5-yl)((3-(2-(o-tolyl)acetamido)-5-(trifluoromethyl)phenyl)-carbamoyl)amide
- (3-(4-(4,6-Dimethylpyrimidin-5-yl)benzyl)-1,2,3-oxadiazol-3-ium-5-yl)((4-methoxy-3-(trifluoromethyl)phenyl)carbamoyl)amide
- (3-(4-(4-Methyl-1-(oxetan-3-yl)-1H-pyrazol-3-yl)benzyl)-1,2,3-oxadiazol-3-ium-5-yl)((2-(trifluoromethyl)pyridin-4-yl)carbamoyl)amide
- rac-(3-((1R,4R)-4-(Methylcarbamoyl)-cyclohexyl)-1,2,3-oxadiazol-3-ium-5-yl)((3-(2-(o-tolyl)acetamido)-5-(trifluoromethyl)phenyl)-carbamoyl)amide
- rac-(3-((1R,3R)-3-(Methylcarbamoyl)-cyclohexyl)-1,2,3-oxadiazol-3-ium-5-yl)((3-(2-(o-tolyl)-acetamido)-5-(trifluoromethyl)phenyl)-carbamoyl)amide
- rac-(3-((1R,3S)-3-(Dimethylcarbamoyl)-cyclohexyl)-1,2,3-oxadiazol-3-ium-5-yl)((3-(2-(o-tolyl)-acetamido)-5-(trifluoromethyl)phenyl)-carbamoyl)amide
- (3-((1R,4R)-4-(Pyrrolidine-1-carbonyl)-cyclohexyl)-1,2,3-oxadiazol-3-ium-5-yl)((3-(2-(o-tolyl)acetamido)-5-(trifluoromethyl)phenyl)-carbamoyl)amide
- (3-((1R,3S)-3-Carbamoylcyclohexyl)-1,2,3-oxadiazol-3-ium-5-yl)((3-(2-(o-tolyl)acetamido)-5-(trifluoromethyl)phenyl)carbamoyl)amide
- (3-((4-(4,6-Dimethylpyrimidin-5-yl)phenyl)methyl)oxadiazol-3-ium-5-yl)-((rac-(1R,3R)-3-(trifluoromethyl)cyclopentyl)-carbamoyl)azanide
- (3-((1R,4R)-4-(Dimethylamino)cyclohexyl)-1,2,3-oxadiazol-3-ium-5-yl)((3-(2-(o-tolyl)-acetamido)-5-(trifluoromethyl)phenyl)-carbamoyl)amide
- (3-(4-(2-Methylpyridin-3-yl)benzyl)-1,2,3-oxadiazol-3-ium-5-yl)((2-(trifluoromethyl)pyridin -4-yl)carbamoyl)amide
- (3-(4-(2,5-Dimethylthiazol-4-yl)benzyl)-1,2,3-oxadiazol-3-ium-5-yl)((2-(trifluoromethyl)pyridin -4-yl)carbamoyl)amide
- (3-(4-(2-Amino-4-methylpyrimidin-5-yl)benzyl)-1,2,3-oxadiazol-3-ium-5-yl)((2-(trifluoromethyl)pyridin-4-yl)carbamoyl)amide
- (3-(4-(4,6-Dimethylpyrimidin-5-yl)benzyl)-1,2,3-oxadiazol-3-ium-5-yl)((4-hydroxy-3-(trifluoromethyl)phenyl)carbamoyl)amide
- (3-(4-(4,6-Dimethylpyrimidin-5-yl)benzyl)-1,2,3-oxadiazol-3-ium-5-yl)((4-(methoxy-carbonyl)-3-(trifluoromethyl)phenyl)carbamoyl) amide
- (3-(4-(2,4-Dimethylpyridin-3-yl)benzyl)-1,2,3-oxadiazol-3-ium-5-yl)((2-(trifluoromethyl)pyridin -4-yl)carbamoyl)amide
- ((4-Acetamido-3-(trifluoromethyl)phenyl)-carbamoyl)(3-(4-(4,6-dimethylpyrimidin-5-yl)benzyl)-1,2,3-oxadiazol-3-ium-5-yl)amide
- (3-(3-(Hydroxymethyl)cyclohexyl)-1,2,3-oxadiazol-3-ium-5-yl)((3-(2-(o-tolyl)acetamido)-5-(trifluoromethyl)phenyl)carbamoyl)amide
- (3-(4-(Pyrimidin-5-yl)benzyl)-1,2,3-oxadiazol-3-ium-5-yl)((2-(trifluoromethyl)pyridin-4-yl)carbamoyl)amide
- (3-((1R,3R)-3-Carbamoylcyclohexyl)-1,2,3-oxadiazol-3-ium-5-yl)((3-(2-(o-tolyl)acetamido)-5-(trifluoromethyl)phenyl)carbamoyl)amide
- ((2-(Trifluoromethyl)pyridin-4-yl)carbamoyl)(3-(4-(1,3,5-trimethyl-1H-pyrazol-4-yl)benzyl)-1,2,3-oxadiazol-3-ium-5-yl)amide
- (3-(4-(4-Methoxy-6-methylpyrimidin-5-yl)benzyl)-1,2,3-oxadiazol-3-ium-5-yl)((2-(trifluoromethyl)pyridin-4-yl)carbamoyl)amide
- ((2-(Trifluoromethyl)pyridin-4-yl)carbamoyl)(3-(4-(4-(trifluoromethyl)pyrimidin-5-yl)benzyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-((R)-2-fluoro-2-(2-fluorophenyl)acetamido)-5-(trifluoromethyl)phenyl)carbamoyl)amide
- (3-(4-(4,6-Dimethylpyrimidin-5-yl)benzyl)-1,2,3-oxadiazol-3-ium-5-yl)((8-(trifluoromethyl)-quinolin-6-yl)carbamoyl)amide
- (3-(4-(4,6-Dimethylpyrimidin-5-yl)benzyl)-1,2,3-oxadiazol-3-ium-5-yl)((4-(methylcarbamoyl)-3-(trifluoromethyl)phenyl)carbamoyl) amide.

Preferably the compound according is an inhibitor of NS2B-NS3 protease of a Flavivirus, preferably an inhibitor of the NS2B-NS3 protease of Zika and/or Dengue and/or West Nile and/or Japan Encephalitis virus, thus inhibiting replication of the above viruses in cells.

It is a further object if the invention a compound as defined above for medical use, preferably for use in treatment and/or prevention of a Flavivirus infection, preferably wherein the Flavivirus is selected from the group consisting of Dengue, West Nile, Zika and Japan Encephalitis virus. In one embodiment, the Flavivirus infection is dengue fever. In a further embodiment, the dengue fever is Dengue virus type 1 (DENV-1), type 2 (DENV-2), type 3 (DENV-3), or type 4 (DENV-4). In one embodiment, the Flavivirus infection is West Nile fever. In one embodiment, Flavivirus infection is Yellow Fever. In one embodiment, the Flavivirus infection is from the Zika virus.

It is a further object of the invention a pharmaceutical composition comprising the compound as defined above and at least one pharmaceutically acceptable excipient.

Preferably the pharmaceutical composition is for use in the treatment and/or prevention of a flavivirus infection, preferably wherein the flavivirus is selected from the group consisting of Dengue, West Nile, Zika and Japan Encephalitis virus.

In a preferred embodiment, said pharmaceutical composition comprises at least one further active compound selected from the group consisting of: antivirals, antibacterials, anti-inflammatory agents, anti-pain agents and antipyretic agents.

It is a further object of the invention a method for the synthesis of the compound of general formula (I) as defined above.

The present invention includes within its scope prodrugs of the compound of formula (I). In general, such prodrugs will be functional derivatives of the compound of formula (I) which are readily convertible *in vivo* into the required compound of formula (I). Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The present invention includes within its scope solvates of the compound of formula (I) and salts thereof, for example, hydrates.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and all tautomeric forms are intended to be encompassed by the scope of the invention, even though only one tautomeric structure is depicted. Specific tautomeric forms of compounds of the invention are depicted below:

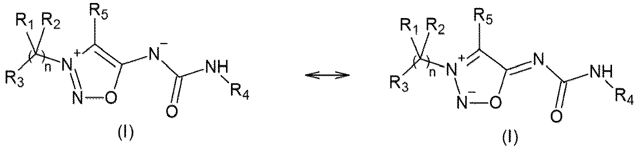

The compounds may exist in different isomeric forms, all of which are encompassed by the present invention.

When any variable occurs more than one time in any constituent, its definition on each occurrence is independent of every other occurrence. Also, combinations of substituents and variables are permissible only if such combinations result in stable compounds.

It is understood that substituents and substitution patterns on the compounds of the instant invention can be selected by one of ordinary skill in the art to provide compounds that are chemically stable and that can be readily synthesized from readily available starting materials by techniques known in the art, as well as those methods set forth below. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents. A substituent on a saturated, partially saturated or unsaturated heterocycle can be attached at any substitutable position.

As used herein, "alkyl" is intended to include both branched and straight-chain saturated aliphatic hydrocarbon groups having the specified number of carbon atoms. For example, "C₁-C₆ alkyl" is defined to include groups having 1, 2, 3, 4, 5 or 6 carbons in a linear or branched arrangement. For example, "C₁-C₆ alkyl" specifically includes methyl, ethyl, *n*-propyl, *i*-propyl, *n*-butyl, *t*-butyl, *i*-butyl, pentyl, hexyl, and so on.

"Cycloalkyl" refers to a non-aromatic hydrocarbon ring system (monocyclic, bicyclic, or polycyclic), including cyclized alkyl and alkenyl groups. The term "Cn-m cycloalkyl" or "(Cn-Cm) cycloalkyl" refers to a cycloalkyl that has n to m ring member carbon atoms. Cycloalkyl groups can include mono- or polycyclic (e.g., having 2, 3, or 4 fused rings) groups and spirocycles. Cycloalkyl groups can have 3, 4, 5, 6, 7, 8, 9, 10, 11, 12, 13, or 14 ring-forming carbons (C3-14). In some embodiments, the cycloalkyl group has 3 to 14 members, 3 to 10 members, 3 to 6 ring members, 3 to 5 ring members, or 3 to 4 ring members. In some embodiments, the cycloalkyl group is monocyclic. In some embodiments, the cycloalkyl group is monocyclic or bicyclic. In some embodiments, the cycloalkyl group is a C3-6 monocyclic cycloalkyl group. Ring-forming carbon atoms of a cycloalkyl group can be optionally oxidized to form an oxo or sulfido group. Cycloalkyl groups also include cycloalkylidenes. In some embodiments, cycloalkyl is cyclopropyl, cyclobutyl, cyclopentyl, or cyclohexyl. Examples of cycloalkyl groups include cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcarnyl, bicyclo[1.1.1]pentanyl, bicyclo[2.1.1]hexanyl, and the like. In some embodiments, the cycloalkyl group is cyclopropyl, cyclobutyl, cyclopentyl, or cyclohexyl. In some embodiments, cycloalkyl includes a single saturated carbocyclic ring of three to eight ring carbons, such as cyclopropyl, cyclobutyl, cyclopentyl, and cyclohexyl. Cycloalkyl may optionally be substituted with one or more substituents, such as one, two, or three substituents. In some embodiments, the cycloalkyl substituent is selected from the group consisting of (C1- C6)alkyl, hydroxy, (C1-C6)alkoxy, halo(C1-C6)alkyl, halo(C1-C6)alkoxy, halo, amino, mono- and di(C1-C6)alkylamino, hetero(C1-C6)alkyl, acyl, aryl, and heteroaryl. A cycloalkyl group can be unsubstituted or optionally substituted. When optionally substituted, one or more hydrogen atoms of the cycloalkyl group (e.g., from 1 to 4, from 1 to 2, or 1) may be replaced with a moiety as halogen, OH, cyano, methyl, trifluoromethyl, amino and the like. In some aspects, a substituted cycloalkyl group can incorporate an exo- or endocyclic alkene (e.g., cyclohex-2-en-1-yl). In some aspects, a cycloalkyl group is unsubstituted or not optionally substituted.

"Alkoxy" represents an alkyl group of indicated number of carbon atoms attached through an oxygen bridge. "Alkoxy" therefore encompasses the definitions of alkyl above. Examples of suitable alkoxy groups include methoxy, ethoxy, *n*-propoxy, *i*-propoxy, *n*-butoxy, *s*-butoxy and *t-*butoxy. The preferred alkoxy group is methoxy.

The terms "haloC₁₋₆alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. Preferred are fluoroC₁₋₆alkyl and fluoroC₁₋₆alkoxy groups, in particular fluoroC₁₋₃alkyl and fluoroC₁₋₃alkoxy groups, for example, CF₃, CHF₂, CH₂F, CH₂CH₂F, CH₂CHF₂, CH₂CF₃, OCF₃, OCHF₂, OCH₂F, OCH₂CH₂F, OCH₂CHF₂ or OCH₂CF₃, and most especially CF₃, OCF₃ and OCHF₂.

The term "hydroxyC₁₋₆alkyl" means a C₁₋₆alkyl group in which one or more (in particular, 1 to 3) hydrogen atoms have been replaced by hydroxy groups. Preferred are CH₂OH, CH₂CHOH and CHOHCH₃.

As used herein, "aryl" or "aromatic ring" is intended to mean any stable monocyclic or bicyclic carbon ring of 6 to 10 atoms, wherein at least one ring is aromatic. Examples of such aryl elements include phenyl, naphthyl, tetrahydronaphthyl, indanyl and tetrahydrobenzo[7]annulene. The preferred aryl group is phenyl or naphthyl, especially phenyl.

As used herein, "heteroaryl" or "heteroaromatic ring" is intended to mean any stable monocyclic or bicyclic ring containing 1, 2, 3 or 4 heteroatoms independently selected from N, O and S, wherein at least one ring is aromatic. In particular the term heteroaryl includes 5 membered aromatic heterocycles containing 1, 2, 3 or 4 heteroatoms independently selected from N, O and S , but not more than one of which is O or S; 6 membered aromatic heterocycles containing 1, 2 or 3 nitrogen atoms; or a 7-13 membered aromatic heterocycle containing heteroatoms independently selected from N, O or S of 5 to 10 atoms, wherein at least one ring is aromatic. Heteroaryl ring also includes partially saturated bicyclic systems, such as indoline, isoindoline, 4,5,6,7-tetrahydropyrazolo[1,5-a]pyrazine; further included are tautomeric structures, such as for example hydroxypyrimidine and hydroxypyrimidones. When optionally substituted, one or more hydrogen atoms of the aryl or heteroaryl ring may be replaced with a moiety as halogen, OH, cyano, methyl, trifluoromethyl, amino and the like.

Examples of particular heteroaryl of this invention are benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl, benzoisoxazolyl, benzoisothiazolyl, chromenyl, chromanyl, isochromanyl, carbazolyl, carbolinyl, cinnolinyl, epoxidyl, furanyl, furazanyl, imidazolyl, imidazothiazolyl, indolinyl, indolyl, indolizinyl, isoindolinyl, indazolyl, isobenzofuranyl, isoindolyl, isoquinolyl, isothiazolyl, isoxazolyl, naphthpyridinyl, oxadiazolyl, oxazolyl, oxazolinyl, oxoquinazolinyl isoxazolinyl, oxetanyl, purinyl, pyranyl, pyrazinyl, pyrazolyl, pyridazinyl, pyridopyridinyl, pyridazinyl, pyridinyl, pyrimidinyl, triazinyl, tetrazinyl, pyrrolyl, quinazolinyl, quinolyl, quinoxalinyl, quinolizinyl, tetrazolyl, tetrazolopyridyl, thiadiazolyl, thiazolidinyl, thiazolyl, thienyl, triazolyl, imidazopyridinyl, phthalazinyl, naphthyridinyl, quinazolinyl, pteridinyl, pyrimidin-4(3H)-one and N oxides thereof. Attachment of a heterocycles substituent can occur via a carbon atom or via a heteroatom.

Biphenyl indicates a phenyl ring substituted with a further phenyl ring; phenyl-heteroaryl indicates a phenyl ring substituted with an heteroaromatic ring; bis heteroaryl indicates and heteroaryl substituted with a further heteroaromatic ring.

The term "heterocyclic ring" refers to a non-aromatic ring radical, which consists of carbon atoms and at least one heteroatom of nitrogen, phosphorus, oxygen or sulfur. The heterocyclic ring may be a mono-, bi-, tri-, or tetracyclic ring system, which may include fused, bridged or spiro ring systems, and the nitrogen, phosphorus, carbon, oxygen, or sulfur atoms in the heterocyclic ring radical may be optionally oxidized to various oxidation states. In addition, the nitrogen atom may be optionally quaternized.

Examples of particular heterocyclic rings of the invention are tetrahydrofuranyl, tetrahydropyranyl, tetrahydrothiopyranyl, tetrahydroisoquinolinyl, azetidinyl, 1,4-dioxanyl, hexahydroazepinyl, piperazinyl, piperidyl, pyridin-2-onyl, pyrrolidinyl, imidazolinyl, pyrazolinyl, pyrrolinyl, morpholinyl, thiomorpholinyl, dihydrobenzoimidazolyl, dihydrobenzofuranyl, dihydrobenzothiophenyl, dihydrobenzoxazolyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, dihydroisochromenyl, dihydroimidazolonyl, dihydrotriazolonyl, dihydrobenzodioxinyl, dihydrothiazolopyrimidinyl, methylenedioxybenzoyl, tetrahydrofuranyl, tetrahydrothienyl, tetrahydroquinolinyl, thiazolidinonyl, imidazolonyl, isoindolinonyl, octahydroquinolizinyl, octahydroisoindolyl, azabicycloheptanyl, chromenonyl, triazolopyrimidinyl, dihydrobenzoxazinyl, thiazolotriazolyl, azoniabicycloheptanyl, azoniabicyclooctanyl. Attachment of a heterocyclyl substituent can occur via a carbon atom or via a heteroatom.

A preferred 4 membered saturated heterocycle is azetidine. A further preferred 4 membered saturated heterocycle is oxetane.

A preferred 5 membered saturated heterocycle is tetrahydrofurane.

Preferred 6 membered saturated or partially saturated heterocycles are pyrrolidinyl, piperidyl, piperazinyl, morpholinyl, thiomorpholinyl and thiazolidinyl.

Preferred 5 membered heteroaryls are thienyl, thiazolyl, pyrazolyl, isoxazolyl, imidazolyl, thiadiazolyl, oxazolyl, triazolyl, tetrazolyl, furyl and oxadiazolyl.

Preferred 6 membered heteroaryls are pyridinyl and pyrymidinyl.

Preferred 8-10 membered heteroaryls are benzothienyl, indolyl, benzothiadiazolyl, benzoxadiazolyl, thiazolotriazolyl, dihydrobenzodioxinyl, dihydrothiazolopyrimidinyl, dihydrobenzoxazinyl, dihydrobenzofuranyl, benzothiazolyl, quinolinyl, isoquinolinyl, benzimidazolyl, benzofuranyl, dihydrobenzoxazolyl, dihydroindolyl, dihydroquinazolinyl, dihydrophthalazinyl, indazolyl, benzisoxazolyl, benzotriazolyl, dihydroisoindolyl, tetrahydronaphthyridinyl, triazolopyrimidinyl and tetrahydroquinolinyl.

As used herein, the term 'halogen' refers to fluorine, chlorine, bromine and iodine, of which fluorine, chlorine and bromine are preferred.

As used herein, the term "amine" refers to linear and cyclic amines, such as methylamine, dimethylamine, diethylamine, isopropylamine, pyrrolidine, pyperidine and the like. A substutuent referred generically as "amine" indicates that an amine radical is linked through its nitrogen to the specific residue. In some instances, it is herein used the term "C₁₋₆alkylamine" which refers to an alkyl group substituted with an amino group, such as -CH₂NH₂, -CH₂NHCH₃, CH₂NHCH₂CH₃, CH₂-pyrrolidine, CH₂-piperidine and the like.

As used herein, an optionally substituted aryl, heteroaryl, cycloalkyl, heterocycloalkyl refer to any of said ring systems as above defined being optionally substituted with groups such as halogen, hydroxy, methyl, trifluoromethyl, amino, methylamino, and the like.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reaction of the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reaction of a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Preferably, a pharmaceutically acceptable salt of this invention contains one equivalent of a compound of formula (I) and 1, 2 or 3 equivalent of an inorganic or organic acid. More particularly, pharmaceutically acceptable salts of this invention are the tartrate, trifluoroacetate or the chloride salts.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, *N*,*N*¹-dibenzylethylenediamine, diethylamine, 2-diethylaminoethanol, 2-dimethylaminoethanol, ethanolamine, ethylenediamine, *N-*ethylmorpholine, *N*-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the invention find use in a variety of applications for human and animal health. The compounds of the invention are flavivirus inhibitors and can be used in the treatment and/or prevention of flavivirus infections.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl *p*-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsion. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants. Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solution. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution may be then introduced into a water and glycerol mixture and processed to form a microemulsion. The injectable solutions or microemulsions may be introduced into a patient's blood stream by local bolus injection.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of inj ectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of the invention are employed. The compounds of the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol. When a compound according to this invention is administered into a human subject, the daily dosage regimen will normally be determined by the prescribing physician with the dosage generally varying according to the age, weight, sex and response of the individual patient, as well as the severity of the patient's symptoms.

The instant compounds are also useful in combination with known therapeutic agents for simultaneous, separate or sequential administration.

The term "administration" and variants thereof (e.g., "administering" a compound) in reference to a compound of the invention means introducing the compound or a prodrug of the compound into the system of the subject in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

When a compound of the present invention is administered into a human subject, the daily dosage regimen will normally be determined by the prescribing physician, with the dosage generally varying according to the age, weight, and response of the individual patient, as well as the severity of the patient's symptoms. In one exemplary application, oral dosages of the present invention will range between about 0.01 mg per kg of body weight per day (mg/kg/day) to about 100 mg/kg/day, preferably 0.01 to 10 mg/kg/day, and most preferably 0.1 to 5.0 mg/kg/day.

It is a further object of the invention a method treating, ameliorating or preventing a Flavivirus infection and related conditions in a subject, preferably wherein the Flavivirus is selected from the group consisting of Dengue, West Nile, Zika and Japan Encephalitis virus, comprising administering to said subject a therapeutically effective amount of the compound as defined above. These and other aspects of the invention will be apparent from the teachings contained herein.

### Chemistry

### General

The compounds, or pharmaceutically acceptable salts thereof, compositions, and methods described herein are further illustrated by the following non-limiting examples.

As used herein, the following abbreviations have the following meanings. If an abbreviation is not defined, it has its generally accepted meaning.

Boc₂O: Di-*tert*-butyl decarbonate; DCM: Dichloromethane; DIPEA: N,N-Diisopropylethylamine; DMF: Dimethylformamide; DMSO: Dimethylsulfoxide; ES⁺: Electrospray Positive Ionisation; EtOAc: Ethyl acetate; EtOH: Ethanol; h: Hour; HATU: Hexafluorophosphate Azabenzotriazole Tetramethyl Uronium; HPLC: High Performance Liquid Chromatography; LiBH₄: Lithium borohydride; LiOH: Lithium hydroxide; LCMS: Liquid Chromatography Mass Spectrometry; KOAc: Potassium acetate; K₂CO₃: Potassium carbonate; K₃PO₄: tripotassium phosphate; MeCN: Acetonitrile; MeOH: Methanol; min: Minute; NaH: Sodium Hydride; NH₃: ammonium; NMP: *N-*Methylpyrrolidone; NMR: Nuclear Magnetic Resonance; NH₂NH₂: Hydrazine; NaCNBH₃: Sodium cyanoborohydride; Pd(dppf)Cl₂: (1,1'-Bis(diphenylphosphino)ferrocene)-palladium(II) dichloride; Pd(OAc)₂: Palladium(II) acetate; PCy₃: triclyclohexylphosphine; Py: pyridine; RP: Reverse Phase; *t*_{R}: Retention time; rt: room temperature; ^{t}BuONO: *tert*-Butyl nitrite; TEA: Triethylamine; TIPS: Triisopropylsilyl ether; TFA: Trifluoroacetic acid; THF: Tetrahydrofurane; UPLC: Ultra High Performance Liquid Chromatography.

### General experimental details

Solvents and reagents were obtained from commercial suppliers and were used without further purification. Flash chromatography purifications were performed on prepacked cartridges on a Biotage system. Purity of final compounds was determined using MS and UPLC. UPLC-MS analyses were performed on a Waters Acquity UPLC^{™}, equipped with a diode array and a ZQ mass spectrometer, using an X-Terra C18 column (5 µm, 4.6 x 50 mm) or a BEH C18 column (1.7 mm, 2.1 x 50 mm). Mobile phase comprised a linear gradient of binary mixtures of H₂O containing 0.1% formic acid (A), and MeCN containing 0.1% formic acid (B). The linear gradient used is: (A): 90% (0.1 min), 90%-0% (2.6 min), 0% (0.3 min), 0%-90% (0.1 min) with a 0.5 mL/min flow. The purity of final compounds was ≥95%. All ¹H spectra were recorded on Bruker AV400 spectrometer at 400 MHz except where indicated. Chemical shift (δ) are reported in parts per million relative to TMS using CDCl₃ as a solvent or relative to the residual solvent signal using DMSO-d₆. Coupling costants (*J*) are reported in Hertz (Hz). Multiplicities are reported as singlet (s), broad (br), doublet (d), doublet of doublet (dd), doublet of doublet of doublet (ddd), triplet (t), doublet of triplet (dt), doublet of doublet of triplet (ddt), triplet of triplet (tt), quartet (q), doublet of quartets (dq) or multiplet (m). Unless indicated, spectra were acquired at 300 K. Temperatures are expressed in degrees Celsius (°C) and are uncorrected. Unless otherwise indicated, commercially available reagents and solvents (HPLC grade) were used without further purification. Where the synthesis of intermediates and starting materials is not described, these compounds are commercially available or can be made from commercially available compounds by standard methods or by extension of the Examples herein. During any of the synthetic sequences described herein it may be necessary and/or desirable to protect sensitive or reactive groups or any of the molecules concerned, this may be achieved by means of conventional protecting groups, such as those described in Protecting Groups in Organic Synthesis (3rd Edition, Greene, T.W. and Wuts, P.G.M.; Wiley Interscience, 1999) and Protecting Groups (Kocienski, P.J.; Thieme, 1994).

### Synthesis of Intermediates 1-9

### Intermediate 1: N-(3-Amino-5-(trifluoromethyl)phenyl)-2-phenylacetamide

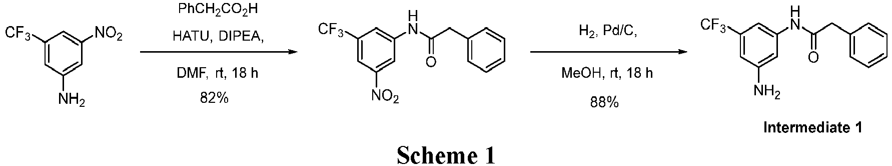

### Step 1: N-(3-Nitro-5-(trifluoromethyl)phenyl)-2-phenylacetamide.

A solution of 3-nitro-5-(trifluoromethyl)aniline (300 mg, 1.46 mmol) and HATU (830 mg, 2.18 mmol) in dry DMF (8 mL) was treated with DIPEA (0.77 mL, 4.37 mmol) followed by 2-phenylacetic acid (297 mg, 2.18 mmol) and the reaction mixture was stirred at rt for 18 h before being diluted with EtOAc (45 mL) and H₂O (15 ml); the organic layer was separated, washed with brine and dried under reduced pressure to get a residue which was purified by flash chromatography on silica gel (eluting with 10-25% EtOAc in petroleum ether) to get the title compound as a yellow solid (387 mg, 82%). ¹H NMR (400 MHz, DMSO-*d*₆) δ 10.98 (s, 1H), 8.76 (br s, 1H), 8.39 (s, 1H), 8.16 (s, 1H), 7.36-7.28 (m, 5H), 3.73 (s, 2H). LCMS (ES⁺) *m*/*z* calculated for C₁₅H₁₁F₃N₂O₃ 324.07, found 323 (M-H)⁻. HPLC *t*_{R} 2.03 min.

### Step 2: N-(3-Amino-5-(trifluoromethyl)phenyl)-2-phenylacetamide (Intermediate 1).

A solution of N-(3-nitro-5-(trifluoromethyl)phenyl)-2-phenylacetamide (387 mg, 1.19 mmol) in MeOH (14 mL) was treated with Pd (10% on C, 40 mg, 0.38 mmol) and stirred under an H₂ atmosphere at rt for 18 h. After removal of H₂ the reaction mixture was filtered on a pad of Celite and the solvent removed under reduced pressure to get a residue which was purified by RP chromatography on silica gel (eluting with 20-55% CH₃CN in H₂O) to get the title compound as a white solid (310 mg, 88%). ¹H NMR (400 MHz, DMSO-*d*₆) δ 10.15 (s, 1H), 7.33-7.32 (m, 4H), 7.28-7.23 (m, 1H), 7.10 (br s, 1H), 7.07 (br s, 1H), 6.53 (br s, 1H), 5.60 (br s, 2H), 3.61 (s, 2H). ¹⁹F NMR (377 MHz, DMSO-*d*₆) δ -61.73 (s, 3F). LCMS (ES⁺) *m*/*z* calculated for C₁₅H₁₃F₃N₂O 294.10, found 293 (M-H)⁻. HPLC *t*_{R} 1.66 min.

### Intermediate 2: N-3-Isocyanato-5-(trifluoromethyl)phenyl)-2-phenylacetamide

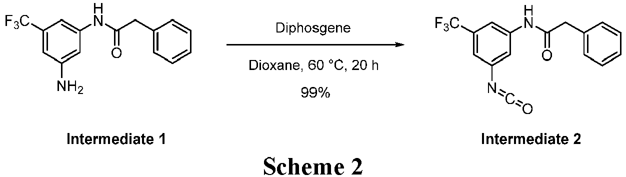

### Step 1: N-(3-Isocyanato-5-(trifluoromethyl)phenyl)-2-phenylacetamide (Intermediate 2).

A solution of *N-*3-amino-5-(trifluoromethyl)phenyl)-2-phenylacetamide (Intermediate **1**, 78.19 mg, 0.27 mmol) and diphosgene (0.1 mL, 0.80 mmol) in dry dioxane (4 mL) was heated at 60 °C for 20 h. After cooling the solvent was removed under reduced pressure to get the title compound (85 mg, 99%) which was used in the next step without further purification.

### Intermediate 3: N-(3-Cyclopropyl-5-isocyanatophenyl)-2-phenylacetamide

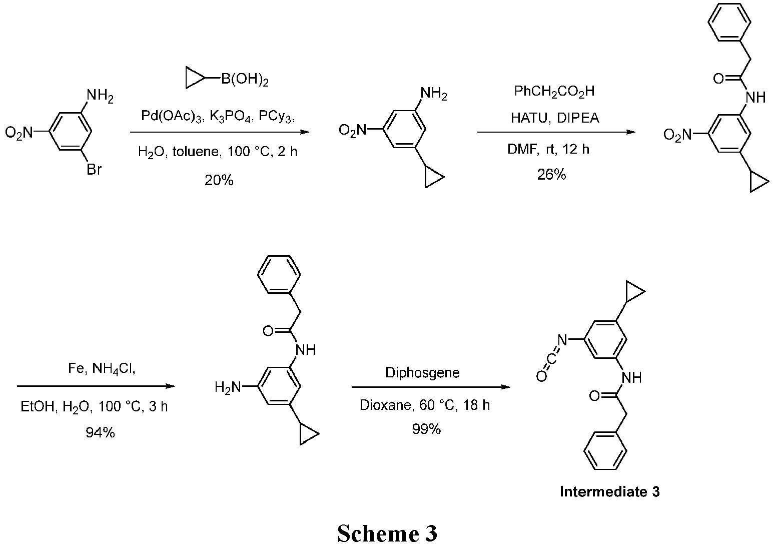

### Step 1: 3-Cyclopropyl-5-nitroaniline.

A solution of 3-bromo-5-nitroaniline (500 mg, 2.3 mmol), PCy₃ (77 mg, 0.28 mmol), cyclopropylboronic acid (297 mg, 3.46 mmol), Pd(OAc)₂ (26 mg, 0.12 mmol) and K₃PO₄ (978 mg, 4.61 mmol) in a degassed mixture of toluene (8 mL) and H₂O (2 mL) was stirred at 100 °C for 2 h. After cooling the mixture was diluted with EtOAC, washed with brine, dried over Na₂SO₄, and concentrated under reduced pressure to get a residue which was purified by flash chromatography on silica gel (eluting with 0-20% EtOAc in petroleum ether) to get the title compound (206 mg, 40% pure, 20%) which was used without further purification. LCMS (ES⁺) *m*/*z* calculated for C₉H₁₀N₂O₂ 178.07, found 179 (M+H)⁺. HPLC *t*_{R} 1.62 min.

### Step 2: N-(3-Cyclopropyl-5-nitrophenyl)-2-phenylacetamide.

A solution of 3-cyclopropyl-5-nitroaniline (206 mg, 1.16 mmol) and HATU (659 mg, 1.73 mmol) in dry DMF (10 mL) was treated with DIPEA (448 mg, 3.47 mmol) followed by 2-phenylacetic acid (189 mg, 1.39 mmol) and the reaction mixture was stirred at rt for 12 h before being diluted with EtOAc; the organic phase was washed with NaHCO₃ (sat aqueous solution), brine and dried under reduced pressure to get a residue which was purified by flash chromatography on silica gel (eluting with 0-25% EtOAc in petroleum ether) to get the title compound as a yellow solid (89.6 mg, 26%). ¹H NMR (400 MHz, DMSO-*d*₆) □ 10.56 (s, 1H), 8.39 (br t, *J* = 1.9 Hz, 1H), 7.63 (br d, *J* = 1.8 Hz, 2H), 7.34-7.33 (m, 4H), 7.29-7.25 (m, 1H), 3.68 (s, 2H), 2.12-2.06 (m, 1H), 1.08-1.03 (m, 2H), 0.76-0.72 (m, 2H). LCMS (ES⁺) *m*/*z* calculated for C₁₇H₁₆N₂O₃ 296.12, found 297 (M+H)⁺. HPLC *t*_{R} 1.94 min.

### Step 3: N-(3-Amino-5-cyclopropylphenyl)-2-phenylacetamide.

A solution of *N*-(3-cyclopropyl-5-nitrophenyl)-2-phenylacetamide (85 mg, 0.29 mmol) and NH₄Cl (38 mg, 0.72 mmol) in a mixture of EtOH (5 mL) and H₂O (2 mL) was treated with iron (80.1 mg, 1.43 mmol) and heated at 100 °C for 3 h before being diluted with EtOAc; the organic phase was washed with NaHCO₃ (sat aqueous solution), brine and dried under reduced pressure to get the title compound (71.7 mg, 94%) which was used without further purification. ¹H NMR (400 MHz, DMSO-*d*₆) □ 9.75 (s, 1H), 7.32-7.31 (m, 4H), 7.28-7.21 (m, 1H), 6.70 (br s, 1H), 6.44 (br s, 1H), 5.99 (br s, 1H), 4.94 (br s, 2H), 3.56 (s, 2H), 1.71-1.65 (m, 1H), 0.86-0.82 (m, 2H), 0.52-0.49 (m, 2H). LCMS (ES⁺) *m*/*z* calculated for C₁₇H₁₈N₂O 266.14, found 267 (M+H)⁺. HPLC *t*_{R} 1.21 min.

### Step 4: N-(3-Cyclopropyl-5-isocyanatophenyl)-2-phenylacetamide (Intermediate 3).

A solution of *N*-(3-amino-5-cyclopropylphenyl)-2-phenylacetamide (75 mg, 0.28 mmol) in dry dioxane (5 mL) was treated with diphosgene (0.1 mL, 0.84 mmol) and stirred at 60 °C for 18 h. After cooling the solvent was removed under reduced pressure to get the title compound (82 mg, 99%) which was used without further purification.

### Intermediate 4: 1-Isocyanato-3-nitro-5-(trifluoromethyl)benzene

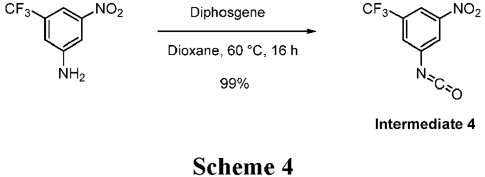

### Step 1: 1-Isocyanato-3-nitro-5-(trifluoromethyl)benzene (Intermediate 4).

A mixture of 3-nitro-5-(trifluoromethyl)aniline (100 mg, 0.49 mmol) and diphosgene (0.17 mL, 1.46 mmol) in dry dioxane (30 mL) was heated at 60 °C for 16 h. After cooling the solvent was removed under reduced pressure to get the title compound (112 mg, 99%) which was used as such without further purification.

### Intermediate 5: tert-Butyl (tert-butoxycarbonyl)(3-isocyanato-5-(trifluoromethyl)phenyl)-Carbamate

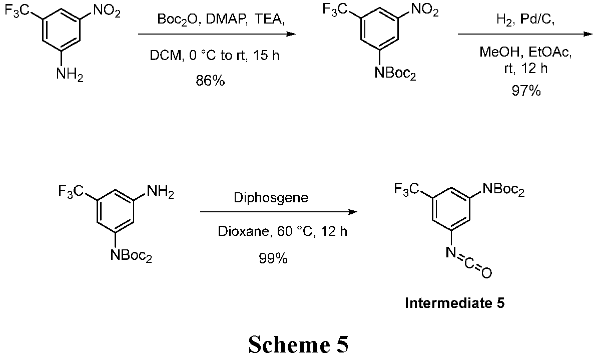

### Step 1: tert-Butyl (tert-butoxycarbonyl)(3-nitro-5-(trifluoromethyl)phenyl)carbamate.

A solution of 3-nitro-5-(trifluoromethyl)aniline (500 mg, 2.43 mmol) in DCM (20 mL) was treated with TEA (0.33 mL, 2.43 mmol), DMAP (296 mg, 2.43 mmol) and Boc₂O (2.12 g, 9.7 mmol) at 0 °C and stirred at rt for 15 h before being washed with H₂O, NaHCO₃ (saturated aqueous solution) and brine, dried over Na₂SO₄, and evaporated under reduced pressure to get a residue which was purified by flash chromatography on silica gel (eluting with 30% EtOAc in petroleum ether) to get the title compound as a light yellow solid (844 mg, 86%). ¹H NMR (400 MHz, DMSO-*d*₆) δ 8.54 (br s, 1H), 8.47 (br s, 1H), 8.34 (br s, 1H), 1.40 (s, 18H). LCMS (ES⁺) *m*/*z* calculated for C₁₇H₂₁F₃N₂O₆ 406.14, found 305 (M-H-Boc)⁻. HPLC *t*_{R} 2.48 min.

### Step 2: tert-Butyl (3-amino-5-(trifluoromethyl)phenyl)(tert-butoxycarbonyl)carbamate.

A solution of tert-butyl (tert-butoxycarbonyl)(3-nitro-5-(trifluoromethyl)phenyl)carbamate (840 mg, 2.07 mmol) in a mixture of MeOH (10 mL) and EtOAc (10 mL) was treated with Pd/C (10% wt, 160 mg, 1.5 mmol) and stirred under H₂ atmosphere at rt for 12 h. After removal of H₂ the solution was filtered on a pad of Celite and the solvent removed under reduced pressure to get the title compound as a white solid (756 mg, 97%). ¹H NMR (400 MHz, DMSO-*d*₆) δ 6.59 (br s, 1H), 6.39 (br s, 1H), 6.36 (br s, 1H), 5.51 (br s, 2H), 1.20 (s, 18H). LCMS (ES⁺) *m*/*z* calculated for C₁₇H₂₃F₃N₂O₄ 376.16, found 277 (M+H-Boc)⁺. HPLC *t*_{R} 2.29 min.

### Step 3: tert-Butyl (tert-butoxycarbonyl)(3-isocyanato-5-(trifluoromethyl)phenyl)carbamate (Intermediate 5).

A mixture of *tert*-butyl (3-amino-5-(trifluoromethyl)phenyl)(*tert-*butoxycarbonyl)carbamate (120.0 mg, 0.32 mmol) and diphosgene (0.06 mL, 0.48 mmol) in dry dioxane (10 mL) was heated at 60 °C for 12 h. After cooling the solvent was removed under reduced pressure to get the title compound (128 mg, 99%) which was used as such in the next reactions.

### Intermediate 6: 2-(3-(Aminomethyl)benzyl)isoindoline-1,3-dione hydrochloride

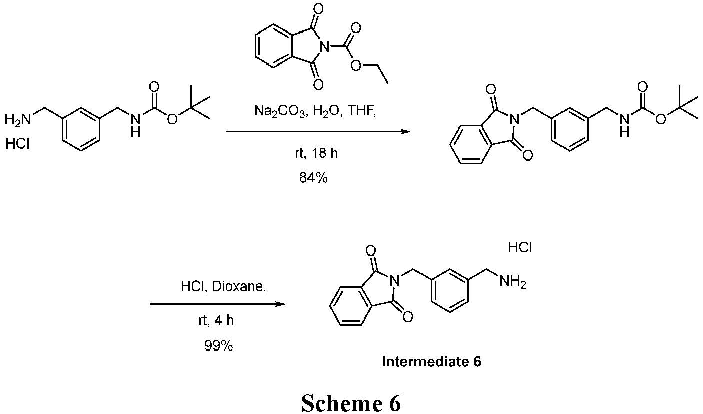

### Step 1: tert-Butyl (3-((1,3-dioxoisoindolin-2-yl)methyl)benzyl)carbamate.

A suspension of *tert*-butyl (3-(aminomethyl)benzyl)carbamate hydrochloride (300 mg, 1.10 mmol) in a mixture of H₂O (15 mL) and THF (10 mL) was treated with ethyl 1,3-dioxoisoindoline-2-carboxylate (289 mg, 1.32 mmol) and Na₂CO₃ (350 mg, 3.3 mmol) and vigorously stirred at rt for 18 h before being treated with H₂O (20 mL) and EtOAc. The organic phase was washed with brine, dried over Na₂SO₄, filtered, and concentrated under reduced pressure to get the title compound as a white solid (340 mg, 84%) which was used without further purification. LCMS (ES⁺) *m*/*z* calculated for C₂₁H₂₂N₂O₄ 366.16. found 367 (M+H)⁺. HPLC *t*_{R} 2.09 min.

### Step 2: 2-(3-(Aminomethyl)benzyl)isoindoline-1,3-dione hydrochloride (Intermediate 6).

A solution of *tert*-butyl (3-((1,3-dioxoisoindolin-2-yl)methyl)benzyl)carbamate (199 mg, 0.54 mmol) in dioxane (5.0 mL) was treated with HCl (4 N in dioxane, 5.0 mL) and stirred at rt for 4 h before being concentrated under reduced pressure to get the title compound (164 mg, 99%) which was used without further purification. ¹H NMR (400 MHz, DMSO-*d*₆) δ 8.19 (br s, 3H), 7.94-7.87 (m, 4H), 7.40-7.35 (m, 4H), 4.80 (s, 2H), 3.99 (s, 2H). LCMS (ES⁺) *m*/*z* calculated for C₁₆H₁₄N₂O₂ 266.11, found 267 (M+H)⁺. HPLC *t*_{R} 0.98 min.

### Intermediate 7: 2-(4-(Aminomethyl)benzyl)isoindoline-1,3-dione hydrochloride

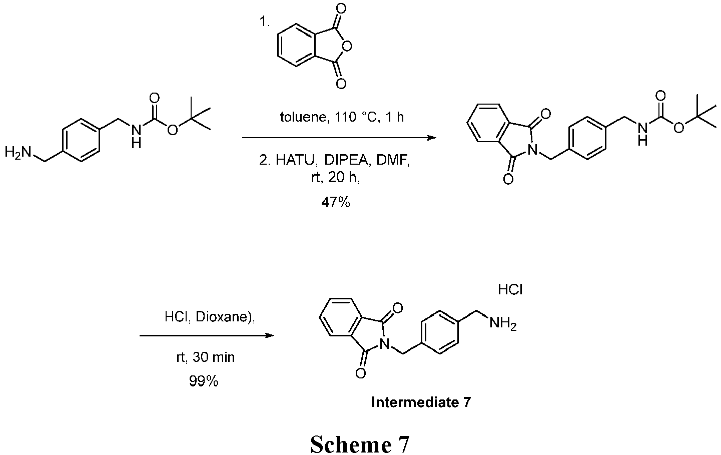

### Step 1: tert-Butyl (4-((1,3-dioxoisoindolin-2-yl)methyl)benzyl)carbamate.

A solution of *tert*-butyl (4-(aminomethyl)benzyl)carbamate (500 mg, 2.12 mmol) and isobenzofuran-1,3-dione (345 mg, 2.33 mmol) in toluene (10 mL) was heated at reflux using a dean stark apparatus for 1 h. After cooling, the excess of solvent was removed under reduced pressure to give a residue which was dissolved in DMF (6.0 mL) and treated with HATU (802 mg, 2.11 mmol) and DIPEA (1.13 mL, 6.33 mmol) and stirred at rt for 20 h before being diluted with H₂O and EtOAc. The organic phase was washed with brine, dried over Na₂SO₄, and evaporated under reduced pressure to get a residue which was purified by flash chromatography on silica gel (eluting with 0-100% EtOAc in petroleum ether) to get the title compound as a yellow solid (364 mg, 47%). ¹H NMR (400 MHz, CDCl₃) δ 7.87-7.84 (m, 2H), 7.74-7.72 (m, 2H), 7.42 (d, *J*=7.9 Hz, 2H), 7.28-7.24 (m, 3H), 4.85 (s, 2H), 4.29 (br d, *J*=5.0 Hz, 2H), 1.46 (s, 9H). LCMS (ES⁺) *m*/*z* calculated for C₂₁H₂₂N₂O₄ 366.16. HPLC *t*_{R} 1.91 min.

### Step 2: 2-(4-(Aminomethyl)benzyl)isoindoline-1,3-dione hydrochloride (Intermediate 7).

A solution of tert-butyl (4-((1,3-dioxoisoindolin-2-yl)methyl)benzyl)carbamate (364 mg, 0.99 mmol) in HCl (4 N in dioxane, 2.0 mL) was stirred at rt for 30 min.before being concentrated under reduced pressure to get the title compound as a yellow powder (300 mg, 99%) which was used without further purification. LCMS (ES⁺) *m*/*z* calculated for C₁₆H₁₄N₂O₂ 266.11, found 267 (M+H)⁺. HPLC *t*_{R} 0.89 min.

### Intermediate 8: (1-(2,2,2-Trifluoroethyl)piperidin-2-yl)methanamine hydrochloride

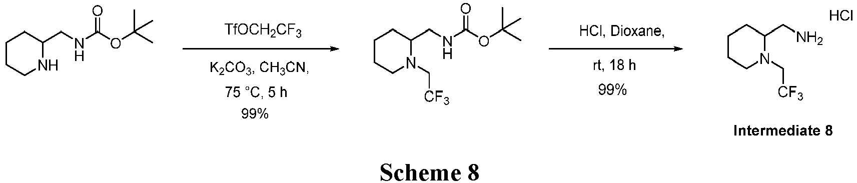

### Step 1: tert-Butyl ((1-(2,2,2-trifluoroethyl)piperidin-2-yl)methyl)carbamate.

A solution of *tert*-butyl (piperidin-2-ylmethyl)carbamate (100 mg, 0.47 mmol) in CH₃CN (2.3 mL) was treated with K₂CO₃ (194 mg, 1.4 mmol) and 2,2,2-trifluoroethyl trifluoromethanesulfonate (0.1 mL, 0.7 mmol) and heated at 75 °C for 5 h. After cooling, the reaction mixture was portioned between EtOAc and H₂O, phases were separated, and the aqueous phase was extracted with EtOAc. Combined organics were washed with brine, dried over Na₂SO₄, and evaporated under reduced pressure to get a residue which was purified by flash chromatography on silica gel (eluting with 0-50% EtOAc in petroleum ether) to get the title compound (138 mg, 99%). ¹H NMR (400 MHz, DMSO-*d*₆) δ 6.74 (br s, 1H), 3.40-3.10 (m, 3H), 3.09-2.82 (m, 2H), 2.55-2.48 (m, 2H), 1.60-1.55 (m, 2H), 1.48-1.40 (m, 2H), 1.38 (s, 9H), 1.36-1.25 (m, 2H). ¹⁹F NMR (377 MHz, DMSO-*d*₆) δ -69.47 (s, 3F). LCMS (ES⁺) *m*/*z* calculated for C₁₃H₂₃F₃N₂O₂ 296.17, found 297 (M+H)⁺. HPLC *t*_{R} 2.05 min.

### Step 2: (1-(2,2,2-Trifluoroethyl)piperidin-2-yl)methanamine hydrochloride (Intermediate 8).

A solution of *tert*-butyl ((1-(2,2,2-trifluoroethyl)piperidin-2-yl)methyl)carbamate (138 mg, 0.47 mmol) in HCl (4 N in dioxane, 1.5 mL) was stirred at rt for 18 h before being concentrated under reduced pressure to get the title compound as an off-white sticky powder (174.8 mg, 99%) which was used without further purification. ¹⁹F NMR (377 MHz, DMSO-*d*₆) δ -69.57 (s, 3F). LCMS (ES⁺) *m*/*z* calculated for C₈H₁₅F₃N₂ 196.12, found 197 (M+H)⁺.

### Intermediate 9: Methyl 3-isocyanato-5-(trifluoromethyl)benzoate

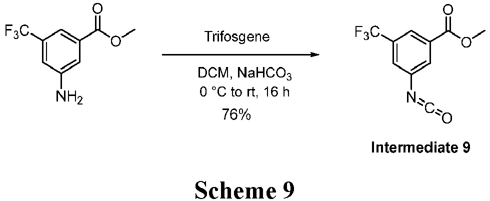

### Step 1: Methyl 3-isocyanato-5-(trifluoromethyl)benzoate (Intermediate 9).

Triphosgene (34 mg, 0.11 mmol) was dissolved in DCM (0.5 mL). In a separate flask, methyl 3-amino-5-(trifluoromethyl)benzoate (50 mg, 0.23 mmol) was dissolved in a 1:1 mixture of saturated aqueous sodium bicarbonate and DCM (2.0 mL) and the mixture was cooled to 0 °C. Stirring was stopped and the triphosgene solution was added directly to the organic phase of the biphasic reaction mixture. The reaction was then stirred at 0 °C for 30 min, warmed to rt and stirred for 16 h. The reaction mixture was transferred to a separatory funnel and extracted with DCM (3 x 20 mL). The organic phase was washed with brine, dried over Na₂SO₄, filtered and evaporated under reduced pressure to get the title compound (65 mg, 65% pure, 76%) as a light-yellow powder which was used without further purification.

### Example 1: (3-(4-(2-Methoxy-4-methylpyrimidin-5-yl)benzyl)-1,2,3-oxadiazol-3-ium-5-yl)((3-(2-phenylacetamido)-5-(trifluoromethyl)phenyl)carbamoyl)amide

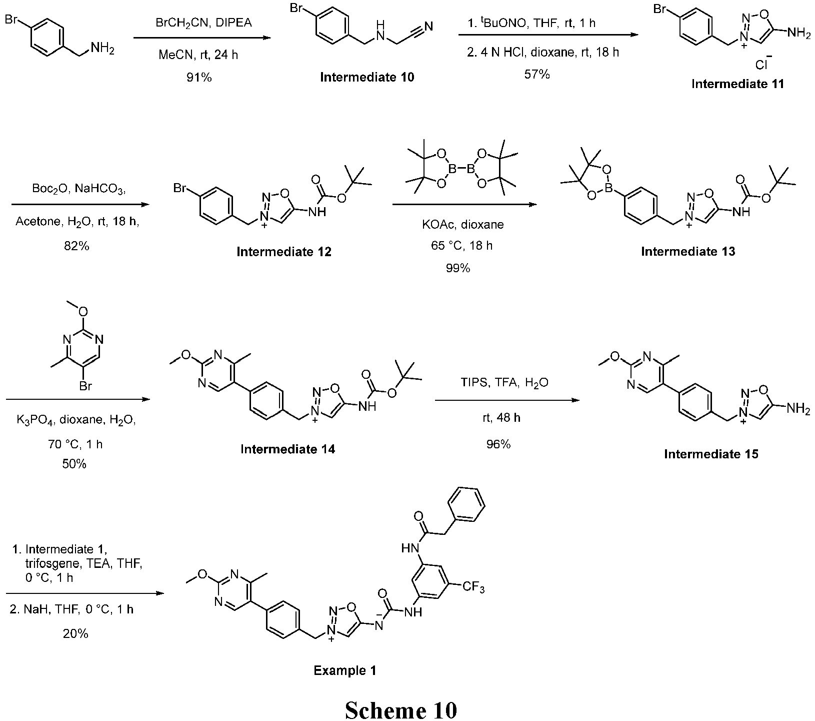

### Step 1: 2-((4-Bromobenzyl)amino)acetonitrile (Intermediate 10).

A suspension of 4-bromobenzylamine (10 g, 53.75 mmol) in CH₃CN (65 mL) was treated with DIPEA (19 mL, 107.5 mmol) and 2-bromoacetonitrile (3.74 mL, 53.75 mmol) and stirred at rt for 24 h. Solvent was removed under reduced pressure and the residue treated with H₂O and extracted with EtOAc. The organic phase was washed with brine, dried over Na₂SO₄, and evaporated under reduced pressure to get the title compound as a light orange oil (11.03 g, 91%). ¹H NMR (400 MHz, DMSO-*d*₆) δ 7.52 (d, *J* = 8.1 Hz, 2H), 7.29 (d, *J* = 8.1 Hz, 2H), 3.73 (br s, 2H), 3.59 (br s, 2H), 3.10 (br s, 1H). LCMS (ES⁺) *m*/*z* calculated for C₉H₉BrN₂ 223.99, found 225-227 (M+H)⁺. HPLC *t*_{R} 1.19 min.

### Step 2: 5-Amino-3-(4-bromobenzyl)-1,2,3-oxadiazol-3-ium chloride (Intermediate 11).

A solution of 2-((4-bromobenzyl)amino)acetonitrile (11 g, 48.87 mmol) in dry THF (25 mL) was treated with *tert*-butyl nitrite (17.5 mL, 146.61 mmol) and stirred at rt for 1 h before being treated with 4 N HCl in dioxane (36.23 mL, 305.43 mmol). The reaction mixture was stirred at rt for 18 h and then quenched with Et₂O. After removal the solvent the title product was obtained as a white powder (8.17 g, 57%). ¹H NMR (400 MHz, DMSO-*d*₆) δ 9.77 (s, 2H), 8.16 (s, 1H), 7.68 (d, *J* = 8.3 Hz, 2H), 7.55 (d, *J* = 8.3 Hz, 2H), 5.91 (s, 2H). LCMS (ES⁺) *m*/*z* calculated for C₉H₉BrN₃O 253.99, found 255-257 (M+H)⁺. HPLC *t*_{R} 0.92 min.

### Step 3: 3-(4-Bromobenzyl)-5-((tert-butoxycarbonyl)amino)-1,2,3-oxadiazol-3-ium (Intermediate 12).

A solution of 5-amino-3-(4-bromobenzyl)-1,2,3-oxadiazol-3-ium chloride (2.32 g, 7.96 mmol) in a mixture of H₂O (40 mL) and acetone (30 mL) was treated with Boc₂O (10.42 g, 47.74 mmol). NaHCO₃ (0.8 g, 9.55 mmol) in H₂O (15 mL) was slowly added to the reaction mixture and the solution was stirred at rt for 18 h before being extracted with EtOAc, washed with H₂O and brine, dried over Na₂SO₄, filtered, and concentrated under reduced pressure to get the title compound as a white powder (2.3 g, 82%). ¹H NMR (400 MHz, DMSO-*d*₆) δ 8.06 (s, 1H), 7.67 (br d, *J* = 8.6 Hz, 2H), 7.52 (br d, *J* = 8.3 Hz, 2H), 5.76 (s, 2H), 1.39 (s, 9H). LCMS (ES⁺) *m*/*z* calculated for C₁₄H₁₆BrN₃O₃ 353.04, found 354-356 (M+H)⁺. HPLC *t*_{R} 1.57 min.

### Step 4: (tert-Butoxycarbonyl)(3-(4-(4,4,5,5-tetramethyl-1,3,2-dioxaborolan-2-yl)benzyl)-1,2,3-oxadiazol-3-ium-5-yl)amide (Intermediate 13).

A solution of 3-(4-bromobenzyl)-5-((*tert*-butoxycarbonyl)amino)-1,2,3-oxadiazol-3-ium (1.7 g, 4.8 mmol), KOAc (1.41 g, 14.4 mmol), Pd(dppf)Cl₂ (356 mg, 0.48 mmol) and 4,4,5,5-tetramethyl-2-(4,4,5,5-tetramethyl-1,3,2-dioxaborolan-2-yl)-1,3,2-dioxaborolane (1.83 g, 7.2 mmol) in dioxane (30 mL) was heated at 65 °C for 18 h before being diluted with EtOAc. The organic phase was washed with H₂O, brine, dried over Na₂SO₄, filtered, and concentrated under reduced pressure to get the title compound (1.92 g, 99%) which was used without further purification. ¹H NMR (400 MHz, DMSO-*d*₆) δ 7.95 (s, 1H), 7.67 (d, *J* = 7.9 Hz, 2H), 7.47 (d, *J* = 7.9 Hz, 2H), 5.73 (s, 2H), 1.31 (s, 9H), 1.22 (s, 12H). LCMS (ES⁺) *m*/*z* calculated for C₂₀H₂₈BN₃O₅ 401.21, found 402 (M+H)⁺. HPLC *t*_{R} 1.81 min.

### Step 5: (tert-Butoxycarbonyl)(3-(4-(2-methoxy-4-methylpyrimidin-5-yl)benzyl)-1,2,3-oxadiazol-3-ium-5-yl)amide (Intermediate 14).

A solution of (*tert*-butoxycarbonyl)(3-(4-(4,4,5,5-tetramethyl-1,3,2-dioxaborolan-2-yl)benzyl)-1,2,3-oxadiazol-3-ium-5-yl)amide (1.7 g, 4.24 mmol), Pd(dppf)Cl₂ (315 mg, 0.42 mmol), 5-bromo-2-methoxy-4-methylpyrimidine (1.12 g, 5.51 mmol) and K₃PO₄ (2.70 g, 12.71 mmol) in a mixture of dioxane (40 mL) and H₂O (4 mL) was stirred at 70 °C for 1 h. After cooling the solution was diluted with EtOAc. The organic phase was washed with H₂O, brine, dried over Na₂SO₄, filtered, and concentrated under reduced pressure to get a residue which was purified by flash chromatography on silica gel (eluting with 10-100% EtOAc in petroleum ether) to get the title compound (835 mg, 50%). LCMS (ES⁺) *m*/*z* calculated for C₂₀H₂₃N₅O₄ 397.18, found 396 (M-H)⁻. HPLC *t*_{R} 1.41 min.

### Step 6: 5-Amino-3-(4-(2-methoxy-4-methylpyrimidin-5-yl)benzyl)-1,2,3-oxadiazol-3-ium 2,2,2-trifluoroacetate (Intermediate 15).

A solution of (*tert*-butoxycarbonyl)(3-(4-(2-methoxy-4-methylpyrimidin-5-yl)benzyl)-1,2,3-oxadiazol-3-ium-5-yl)amide (835.0 mg, 2.1 mmol) in a mixture of TFA/DCM/H₂O (1.0 mL/8.5 mL/0.5 mL) was treated with TIPS (400 mg, 2.1 mmol) and stirred at rt for 48 h. After removal of the solvent under reduced pressure the title compound was obtained (835 mg, 96%). ¹H NMR (400 MHz, DMSO-*d*₆) δ 9.34 (s, 2H), 8.25 (s, 1H), 7.93 (s, 1H), 7.52 (d, *J* = 8.1 Hz, 2H), 7.38 (d, *J* = 8.3 Hz, 2H), 5.79 (s, 2H), 3.79 (s, 3H), 2.23 (s, 3H). LCMS (ES⁺) *m*/*z* calculated for C₁₅H₁₆N₅O₂⁺ 298.13, found 298 (M+H)⁺. HPLC *t*_{R} 0.96 min.

### Step 7: (3-(4-(2-Methoxy-4-methylpyrimidin-5-yl)benzyl)-1,2,3-oxadiazol-3-ium-5-yl)((3-(2-phenylacetamido)-5-(trifluoromethyl)phenyl)carbamoyl)amide (Example 1).

A solution of *N*-(3-amino-5-(trifluoromethyl)phenyl)-2-phenylacetamide (Intermediate **1**; 53.52 mg, 0.18 mmol) in THF (3 mL) at 0°C was treated with TEA (0.05 mL, 0.36 mmol) and trifosgene (35.98 mg, 0.12 mmol) and stirred for 1 h. Diethyl ether (2 mL) was added and the mixture was stirred for 5 minutes before being filtered to afford a clear solution (A). In the meanwhile a solution of 5-amino-3-(4-(2-methoxy-4-methylpyrimidin-5-yl)benzyl)-1,2,3-oxadiazol-3-ium 2,2,2-trifluoroacetate (50.0 mg, 0.12 mmol) in THF (3.0 mL) at 0 °C was treated with NaH (60% in mineral oil; 5.82 mg, 0.24 mmol). Solution (A) was added dropwise, and the mixture stirred for 5 minutes before being diluted with EtOAc. The organic phase was washed with H₂O, brine, dried over Na₂SO₄, filtered, and concentrated under reduced pressure to get a residue which was purified by flash chromatography on silica gel (eluting with 1-6% MeOH in DCM) to get the title compound as a light orange solid (15 mg, 20%). ¹H NMR (400 MHz, DMSO-*d*₆) δ 10.34 (s, 1H), 9.67 (s, 1H), 8.35 (s, 1H), 8.14 (s, 1H), 7.91 (br s, 1H), 7.69 (br s, 1H), 7.62 (br d, *J* = 8.1 Hz, 2H), 7.46 (br d, *J* = 8.1 Hz, 2H), 7.26-7.23 (m, 5H), 7.20-7.16 (m, 1H), 5.78 (s, 2H), 3.87 (s, 3H), 3.57 (s, 2H), 2.32 (s, 3H). ¹⁹F NMR (377 MHz, DMSO-*d*₆) δ -61.59 (s, 3F). LCMS (ES⁺) *m*/*z* calculated for C₃₁H₂₆F₃N₇O₄ 617.20; found 616 (M-H)⁻. HPLC *t*_{R} 2.00 min.

### Example 3: ((3-Amino-5-(trifluoromethyl)phenyl)carbamoyl)(3-(4-(2-methoxy-4-methylpyrimidin-5-yl)benzyl)-1,2,3-oxadiazol-3-ium-5-yl)amide

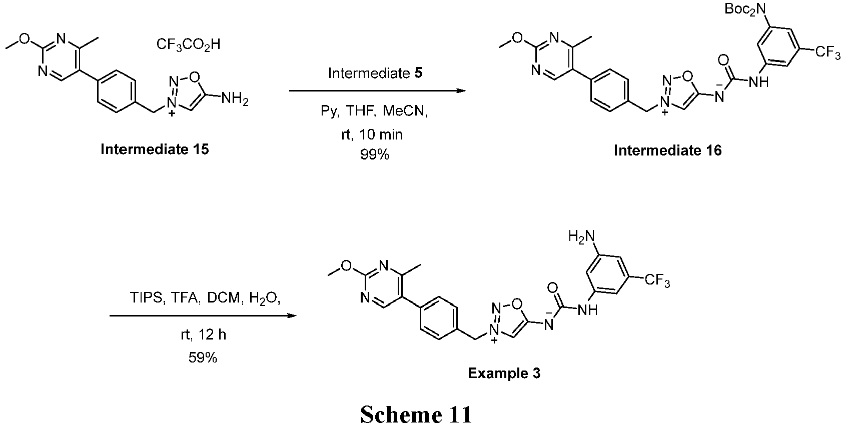

### Step 1: (3-(Bis(tert-butoxycarbonyl)amino)-5-(trifluoromethyl)phenyl)carbamoyl)(3-(4-(2-methoxy-4-methylpyrimidin-5-yl)benzyl)-1,2,3-oxadiazol-3-ium-5-yl)amide (Intermediate 16).

A solution of 5-amino-3-(4-(2-methoxy-4-methylpyrimidin-5-yl)benzyl)-1,2,3-oxadiazol-3-ium 2,2,2-trifluoroacetate (Intermediate **15**) (50 mg, 0.12 mmol) in MeCN (2 mL) was treated with a solution of *tert*-butyl (tert-butoxycarbonyl)(3-isocyanato-5-(trifluoromethyl)phenyl)carbamate (Intermediate **5**) (97 mg, 0.24 mmol) in THF (2 mL) followed by pyridine (0.04 mL, 0.49 mmol) and stirred at rt for 10 min.before being diluted with EtOAc and H₂O. The phases were separated, and the organic phase was washed with H₂O and brine, dried over Na₂SO₄, filtered, and concentrated under reduced pressure to get the title compound (84.8 mg, 99%) which was used in the next step without further purification. LCMS (ES⁺) *m*/*z* calculated for C₃₃H₃₆F₃N₇O₇ 699.26, found 698 (M-H)⁻. HPLC *t*_{R} 2.59 min.

### Step 2: ((3-Amino-5-(trifluoromethyl)phenyl)carbamoyl)(3-(4-(2-methoxy-4-methylpyrimidin-5-yl)benzyl)-1,2,3-oxadiazol-3-ium-5-yl)amide (Example 3).

A solution of ((3-(bis(*tert*-butoxycarbonyl)amino)-5-(trifluoromethyl)phenyl)carbamoyl)(3-(4-(2-methoxy-4-methylpyrimidin-5-yl)benzyl)-1,2,3-oxadiazol-3-ium-5-yl)amide (Intermediate **16**) (50 mg, 0.07 mmol) in a mixture of TFA/DCM/H₂O (1.0 mL/8.5 mL/0.5 mL) was treated with TIPS (catalytic amount) and stirred at rt for 12 h before being diluted with EtOAc and washed with NaHCO₃ (saturated aqueous solution), H₂O, and brine. After removal of the solvent under reduced pressure the title compound was obtained after purification by automated RP-HPLC using water (+ 0.1% TFA) and CH₃CN (+ 0.1% TFA) as eluents (C18 column). Fractions containing product were lyophilized to give the title compound as a yellow solid (25.9 mg, 59%). ¹H NMR (400 MHz, DMSO-*d*₆) δ 9.48 (s, 1H), 8.43 (s, 1H), 8.22 (s, 1H), 7.69 (br d, *J* = 8.3 Hz, 2H), 7.53 (br d, *J* = 8.3 Hz, 2H), 7.33 (br s, 1H), 7.09 (br s, 1H), 6.49 (br s, 1H), 5.85 (s, 2H), 3.94 (s, 3H), 2.39 (s, 3H). ¹⁹F NMR (377 MHz, DMSO-*d*₆) δ -61.60 (s, 3F). LCMS (ES⁺) *m*/*z* calculated for C₂₃H₂₀F₃N₇O₃ 499.16, found 498 (M-H)⁻. HPLC *t*_{R} 1.74 min.

### General procedures A and B for the synthesis of Examples 8-11, 15-22

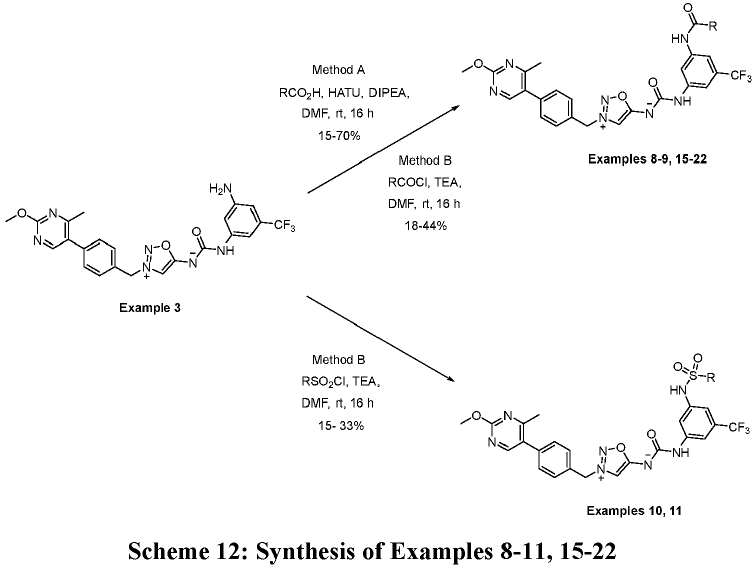

### General procedure A. Step 1.

A solution of ((3-amino-5-(trifluoromethyl)phenyl)carbamoyl)(3-(4-(2-methoxy-4-methylpyrimidin-5-yl)benzyl)-1,2,3-oxadiazol-3-ium-5-yl)amide (**Example 3**) (5.0 mg, 0.01 mmol, 1 eq.) and HATU (5.7 mg, 0.015 mmol, 1.5 eq.) in dry DMF (1.0 mL) was treated with DIPEA (0.01 mL, 0.03 mmol, 3 eq.) followed by the corresponding carboxylic acid (0.015 mmol, 1.5 eq.) and the reaction mixture was stirred at rt for 16 h. The title compound was obtained after purification by automated RP-HPLC using water (+ 0.1% TFA) and MeCN (+ 0.1% TFA) as eluents (C18 column). Fractions containing product were lyophilized to give the desired compound.

### General procedure B. Step 2.

A solution of ((3-amino-5-(trifluoromethyl)phenyl)carbamoyl)(3-(4-(2-methoxy-4-methylpyrimidin-5-yl)benzyl)-1,2,3-oxadiazol-3-ium-5-yl)amide (**Example 3**) (5.0 mg, 0.01 mmol, 1 eq.) and the corresponding acid chloride or sulphonyl chloride (0.02 mmol, 2 eq.) in dry THF (1.5 mL) was treated with TEA (0.004 mL, 0.03 mmol, 3 eq.) and the reaction mixture was stirred at rt for 16 h. The title compound was obtained after purification by automated RP-HPLC using water (+ 0.1% TFA) and CH₃CN (+ 0.1% TFA) as eluents (C18 column). Fractions containing product were lyophilized to give the desired compound.

The following examples were synthesized according to the above procedure (Scheme 12) by reaction using the appropriate starting materials.

### Example 8: (3-(2-Cyclohexylacetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide.

The title compound was obtained as a white solid by using 2-cyclohexylacetic acid (Method A). LCMS (ES⁺) *m*/*z* calculated for C₃₁H₃₂F₃N₇O₄ 623.25, found 624 (M+H)⁺. HPLC *t*_{R} 2.27 min.

### Example 9: (3-(4-(2-Methoxy-4-methylpyrimidin-5-yl)benzyl)-1,2,3-oxadiazol-3-ium-5-yl)((3-(3-phenylpropanamido)-5-(trifluoromethyl)phenyl)carbamoyl)amide.

The title compound was obtained as a white solid by using 3-phenylpropanoic acid (Method A). LCMS (ES⁺) *m*/*z* calculated for C₃₂H₂₈F₃N₇O₄ 631.22, found 632 (M+H)⁺. HPLC *t*_{R} 2.13 min.

### Example 10: (3-(4-(2-Methoxy-4-methylpyrimidin-5-yl)benzyl)-1,2,3-oxadiazol-3-ium-5-yl)((3-((phenylmethyl)sulfonamido)-5-(trifluoromethyl)phenyl)carbamoyl)amide.

The title compound was obtained as a white solid by using phenylmethanesulfonyl chloride (Method B). LCMS (ES⁺) *m*/*z* calculated for C₃₀H₂₆F₃N₇O₅S 653.17, found 652 (M-H)⁻. HPLC *t*_{R} 2.05 min.

### Example 11: (3-Acetamido-5-(trifluoromethyl)phenyl)carbamoyl)(3-(4-(2-methoxy-4-methylpyrimidin-5-yl)benzyl)-1,2,3-oxadiazol-3-ium-5-yl)amide.

The title compound was obtained as a white solid by using acetyl chloride (Method B). LCMS (ES⁺) *m*/*z* calculated for C₂₅H₂₂F₃N₇O₄ 541.17, found 542 (M+H)⁺. HPLC *t*_{R} 1.76 min.

### Example 15: (3-Benzamido-5-(trifluoromethyl)phenyl)carbamoyl)(3-(4-(2-methoxy-4-methylpyrimidin-5-yl)benzyl)-1,2,3-oxadiazol-3-ium-5-yl)amide.

The title compound was obtained as a white solid by using 3-phenylpropanoic acid (Method A). LCMS (ES⁺) *m*/*z* calculated for C₃₀H₂₄F₃N₇O₄ 603.18, found 604 (M+H)⁺. HPLC *t*_{R} 2.09 min.

### Example 16: (3-(4-(2-Methoxy-4-methylpyrimidin-5-yl)benzyl)-1,2,3-oxadiazol-3-ium-5-yl)((3-(2-(4-methoxyphenyl)acetamido)-5-(trifluoromethyl)phenyl)carbamoyl)amide.

The title compound was obtained by using 2-(4-methoxyphenyl)acetic acid (Method A). LCMS (ES⁺) *m*/*z* calculated for C₃₂H₂₈F₃N₇O₅ 647.21, found 648 (M+H)⁺. HPLC *t*_{R} 2.06 min.

### Example 17: (3-(2-(4-Chlorophenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide.

The title compound was obtained by using 2-(4-chlorophenyl)acetic acid (Method A). LCMS (ES⁺) *m*/*z* calculated for C₃₁H₂₅ClF₃N₇O₄ 651.16, found 652 (M+H)⁺. HPLC *t*_{R} 2.21 min.

### Example 18: (3-(Cyclopentanecarboxamido)-5-(trifluoromethyl)phenyl)carbamoyl)(3-(4-(2-methoxy-4-methylpyrimidin-5-yl)benzyl)-1,2,3-oxadiazol-3-ium-5-yl)amide.

The title compound was obtained by using cyclopentanecarboxylic acid (Method A). LCMS (ES⁺) *m*/*z* calculated for C₂₉H₂₈F₃N₇O₄ 595.22, found 596 (M+H)⁺. HPLC *t*_{R} 2.12 min.

### Example 19: (3-(4-(2-Methoxy-4-methylpyrimidin-5-yl)benzyl)-1,2,3-oxadiazol-3-ium-5-yl)((3-(2-(pyridin-3-yl)acetamido)-5-(trifluoromethyl)phenyl)carbamoyl)amide.

The title compound was obtained by using 2-(pyridin-3-yl)acetic acid (Method A). LCMS (ES⁺) *m*/*z* calculated for C₃₀H₂₅F₃N₈O₄ 618.20, found 617 (M-H)⁻. HPLC *t*_{R} 1.47 min.

### Example 20: (3-(2-(Benzo[d]isoxazol-3-yl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide.

The title compound was obtained by using 2-(benzo[d]isoxazol-3-yl)acetic acid (Method A). LCMS (ES⁺) *m*/*z* calculated for C₃₂H₂₅F₃N₈O₅ 658.19, found 659 (M+H)⁺. HPLC *t*_{R} 2.06 min.

### Example 21: ((3-(2-(2-Chlorophenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide.

The title compound was obtained by using 2-(2-chlorophenyl)acetic acid (Method A). LCMS (ES⁺) *m*/*z* calculated for C₃₁H₂₅ClF₃N₇O₄ 651.16, found 650 (M-H)⁻. HPLC *t*_{R} 2.13 min.

### Example 22: (3-(2-(4-(Aminomethyl)phenyl)acetamido)-5-(trifluoromethyl)phenyl)-carbamoyl)(3-(4-(2-methoxy-4-methylpyrimidin-5-yl)benzyl)-1,2,3-oxadiazol-3-ium-5-yl)amide.

The title compound was obtained as a white solid by using 2-[4-[(tert-butoxycarbonylamino)methyl]phenyl]acetic acid (Method A - crude material was treated with TFA in DCM before HPLC purification). LCMS (ES⁺) *m*/*z* calculated for C₃₂H₂₉F₃N₈O₄ 646.23, found 645 (M-H)⁻. HPLC *t*_{R} 1.49 min.

### Example 4: (3-Methyl-1,2,3-oxadiazol-3-ium-5-yl)((3-(2-phenylacetamido)-5-(trifluoromethyl)phenyl)carbamoyl)amide

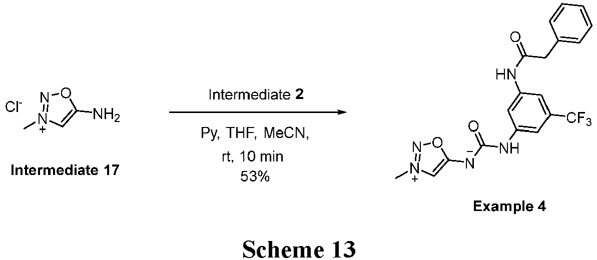

### Step 1: (3 Methyl-1,2,3-oxadiazol-3-ium-5-yl)((3-(2-phenylacetamido)-5-(trifluoromethyl)-phenyl)carbamoyl)amide (Example 4).

A solution of 5-amino-3-methyl-1,2,3-oxadiazol-3-ium chloride (Intermediate **17**) prepared as reported in the synthesis of **Example 1** (Scheme 10 steps 1 and 2) (30 mg, 0.22 mmol) and N-(3-Isocyanato-S-(trifluoromethyl)phenyl)-2-phenylacetamide (Intermediate **2**) (141 mg, 0.44 mmol) in THF (4 mL) was treated with pyridine (0.07 mL, 0.88 mmol) at 0 °C and stirred at rt for 1 h before being diluted with EtOAc. The organic phase was washed with NaHCO₃ (sat aqueous solution) and brine, dried over Na₂SO₄, filtered, and concentrated under reduced pressure. The title compound was obtained after purification by automated RP-HPLC using water (+ 0.1% TFA) and CH₃CN (+ 0.1% TFA) as eluents (C18 column). Fractions containing product were lyophilized to give the title compound as a white powder (49.0 mg, 53%). ¹H NMR (400 MHz, DMSO-*d*₆) δ 10.24 (s, 1H), 9.50 (s, 1H), 7.89 (s, 1H), 7.80 (s, 1H), 7.57 (s, 1H), 7.51 (s, 1H), 7.15-7.14 (m, 4H), 7.09-7.04 (m, 1H), 4.04 (s, 3H), 3.47 (s, 2H). ¹⁹F NMR (377 MHz, DMSO-*d*₆) δ - 61.57 (s, 3F). LCMS (ES⁺) *m*/*z* calculated for C₁₉H₁₆F₃N₅O₃ 419.12, found 420 (M+H)⁺. HPLC *t*_{R} 1.73 min.

### Example 12: (3-((5-Bromopyridin-2-yl)methyl)-1,2,3-oxadiazol-3-ium-5-yl)((3-(2-phenylacetamido)-5-(trifluoromethyl)phenyl)carbamoyl)amide

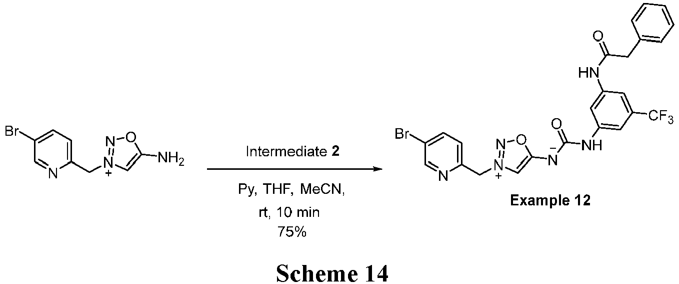

The above example was prepared following procedure reported for the synthesis of **Example 1** (Scheme 10, steps 1, 2) using (5-bromopyridin-2-yl)methanamine dihydrochloride in step 1 and followed by reaction with intermediate **2** (as reported for the synthesis of **Example 4**). The title compound was obtained as an orange solid (2.2 mg, 44%). ¹H NMR (400 MHz, DMSO-*d*₆) δ 10.42 (s, 1H), 9.75 (s, 1H), 8.76 (br d, *J* = 2.2 Hz, 1H), 8.21 (dd, *J₁* = 8.4 Hz, *J₂* = 2.3 Hz, 1H), 8.15 (s, 1H), 7.99 (br s, 1H), 7.75 (br s, 1H), 7.70 (br s, 1H), 7.65 (br d, *J* = 8.3 Hz, 1H), 7.33 (br d, *J* = 4.4 Hz, 4H), 7.27-7.24 (m, 1H), 5.94 (s, 2H), 3.65 (s, 2H). ¹⁹F NMR (377 MHz, DMSO-*d*₆) δ -61.58 (s, 3F). LCMS (ES⁺) *m*/*z* calculated for C₂₄H₁₈BrF₃N₆O₃ 574.06, found 575-577 (M+H)⁺. HPLC *t*_{R} 1.92 min.

### Example 13: (3-((5-(2-Methoxy-4-methylpyrimidin-5-yl)pyridin-2-yl)methyl)-1,2,3-oxadiazol-3-ium-5-yl)((3-(2-phenylacetamido)-5-(trifluoromethyl)phenyl)carbamoyl)amide.

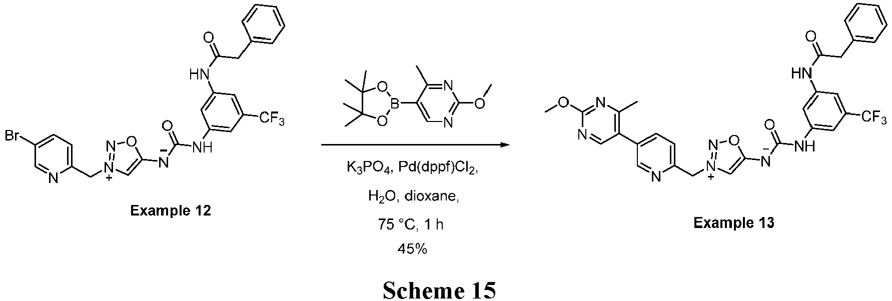

A solution of (3-((5-bromopyridin-2-yl)methyl)-1,2,3-oxadiazol-3-ium-5-yl)((3-(2-phenylacetamido)-5-(trifluoromethyl)phenyl)carbamoyl)amide **(Example 12**; 20.0 mg, 0.035 mmol, 1 eq), appropriate boronic ester (0.035 mmol, 1 eq), Pd(dppf)Cl₂ (2.87 mg, 0.0035 mmol, 0.1 eq) and K₃PO₄ (22.14 mg, 0.105 mmol, 3 eq) in a mixture of dioxane (1 mL) and H₂O (0.1 mL) was heated at 75 °C for 1 h before being cooled at rt and filtered on a pad of solca flock. After removal of solvent under reduced pressure the title compound was obtained after purification by automated RP-HPLC using water (+ 0.1% TFA) and CH₃CN (+ 0.1% TFA) as eluents (C18 column). Fractions containing product were lyophilized to give the desired compound. The title compound was obtained as a white solid by using 2-methoxy-4-methyl-5-(4,4,5,5-tetramethyl-1,3,2-dioxaborolan-2-yl)pyrimidine. ¹H NMR (400 MHz, DMSO-*d*₆) δ 10.42 (s, 1H), 9.76 (s, 1H), 8.68 (d, *J* = 1.8 Hz, 1H), 8.50 (s, 1H), 8.22 (s, 1H), 8.05 (dd, *J₁* = 8.0 Hz, *J₂* = 2.3 Hz, 1H), 8.00 (br s, 1H), 7.78 (d, *J* = 8.3 Hz, 1H), 7.75 (br s, 1H), 7.70 (br s, 1H), 7.33 (d, *J* = 4.4 Hz, 4H), 7.28-7.24 (m, 1H), 6.02 (s, 2H), 3.95 (s, 3H), 3.65 (s, 2H), 2.41 (s, 3H). ¹⁹F NMR (377 MHz, DMSO-*d*₆) δ -61.59 (s, 3F). LCMS (ES⁺) *m*/*z* calculated for C₃₀H₂₅F₃N₈O₄ 618.20, found 619 (M+H)⁺. HPLC *t*_{R} 1.77 min.

The following examples were synthesized according to the above procedure (Scheme 14) by reaction using the appropriate starting materials.

### Example 5: (3-((5-(2-(Aminomethyl)phenyl)pyridin-2-yl)methyl)-1,2,3-oxadiazol-3-ium-5-yl)((3-(2-phenylacetamido)-5-(trifluoromethyl)phenyl)carbamoyl)amide.

The title compound was obtained as a white solid by using tert-butyl (2-(4,4,5,5-tetramethyl-1,3,2-dioxaborolan-2-yl)benzyl)carbamate and treating the crude material with HCl (4.0 M in 1,4-dioxane) before HPLC purification. ¹H NMR (400 MHz, DMSO-*d*₆) δ 10.43 (s, 1H), 9.77 (s, 1H), 8.64 (s, 1H), 8.22 (s, 1H), 8.13 (br s, 3H), 8.02-7.95 (m, 2H), 7.81-7.79 (m, 2H), 7.67-7.65 (m, 2H), 7.59-7.49 (m, 2H), 7.40-7.38 (m, 1H), 7.33 (d, *J* = 4.4 Hz, 4H), 7.27-7.23 (m, 1H), 6.03 (s, 2H), 3.99-3.95 (m, 2H), 3.65 (s, 2H). ¹⁹F NMR (377 MHz, DMSO-*d*₆) δ -61.58 (s, 3F). LCMS (ES⁺) *m*/*z* calculated for C₃₁H₂₆F₃N₇O₃ 601.20, found 602 (M+H)⁺. HPLC *t*_{R} 1.39 min.

### Example 6: (3-((5-(1-Methyl-1H-imidazol-5-yl)pyridin-2-yl)methyl)-1,2,3-oxadiazol-3-ium-5-yl)((3-(2-phenylacetamido)-5-(trifluoromethyl)phenyl)carbamoyl)amide.

The title compound was obtained as a white solid by using 1-methyl-5-(4,4,5,5-tetramethyl-1,3,2-dioxaborolan-2-yl)-1*H*-imidazole. LCMS (ES⁺) *m*/*z* calculated for C₂₈H₂₃F₃N₈O₃ 576.18, found 577 (M+H)⁺. HPLC *t*_{R} 1.30 min.

### Example 14: (3-((5-(Isoindolin-4-yl)pyridin-2-yl)methyl)-1,2,3-oxadiazol-3-ium-5-yl)((3-(2-phenylacetamido)-5-(trifluoromethyl)phenyl)carbamoyl)amide.

The title compound was obtained as a white solid by using *tert*-butyl 4-(4,4,5,5-tetramethyl-1,3,2-dioxaborolan-2-yl)isoindoline-2-carboxylate and treating the crude material with HCl (4.0 M in 1,4-dioxane) before HPLC purification. ¹H NMR (400 MHz, DMSO-*d*₆) δ 10.42 (s, 1H), 9.76 (s, 1H), 9.45 (br s, 2H), 8.76 (s, 1H), 8.22 (s, 1H), 8.08 (br d, *J* = 8.3 Hz, 1H), 8.00 (s, 1H), 7.82 (s, 1H), 7.79 (br s, 1H), 7.67 (br s, 1H), 7.65-7.59 (m, 1H), 7.58-7.52 (m, 4H), 7.33 (d, *J* = 4.4 Hz, 4H), 7.28-7.24 (m, 1H), 6.03 (s, 2H), 4.68-4.64 (m, 2H), 4.62-4.58 (m, 2H), 3.65 (s, 2H). ¹⁹F NMR (377 MHz, DMSO-*d*₆) δ -61.59 (s, 3F). LCMS (ES⁺) *m*/*z* calculated for C₃₂H₂₆F₃N₇O₃ 613.20, found 614 (M+H)⁺. HPLC *t*_{R} 1.35 min.

### Example 25: (3-((5-(2-Amino-4-methylpyrimidin-5-yl)pyridin-2-yl)methyl)-1,2,3-oxadiazol-3-ium-5-yl)((3-(2-phenylacetamido)-5-(trifluoromethyl)phenyl)carbamoyl)amide.

The title compound was obtained as a yellow solid by using 4-methyl-5-(4,4,5,5-tetramethyl-1,3,2-dioxaborolan-2-yl)pyrimidin-2-amine. ¹H NMR (400 MHz, DMSO-*d*₆) δ 10.43 (s, 1H), 9.75 (s, 1H), 8.62 (s, 1H), 8.19 (s, 1H), 8.13 (s, 1H), 7.99 (s, 1H), 7.96 (br d, *J* = 8.1 Hz, 1H), 7.75-7.73 (m, 1H), 7.71 (br s, 1H), 7.60-7.48 (m, 2H), 7.33 (d, *J* = 4.4 Hz, 4H), 7.28-7.24 (m, 1H), 6.76 (s, 2H), 5.98 (s, 2H), 3.65 (s, 2H), 2.26 (s, 3H). ¹⁹F NMR (377 MHz, DMSO-*d*₆) δ -61.58 (s, 3F). LCMS (ES⁺) *m*/*z* calculated for C₂₉H₂₄F₃N₉O₃ 603.20, found 604 (M+H)⁺. HPLC *t*_{R} 1.57 min.

### Example 28: (3-Benzyl-1,2,3-oxadiazol-3-ium-5-yl)((3-(2-phenylacetamido)-5-(trifluoro-methyl)phenyl)carbamoyl)amide

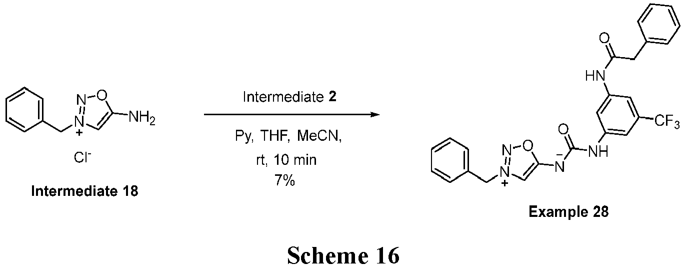

### Step 1: (3-Benzyl-1,2,3-oxadiazol-3-ium-5-yl)((3-(2-phenylacetamido)-5-(trifluoromethyl)-phenyl)carbamoyl)amide (Example 28).

### A solution of 5-amino-3-benzyl-1,2,3-oxadiazol-3-ium chloride (Intermediate 18) (prepared as reported in the synthesis of Example 1 (Scheme 10 steps 1 and 2) (50 mg, 0.28 mmol) and N-(3-Isocyanato-S-(trifluoromethyl)phenyl)-2-phenylacetamide (Intermediate 2) (136 mg, 0.43 mmol) in THF (10 mL) was treated with pyridine (0.02 mL, 0.28 mmol) at 0 °C and stirred at rt for 1 h before being diluted with EtOAc. The organic phase was washed with NaHCO₃ (sat aqueous solution) and brine, dried over Na₂SO₄, filtered, and concentrated under reduced pressure. The title compound was obtained after purification by automated RP-HPLC using water (+ 0.1% TFA) and CH₃CN (+ 0.1% TFA) as eluents (C18 column). Fractions containing product were lyophilized to give the title compound as a yellow powder (9.2 mg, 7%). ¹H NMR (400 MHz, DMSO-d₆) δ 11.25 (s, 1H), 1.51 (s, 1H), 10.32 (s, 1H), 7.97 (s, 1H), 7.78 (s, 1H), 7.74 (s, 1H), 7.59-7.57 (m. 1H), 7.52 (s, 1H), 7.49-7.46 (m, 2H), 7.36-7.31 (m, 6H), 7.30-7.24 (m, 4H), 4.96 (s, 2H), 3.66 (s, 2H). ¹⁹F NMR (377 MHz, DMSO-d₆) δ -61.77 (s, 3F). LCMS (ES⁺) m/z calculated for C₂₅H₂₀F₃N₅O₃ 495.15, found 496 (M+H)⁺. HPLC t_{R} 1.94 min.

### Example 7: ((3-(2-Phenylacetamido)-5-(trifluoromethyl)phenyl)carbamoyl)(3-(pyridin-2-ylmethyl)-1,2,3-oxadiazol-3-ium-5-yl)amide

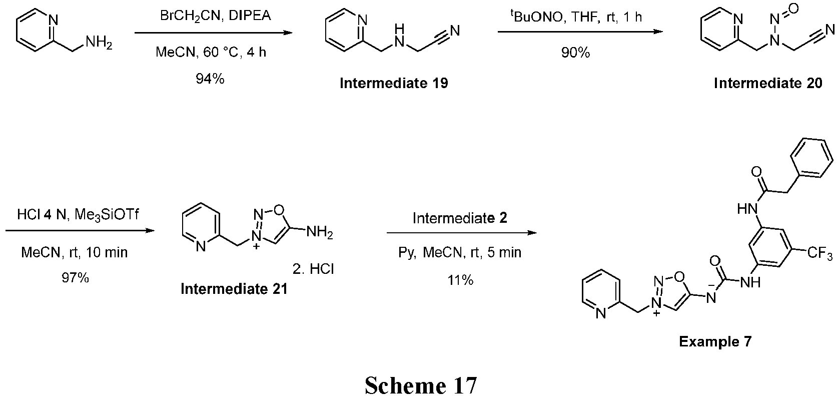

### Step 1: 2-((Pyridin-2-ylmethyl)amino)acetonitrile (Intermediate 19).

A suspension of pyridin-2-ylmethanamine dihydrochloride (500 mg, 4.62 mmol) in MeCN (5 mL) was treated with DIPEA (3.27 mL, 18.49 mmol) and 2-bromoacetonitrile (0.32 mL, 4.62 mmol) and stirred at 60 °C for 4 h. After cooling the reaction mixture was treated with H₂O and extracted with EtOAc. The organic phase was washed with brine, dried over Na₂SO₄, and evaporated under reduced pressure to get the title compound as a brown oil (750 mg, 85% pure, 94%) which was used without further purification. ¹H NMR (400 MHz, CDCl₃) δ 10.40 (br s, 1H), 8.53 (d, *J=* 4.4 Hz, 1H), 7.65 (br t, *J* = 7.7 Hz, 1H), 7.27 (d, *J* = 7.7 Hz, 1H), 7.19-7.16 (m, 1H), 4.00 (br s, 2H), 3.63 (br s, 2H).

### Step 2: N-(Cyanomethyl)-N-(pyridin-2-ylmethyl)nitrous amide (Intermediate 20).

A solution of 2-((pyridin-2-ylmethyl)amino)acetonitrile (750 mg, 4.23 mmol) in THF (5 mL) was treated with tert-butyl nitrite (1.51 mL, 12.69 mmol) and stirred at rt for 1 h before being treated with H₂O and extracted with EtOAc. The organic phase was washed with brine, dried over Na₂SO₄, and evaporated under reduced pressure to get the title compound as a brown oil (667 mg, 90%) which was used without further purification. ¹H NMR (400 MHz, CDCl₃) δ 8.54 (d, *J=* 4.4 Hz, 1H), 7.75-7.70 (m, 1H), 7.33 (d, *J* = 7.7 Hz, 1H), 7.28-7.23 (m, 1H), 5.54 (s, 2H), 4.47 (s, 2H). LCMS (ES⁺) *m*/*z* calculated for C₈H₈N₄O 176.07, found 177 (M+H)⁺. HPLC *t*_{R} 0.78 min.

### Step 3: 5-Amino-3-(pyridin-1-ium-2-ylmethyl)-1,2,3-oxadiazol-3-ium chloride (Intermediate 21).

A solution of *N*-(cyanomethyl)-*N*-(pyridin-2-ylmethyl)nitrous amide (667 mg, 3.79 mmol) in MeCN (7 mL) was treated with 4 N HCl in dioxane (1.89 mL, 7.57 mmol). The reaction mixture was stirred at rt for 5 min. and then treated with trimethylsilyl trifluoromethanesulfonate (2.06 mL, 11.36 mmol) and stirred at rt for further 5 min. before being treated with H₂O (20 mL). After removal of the solvent by lyophilization the title product was obtained as a yellow powder (2.0 g, 46% pure, 97%) and used without further purification. ¹H NMR (400 MHz, DMSO-*d*₆) δ 9.49 (s, 2H), 8.60 (d, *J* = 4.0 Hz, 1H), 8.07 (s, 1H), 7.95 (br t, *J=* 7.8 Hz, 1H), 7.67 (d, *J=* 7.9 Hz, 1H), 7.48 (dd, *J*₁ = 7.1 Hz, *J*₂ = 5.4 Hz, 1H), 6.05 (s, 2H).

### Step 4: (3-(2-Phenylacetamido)-5-(trifluoromethyl)phenyl)carbamoyl)(3-(pyridin-2-ylmethyl)-1,2,3-oxadiazol-3-ium-5-yl)amide (Example 7).

A solution of 5-amino-3-(pyridin-1-ium-2-ylmethyl)-1,2,3-oxadiazol-3-ium chloride (100 mg, 0.20 mmol) and *N*-(3-isocyanato-5-(trifluoromethyl)phenyl)-2-phenylacetamide (Intermediate **2**) (77 mg, 0.24 mmol) in MeCN (2 mL) was treated with pyridine (0.13 mL, 1.63 mmol) and stirred at rt for 5 min. before being diluted with DCM and H₂O. The phases were separated, and the organic phase was washed with H₂O and brine, dried over Na₂SO₄, filtered, and concentrated under reduced pressure. The title compound was obtained after purification by automated RP-HPLC using water (+ 0.1% TFA) and CH₃CN (+ 0.1% TFA) as eluents (C18 column). Fractions containing product were lyophilized to give the title compound as a white solid (11.0 mg, 11%). ¹H NMR (400 MHz, DMSO-*d*₆) δ 10.31 (s, 1H), 9.64 (s, 1H), 8.50 (br d, *J=* 4.0 Hz, 1H), 8.04 (s, 1H), 7.88 (s, 1H), 7.82 (br t, *J* = 7.8 Hz, 1H), 7.61 (br d, *J* = 10.7 Hz, 2H), 7.54 (br d, *J* = 7.9 Hz, 1H), 7.35 (dd, *J*₁ = 7.2 Hz, *J*₂ = 5.3 Hz, 1H), 7.22-7.21 (m, 4H), 7.17-7.11 (m, 1H), 5.84 (s, 2H), 3.54 (s, 2H). ¹⁹F NMR (377 MHz, DMSO-*d*₆) δ -61.58 (s, 3F). LCMS (ES⁺) *m*/*z* calculated for C₂₄H₁₉F₃N₆O₃ 496.15, found 497 (M+H)⁺. HPLC *t*_{R} 1.72 min.

### Synthesis of Examples 29-51, 54, 64-80, 82-86, 88.

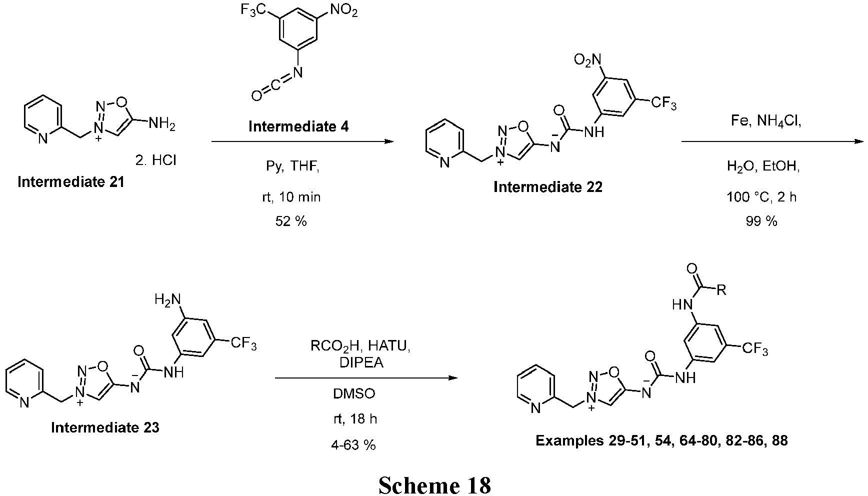

### Step 1: (3-Nitro-5-(trifluoromethyl)phenyl)carbamoyl)(3-(pyridin-2ylmethyl)-1,2,3-oxadiazol-3-ium-5-yl)amide (Intermediate 22).

A solution of 5-amino-3-(pyridin-1-ium-2-ylmethyl)-1,2,3-oxadiazol-3-ium chloride (Intermediate **21**, 800.0 mg, 3.74 mmol) in dry THF (20 mL) was treated with a solution of 1-isocyanato-3-nitro-5-(trifluoromethyl)benzene (Intermediate **4**; 1.3 g, 5.62 mmol) in THF (7.0 mL) followed by pyridine (0.91 mL, 11.23 mmol) and the reaction mixture was stirred at rt for 10 min.before being diluted with EtOAc. The organic phase was washed with H₂O and brine, dried over Na₂SO₄, filtered, and concentrated under reduced pressure. The title compound was obtained after purification by flash chromatography on silica gel (eluting with 30-100% EtOAc in petroleum ether) to get the title compound as a yellow solid (800 mg, 52%). ¹H NMR (400 MHz, DMSO-*d*₆) δ 10.21 (s, 1H), 8.84 (s, 1H), 8.61 (br d, *J* = 4.8 Hz, 1H), 8.41 (s, 1H), 8.30 (s, 1H), 7.97 (s, 1H), 7.94 (br t, *J* = 7.7 Hz, 1H), 7.66 (br d, *J* = 7.9 Hz, 1H), 7.47 (br t, *J* = 6.1 Hz, 1H), 5.97 (s, 2H). ¹⁹F NMR (377 MHz, DMSO-*d*₆) δ -61.74 (s, 3F). LCMS (ES⁺) *m*/*z* calculated for C₁₆H₁₁F₃N₆O₄ 408.08, found 409 (M+H)⁺. HPLC *t*_{R} 1.74 min.

### Step 2: (3-Amino-S-(trifluoromethyl)phenyl)carbamoyl)(3-(pyridin-2-ylmethyl)-1,2,3-oxadiazol-3-ium-5-yl)amide (Intermediate 23).

A solution of ((3-nitro-5-(trifluoromethyl)phenyl)carbamoyl)(3-(pyridin-2-ylmethyl)-1,2,3-oxadiazol-3-ium-5-yl)amide (Intermediate **22**, 500.0 mg, 1.22 mmol) and NH₄Cl (327.5 mg, 6.12 mmol) in a mixture of EtOH (15 mL) and H₂O (7 mL) was treated with iron (683.9 mg, 12.25 mmol) and heated at 100 °C for 2 h before being diluted with EtOAc, the organic phase was washed with H₂O, brine and dried under reduced pressure to get the title compound (463 mg, 99%) which was used without further purification. ¹H NMR (400 MHz, DMSO-*d*₆) δ 9.40 (s, 1H), 8.61 (br d, *J* = 4.2 Hz, 1H), 8.10 (s, 1H), 7.93 (br t, *J* = 7.7 Hz, 1H), 7.65 (br d, *J* = 7.7 Hz, 1H), 7.46 (dd, *J₁* = 7.5 Hz, *J₂* = 4.8 Hz, 1H), 7.23 (s, 1H), 7.03 (s, 1H), 6.41 (s, 1H), 5.92 (s, 2H), 5.42 (br s, 2H). ¹⁹F NMR (377 MHz, DMSO-*d*₆) δ -61.56 (s, 3F). LCMS (ES⁺) *m*/*z* calculated for C₁₆H₁₃F₃N₆O₂ 378.11, found 379 (M+H)⁺. HPLC *t*_{R} 1.30 min.

### Step 3: Coupling amide.

General procedure: A solution of ((3-amino-5-(trifluoromethyl)phenyl)carbamoyl)(3-(pyridin-2-ylmethyl)-1,2,3-oxadiazol-3-ium-5-yl)amide (20.0 mg, 0.05 mmol, 1 eq) and HATU (30.2 mg, 0.08 mmol, 1.5 eq) in dry DMSO (0.8 mL) was treated with DIPEA (20.5 mg, 0.16 mmol, 3 eq) followed by the appropriate carboxylic acid (0.06 mmol, 1.2 eq) and the reaction mixture was stirred at rt for 18 h. The title compound was obtained after purification by automated RP-HPLC using water (+ 0.1% TFA) and CH₃CN (+ 0.1% TFA) as eluents (C18 column). Fractions containing product were lyophilized to give the desired compound.

The following examples were synthesized according to the above procedure (Scheme 18) by reaction using the appropriate starting materials in Step 3.

### Example 29: ((3-(1H Benzo[d]imidazole-5-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 1*H*-benzo[d]imidazole-5-carboxylic acid. LCMS (ES⁺) *m*/*z* calculated for C₂₄H₁₇F₃N₈O₃ 522.14, found 523 (M+H)⁺. HPLC *t*_{R} 1.31 min.

### Example 30: (3-(5-(Morpholinomethyl)furan-2-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 5-(morpholinomethyl)furan-2-carboxylic acid. LCMS (ES⁺) *m*/*z* calculated for C₂₆H₂₄F₃N₇O₅ 571.18, found 572 (M+H)⁺. HPLC *t*_{R} 1.17 min.

### Example 31: (3-(3-Phenoxybenzamido)-5-(trifluoromethyl)phenyl)carbamoyl)(3-(pyridin-2-ylmethyl)-1,2,3-oxadiazol-3-ium-5-yl)amide.

The title compound was obtained by using 3-phenoxybenzoic acid. LCMS (ES⁺) *m*/*z* calculated for C₂₉H₂₁F₃N₆O₄ 574.16, found 575 (M+H)⁺. HPLC *t*_{R} 2.08 min.

### Example 32: (3-(2-Phenylpropanamido)-5-(trifluoromethyl)phenyl)carbamoyl)(3-(pyridin-2-ylmethyl)-1,2,3-oxadiazol-3-ium-5-yl)amide.

The title compound was obtained by using 2-phenylpropanoic acid. LCMS (ES⁺) *m*/*z* calculated for C₂₅H₂₁F₃N₆O₃ 510.16, found 511 (M+H)⁺. HPLC *t*_{R} 1.89 min.

### Example 33: (3-(2-(5-Methyl-2-phenyloxazol-4-yl)acetamido)-5-(trifluoromethyl)phenyl)-carbamoyl)(3-(pyridin-2-ylmethyl)-1,2,3-oxadiazol-3-ium-5-yl)amide.

The title compound was obtained by using 2-(5-methyl-2-phenyloxazol-4-yl)acetic acid. LCMS (ES⁺) *m*/*z* calculated for C₂₈H₂₂F₃N₇O₄ 577.17, found 578 (M+H)⁺. HPLC *t*_{R} 1.91 min.

### Example 34: (3-(1-Phenyl-1H-pyrazole-5-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 1-phenyl-1*H-*pyrazole-5-carboxylic acid. LCMS (ES⁺) *m*/*z* calculated for C₂₆H₁₉F₃N₈O₃ 548.15, found 549 (M+H)⁺. HPLC *t*_{R} 1.74 min.

### Example 35: (3-(1-Benzyl-1H-pyrazole-4-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 1-benzyl-1*H*-pyrazole-4-carboxylic acid. LCMS (ES⁺) *m*/*z* calculated for C₂₇H₂₁F₃N₈O₃ 562.17, found 563 (M+H)⁺. HPLC *t*_{R} 1.74 min.

### Example 36: (3-(2-(1H-pyrrolo[3,2-b]pyridin-3-yl)acetamido)-5-(trifluoromethyl)phenyl)-carbamoyl)(3-(pyridin-2-ylmethyl)-1,2,3-oxadiazol-3-ium-5-yl)amide.

The title compound was obtained by using 2-(1*H*-pyrrolo[3,2-b]pyridin-3-yl)acetic acid. LCMS (ES⁺) *m*/*z* calculated for C₂₅H₁₉F₃N₈O₃ 536.15, found 537 (M+H)⁺. HPLC *t*_{R} 1.13 min.

### Example 37: (3-(6-Cyanoimidazo[1,2-a]pyridine-2-carboxamido)-5-(trifluoromethyl)-phenyl)carbamoyl)(3-(pyridin-2-ylmethyl)-1,2,3-oxadiazol-3-ium-5-yl)amide.

The title compound was obtained by using 6-cyanoimidazo[1,2-a]pyridine-2-carboxylic acid. LCMS (ES⁺) *m*/*z* calculated for C₂₅H₁₆F₃N₉O₃ 547.13, found 548 (M+H)⁺. HPLC *t*_{R} 1.61 min.

### Example 38: (3-(Pyridin-2-ylmethyl)-1,2,3-oxadiazol-3-ium-5-yl)((3-(4,5,6,7-tetrahydro-1H-indazole-7-carboxamido)-5-(trifluoromethyl)phenyl)carbamoyl)amide.

The title compound was obtained by using 4,5,6,7-tetrahydro-1*H*-indazole-7-carboxylic acid. LCMS (ES⁺) *m*/*z* calculated for C₂₄H₂₁F₃N₈O₃ 526.17, found 527 (M+H)⁺. HPLC *t*_{R} 1.54 min.

### Example 39: (3-(5-Methyl-5,6-dihydro-4H-thieno[2,3-c]pyrrole-2-carboxamido)-5-(trifluoromethyl)phenyl)carbamoyl)(3-(pyridin-2-ylmethyl)-1,2,3-oxadiazol-3-ium-5-yl)amide.

The title compound was obtained by using 5-methyl-5,6-dihydro-4*H*-thieno[2,3-c]pyrrole-2-carboxylic acid. LCMS (ES⁺) *m*/*z* calculated for C₂₄H₂₀F₃N₇O₃S 543.13, found 544 (M+H)⁺. HPLC *t*_{R} 0.89 min.

### Example 40: (3-(2-(1-Methyl-1H-pyrazol-3-yl)acetamido)-5-(trifluoromethyl)phenyl)-carbamoyl)(3-(pyridin-2-ylmethyl)-1,2,3-oxadiazol-3-ium-5-yl)amide.

The title compound was obtained by using 2-(1-methyl-1*H*-pyrazol-3-yl)acetic acid. LCMS (ES⁺) *m*/*z* calculated for C₂₂H₁₉F₃N₈O₃ 500.15, found 501 (M+H)⁺. HPLC *t*_{R} 1.43 min.

### Example 41: ((3-(2-(2-Methylthiazol-4-yl)acetamido)-5-(trifluoromethyl)phenyl)-carbamoyl)(3-(pyridin-2-ylmethyl)-1,2,3-oxadiazol-3-ium-5-yl)amide.

The title compound was obtained by using 2-(2-methylthiazol-4-yl)acetic acid. LCMS (ES⁺) *m*/*z* calculated for C₂₂H₁₈F₃N₇O₃S 517.11, found 518 (M+H)⁺. HPLC *t*_{R} 1.52 min.

### Example 42: (3-(2-(6-Aminopyridin-3-yl)acetamido)-5-(trifluoromethyl)phenyl)-carbamoyl)(3-(pyridin-2-ylmethyl)-1,2,3-oxadiazol-3-ium-5-yl)amide.

The title compound was obtained by using 2-(6-aminopyridin-3-yl)acetic acid. LCMS (ES⁺) *m*/*z* calculated for C₂₃H₁₉F₃N₈O₃ 512.15, found 513 (M+H)⁺. HPLC *t*_{R} 1.11 min.

### Example 43: (3-(2-(Phenylsulfonyl)acetamido)-5-(trifluoromethyl)phenyl)carbamoyl)(3-(pyridin-2-ylmethyl)-1,2,3-oxadiazol-3-ium-5-yl)amide.

The title compound was obtained by using 2-(phenylsulfonyl)acetic acid. LCMS (ES⁺) *m*/*z* calculated for C₂₄H₁₉F₃N₆O₅S 560.11, found 561 (M+H)⁺. HPLC *t*_{R} 1.61 **min.Example 44: (3-(Isoxazole-3-carboxamido)-5-(trifluoromethyl)phenyl)carbamoyl)(3-(pyridin-2-ylmethyl)-1,2,3-oxadiazol-3-ium-5-yl)amide.**

The title compound was obtained by using isoxazole-3-carboxylic acid. LCMS (ES⁺) *m*/*z* calculated for C₂₀H₁₄F₃N₇O₄ 473.11, found 474 (M+H)⁺. HPLC *t*_{R} 1.57 min.

### Example 45: (3-(Pyrazine-2-carboxamido)-5-(trifluoromethyl)phenyl)carbamoyl)(3-(pyridin-2-ylmethyl)-1,2,3-oxadiazol-3-ium-5-yl)amide.

The title compound was obtained by using pyrazine-2-carboxylic acid. LCMS (ES⁺) *m*/*z* calculated for C₂₁H₁₅F₃N₈O₃ 484.12, found 485 (M+H)⁺. HPLC *t*_{R} 1.53 min.

### Example 46: (3-(3-Phenylpropiolamido)-5-(trifluoromethyl)phenyl)carbamoyl)(3-(pyridin-2-ylmethyl)-1,2,3-oxadiazol-3-ium-5-yl)amide.

The title compound was obtained by using 3-phenylpropiolic acid. LCMS (ES⁺) *m*/*z* calculated for C₂₅H₁₇F₃N₆O₃ 506.13, found 507 (M+H)⁺. HPLC *t*_{R} 1.88 min.

### Example 47: (3-(Bicyclo[4.2.0]octa-1(6),2,4-triene-7-carboxamido)-5-(trifluoromethyl)-phenyl)carbamoyl)(3-(pyridin-2-ylmethyl)-1,2,3-oxadiazol-3-ium-5-yl)amide.

The title compound was obtained by using bicyclo[4.2.0]octa-1(6),2,4-triene-7-carboxylic acid. LCMS (ES⁺) *m*/*z* calculated for C₂₅H₁₉F₃N₆O₃ 508.15, found 509 (M+H)⁺. HPLC *t*_{R} 1.82 min.

### Example 48: (3-(1-Isopropyl-1H-pyrazole-3-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 1-isopropyl-1*H-*pyrazole-3-carboxylic acid. LCMS (ES⁺) *m*/*z* calculated for C₂₃H₂₁F₃N₈O₃ 514.17, found 515 (M+H)⁺. HPLC *t*_{R} 1.59 min.

### Example 49: (3-(1-Methylazetidine-3-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 1-methylazetidine-3-carboxylic acid. LCMS (ES⁺) *m*/*z* calculated for C₂₁H₂₀F₃N₇O₃ 475.16, found 476 (M+H)⁺. HPLC *t*_{R} 1.07 min.

### Example 50: (3-(2-Phenylcyclopropane-1-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 2-phenylcyclopropane-1-carboxylic acid. LCMS (ES⁺) *m*/*z* calculated for C₂₆H₂₁F₃N₆O₃ 522.16, found 523 (M+H)⁺. HPLC *t*_{R} 1.93 min.

### Example 51: (3-(2-(Bicyclo[2.1.1]hexan-2-yl)acetamido)-5-(trifluoromethyl)phenyl)-carbamoyl)(3-(pyridin-2-ylmethyl)-1,2,3-oxadiazol-3-ium-5-yl)amide.

The title compound was obtained by using 2-(bicyclo[2.1.1]hexan-2-yl)acetic acid. LCMS (ES⁺) *m*/*z* calculated for C₂₄H₂₃F₃N₆O₃ 500.18, found 501 (M+H)⁺. HPLC *t*_{R} 1.93 min.**Example 54: (3-(2-Cyclobutylacetamido)-5-(trifluoromethyl)phenyl)carbamoyl)(3-(pyridin-2-ylmethyl)-1,2,3-oxadiazol-3-ium-5-yl)amide.**

The title compound was obtained as a beige solid by using 2-cyclobutylacetic acid. ¹H NMR (400 MHz, DMSO-*d*₆) δ 10.09 (s, 1H), 9.72 (s, 1H), 8.61 (br d, *J* = 3.7 Hz, 1H), 8.13 (s, 1H), 7.95 (br s, 2H), 7.72 (br d, *J* = 10.9 Hz, 2H), 7.66 (br d, *J* = 7.9 Hz, 1H), 7.48-7.45 (m, 1H), 5.95 (s, 2H), 2.71-2.63 (m, 1H), 2.43 (br d, *J* = 7.9 Hz, 2H), 2.10-2.01 (m, 2H), 1.87-1.79 (m, 2H), 1.76-1.69 (m, 2H). ¹⁹F NMR (377 MHz, DMSO-*d*₆) δ -61.53 (s, 3F). LCMS (ES⁺) *m*/*z* calculated for C₂₂H₂₁F₃N₆O₃ 474.16, found 475 (M+H)⁺. HPLC *t*_{R} 1.78 min.

### Example 64: (3-(5,5-Dioxido-6,7-dihydro-4H-pyrazolo[5,1-c][1,4]thiazine-2-carboxamido)-5-(trifluoromethyl)phenyl)carbamoyl)(3-(pyridin-2-ylmethyl)-1,2,3-oxadiazol-3-ium-5-yl)amide.

The title compound was obtained by using 6,7-dihydro-4*H*-pyrazolo[5,1-c][1,4]thiazine-2-carboxylic acid 5,5-dioxide. LCMS (ES⁺) *m*/*z* calculated for C₂₃H₁₉F₃N₈O₅S 576.12, found 577 (M+H)⁺. HPLC *t*_{R} 1.46 min.

### Example 65: (3-(2-(2-(Difluoromethyl)-1H-benzo[d]imidazol-1-yl)acetamido)-5-(trifluoromethyl)phenyl)carbamoyl)(3-(pyridin-2-ylmethyl)-1,2,3-oxadiazol-3-ium-5-yl)amide.

The title compound was obtained by using 2-(2-(difluoromethyl)-1*H*-benzo[d]imidazol-1-yl)acetic acid. LCMS (ES⁺) *m*/*z* calculated for C₂₆H₁₉F₅N₈O₃ 586.15, found 587 (M+H)⁺. HPLC *t*_{R} 1.67 min.

### Example 66: (3-(4-Hydroxybicyclo[2.2.1]heptane-1-carboxamido)-5-(trifluoromethyl)-phenyl)carbamoyl)(3-(pyridin-2-ylmethyl)-1,2,3-oxadiazol-3-ium-5-yl)amide.

The title compound was obtained by using 4-hydroxybicyclo[2.2.1]heptane-1-carboxylic acid. LCMS (ES⁺) *m*/*z* calculated for C₂₄H₂₃F₃N₆O₄ 516.17, found 517 (M+H)⁺. HPLC *t*_{R} 1.40 min.

### Example 67: (3-(7-Methyl-2,3-dihydrobenzofuran-3-carboxamido)-5-(trifluoromethyl)-phenyl)carbamoyl)(3-(pyridin-2-ylmethyl)-1,2,3-oxadiazol-3-ium-5-yl)amide.

The title compound was obtained by using 7-methyl-2,3-dihydrobenzofuran-3-carboxylic acid. LCMS (ES⁺) *m*/*z* calculated for C₂₆H₂₁F₃N₆O₄ 538.16, found 539 (M+H)⁺. HPLC *t*_{R} 1.89 min.

### Example 68: (3-(3-Benzylcyclobutane-1-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 3-benzylcyclobutane-1-carboxylic acid. LCMS (ES⁺) *m*/*z* calculated for C₂₈H₂₅F₃N₆O₃ 550.19, found 551 (M+H)⁺. HPLC *t*_{R} 2.08 min.**Example 69: (3-((1*S*,3*S*)-3-Hydroxy-3-(pyridin-2-yl)cyclobutane-1-carboxamido)-5-(trifluoromethyl)phenyl)carbamoyl)(3-(pyridin-2-ylmethyl)-1,2,3-oxadiazol-3-ium-5-yl)amide.**

The title compound was obtained by using (1*S*,3*S*)-3-hydroxy-3-(pyridin-2-yl)cyclobutane-1-carboxylic acid. LCMS (ES⁺) *m*/*z* calculated for C₂₆H₂₂F₃N₇O₄ 553.17, found 554 (M+H)⁺. HPLC *t*_{R} 1.18 min.

### Example 70: (3-(1-((5-Methylfuran-2-yl)methyl)piperidine-4-carboxamido)-5-(trifluoromethyl)phenyl)carbamoyl)(3-(pyridin-2-ylmethyl)-1,2,3-oxadiazol-3-ium-5-yl)amide.

The title compound was obtained by using 1-((5-methylfuran-2-yl)methyl)piperidine-4-carboxylic acid. LCMS (ES⁺) *m*/*z* calculated for C₂₈H₂₈F₃N₇O₄ 583.22, found 584 (M+H)⁺. HPLC *t*_{R} 1.23 min.

### Example 71: (3-(1-(3-Cyano-1H-pyrazol-1-yl)cyclopropane-1-carboxamido)-5-(trifluoromethyl)phenyl)carbamoyl)(3-(pyridin-2-ylmethyl)-1,2,3-oxadiazol-3-ium-5-yl)amide.

The title compound was obtained by using 1-(3-cyano-1*H*-pyrazol-1-yl)cyclopropane-1-carboxylic acid. LCMS (ES⁺) *m*/*z* calculated for C₂₄H₁₈F₃N₉O₃ 537.15, found 538 (M+H)⁺. HPLC *t*_{R} 1.71 min.

### Example 72: (3-(2-((4-Methyl-4H-1,2,4-triazol-3-yl)methoxy)acetamido)-5-(trifluoromethyl)phenyl)carbamoyl)(3-(pyridin-2-ylmethyl)-1,2,3-oxadiazol-3-ium-5-yl)amide.

The title compound was obtained by using 2-((4-methyl-4*H*-1,2,4-triazol-3-yl)methoxy)acetic acid. LCMS (ES⁺) *m*/*z* calculated for C₂₂H₂₀F₃N₉O₄ 531.16, found 532 (M+H)⁺. HPLC *t*_{R} 1.23 min.

### Example 73: (3-(3-(Cyclopentyloxy)propanamido)-5-(trifluoromethyl)phenyl)-carbamoyl)(3-(pyridin-2-ylmethyl)-1,2,3-oxadiazol-3-ium-5-yl)amide.

The title compound was obtained by using 3-(cyclopentyloxy)propanoic acid. LCMS (ES⁺) *m*/*z* calculated for C₂₄H₂₅F₃N₆O₄ 518.19, found 519 (M+H)⁺. HPLC *t*_{R} 1.83 min.

### Example 74: (3-((1R,4S,5S)-4-(3-methoxyphenyl)-2-oxabicyclo[2.1.1]hexane-5-carboxamido)-5-(trifluoromethyl)phenyl)carbamoyl)(3-(pyridin-2-ylmethyl)-1,2,3-oxadiazol-3-ium-5-yl)amide.

The title compound was obtained by using (1*R*,4*S*,5*S*)-4-(3-methoxyphenyl)-2-oxabicyclo[2.1.1]hexane-5-carboxylic acid. LCMS (ES⁺) *m*/*z* calculated for C₂₉H₂₅F₃N₆O₅ 594.18, found 595 (M+H)⁺. HPLC *t*_{R} 1.86 min. **Example 75: (3-(1-Cyclopropyl-1*H*-imidazole-5-carboxamido)-5-(trifluoromethyl)phenyl)-carbamoyl)(3-(pyridin-2-ylmethyl)-1,2,3-oxadiazol-3-ium-5-yl)amide.**

The title compound was obtained by using 1-cyclopropyl-1*H-*imidazole-5-carboxylic acid. LCMS (ES⁺) *m*/*z* calculated for C₂₃H₁₉F₃N₈O₃ 512.15, found 511 (M-H)⁻. HPLC *t*_{R} 1.23 min.

### Example 76: (3-(2,2-Difluoro-3-(pyrrolidin-1-yl)propanamido)-5-(trifluoromethyl)phenyl)-carbamoyl)(3-(pyridin-2-ylmethyl)-1,2,3-oxadiazol-3-ium-5-yl)amide.

The title compound was obtained by using 2,2-difluoro-3-(pyrrolidin-1-yl)propanoic acid. LCMS (ES⁺) *m*/*z* calculated for C₂₃H₂₂F₅N₇O₃ 539.17, found 540 (M+H)⁺. HPLC *t*_{R} 1.15 min.

### Example 77: (3-(1-Cyclopropylazetidine-3-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 1-cyclopropylazetidine-3-carboxylic acid. LCMS (ES⁺) *m*/*z* calculated for C₂₃H₂₂F₃N₇O₃ 501.17, found 502 (M+H)⁺. HPLC *t*_{R} 1.09 min.

### Example 78: (3-(Pyridin-2-ylmethyl)-1,2,3-oxadiazol-3-ium-5-yl)((3-(4-(1-sulfamoylethyl)-benzamido)-5-(trifluoromethyl)phenyl)carbamoyl)amide.

The title compound was obtained by using 4-(1-sulfamoylethyl)benzoic acid. LCMS (ES⁺) *m*/*z* calculated for C₂₅H₂₂F₃N₇O₅S 589.14, found 590 (M+H)⁺. HPLC *t*_{R} 1.51 min.

### Example 79: (3-(2,3-Dihydrofuro[3,2-b]pyridine-5-carboxamido)-5-(trifluoromethyl)-phenyl)carbamoyl)(3-(pyridin-2-ylmethyl)-1,2,3-oxadiazol-3-ium-5-yl)amide.

The title compound was obtained by using 2,3-dihydrofuro[3,2-b]pyridine-5-carboxylic acid. LCMS (ES⁺) *m*/*z* calculated for C₂₄H₁₈F₃N₇O₄ 525.14, found 526 (M+H)⁺. HPLC *t*_{R} 1.77 min.

### Example 80: (3-(Pyridin-2-ylmethyl)-1,2,3-oxadiazol-3-ium-5-yl)((3-(5,6,7,8-tetrahydroimidazo [1,5-a] pyridine-7-carboxamido)-5-(trifluoromethyl)phenyl)carbamoyl)amide.

The title compound was obtained by using 5,6,7,8-tetrahydroimidazo[1,5-a]pyridine-7-carboxylic acid. LCMS (ES⁺) *m*/*z* calculated for C₂₄H₂₁F₃N₈O₃ 526.17, found 527 (M+H)⁺. HPLC *t*_{R} 1.10 min.

### Example 82: (3-(5-(Piperidin-1-ylsulfonyl)pentanamido)-5-(trifluoromethyl)phenyl)-carbamoyl)(3-(pyridin-2-ylmethyl)-1,2,3-oxadiazol-3-ium-5-yl)amide.

The title compound was obtained by using 5-(piperidin-1-ylsulfonyl)pentanoic acid. LCMS (ES⁺) *m*/*z* calculated for C₂₆H₃₀F₃N₇O₅S 609.20, found 610 (M+H)⁺. HPLC *t*_{R} 1.73 min.

### Example 83: (3-(3-(4-Chlorophenoxy)oxetane-3-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 3-(4-chlorophenoxy)oxetane-3-carboxylic acid. LCMS (ES⁺) *m*/*z* calculated for C₂₆H₂₀ClF₃N₆O₅ 588.11, found 589 (M+H)⁺. HPLC *t*_{R} 1.94 min.**Example 84: (3-(2,6-Dioxaspiro[4.5]decane-7-carboxamido)-5-(trifluoromethyl)phenyl)-carbamoyl)(3-(pyridin-2-ylmethyl)-1,2,3-oxadiazol-3-ium-5-yl)amide.**

The title compound was obtained by using 2,6-dioxaspiro[4.5]decane-7-carboxylic acid. LCMS (ES⁺) *m*/*z* calculated for C₂₅H₂₅F₃N₆O₅ 546.18, found 547 (M+H)⁺. HPLC *t*_{R} 1.68 min.

### Example 85: (3-(3,3-Difluorospiro[3.3]heptane-1-carboxamido)-5-(trifluoromethyl)-phenyl)carbamoyl)(3-(pyridin-2-ylmethyl)-1,2,3-oxadiazol-3-ium-5-yl)amide.

The title compound was obtained by using 3,3-difluorospiro[3.3]heptane-1-carboxylic acid. LCMS (ES⁺) *m*/*z* calculated for C₂₄H₂₁F₅N₆O₃ 536.16, found 537 (M+H)⁺. HPLC *t*_{R} 1.95 min.

### Example 86: (3-(Pyridin-2-ylmethyl)-1,2,3-oxadiazol-3-ium-5-yl)((3-(2-(3-(pyrrolidin-1-yl)oxetan-3-yl)acetamido)-5-(trifluoromethyl)phenyl)carbamoyl)amide.

The title compound was obtained by using 2-(3-(pyrrolidin-1-yl)oxetan-3-yl)acetic acid. LCMS (ES⁺) *m*/*z* calculated for C₂₅H₂₆F₃N₇O₄ 545.20, found 546 (M+H)⁺. HPLC *t*_{R} 1.08 min.

### Example 88: (3-(4,4-Dioxido-1,4-oxathiane-2-carboxamido)-5-(trifluoromethyl)phenyl)-carbamoyl)(3-(pyridin-2-ylmethyl)-1,2,3-oxadiazol-3-ium-5-yl)amide.

The title compound was obtained by using 1,4-oxathiane-2-carboxylic acid 4,4-dioxide. LCMS (ES⁺) *m*/*z* calculated for C₂₁H₁₉F₃N₆O₆S 540.10, found 541 (M+H)⁺. HPLC *t*_{R} 1.44 min.

### Example 110: ((3-(2-Phenylacetamido)-5-(trifluoromethyl)phenyl)carbamoyl)(3-(piperidin-3-ylmethyl)-1,2,3-oxadiazol-3-ium-5-yl)amide

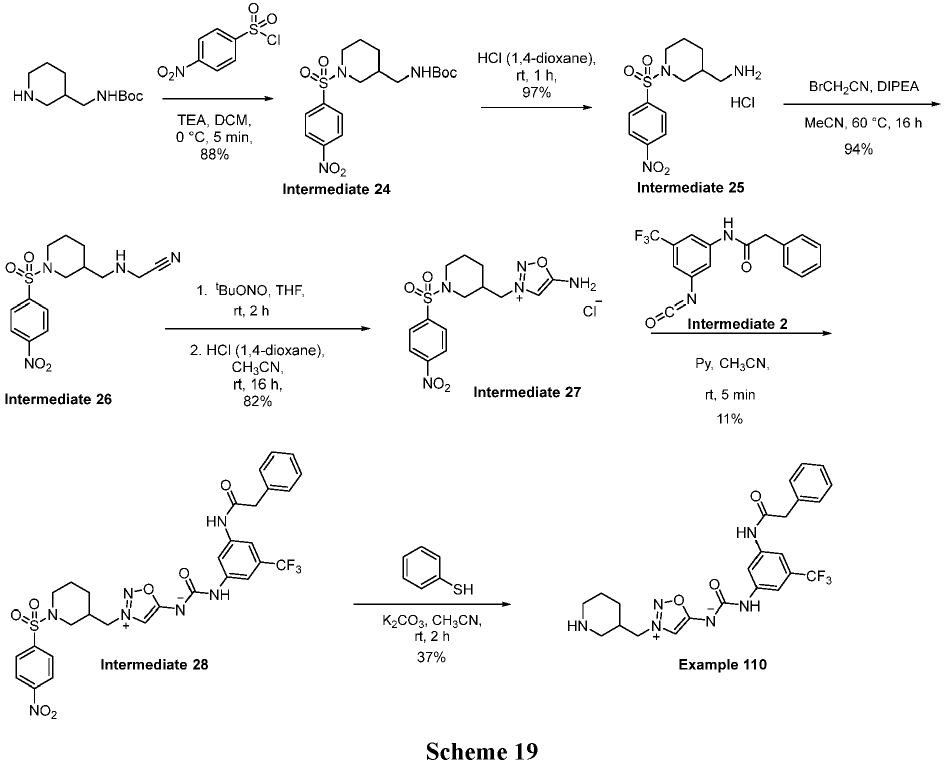

### Step 1: tert-Butyl ((1-((4-nitrophenyl)sulfonyl)piperidin-3-yl)methyl)carbamate (Intermediate 24).

A solution of tert-butyl (piperidin-3-ylmethyl)carbamate (410.0 mg, 1.91 mmol) in DCM (19.0 mL) was treated with TEA (0.8 mL, 5.74 mmol) and 4-nitrobenzenesulfonyl chloride (424.0 mg, 1.91 mmol) and stirred at 0°C for 5 min.before being diluted with DCM and washed with H₂O (3x), brine, dried over Na₂SO₄, filtered and concentrated under reduced pressure to afford the title compound as a light yellow powder (680 mg, 88%) which was used without further purification. ¹H NMR (400 MHz, DMSO-*d₆*) δ 8.24 (d, *J* = 8.8 Hz, 2H), 7.77 (d, *J* = 8.8 Hz, 2H), 6.75-6.70 (m, 1H), 3.39-3.33 (m, 1H), 2.69-2.62 (m, 2H), 2.54-2.47 (m, 1H), 2.15-2.09 (m, 1H), 1.85-1.80 (m, 1H), 1.50-1.47 (m, 1H), 1.40-1.36 (m, 2H), 1.20-1.16 (m, 1H), 1.18 (s, 9H), 0.72-0.66 (m, 1H). HPLC *t*_{R} 1.87 min.

### Step 2: (1-((4-Nitrophenyl)sulfonyl)piperidin-3-yl)methanamine hydrochloride (Intermediate 25).

A solution of *tert*-butyl (1-((4-nitrophenyl)sulfonyl)piperidin-3-yl)methyl)carbamate (Intermediate **24**, 680 mg, 1.70 mmol) in HCl (4 N in dioxane; 6.38 mL, 25.53 mmol) was stirred at rt for 1 h. After removal of the solvent under reduced pressure the title compound was obtained as a yellow solid (560 mg, 97%) which was used without further purification. ¹H NMR (400 MHz, DMSO-*d₆*) δ 8.47 (d, *J* = 8.8 Hz, 2H), 8.01 (d, *J* = 8.8 Hz, 2H), 3.72 (br d, *J* = 11.4 Hz, 1H), 3.56-3.52 (m, 1H), 3.33-3.30 (m, 1H), 3.05-2.90 (m, 2H), 2.71-2.66 (m, 1H), 2.40-2.33 (m, 1H), 2.19-2.13 (m, 1H), 1.84-1.66 (m, 3H), 1.51-1.43 (m, 1H), 1.02-0.94 (m, 1H). LCMS (ES⁺) *m*/*z* calculated for C₁₂H₁₇N₃O₄S 299.09, found 300 (M+H)⁺. HPLC *t*_{R} 0.86 min.

### Step 3: 2-(((1-((4-Nitrophenyl)sulfonyl)piperidin-3-yl)methyl)amino)acetonitrile (Intermediate 26).

The title compound was prepared according to the procedure reported in the synthesis of **Example 1** (Scheme 10) in step 1 and obtained as a white powder (541.41 mg, 94%) which was used without further purification. LCMS (ES⁺) *m*/*z* calculated for C₁₄H₁₈N₄O₄S 338.10, found 339 (M+H)⁺. HPLC *t*_{R} 1.23 min.

### Step 4: 5-Amino-3-((1-((4-nitrophenyl)sulfonyl)piperidin-3-yl)methyl)-1,2,3-oxadiazol-3-ium chloride (Intermediate 27).

The title compound was prepared according to the procedure reported in the synthesis of **Example 1** (Scheme 10) in step 2 & 3 and obtained as a brown oil (538 mg, 82%) which was used without further purification. LCMS (ES⁺) *m*/*z* calculated for C₁₄H₁₈N₅O₅S⁺ 368.10, found 368 (M+H)⁺. HPLC *t*_{R} 0.99 min.

### Step 5: (3-((1-((4-Nitrophenyl)sulfonyl)piperidin-3-yl)methyl)-1,2,3-oxadiazol-3-ium-5-yl)((3-(2-phenylacetamido)-5-(trifluoromethyl)phenyl)carbamoyl)amide (Intermediate 28).

The title compound was prepared according to the procedure reported in the synthesis of **Example 1** (Scheme 10) in step 7 and obtained as a white powder (25 mg, 11%). LCMS (ES⁺) *m*/*z* calculated for C₃₀H₂₈F₃N₇O₇S 687.17, found 688 (M+H)⁺. HPLC *t*_{R} 1.99 min.

### Step 6: (3-(2-Phenylacetamido)-5-(trifluoromethyl)phenyl)carbamoyl)(3-(piperidin-3-ylmethyl)-1,2,3-oxadiazol-3-ium-5-yl)amide (Example 110).

A suspension of (3-((1-((4-nitrophenyl)sulfonyl)piperidin-3-yl)methyl)-1,2,3-oxadiazol-3-ium-5-yl)((3-(2-phenylacetamido)-5-(trifluoromethyl)phenyl)carbamoyl)amide (14.0 mg, 0.02 mmol) and K₂CO₃ (2.81 mg, 0.02 mmol) in CH₃CN (1.8 mL) was treated with benzenethiol (0.01 mL, 0.10 mmol) and stirred at rt for 2 h. The excess of solvent was removed under reduced pressure to give a residue which was purified by automated RP-HPLC using water (+ 0.1% TFA) and CH₃CN (+ 0.1% TFA) as eluents (C18 column). Fractions containing product were lyophilized to give the title compound as a white powder (3.8 mg, 37%). ¹H NMR (400 MHz, DMSO-*d*₆) δ 10.42 (s, 1H), 9.77 (s, 1H), 8.69 (br d, *J* = 9.7 Hz, 1H), 8.34 (br d, *J* = 9.7 Hz, 1H), 8.22 (s, 1H), 8.01 (br s, 1H), 7.83 (br s, 1H), 7.63 (br s, 1H), 7.34-7.33 (m, 4H), 7.29-7.24 (m, 1H), 4.57-4.55 (m, 2H), 3.65 (s, 2H), 3.25 (br t, *J* = 13.2 Hz, 2H), 2.82-2.72 (m, 2H), 2.45-2.37 (m, 1H), 1.85-1.78 (m, 2H), 1.66-1.59 (m, 1H), 1.36-1.24 (m, 1H). ¹⁹F NMR (377 MHz, DMSO-*d*₆) δ -61.60 (s, 3F). LCMS (ES⁺) *m*/*z* calculated for C₂₄H₂₅F₃N₆O₃ 502.19, found 503 (M+H)⁺. HPLC *t*_{R} 1.22 min.

The following compounds were prepared according to the procedure described above (Scheme 18) using the appropriate reagents in the first step.

### Example 105: (3-((1-Methylpiperidin-2-yl)methyl)-1,2,3-oxadiazol-3-ium-5-yl)((3-(2-phenylacetamido)-5-(trifluoromethyl)phenyl)carbamoyl)amide.

The title compound was obtained as a white powder. ¹H NMR (400 MHz, DMSO-*d*₆) δ 10.42 (s, 1H), 9.81 (s, 1H), 8.33 (s, 1H), 8.02 (s, 1H), 7.85 (s, 1H), 7.62 (s, 1H), 7.34 (br d, *J=* 4.2 Hz, 4H), 7.29-7.24 (m, 1H), 5.09-4.95 (m, 2H), 3.86-3.76 (m, 1H), 3.65 (s, 2H), 2.95 (br s, 3H), 2.90-2.84 (m, 2H), 1.84-1.70 (m, 3H), 1.67-1.48 (m, 3H). ¹⁹F NMR (377 MHz, DMSO-*d*₆) δ -61.61 (s, 3F). LCMS (ES⁺) *m*/*z* calculated for C₂₅H₂₇F₃N₆O₃ 516.21, found 517 (M+H)⁺. HPLC *t*_{R} 1.23 min.

### Example 106: (3-((1-Methylpiperidin-4-yl)methyl)-1,2,3-oxadiazol-3-ium-5-yl)((3-(2-phenylacetamido)-5-(trifluoromethyl)phenyl)carbamoyl)amide.

The title compound was obtained as a white powder. ¹H NMR (400 MHz, DMSO-*d*₆+TFA) δ 10.45 (s, 1H), 9.89 (s, 1H), 8.29 (s, 1H), 8.01 (s, 1H), 7.81 (s, 1H), 7.65 (s, 1H), 7.33 (br d, *J* = 4.2 Hz, 4H), 7.29-7.23 (m, 1H), 4.57 (br d, *J* = 6.8 Hz, 2H), 3.66 (s, 2H), 3.47-3.44 (br m, 2H), 2.96-2.88 (m, 3H), 2.76-2.75 (m, 3H), 2.30-2.20 (m, 1H), 1.91-1.86 (m, 2H), 1.52-1.43 (m, 2H). ¹⁹F NMR (377 MHz, DMSO-*d*₆) δ -61.59 (s, 3F). LCMS (ES⁺) *m*/*z* calculated for C₂₅H₂₇F₃N₆O₃ 516.21, found 517 (M+H)⁺. HPLC *t*_{R} 1.22 min.

### Example 115: (3-((1,4-oxazepan-2-yl)methyl)-1,2,3-oxadiazol-3-ium-5-yl)((3-(2-phenylacetamido)-5-(trifluoromethyl)phenyl)carbamoyl)amide.

The title compound was obtained as a white powder. LCMS (ES⁺) *m*/*z* calculated for C₂₄H₂₅F₃N₆O₄ 518.19, found 519 (M+H)⁺. HPLC *t*_{R} 1.31 min.

### Example 120: (3-(2-Phenylacetamido)-5-(trifluoromethyl)phenyl)carbamoyl)(3-(piperidin-4-ylmethyl)-1,2,3-oxadiazol-3-ium-5-yl)amide.

The title compound was obtained as a white powder. ¹H NMR (400 MHz, DMSO-*d*₆) δ 10.35 (s, 1H), 9.69 (s, 1H), 8.47 (br d, *J* = 9.0 Hz, 1H), 8.12 (br s, 2H), 7.93 (s, 1H), 7.75 (s, 1H), 7.56 (s, 1H), 7.26 (br d, *J* = 4.2 Hz, 4H), 7.21-7.15 (m, 1H), 4.48 (br d, *J* = 6.6 Hz, 2H), 3.58 (s, 2H), 3.25-3.22 (m, 2H), 2.85-2.76 (m, 2H), 2.30-2.22 (m, 1H), 1.74 (br d, *J* = 13.1 Hz, 2H), 1.39-1.31 (m, 2H). ¹⁹F NMR (377 MHz, DMSO-*d*₆) δ -61.60 (s, 3F). LCMS (ES⁺) *m*/*z* calculated for C₂₄H₂₅F₃N₆O₃ 502.19, found 503 (M+H)⁺. HPLC *t*_{R} 1.28 min.

### Example 121: (3-(2-Phenylacetamido)-5-(trifluoromethyl)phenyl)carbamoyl)(3-(piperidin-2-ylmethyl)-1,2,3-oxadiazol-3-ium-5-yl)amide.

The title compound was obtained as a white powder. ¹H NMR (400 MHz, DMSO-*d*₆) δ 10.36 (s, 1H), 9.72 (s, 1H), 8.65-8.56 (br m, 2H), 8.18 (br s, 1H), 7.95 (s, 1H), 7.76 (s, 1H), 7.55 (s, 1H), 7.26 (br d, *J* = 4.4 Hz, 4H), 7.22-7.16 (m, 1H), 4.80-4.76 (m, 1H), 4.60 (br dd, *J1* = 14.4 Hz, *J2* = 8.7 Hz, 1H), 3.58 (s, 2H), 3.28 (br d, *J* = 12.3 Hz, 1H), 2.88-2.79 (m, 2H), 1.93-1.84 (m, 1H), 1.76-1.65 (m, 2H), 1.50-1.41 (m, 3H). ¹⁹F NMR (377 MHz, DMSO-*d*₆) δ -61.61 (s, 3F). LCMS (ES⁺) *m*/*z* calculated for C₂₄H₂₅F₃N₆O₃ 502.19, found 503 (M+H)⁺. HPLC *t*_{R} 1.30 min.

### Example 127: (3-Cyclopropyl-5-(2-phenylacetamido)phenyl)carbamoyl)(3-((1-methyl-piperidin-2-yl)methyl)-1,2,3-oxadiazol-3-ium-5-yl)amide.

The title compound was obtained as a light-yellow powder. ¹H NMR (400 MHz, DMSO-*d*₆) δ 9.98 (s, 1H), 9.32 (br s, 1H), 8.27 (s, 1H), 7.70 (s, 1H), 7.33 (br d, *J=* 4.2 Hz, 4H), 7.29-7.23 (m, 1H), 7.08 (s, 1H), 6.92 (s, 1H), 5.10-4.86 (m, 2H), 3.60 (s, 2H), 3.31-3.24 (br m, 1H), 3.11-3.05 (m, 1H), 2.95 (br s, 3H), 2.89-2.81 (m, 1H), 2.05-2.02 (m, 1H), 1.85-1.74 (m, 3H), 1.67-1.46 (m, 3H), 0.95-0.86 (m, 2H), 0.56-0.53 (m, 2H). ¹⁹F NMR (377 MHz, DMSO-*d*₆) δ -74.10 (s, 3F). LCMS (ES⁺) ***m*/*z*** calculated for C₂₇H₃₂N₆O₃ 488.25, found 489 (M+H)⁺. HPLC *t*_{R} 1.17 min.

### Example 117: (3-((1-Methylpiperidin-3-yl)methyl)-1,2,3-oxadiazol-3-ium-5-yl)((3-(2-phenylacetamido)-5-(trifluoromethyl)phenyl)carbamoyl)amide

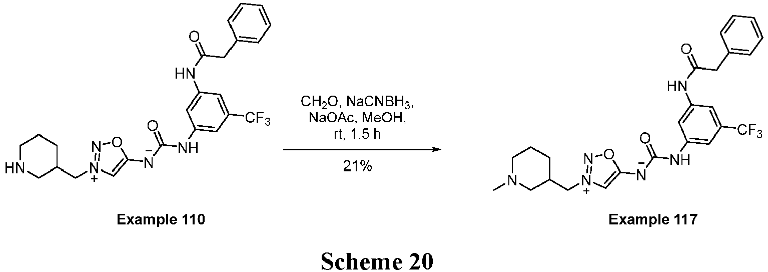

### Step 1: (3-((1-Methylpiperidin-3-yl)methyl)-1,2,3-oxadiazol-3-ium-5-yl)((3-(2 phenylacetamido) -5-(trifluoromethyl)phenyl)carbamoyl)amide (Example 117).

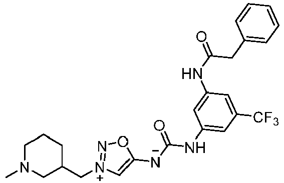

A suspension of ((3-(2-phenylacetamido)-5-(trifluoromethyl)phenyl)carbamoyl)(3-(piperidin-3-ylmethyl)-1,2,3-oxadiazol-3-ium-5-yl)amide (**Example 110**, 26.0 mg, 0.05 mmol) in MeOH (0.5 mL) was treated with formaldehyde (4.99 uL, 0.18 mmol) followed by NaOAc (7.09 mg, 0.05 mmol) and the mixture was stirred at rt for 30 min.before being treated with NaCNBH₃ (9.75 mg, 0.10 mmol) and stirred at rt for 1 h. The reaction mixture was filtered through a pad of cellulose and the filtrate was concentrated under reduced pressure to get a residue which was purified by automated RP-HPLC using water (+ 0.1% TFA) and CH₃CN (+ 0.1% TFA) as eluents (C18 column). Fractions containing product were lyophilized to give the title compound as a white powder (8.2 mg, 21%). ¹H NMR (400 MHz, DMSO-*d*₆) δ 10.42 (s, 1H), 9.77 (br s, 1H), 9.33 (br s, 1H), 8.23 (s, 1H), 8.02 (br s, 1H), 7.83 (br s, 1H), 7.62 (br s, 1H), 7.34-7.33 (m, 4H), 7.29-7.24 (m, 1H), 4.62-4.50 (m, 3H), 3.65 (s, 2H), 3.40 (br d, *J* = 11.4 Hz, 2H), 2.86-2.77 (m, 4H), 2.46-2.41 (m, 1H), 1.93-1.89 (m, 1H), 1.83-1.80 (m, 1H), 1.70-1.63 (m, 1H), 1.28-1.21 (m, 1H). ¹⁹F NMR (377 MHz, DMSO-*d*₆) δ -61.60 (s, 3F). LCMS (ES⁺) *m*/*z* calculated for C₂₅H₂₇F₃N₆O₃ 516.21, found 517 (M+H)⁺. HPLC *t*_{R} 1.25 min.

### Example 89: ((3-Cyclopropyl-5-(2-phenylacetamido)phenyl)carbamoyl)(3-(pyridin-2-ylmethyl)-1,2,3-oxadiazol-3-ium-5-yl)amide

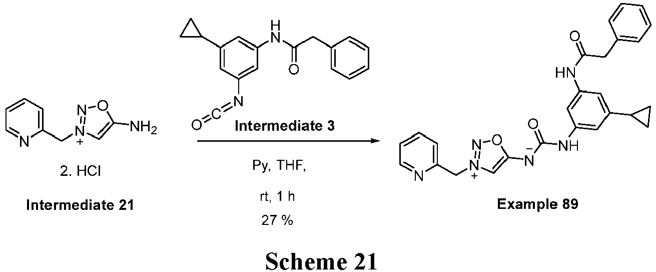

### Step 1: (3-Cyclopropyl-5-(2-phenylacetamido)phenyl)carbamoyl)(3-(pyridin-2-ylmethyl)-1,2,3-oxadiazol-3-ium-5-yl)amide (Example 89).

A solution of 5-amino-3-(pyridin-1-ium-2-ylmethyl)-1,2,3-oxadiazol-3-ium chloride (Intermediate **21**; 40.0 mg, 0.19 mmol) and *N*-(3-cyclopropyl-5-isocyanatophenyl)-2-phenylacetamide (Intermediate **3**; 82.1 mg, 0.28 mmol) in THF (12 mL) was treated with pyridine (0.05 mL, 0.56 mmol) at 0 °C and stirred at rt for 1 h before being diluted with EtOAc. The organic phase was washed with NaHCO₃ (sat aqueous solution) and brine, dried over Na₂SO₄, filtered, and concentrated under reduced pressure. The title compound was obtained after purification by automated RP-HPLC using water (+ 0.1% TFA) and CH₃CN (+ 0.1% TFA) as eluents (C18 column). Fractions containing product were lyophilized to give the title compound as a light beige powder (24.0 mg, 27%). ¹H NMR (400 MHz, DMSO-*d*₆) δ 10.01 (s, 1H), 9.360 (s, 1H), 8.61 (s, 1H), 8.21 (s, 1H), 7.93 (s, 1H), 7.64 (s, 1H), 7.47 (s, 1H), 7.32-7.24 (br m, 5H), 7.02 (s, 1H), 5.97 (s, 2H), 3.60 (s, 2H), 1.79-1.77 (m, 1H), 0.90 (s, 2H), 0.54 (s, 2H). LCMS (ES⁺) *m*/*z* calculated for C₂₆H₂₄N₆O₃ 468.19, found 469 (M+H)⁺. HPLC *t*_{R} 1.57 min.

The above procedure was used for the preparation of the following examples:

### Example 57: ((3-Methyl-5-(2-phenylacetamido)phenyl)carbamoyl)(3-(pyridin-2-ylmethyl)-1,2,3-oxadiazol-3-ium-5-yl)amide (trifluoroacetate salt)

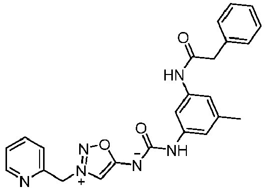

The title compound was prepared from *N*-(3-isocyanato-5-methylphenyl)-2-phenylacetamide (prepared from 3-methyl-5-nitroaniline following procedure reported in the synthesis of Intermediate **3** in which step 3 is performed using H₂ on Pd/C in EtOAc) and 5-amino-3-(pyridin-1-ium-2-ylmethyl)-1,2,3-oxadiazol-3-ium chloride (Intermediate **21**), final compound was obtained as a yellow powder (16.6 mg, 13%). ¹H NMR (400 MHz, DMSO-*d*₆) δ 9.93 (s, 1H), 9.24 (s, 1H), 8.54 (d, *J* = 4.6 Hz, 1H), 8.02 (s, 1H), 7.86 (br t, *J* = 7.7 Hz, 1H), 7.61 (s, 1H), 7.58 (br d, *J* = 7.9 Hz, 1H), 7.39 (dd, *J₁* = 7.1 Hz, *J₂* = 5.2 Hz, 1H), 7.26-7.24 (m, 4H), 7.21-7.14 (m, 1H), 7.05 (s, 1H), 6.97 (s, 1H), 5.87 (s, 2H), 3.53 (s, 2H), 2.11 (s, 3H). LCMS (ES⁺) *m*/*z* calculated for C₂₄H₂₂N₆O₃ 442.18, found 443 (M+H)⁺. HPLC *t*_{R} 1.46 min.

### Example 58: ((3-Chloro-5-(2-phenylacetamido)phenyl)carbamoyl)(3-(pyridin-2-ylmethyl)-1,2,3-oxadiazol-3-ium-5-yl)amide (trifluoroacetate salt)

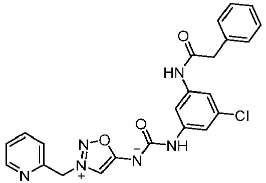

The title compound was prepared from *N*-(3-chloro-5-isocyanatophenyl)-2-phenylacetamide (prepared from 3-chloro-5-nitroaniline following procedure reported in the synthesis of Intermediate **3** in which step 3 is performed using H₂ on Pd/C in EtOAc) and 5-amino-3-(pyridin-1-ium-2-ylmethyl)-1,2,3-oxadiazol-3-ium chloride (Intermediate **21**) final compound was obtained as a white powder (30.2 mg, 28%). ¹H NMR (400 MHz, DMSO-*d*₆) δ 10.28 (s, 1H), 9.60 (s, 1H), 8.61 (d, *J* = 4.6 Hz, 1H), 8.09 (s, 1H), 7.93 (br t, *J* = 7.7 Hz, 1H), 7.71 (s, 1H), 7.65 (br d, *J* = 7.9 Hz, 1H), 7.48-7.45 (m, 2H), 7.39 (s, 1H), 7.33-7.32 (m, 4H), 7.28-7.23 (m, 1H), 5.95 (s, 2H), 3.63 (s, 2H). LCMS (ES⁺) *m*/*z* calculated for C₂₃H₁₉ClN₆O₃ 462.12, found 463 (M+H)⁺. HPLC *t*_{R} 1.62 min.

### Example 130: ((3-((1R,2R)-2-Methylcyclopropyl)-5-(2-phenylacetamido)phenyl)-carbamoyl)(3-(pyridin-2-ylmethyl)-1,2,3-oxadiazol-3-ium-5-yl)amide (trifluoroacetate salt)

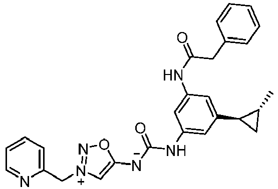

The title compound was prepared from *N*-(3-isocyanato-5-((1*R*,2*R*)-2-methylcyclopropyl)phenyl)-2-phenylacetamide (prepared from 4,4,5,5-tetramethyl-2-((1*R*,2*R*)-2-methylcyclopropyl)-1,3,2-dioxaborolane following procedure reported in the synthesis of **Intermediate 3**) and 5-amino-3-(pyridin-1-ium-2-ylmethyl)-1,2,3-oxadiazol-3-ium chloride (Intermediate **21**) in CH₃CN as solvent and obtained as a beige powder (43.0 mg, 38%). ¹H NMR (400 MHz, DMSO-*d*₆) δ 10.00 (br s, 1H), 9.35 (br s, 1H), 8.61 (br s, 1H), 8.20 (br s, 1H), 7.94 (br t, *J* = 6.9 Hz, 1H), 7.67-7.62 (m, 2H), 7.47 (br s, 1H), 7.33-7.32 (m, 4H), 7.27-7.23 (m, 1H), 6.98 (br d, *J* = 8.8 Hz, 2H), 5.96 (br s, 2H), 3.59 (br s, 2H), 1.49-1.45 (m, 1H), 1.12 (br d, *J* = 5.0 Hz, 3H), 0.95-0.89 (m, 1H), 0.76-0.68 (m, 2H). LCMS (ES⁺) *m*/*z* calculated for C₂₇H₂₆N₆O₃ 482.21, found 483 (M+H)⁺. HPLC *t*_{R} 1.69 min.

### Example 98: (3-(4-(Methoxycarbonyl)benzyl)-1,2,3-oxadiazol-3-ium-5-yl)((3-(2-phenyl-acetamido)-5-(trifluoromethyl)phenyl)carbamoyl)amide

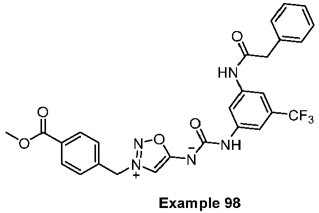

The above example was prepared following the procedure reported for **Example 4** (Scheme 13) using Intermediate **2** and 5-amino-3-(4-(methoxycarbonyl)benzyl)-1,2,3-oxadiazol-3-ium chloride (prepared as described in the synthesis of **Example 1**, scheme 10, in step 1 and 2 from methyl 4-(aminomethyl)benzoate). The title compound was obtained after purification by automated RP-HPLC using water (+ 0.1% TFA) and CH₃CN (+ 0.1% TFA) as eluents (C18 column). Fractions containing product were lyophilized to give the title compound as a white solid (1.8 mg, 44%). ¹H NMR (400 MHz, DMSO-*d*₆) δ 10.42 (s, 1H), 9.76 (s, 1H), 8.20 (s, 1H), 8.04 (d, *J* = 8.3 Hz, 2H), 7.98 (s, 1H), 7.75 (s, 1H), 7.70 (br d, *J* = 8.1 Hz, 3H), 7.33 (br d, *J* = 4.4 Hz, 4H), 7.29-7.23 (m, 1H), 5.90 (s, 2H), 3.87 (s, 3H), 3.65 (s, 2H). ¹⁹F NMR (377 MHz, DMSO-*d*₆) δ -61.59 (s, 3F). LCMS (ES⁺) *m*/*z* calculated for C₂₇H₂₂F₃N₅O₅ 553.16, found 554 (M+H)⁺. HPLC *t*_{R} 1.92 min.

### Example 102: (3-(4-(Hydroxymethyl)benzyl)-1,2,3-oxadiazol-3-ium-5-yl)((3-(2-phenylacetamido)-5-(trifluoromethyl)phenyl)carbamoyl)amide

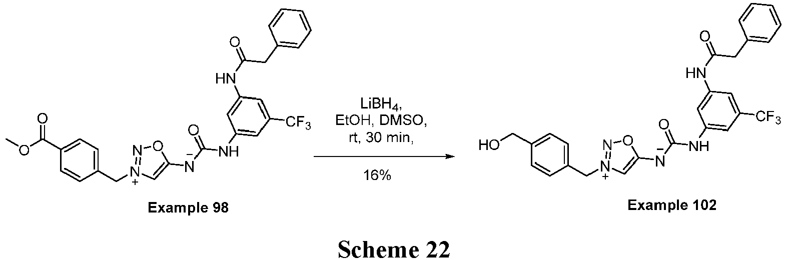

### Step 1: (3-(4-(Hydroxymethyl)benzyl)-1,2,3-oxadiazol-3-ium-5-yl)((3-(2-phenylacetamido)-5-(trifluoromethyl)phenyl)carbamoyl)amide (Example 102).

A solution of (3-(4-(methoxycarbonyl)benzyl)-1,2,3-oxadiazol-3-ium-5-yl)((3-(2-phenylacetamido)-5-(trifluoromethyl)phenyl)carbamoyl)amide **(Example 98**; 20.0 mg, 0.04 mmol) in a mixture of EtOH (1.0 mL) and DMSO (0.5 mL) was treated with LiBH₄ (3.15 mg, 0.14 mmol) and stirred at rt for 30 min before being diluted with H₂O and EtOAc. The phases were separated and the organic phase was washed with brine, dried over Na₂SO₄, filtered, and concentrated under reduced pressure. The title compound was obtained after purification by automated RP-HPLC using water (+ 0.1% TFA) and CH₃CN (+ 0.1% TFA) as eluents (C18 column). Fractions containing product were lyophilized to give the title compound as a white solid (3.0 mg, 16%). ¹H NMR (400 MHz, DMSO-*d*₆) δ 10.39 (s, 1H), 9.71 (br s, 1H), 8.05 (s, 1H), 7.96 (br s, 1H), 7.68 (br d, *J* = 8.6 Hz, 2H), 7.51 (br d, *J* = 7.9 Hz, 2H), 7.38 (br d, *J* = 7.7 Hz, 2H), 7.30 (br d, *J* = 4.0 Hz, 4H), 7.25-7.20 (m, 1H), 5.74 (s, 2H), 4.50 (s, 2H), 3.62 (s, 2H). ¹⁹F NMR (377 MHz, DMSO-*d*₆) δ -61.59 (s, 3F). LCMS (ES⁺) *m*/*z* calculated for C₂₆H₂₂F₃N₅O₄ 525.16, found 526 (M+H)⁺. HPLC *t*_{R} 1.65 min.

### Example 101: (3-(4-Carboxybenzyl)-1,2,3-oxadiazol-3-ium-5-yl)((3-(2-phenylacetamido)-5-(trifluoromethyl)phenyl)carbamoyl)amide

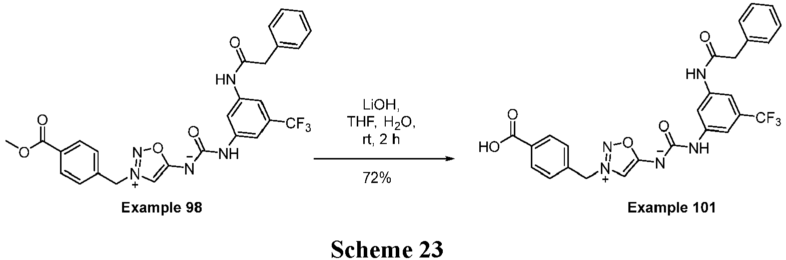

### Step 1: (3-(4-Carboxybenzyl)-1,2,3-oxadiazol-3-ium-5-yl)((3-(2-phenylacetamido)-5-(trifluoromethyl)phenyl)carbamoyl)amide (Example 101)

A solution of (3-(4-(methoxycarbonyl)benzyl)-1,2,3-oxadiazol-3-ium-5-yl)((3-(2-phenylacetamido)-5-(trifluoromethyl)phenyl)carbamoyl)amide (**Example 98**; 25.0 mg, 0.05 mmol) in THF (0.25 mL) and H₂O (0.25 mL) was treated with lithium hydroxide hydrate (4.74 mg, 0.11 mmol) and stirred for 2 h at rt before being diluted with H₂O and EtOAc. The phases were separated, and the aqueous phase was acidified to pH 5 with HCl (1N) and extracted with EtOAc. The organic phase was washed with brine, dried over Na₂SO₄, filtered, and concentrated under reduced pressure. The title compound was obtained after lyophilization as a yellow solid (17.8 mg, 72%). ¹H NMR (400 MHz, DMSO-*d*₆) δ 10.48 (s, 1H), 9.81 (br s, 1H), 8.23 (s, 1H), 8.07 (br d, *J* = 8.1 Hz, 2H), 8.04 (br s, 1H), 7.78 (br d, *J* = 14.5 Hz, 2H), 7.71 (br d, *J* = 8.1 Hz, 2H), 7.39 (br d, *J* = 4.4 Hz, 4H), 7.33-7.29 (m, 1H), 5.94 (s, 2H), 3.71 (s, 2H). ¹⁹F NMR (377 MHz, DMSO-*d*₆) δ -61.59 (s, 3F). LCMS (ES⁺) *m*/*z* calculated for C₂₆H₂₀F₃N₅O₅ 539.14, found 540 (M+H)⁺. HPLC *t*_{R} 1.66 min.

### Example 104: (3-(4-Carbamoylbenzyl)-1,2,3-oxadiazol-3-ium-5-yl)((3-(2-phenylacetamido)-5-(trifluoromethyl)phenyl)carbamoyl)amide

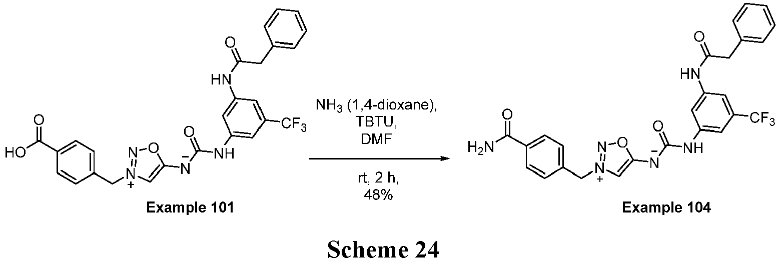

### Step 1: (3-(4-Carbamoylbenzyl)-1,2,3-oxadiazol-3-ium-5-yl)((3-(2-phenylacetamido)-5-(trifluoromethyl)phenyl)carbamoyl)amide (Example 104).

A solution of (3-(4-carboxybenzyl)-1,2,3-oxadiazol-3-ium-5-yl)((3-(2-phenylacetamido)-5-(trifluoromethyl)phenyl)carbamoyl)amide (**Example 101**; 15.0 mg, 0.03 mmol) in DMF (0.5 mL) was treated with TBTU (13.39 mg, 0.04 mmol) followed by NH₃ (0.5 M in dioxane; 0.46 mL, 0.14 mmol) and the resulting solution was stirred at rt for 2 h. Solvent was removed under reduced pressure and the residue dissolved in MeOH and passed through a SiCO₃ cartridge (2.0 g). Solvent was removed under reduced pressure and the title compound was obtained after purification by automated RP-HPLC using water (+ 0.1% TFA) and CH₃CN (+ 0.1% TFA) as eluents (C18 column). Fractions containing product were lyophilized to give the title compound as a white solid (7.3 mg, 48%). ¹H NMR (400 MHz, DMSO-*d*₆) δ 10.23 (s, 1H), 9.56 (br s, 1H), 7.97 (s, 1H), 7.84 (br s, 1H), 7.79 (s, 1H), 7.74 (br d, *J* = 8.3 Hz, 2H), 7.52 (br d, *J* = 16.0 Hz, 2H), 7.44 (br d, *J* = 8.3 Hz, 2H), 7.27 (br s, 1H), 7.14 (br d, *J* = 4.4 Hz, 4H), 7.09-7.03 (m, 1H), 5.66 (s, 2H), 3.45 (s, 2H). ¹⁹F NMR (377 MHz, DMSO-*d*₆) δ -61.59 (s, 3F). LCMS (ES⁺) *m*/*z* calculated for C₂₆H₂₁F₃N₆O₄ 538.16, found 539 (M+H)⁺. HPLC *t*_{R} 1.57 min.

### Example 109: (3-(4-(2-Methoxy-2-oxoethyl)benzyl)-1,2,3-oxadiazol-3-ium-5-yl)((3-(2-phenylacetamido)-5-(trifluoromethyl)phenyl)carbamoyl)amide

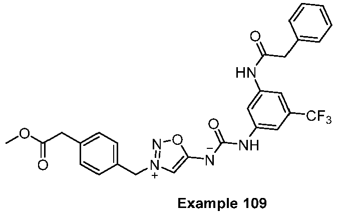

The above example was prepared following the procedure reported for **Example 4** (Scheme 13) using Intermediate **2** and 5-amino-3-(4-(2-methoxy-2-oxoethyl)benzyl)-1,2,3-oxadiazol-3-ium chloride ((prepared as described in the synthesis of **Example 1**, scheme 10, in step 1 and 2 from from methyl 2-(4-(aminomethyl)phenyl)acetate hydrochloride). The title compound was obtained after purification by flash chromatography on silica gel (eluting with 10-100% EtOAc in petroleum ether) as a yellow solid (8.0 mg, 48%). ¹H NMR (400 MHz, DMSO-*d*₆) δ 10.42 (s, 1H), 9.73 (s, 1H), 8.10 (s, 1H), 7.98 (br s, 1H), 7.73 (br s, 1H), 7.70 (br s, 1H), 7.53 (d, *J* = 8.1 Hz, 2H), 7.36 (br d, *J* = 8.3 Hz, 2H), 7.33 (br d, *J* = 4.4 Hz, 4H), 7.28-7.23 (m, 1H), 5.77 (s, 2H), 3.73 (s, 2H), 3.65 (s, 2H), 3.62 (s, 3H). ¹⁹F NMR (377 MHz, DMSO-*d*₆) δ -61.58 (s, 3F). LCMS (ES⁺) *m*/*z* calculated for C₂₈H₂₄F₃N₅O₅ 567.17, found 568 (M+H)⁺. HPLC *t*_{R} 1.88 min.

### Example 111: (3-(4-(2-Hydroxyethyl)benzyl)-1,2,3-oxadiazol-3-ium-5-yl)((3-(2-phenylacetamido)-5-(trifluoromethyl)phenyl)carbamoyl)amide

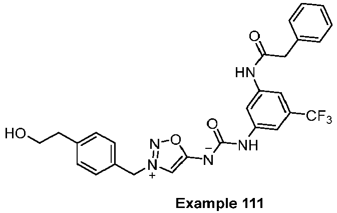

The above example was prepared following the procedure reported for **Example 102** (scheme 22) using **Example 109** in EtOH at 40 °C for 30 min. The title compound was obtained after purification by automated RP-HPLC using water (+ 0.1% TFA) and CH₃CN (+ 0.1% TFA) as eluents (C18 column). Fractions containing product were lyophilized to give the title compound as a white solid (5.2 mg, 96% pure, 21%). ¹H NMR (400 MHz, DMSO-*d*₆) δ 10.36 (s, 1H), 9.68 (s, 1H), 8.04 (s, 1H), 7.92 (br s, 1H), 7.67 (br s, 1H), 7.64 (br s, 1H), 7.42 (d, *J* = 8.1 Hz, 2H), 7.28-7.24 (m, 6H), 7.22-7.17 (m, 1H), 5.68 (s, 2H), 3.59 (s, 2H), 3.54 (t, *J* = 6.6 Hz, 2H), 2.68 (t, *J* = 6.8 Hz, 2H). ¹⁹F NMR (377 MHz, DMSO-*d*₆) δ -61.59 (s, 3F). LCMS (ES⁺) *m*/*z* calculated for C₂₇H₂₄F₃N₅O₄ 539.18, found 540 (M+H)⁺. HPLC *t*_{R} 1.68 min.

### Example 113: (3-((5-(Methoxycarbonyl)pyridin-2-yl)methyl)-1,2,3-oxadiazol-3-ium-5-yl)((3-(2-phenylacetamido)-5-(trifluoromethyl)phenyl)carbamoyl)amide

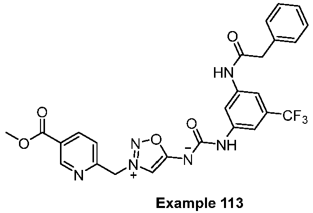

The above example was prepared following the procedure reported for **Example 4** (Scheme 13) using Intermediate **2** and 5-amino-3-((5-(methoxycarbonyl)pyridin-2-yl)methyl)-1,2,3-oxadiazol-3-ium (prepared as described in the synthesis of **Example 1**, scheme 10, in step 1 and 2 from methyl 6-(aminomethyl)nicotinate hydrochloride). The title compound was obtained after purification by flash chromatography on silica gel (eluting with 0-100% EtOAc in petroleum ether) as a yellow solid (2.6 mg, 46%). ¹H NMR (400 MHz, DMSO-*d*₆) δ 10.43 (s, 1H), 9.76 (s, 1H), 9.09 (s, 1H), 8.42 (br d, *J* = 8.3 Hz, 1H), 8.20 (s, 1H), 7.98 (br s, 1H), 7.79 (br d, *J* = 8.3 Hz, 1H), 7.75 (br s, 1H), 7.70 (br s, 1H), 7.33 (br d, *J* = 4.2 Hz, 4H), 7.28-7.23 (m, 1H), 6.07 (s, 2H), 3.90 (s, 3H), 3.65 (s, 2H). ¹⁹F NMR (377 MHz, DMSO-*d*₆) δ -61.59 (s, 3F). LCMS (ES⁺) *m*/*z* calculated for C₂₆H₂₁F₃N₆O₅ 554.15, found 555 (M+H)⁺. HPLC *t*_{R} 1.83 min.

### Example 116: (3-((5-(Hydroxymethyl)pyridin-2-yl)methyl)-1,2,3-oxadiazol-3-ium-5-yl)((3-(2-phenylacetamido)-5-(trifluoromethyl)phenyl)carbamoyl)amide

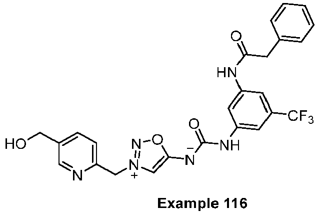

The above example was prepared following the procedure reported for **Example 102** (scheme 22) using **Example 113** in EtOH at rt for 30 min. The title compound was obtained after purification by automated RP-HPLC using water (+ 0.1% TFA) and CH₃CN (+ 0.1% TFA) as eluents (C18 column). Fractions containing product were lyophilized to give the title compound as a white solid (6.5 mg, 34%). ¹H NMR (400 MHz, DMSO-*d*₆) δ 10.43 (s, 1H), 9.75 (s, 1H), 8.55 (s, 1H), 8.10 (s, 1H), 7.99 (br s, 1H), 7.85 (br d, *J* = 7.5 Hz, 1H), 7.72 (br d, *J* = 6.1 Hz, 2H), 7.63 (br d, *J* = 7.9 Hz, 1H), 7.33 (br d, *J* = 4.2 Hz, 4H), 7.28-7.23 (m, 1H), 5.92 (s, 2H), 4.56 (s, 2H), 3.65 (s, 2H). ¹⁹F NMR (377 MHz, DMSO-*d*₆) δ -61.58 (s, 3F). LCMS (ES⁺) *m*/*z* calculated for C₂₅H₂₁F₃N₆O₄ 526.16, found 527 (M+H)⁺. HPLC *t*_{R} 1.49 min.

### Example 108: (3-(3-(Aminomethyl)benzyl)-1,2,3-oxadiazol-3-ium-5-yl)((3-(2-phenylacetamido)-5-(trifluoromethyl)phenyl)carbamoyl)amide

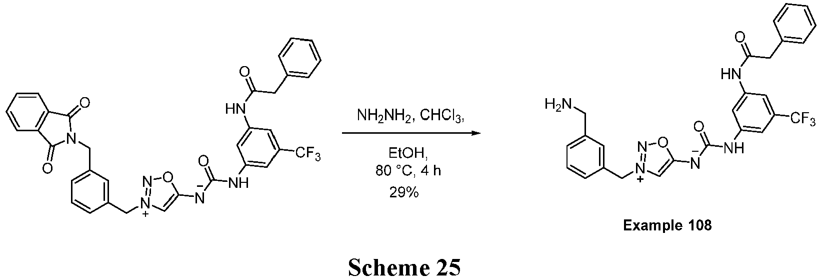

### Step 1: (3-(3-(Aminomethyl) benzyl)-!, 2,3-oxadiazol-3-ium-5-yl)((3-(2-phenylacetamido)-5-(trifluoromethyl)phenyl)carbamoyl)amide (Example 108).

A solution of (3-(3-((1,3-dioxoisoindolin-2-yl)methyl)benzyl)-1,2,3-oxadiazol-3-ium-5-yl)((3-(2-phenylacetamido)-5-(trifluoromethyl)phenyl)carbamoyl)amide (prepared according to the procedure reported for the synthesis of **Example 2** starting from 2-(3-(aminomethyl)benzyl)isoindoline-1,3-dione hydrochloride (**Intermediate 6**); 30.0 mg, 0.05 mmol) in a mixture of EtOH (3.0 mL) and CHCl₃ (5.0 mL) was treated with hydrazine hydrate (11.5 mg, 0.23 mmol) and stirred at rt at 80 °C for 4 h, After removal of solvent under reduced pressure the title compound was obtained after purification by automated RP-HPLC using water (+ 0.1% TFA) and CH₃CN (+ 0.1% TFA) as eluents (C18 column). Fractions containing product were lyophilized to give the title compound as a white powder (7.0 mg, 29%). ¹H NMR (400 MHz, DMSO-*d*₆) δ 10.41 (s, 1H), 9.75 (s, 1H), 8.12 (br s, 4H), 8.00 (br s, 1H), 7.78 (br s, 1H), 7.63 (br s, 2H), 7.57-7.54 (m, 3H), 7.34-7.33 (m, 4H), 7.28-7.24 (m, 1H), 5.82 (s, 2H), 4.09-4.05 (m, 2H), 3.65 (s, 2H). ¹⁹F NMR (377 MHz, DMSO-*d*₆) δ -61.59 (s, 3F). LCMS (ES⁺) *m*/*z* calculated for C₂₆H₂₃F₃N₆O₃ 524.18, found 525 (M+H)⁺. HPLC *t*_{R} 1.34 min.

The following compounds were prepared according to the procedure described above using the appropriate starting amine prepared as reported for the synthesis of Intermediate **7** (Example 56) or Intermediate **8** (Example 142).

### Example 114: (3-(2-(Aminomethyl)benzyl)-1,2,3-oxadiazol-3-ium-5-yl)((3-(2-phenylacetamido)-5-(trifluoromethyl)phenyl)carbamoyl)amide.

The title compound was obtained as a white powder. LCMS (ES⁺) *m*/*z* calculated for C₂₆H₂₃F₃N₆O₃ 524.18, found 525 (M+H)⁺. HPLC *t*_{R} 1.40 min.

### Example 118: (3-((3-Aminocyclobutyl)methyl)-1,2,3-oxadiazol-3-ium-5-yl)((3-(2-phenylacetamido)-5-(trifluoromethyl)phenyl)carbamoyl)amide.

The title compound was obtained as a white powder. ¹H NMR (400 MHz, DMSO-*d*₆) δ 10.42 (s, 1H), 9.75 (s, 1H), 8.15 (d, *J* = 9.4 Hz, 1H), 7.99 (br s, 3H), 7.89 (br s, 1H), 7.81 (br s, 1H), 7.65 (s, 1H), 7.34 (br d, *J* = 4.4 Hz, 4H), 7.28-7.24 (m, 1H), 4.72 (br d, *J* = 7.9 Hz, 1H), 4.59 (br d, *J* = 6.6 Hz, 1H), 3.65 (s, 2H), 3.05-2.96 (m, 1H), 2.76-2.70 (m, 1H), 2.39-2.32 (m, 1H), 2.28-2.18 (m, 2H), 1.99-1.90 (m, 1H). ¹⁹F NMR (377 MHz, DMSO-*d*₆) δ -61.58 (s, 3F). LCMS (ES⁺) *m*/*z* calculated for C₂₃H₂₃F₃N₆O₃ 488.18, found 489 (M+H)⁺. HPLC *t*_{R} 1.22 min.

### Example 56: (3-(4-(Aminomethyl)benzyl)-1,2,3-oxadiazol-3-ium-5-yl)((3-(2-phenylacetamido)-5-(trifluoromethyl)phenyl)carbamoyl)amide.

The title compound was obtained as a white powder. ¹H NMR (400 MHz, DMSO-*d*₆) δ 10.42 (s, 1H), 9.74 (s, 1H), 8.14 (br s, 3H), 8.09 (s, 1H), 7.99 (s, 1H), 7.77 (s, 1H), 7.65-7.63 (m, 3H), 7.54 (br d, *J* = 7.9 Hz, 2H), 7.33 (br d, *J* = 4.4 Hz, 4H), 7.29-7.23 (m, 1H), 5.81 (s, 2H), 4.09-4.05 (m, 2H), 3.65 (s, 2H). ¹⁹F NMR (377 MHz, DMSO-*d*₆) δ -61.60 (s, 3F). LCMS (ES⁺) *m*/*z* calculated for C₂₆H₂₃F₃N₆O₃ 524.18, found 523 (M-H)⁻. HPLC *t*_{R} 1.32 min.

### Example 142: ((3-(2-Phenylacetamido)-5-(trifluoromethyl)phenyl)carbamoyl)(3-((1-(2,2,2-trifluoroethyl)piperidin-2-yl)methyl)-1,2,3-oxadiazol-3-ium-5-yl)amide (trifluoroacetate salt)

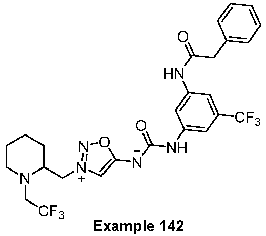

The above example was prepared following the procedure reported for **Example 4** (a mixture of CH₃CN and DMSO was used as solvent) using Intermediate **2** and 5-amino-3-((1-(2,2,2-trifluoroethyl)piperidin-2-yl)methyl)-1,2,3-oxadiazol-3-ium chloride (prepared as described in the synthesis of **Example 1** in step 1 and 2 from Intermediate **8**; step 1 was performed at 60 °C). The title compound was obtained after purification by automated RP-HPLC using water (+ 0.1% TFA) and CH₃CN (+ 0.1% TFA) as eluents (C18 column). Fractions containing product were lyophilized to give the title compound as a pale orange solid (15.5 mg, 92% pure, 4%). ¹H NMR (400 MHz, DMSO-*d*₆) δ 10.23 (s, 1H), 9.51 (s, 1H), 7.99 (s, 1H), 7.78 (s, 1H), 7.59 (s, 1H), 7.48 (s, 1H), 7.14-7.13 (br m, 5H), 7.08-7.03 (m, 1H), 4.70-4.65 (m, 1H), 4.45-4.41 (m, 1H), 3.45 (s, 2H), 3.28-3.12 (m, 3H), 2.85-2.79 (m, 1H), 2.46-2.39 (m, 1H), 1.52-1.38 (m, 2H), 1.35-1.14 (m, 4H). ¹⁹F NMR (377 MHz, DMSO-*d*₆) δ -61.59 (s, 3F), -70.95 (s, 3F). LCMS (ES⁺) *m*/*z* calculated for C₂₆H₂₆F₆N₆O₃ 584.20, found 585 (M+H)⁺. HPLC *t*_{R} 2.11 min.

### Example 112: (3-(9-Amino-3-oxabicyclo[3.3.1]nonan-7-yl)-1,2,3-oxadiazol-3-ium-5-yl)((3-(2-phenylacetamido)-5-(trifluoromethyl)phenyl)carbamoyl)amide.

The title compound was obtained as a white powder. LCMS (ES⁺) *m*/*z* calculated for C₂₆H₂₇F₃N₆O₄ 544.20, found 545 (M+H)⁺. HPLC *t*_{R} 1.22 min.

### Example 140: ((3-(Benzylcarbamoyl)-5-(trifluoromethyl)phenyl)carbamoyl)(3-((1-(2-ethoxy-2-oxoethyl)piperidin-4-yl)methyl)-1,2,3-oxadiazol-3-ium-5-yl)amide (trifluoroacetate salt)

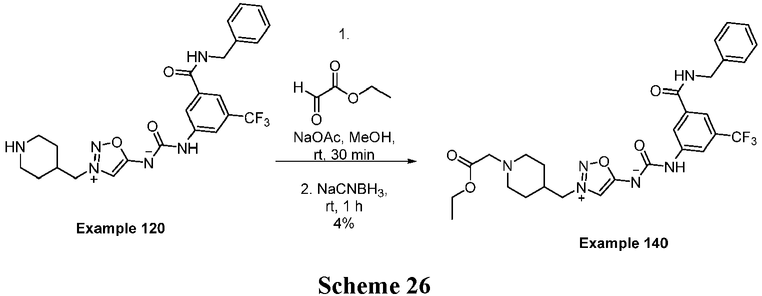

### Step 1: (3-(Benzylcarbamoyl)-5-(trifluoromethyl)phenyl)carbamoyl)(3-((1-(2-ethoxy-2-oxo-ethyl)piperidin-4-yl)methyl)-1,2,3-oxadiazol-3-ium-5-yl)amide (trifluoroacetate salt) (Example 140).

A suspension of ((3-(benzylcarbamoyl)-5-(trifluoromethyl)phenyl)carbamoyl)(3-(piperidin-4-ylmethyl)-1,2,3-oxadiazol-3-ium-5-yl)amide (**Example 120**; 78.0 mg, 0.16 mmol) in MeOH (1.5 mL) was treated with ethyl 2-oxoacetate (0.22 mL, 0.54 mmol) followed by NaOAc (21.28 mg, 0.16 mmol) and the mixture was stirred at rt for 30 min. before being treated with NaCNBH₃ (29.26 mg, 0.47 mmol) and stirred at rt for 1 h. The mixture was then filtered on a pad of solca flok and concentrated under reduced pressure. The title compound was obtained after purification by automated RP-HPLC using water (+ 0.1% TFA) and CH₃CN (+ 0.1% TFA) as eluents (C18 column). Fractions containing product were lyophilized to give the title compound as a white solid (4.4 mg, 4%). ¹H NMR (400 MHz, DMSO-*d*₆) δ 10.42 (s, 1H), 9.91 (br s, 1H), 9.77 (br s, 1H), 8.21 (s, 1H), 8.00 (s, 1H), 7.83 (s, 1H), 7.63 (s, 1H), 7.34-7.32 (m, 4H), 7.29-7.23 (m, 1H), 4.55 (br d, *J* = 5.7 Hz, 2H), 4.27-4.16 (m, 4H), 3.65 (s, 2H), 3.55-3.51 (br m, 2H), 3.05-2.97 (m, 2H), 2.29-2.22 (m, 1H), 1.89 (br d, *J* = 15.6 Hz, 2H), 1.65-1.59 (m, 2H), 1.25 (br t, *J* = 6.9 Hz, 3H). ¹⁹F NMR (377 MHz, DMSO-*d*₆) δ -61.60 (s, 3F). LCMS (ES⁺) *m*/*z* calculated for C₂₈H₃₁F₃N₆O₅ 588.23, found 589 (M+H)⁺. HPLC *t*_{R} 1.36 min.

### Example 141: ((3-(Benzylcarbamoyl)-5-(trifluoromethyl)phenyl)carbamoyl)(3-((1-(2-hydroxyethyl)piperidin-4-yl)methyl)-1,2,3-oxadiazol-3-ium-5-yl)amide (trifluoroacetate salt)

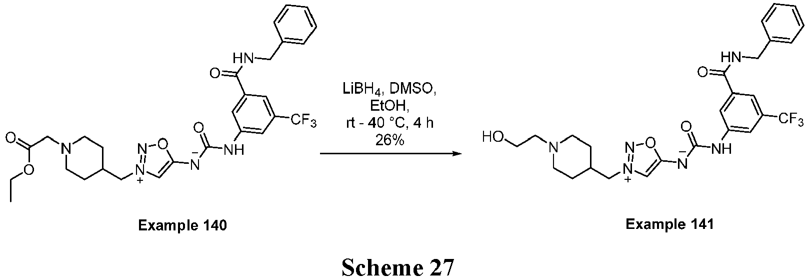

### Step 1: ((3-(Benzylcarbamoyl)-5-(trifluoromethyl)phenyl)carbamoyl)(3-((1-(2-hydroxyethyl)-piperidin-4-yl)methyl)-1,2,3-oxadiazol-3-ium-5-yl)amide (trifluoroacetate salt) (Example 141).

A solution of ((3-(benzylcarbamoyl)-5-(trifluoromethyl)phenyl)carbamoyl)(3-((1-(2-ethoxy-2-oxoethyl)piperidin-4-yl)methyl)-1,2,3-oxadiazol-3-ium-5-yl)amide **(Example 140**; 19.0 mg, 0.03 mmol) in a mixture of EtOH (4 mL) and DMSO (1 mL) was treated with LiBH₄ (42.18 mg, 1.95 mmol) and stirred at rt for 1 h and at 40 °C for 3 h before being cooled to rt and diluted with EtOAc and H₂O. The organic phase was washed with brine, dried over Na₂SO₄, filtered, and concentrated under reduced pressure. The title compound was obtained after purification by automated RP-HPLC using water (+ 0.1% TFA) and CH₃CN (+ 0.1% TFA) as eluents (C18 column). Fractions containing product were lyophilized to give the title compound as a white solid (6.7 mg, 26%). ¹H NMR (400 MHz, DMSO-*d*₆) δ 10.51 (s, 1H), 9.86 (br s, 1H), 9.19 (br s, 1H), 8.30 (s, 1H), 8.10 (s, 1H), 7.92 (s, 1H), 7.72 (s, 1H), 7.43-7.41 (m, 4H), 7.39-7.34 (m, 1H), 4.64 (br d, *J=* 6.1 Hz, 2H), 3.84-3.79 (m, 2H), 3.75 (s, 2H), 3.63-3.59 (br m, 2H), 3.23-3.20 (m, 2H), 3.09-3.00 (m, 2H), 2.39-2.35 (m, 1H), 2.01-1.93 (m, 2H), 1.71-1.62 (m, 2H). ¹⁹F NMR (377 MHz, DMSO-*d*₆) δ -61.59 (s, 3F). LCMS (ES⁺) *m*/*z* calculated for C₂₆H₂₉F₃N₆O₄ 546.22, found 547 (M+H)⁺. HPLC *t*_{R} 1.25 min.

Table 1 below includes all compounds prepared according to the above procedures or by modifications of the same through conventional chemistry.

| **Example N.** | **IUPAC name** | **Structure** | **MW** |
|---|---|---|---|
| 1 | (3-(4-(2-Methoxy-4-methylpyrimidin-5-yl)benzyl)-1,2,3-oxadiazol-3-ium-5-yl)((3-(2-phenylacetamido)-5-(trifluoromethyl)phenyl)carbamoyl)amide | 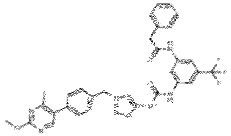 | 617.58 |
| 2 | ((3-((Cyclohexylmethyl)sulfonamido)-5-(trifluoromethyl)phenyl)carbamoyl)(3-(4-(2-methoxy-4-methylpyrimidin-5-yl)benzyl)-1,2,3-oxadiazol-3-ium-5-yl)amide | 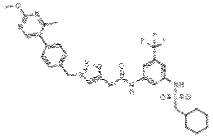 | 659.68 |
| 3 | ((3-Amino-5-(trifluoromethyl)phenyl)-carbamoyl)(3-(4-(2-methoxy-4-methylpyrimidin-5-yl)benzyl)-1,2,3-oxadiazol-3-ium-5-yl)amide | 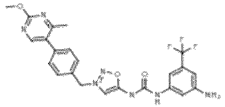 | 499.45 |
| 4 | (3-Methyl-1,2,3-oxadiazol-3-ium-5-yl)((3-(2-phenylacetamido)-5-(trifluoromethyl)phenyl)-carbamoyl)amide | 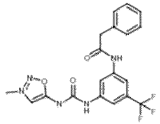 | 419.36 |
| 5 | (3-((5-(2-(Aminomethyl)phenyl)pyridin-2-yl)methyl)-1,2,3-oxadiazol-3-ium-5-yl)((3-(2-phenylacetamido)-5-(trifluoromethyl)phenyl-)carbamoyl)amide | 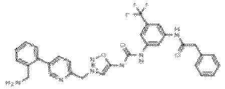 | 601.58 |
| 6 | (3-((5-(1-Methyl-1H-imidazol-5-yl)pyridin-2-yl)methyl)-1,2,3-oxadiazol-3-ium-5-yl)((3-(2-phenylacetamido)-5-(trifluoromethyl)-phenyl)carbamoyl)amide | 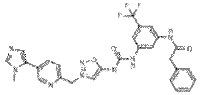 | 576.53 |
| 7 | ((3-(2-Phenylacetamido)-5-(trifluoromethyl)-phenyl)carbamoyl)(3-(pyridin-2-ylmethyl)-1,2,3-oxadiazol-3-ium-5-yl)amide | 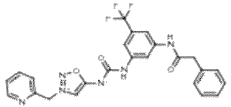 | 496.44 |
| 8 | ((3-(2-Cyclohexylacetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide | 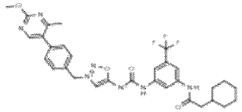 | 623.63 |
| 9 | (3-(4-(2-Methoxy-4-methylpyrimidin-5-yl)benzyl)-1,2,3-oxadiazol-3-ium-5-yl)((3-(3- phenylpropanamido)-5-(trifluoromethyl)phenyl)carbamoyl)amide | 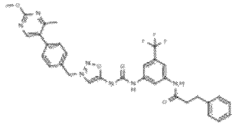 | 631.6 |
| 10 | (3-(4-(2-Methoxy-4-methylpyrimidin-5-yl)benzyl)-1,2,3-oxadiazol-3-ium-5-yl)((3-((phenylmethyl)sulfonamido)-5-(trifluoromethyl)phenyl)carbamoyl)amide | 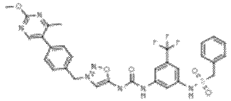 | 653.63 |
| 11 | ((3-Acetamido-5-(trifluoromethyl)phenyl)-carbamoyl)(3-(4-(2-methoxy-4-methylpyrimidin-5-yl)benzyl)-1,2,3-oxadiazol-3-ium-5-yl)amide | 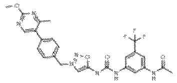 | 541.48 |
| 12 | (3-((5-Bromopyridin-2-yl)methyl)-1,2,3-oxadiazol-3-ium-5-yl)((3-(2-phenylacetamido)-5-(trifluoromethyl)phenyl)carbamoyl)amide | 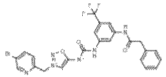 | 575.34 |
| 13 | (3-((5-(2-Methoxy-4-methylpyrimidin-5-yl)-pyridin-2-yl)methyl)-1,2,3-oxadiazol-3-ium-5-yl)((3-(2-phenylacetamido)-5-(trifluoromethyl)-phenyl)carbamoyl)amide | 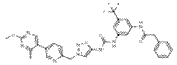 | 618.57 |
| 14 | (3-((5-(Isoindolin-4-yl)pyridin-2-yl)methyl)-1,2,3-oxadiazol-3-ium-5-yl)((3-(2-phenylacetamido)-5-(trifluoromethyl)phenyl)carbamoyl)-amide | 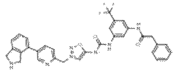 | 613.59 |
| 15 | ((3-Benzamido-5-(trifluoromethyl)phenyl)-carbamoyl)(3-(4-(2-methoxy-4-methylpyrimidin-5-yl)benzyl)-1,2,3-oxadiazol-3-ium-5-yl)amide | 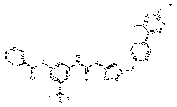 | 603.55 |
| 16 | (3-(4-(2-Methoxy-4-methylpyrimidin-5-yl)benzyl)-1,2,3-oxadiazol-3-ium-5-yl)((3-(2-(4-methoxyphenyl)acetamido)-5-(trifluoromethyl)-phenyl)carbamoyl)amide | 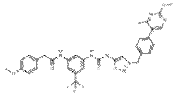 | 647.6 |
| 17 | ((3-(2-(4-Chlorophenyl)acetamido)-5- (trifluoromethyl)phenyl)carbamoyl)(3-(4-(2-methoxy-4-methylpyrimidin-5-yl)benzyl)-1,2,3-oxadiazol-3-ium-5-yl)amide | 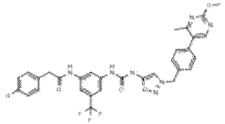 | 652.02 |
| 18 | ((3-(Cyclopentanecarboxamido)-5-(trifluoromethyl)phenyl)carbamoyl)(3-(4-(2-methoxy-4-methylpyrimidin-5-yl)benzyl)-1,2,3-oxadiazol-3-ium-5-yl)amide | 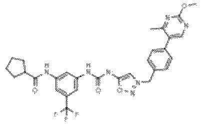 | 595.57 |
| 19 | (3-(4-(2-Methoxy-4-methylpyrimidin-5-yl)benzyl)-1,2,3-oxadiazol-3-ium-5-yl)((3-(2-(pyridin-3-yl)acetamido)-5-(trifluoromethyl)phenyl)carbamoyl)amide | 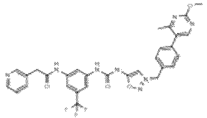 | 618.57 |
| 20 | ((3-(2-(Benzo[d]isoxazol-3-yl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide | 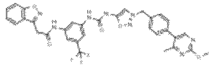 | 658.59 |
| 21 | ((3-(2-(2-Chlorophenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide | 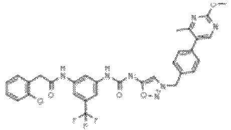 | 652.02 |
| 22 | ((3-(2-(4-(Aminomethyl)phenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide | 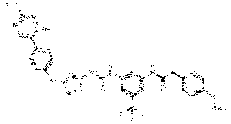 | 646.62 |
| 23 | (*R*)-(3-(2-Amino-1-(4-(3,5-dimethylisoxazol-4-yl)phenyl)ethyl)-1,2,3-oxadiazol-3-ium-5-yl)((3- (2-phenylacetamido)-5-(trifluoromethyl)phenyl)-carbamoyl)amide | 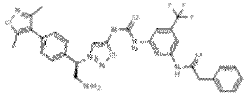 | 619.59 |
| 24 | (*S*)-(3-(2-Amino-1-(4-(3,5-dimethylisoxazol-4-yl)phenyl)ethyl)-1,2,3-oxadiazol-3-ium-5-yl)((3- (2-phenylacetamido)-5-(trifluoromethyl)phenyl)carbamoyl)amide | 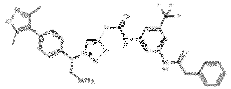 | 619.59 |
| 25 | (3-((5-(2-Amino-4-methylpyrimidin-5-yl)pyridin-2-yl)methyl)-1,2,3-oxadiazol-3-ium-5-yl)((3-(2-phenylacetamido)-5-(trifluoromethyl)-phenyl)carbamoyl)amide | 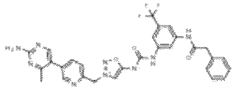 | 603.55 |
| 26 | (3-((1*R*,3*R*)-3-Aminocyclobutyl)-1,2,3-oxadiazol-3-ium-5-yl)((3-(2-phenylacetamido)-5-(trifluoromethyl)phenyl)carbamoyl)amide | 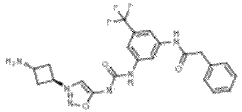 | 474.44 |
| 27 | ((3-(2-(4-(Aminomethyl)phenyl)acetamido)-5-(trifluoromethyl)phenyl)carbamoyl)(3-(pyridin-2-ylmethyl)-1,2,3-oxadiazol-3-ium-5-yl)amide | 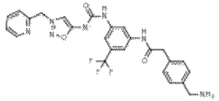 | 525.48 |
| 28 | (3-Benzyl-1,2,3-oxadiazol-3-ium-5-yl)((3-(2-phenylacetamido)-5-(trifluoromethyl)phenyl)-carbamoyl)amide | 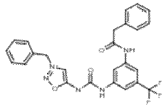 | 495.45 |
| 29 | ((3-(1*H*-Benzo[d]imidazole-5-carboxamido)-5- (trifluoromethyl)phenyl)carbamoyl)(3-(pyridin-2-ylmethyl)-1,2,3-oxadiazol-3-ium-5-yl)amide | 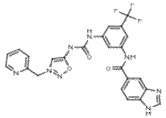 | 522.44 |
| 30 | 5-(3-(3-(5-(Morpholinomethyl)furan-2-carboxamido)-5-(trifluoromethyl)phenyl)ureido)-3-(pyridin-2-ylmethyl)-1,2,3 -oxadiazol-3-ium | 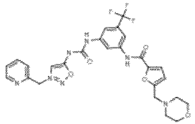 | 572.52 |
| 31 | ((3-(3-Phenoxybenzamido)-5-(trifluoromethyl)-phenyl)carbamoyl)(3-(pyridin-2-ylmethyl)-1,2,3-oxadiazol-3-ium-5-yl)amide | 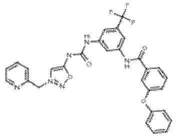 | 574.51 |
| 32 | ((3-(2-Phenylpropanamido)-5-(trifluoromethyl)-phenyl)carbamoyl)(3-(pyridin-2-ylmethyl)-1,2,3-oxadiazol-3-ium-5-yl)amide | 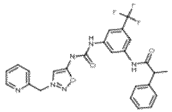 | 510.47 |
| 33 | ((3-(2-(5-Methyl-2-phenyloxazol-4-yl)acetamido)-5-(trifluoromethyl)phenyl)-carbamoyl)(3-(pyridin-2-ylmethyl)-1,2,3-oxadiazol-3-ium-5-yl)amide | 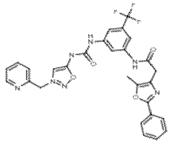 | 577.51 |
| 34 | ((3-(1-Phenyl-1H-pyrazole-5-carboxamido)-5-(trifluoromethyl)phenyl)carbamoyl)(3-(pyridin-2-ylmethyl)-1,2,3-oxadiazol-3-ium-5-yl)amide | 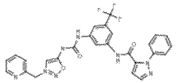 | 548.48 |
| 35 | ((3-(1-Benzyl-1*H*-pyrazole-4-carboxamido)-5-(trifluoromethyl)phenyl)carbamoyl)(3-(pyridin-2-ylmethyl)-1,2,3-oxadiazol-3-ium-5-yl)amide | 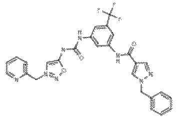 | 562.5 |
| 36 | ((3-(2-(1*H*-Pyrrolo[3,2-b]pyridin-3-yl)acetamido)-5-(trifluoromethyl)phenyl)-carbamoyl)(3-(pyridin-2-ylmethyl)-1,2,3-oxadiazol-3-ium-5-yl)amide | 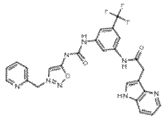 | 536.47 |
| 37 | ((3-(6-Cyanoimidazo[1,2-a]pyridine-2-carboxamido)-5-(trifluoromethyl)-phenyl)carbamoyl)(3-(pyridin-2-ylmethyl)-1,2,3-oxadiazol-3-ium-5-yl)amide | 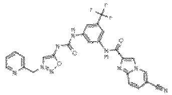 | 547.45 |
| 38 | (3-(Pyridin-2-ylmethyl)-1,2,3-oxadiazol-3-ium-5-yl)((3-(4,5,6,7-tetrahydro-1*H*-indazole-7-carboxamido)-5-(trifluoromethyl)phenyl)-carbamoyl)amide | 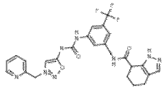 | 526.47 |
| 39 | ((3-(5-Methyl-5,6-dihydro-4*H*-thieno[2,3-c]pyrrole-2-carboxamido)-5-(trifluoromethyl)phenyl)carbamoyl)(3-(pyridin-2-ylmethyl)-1,2,3-oxadiazol-3-ium-5-yl)amide | 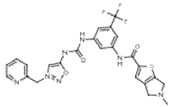 | 543.52 |
| 40 | ((3-(2-(1-Methyl-1*H*-pyrazol-3-yl)acetamido)-5 - (trifluoromethyl)phenyl)carbamoyl)(3-(pyridin-2-ylmethyl)-1,2,3-oxadiazol-3-ium-5-yl)amide | 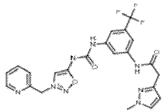 | 500.43 |
| 41 | ((3-(2-(2-Methylthiazol-4-yl)acetamido)-5-(trifluoromethyl)phenyl)-carbamoyl)(3-(pyridin-2-ylmethyl)-1,2,3-oxadiazol-3-ium-5-yl)amide | 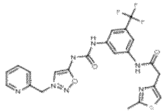 | 517.48 |
| 42 | ((3-(2-(6-Aminopyridin-3-yl)acetamido)-5-(trifluoromethyl)phenyl)carbamoyl)(3-(pyridin-2-ylmethyl)-1,2,3-oxadiazol-3-ium-5-yl)amide | 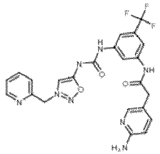 | 512.44 |
| 43 | ((3-(2-(Phenylsulfonyl)acetamido)-5-(trifluoromethyl)phenyl)carbamoyl)(3-(pyridin-2-ylmethyl)-1,2,3-oxadiazol-3-ium-5-yl)amide | 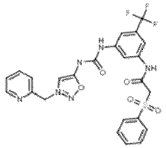 | 560.51 |
| 44 | ((3 -(Isoxazole -3-carboxamido)-5-(trifluoromethyl)-phenyl)carbamoyl)(3-(pyridin-2-ylmethyl)-1,2,3-oxadiazol-3-ium-5-yl)amide | 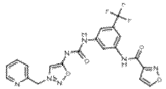 | 473.36 |
| 45 | ((3-(Pyrazine-2-carboxamido)-5-(trifluoromethyl)-phenyl)carbamoyl)(3-(pyridin-2-ylmethyl)-1,2,3-oxadiazol-3-ium-5-yl)amide | 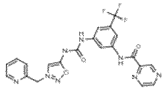 | 484.39 |
| 46 | ((3-(3-Phenylpropiolamido)-5-(trifluoromethyl)-phenyl)carbamoyl)(3-(pyridin-2-ylmethyl)-1,2,3-oxadiazol-3-ium-5-yl)amide | 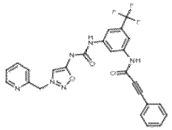 | 506.44 |
| 47 | ((3-(Bicyclo[4.2.0]octa-1(6),2,4-triene-7-carboxamido)-5-(trifluoromethyl)phenyl)-carbamoyl)(3-(pyridin-2-ylmethyl)-1,2,3-oxadiazol-3-ium-5-yl)amide | 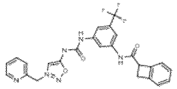 | 508.45 |
| 48 | ((3-(1-Isopropyl-1*H*-pyrazole-3-carboxamido)-5-(trifluoromethyl)phenyl)carbamoyl)(3-(pyridin-2-ylmethyl)-1,2,3-oxadiazol-3-ium-5-yl)amide | 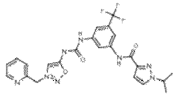 | 514.46 |
| 49 | ((3-(1-Methylazetidine-3 -carboxamido)-5 - (trifluoromethyl)phenyl)carbamoyl)(3-(pyridin-2-ylmethyl)-1,2,3-oxadiazol-3-ium-5-yl)amide | 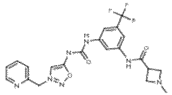 | 475.42 |
| 50 | ((3-(2-Phenylcyclopropane-1-carboxamido)-5-(trifluoromethyl)phenyl)carbamoyl)(3-(pyridin-2-ylmethyl)-1,2,3-oxadiazol-3-ium-5-yl)amide | 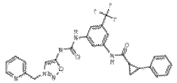 | 522.48 |
| 51 | ((3-(3-(Methoxycarbonyl)bicyclo[1.1.1]pentane-1-carboxamido)-5-(trifluoromethyl)phenyl)-carbamoyl)(3-(pyridin-2-ylmethyl)-1,2,3-oxadiazol-3-ium-5-yl)amide | 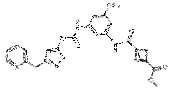 | 530.46 |
| 52 | ((3-(3-Carboxybicyclo[1.1.1]pentane-1-carboxamido)-5-(trifluoromethyl)phenyl)-carbamoyl)(3-(pyridin-2-ylmethyl)-1,2,3-oxadiazol-3-ium-5-yl)amide | 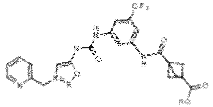 | 516.43 |
| 53 | ((3-(Phenethylamino)-5-(trifluoromethyl)-phenyl)carbamoyl)(3-(pyridin-2-ylmethyl)-1,2,3-oxadiazol-3-ium-5-yl)amide | 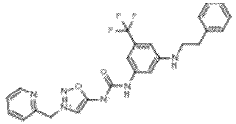 | 482.46 |
| 54 | ((3-(2-Cyclobutylacetamido)-5-(trifluoromethyl)phenyl)carbamoyl)(3-(pyridin-2-ylmethyl)-1,2,3-oxadiazol-3-ium-5-yl)amide | 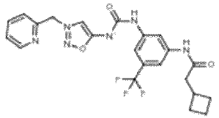 | 474.44 |
| 55 | (3-((1*R*3*R*)-3-(Dimethylamino)cyclobutyl)-1,2,3-oxadiazol-3-ium-5-yl)((3-(2-phenyl-acetamido)-5-(trifluoromethyl)phenyl)-carbamoyl)amide | 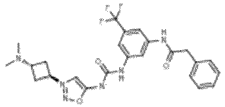 | 502.49 |
| 56 | (3-(4-(Aminomethyl)benzyl)-1,2,3-oxadiazol-3- ium-5-yl)((3-(2-phenylacetamido)-5-(trifluoromethyl)phenyl)carbamoyl)amide | 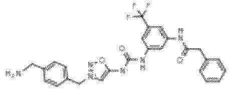 | 524.49 |
| 57 | ((3-Methyl-5-(2-phenylacetamido)phenyl)-carbamoyl)(3-(pyridin-2-ylmethyl)-1,2,3-oxadiazol-3-ium-5-yl)amide | 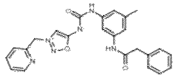 | 442.47 |
| 58 | ((3-Chloro-5-(2-phenylacetamido)phenyl)-carbamoyl)(3-(pyridin-2-ylmethyl)-1,2,3-oxadiazol-3-ium-5-yl)amide | 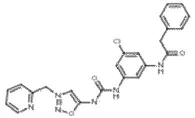 | 463.9 |
| 59 | (3-((1*S*,4*S*)-4-(Aminomethyl)cyclohexyl)-1,2,3-oxadiazol-3-ium-5-yl)((3-(2-phenyl-acetamido)-5-(trifluoromethyl)phenyl)-carbamoyl)amide | 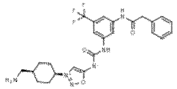 | 516.52 |
| 60 | (3-((1*S*,4*S*)- 4-((1,3-Dioxoisoindolin-2-yl)methyl)cyclohexyl)-1,2,3-oxadiazol-3-ium-5- yl)((3-(2-phenylacetamido)-5-(trifluoromethyl)-phenyl)carbamoyl)amide | 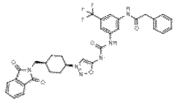 | 646.62 |
| 61 | (3-((1*S*,4*S*)- -4-((Dimethylamino)methyl)cyclohexyl)-1,2,3-oxadiazol-3-ium-5-yl)((3-(2-phenylacetamido)-5-(trifluoromethyl)phenyl)carbamoyl)amide | 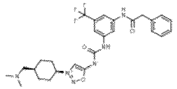 | 544.57 |
| 62 | (3-((1*S*,4*S*)-4-(Acetamidomethyl)cyclohexyl)-1,2,3-oxadiazol-3-ium-5-yl)((3-(2-phenylacetamido)-5-(trifluoromethyl)phenyl)carbamoyl)amide | 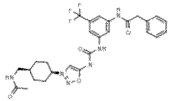 | 558.55 |
| 63 | ((3-(2-(Bicyclo[2.1.1]hexan-2-yl)acetamido)-5-(trifluoromethyl)phenyl)carbamoyl)(3-(pyridin-2-ylmethyl)-1,2,3-oxadiazol-3-ium-5-yl)amide | 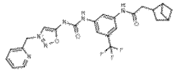 | 500.47 |
| 64 | ((3-(5,5-Dioxido-6,7-dihydro-4*H*-pyrazolo[5,1-c][1,4]thiazine-2-carboxamido)-5-(trifluoromethyl)phenyl)carbamoyl)(3-(pyridin-2-ylmethyl)-1,2,3-oxadiazol-3-ium-5-yl)amide | 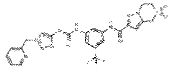 | 576.51 |
| 65 | ((3-(2-(2-(Difluoromethyl)-1H-benzo[d]imidazol-1-yl)acetamido)-5-(trifluoromethyl)phenyl)carbamoyl)(3-(pyridin-2-ylmethyl)-1,2,3-oxadiazol-3-ium-5-yl)amide | 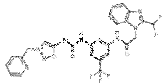 | 586.47 |
| 66 | ((3-(4-Hydroxybicyclo[2.2.1]heptane-1-carboxamido)-5-(trifluoromethyl)phenyl)-carbamoyl)(3-(pyridin-2-ylmethyl)-1,2,3-oxadiazol-3-ium-5-yl)amide | 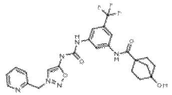 | 516.47 |
| 67 | ((3-(7-Methyl-2,3-dihydrobenzofuran-3-carboxamido)-5-(trifluoromethyl)phenyl)-carbamoyl)(3-(pyridin-2-ylmethyl)-1,2,3-oxadiazol-3-ium-5-yl)amide | 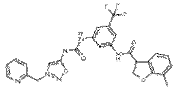 | 538.48 |
| 68 | ((3-(3-Benzylcyclobutane-1-carboxamido)-5-(trifluoromethyl)phenyl)carbamoyl)(3-(pyridin-2-ylmethyl)-1,2,3-oxadiazol-3-ium-5-yl)amide | 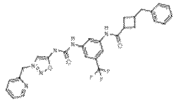 | 550.53 |
| 69 | ((3-((1*S*,3*S*)-3-hydroxy-3-(pyridin-2-yl)cyclobutane-1-carboxamido)-5-(trifluoromethyl)phenyl)carbamoyl)(3-(pyridin-2-ylmethyl)-1,2,3-oxadiazol-3-ium-5-yl)amide | 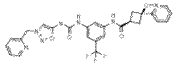 | 553.49 |
| 70 | ((3-(1-((5-Methylfuran-2-yl)methyl)piperidine-4-carboxamido)-5-(trifluoromethyl)phenyl)-carbamoyl)(3-(pyridin-2-ylmethyl)-1,2,3-oxadiazol-3-ium-5-yl)amide | 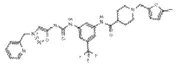 | 583.56 |
| 71 | ((3-(1-(3-Cyano-1*H*-pyrazol-1-yl)cyclopropane-1-carboxamido)-5-(trifluoromethyl)phenyl)carbamoyl)(3-(pyridin-2-ylmethyl)-1,2,3-oxadiazol-3-ium-5-yl)amide | 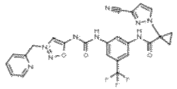 | 537.45 |
| 72 | ((3-(2-((4-Methyl-4*H*-1,2,4-triazol-3-yl)methoxy)acetamido)-5-(trifluoromethyl)-phenyl)carbamoyl)(3-(pyridin-2-ylmethyl)-1,2,3-oxadiazol-3-ium-5-yl)amide | 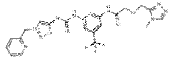 | 531.45 |
| 73 | ((3-(3-(Cyclopentyloxy)propanamido)-5-(trifluoromethyl)phenyl)carbamoyl)(3-(pyridin-2-ylmethyl)-1,2,3-oxadiazol-3-ium-5-yl)amide | 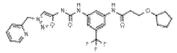 | 518.49 |
| 74 | ((3-((1*S*,4*R*,5*R*)-4-(3-Methoxyphenyl)-2-oxabicyclo[2.1.1]hexane-5-carboxamido)-5-(trifluoromethyl)phenyl)carbamoyl)(3-(pyridin-2-ylmethyl)-1,2,3-oxadiazol-3-ium-5-yl)amide | 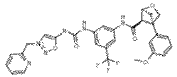 | 594.54 |
| 75 | ((3-(1-Cyclopropyl-1*H*-imidazole-5-carboxamido)-5-(trifluoromethyl)phenyl)-carbamoyl)(3-(pyridin-2-ylmethyl)-1,2,3-oxadiazol-3-ium-5-yl)amide | 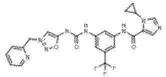 | 512.44 |
| 76 | ((3-(2,2-Difluoro-3-(pyrrolidin-1-yl)propanamido)-5-(trifluoromethyl)phenyl)-carbamoyl)(3-(pyridin-2-ylmethyl)-1,2,3-oxadiazol-3-ium-5-yl)amide | 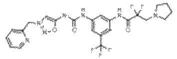 | 539.46 |
| 77 | ((3-(1-Cyclopropylazetidine-3-carboxamido)-5- (trifluoromethyl)phenyl)carbamoyl)(3-(pyridin-2-ylmethyl)-1,2,3-oxadiazol-3-ium-5-yl)amide | 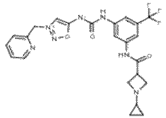 | 501.46 |
| 78 | (3-(Pyridin-2-ylmethyl)-1,2,3-oxadiazol-3-ium-5-yl)((3-(4-(1-sulfamoylethyl)benzamido)-5-(trifluoromethyl)phenyl)carbamoyl)amide | 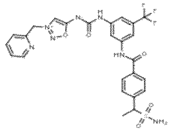 | 589.55 |
| 79 | ((3 -(2,3-Dihydrofuro[3,2-b]pyridine-5- carboxamido)-5-(trifluoromethyl)phenyl)-carbamoyl)(3-(pyridin-2-ylmethyl)-1,2,3-oxadiazol-3-ium-5-yl)amide | 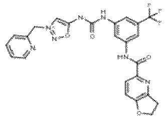 | 525.44 |
| 80 | (3-(Pyridin-2-ylmethyl)-1,2,3-oxadiazol-3-ium-5-yl)((3-(5,6,7,8-tetrahydroimidazo[1,5-a]pyridine-7-carboxamido)-5-(trifluoromethyl) phenyl)carbamoyl)amide | 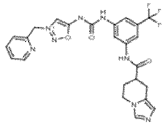 | 526.47 |
| 81 | ((3-(3,4-Dihydro-1*H*-pyrrolo[2,1-c][1,4]oxazine-8-carboxamido)-5-(trifluoromethyl)- phenyl)carbamoyl)(3-(pyridin-2-ylmethyl)-1,2,3-oxadiazol-3-ium-5-yl)amide | 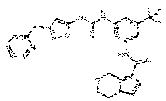 | 527.46 |
| 82 | ((3-(5-(Piperidin-1-ylsulfonyl)pentanamido)-5- (trifluoromethyl)phenyl)carbamoyl)(3-(pyridin-2-ylmethyl)-1,2,3-oxadiazol-3-ium-5-yl)amide | 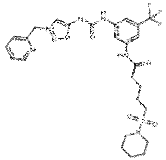 | 609.62 |
| 83 | ((3-(3-(4-Chlorophenoxy)oxetane-3-carboxamido)-5-(trifluoromethyl)phenyl)-carbamoyl)(3-(pyridin-2-ylmethyl)-1,2,3-oxadiazol-3-ium-5-yl)amide | 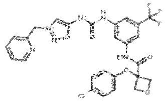 | 588.92 |
| 84 | ((3-(2,6-Dioxaspiro[4.5]decane-7-carboxamido)-5-(trifluoromethyl)phenyl)carbamoyl)(3-(pyridin-2-ylmethyl)-1,2,3-oxadiazol-3-ium-5-yl)amide | 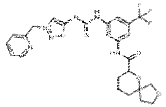 | 546.5 |
| 85 | ((3-(3,3-Difluorospiro[3.3]heptane-1-carboxamido)-5-(trifluoromethyl)phenyl)-carbamoyl)(3-(pyridin-2-ylmethyl)-1,2,3-oxadiazol-3-ium-5-yl)amide | 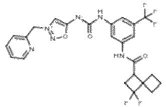 | 536.45 |
| 86 | (3-(Pyridin-2-ylmethyl)-1,2,3-oxadiazol-3-ium-5-yl)((3-(2-(3-(pyrrolidin-1-yl)oxetan-3- yl)acetamido)-5-(trifluoromethyl)phenyl) carbamoyl)amide | 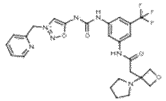 | 545.51 |
| 87 | (3-(Pyridin-2-ylmethyl)-1,2,3-oxadiazol-3-ium-5-yl)((3-(2,4,5,6-tetrahydrocyclopenta[c]pyrrole-1-carboxamido)-5 -(trifluoromethyl)phenyl)-carbamoyl)amide | 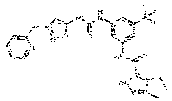 | 511.46 |
| 88 | ((3-(4,4-Dioxido-1,4-oxathiane-2-carboxamido)-5-(trifluoromethyl)phenyl)carbamoyl)(3-(pyridin-2-ylmethyl)-1,2,3-oxadiazol-3-ium-5-yl)amide | 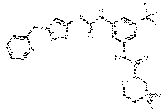 | 540.47 |
| 89 | ((3-Cyclopropyl-5-(2-phenylacetamido)phenyl)-carbamoyl)(3-(pyridin-2-ylmethyl)-1,2,3-oxadiazol-3-ium-5-yl)amide | 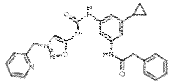 | 468.51 |
| 90 | ((3-(2-(Benzylamino)-2-oxoethyl)-5-(trifluoromethyl)phenyl)carbamoyl)(3-(pyridin-2-ylmethyl)-1,2,3-oxadiazol-3-ium-5-yl)amide | 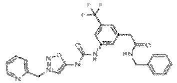 | 510.47 |
| 91 | (3-((1*S*,4*S*)-4-Aminocyclohexyl)-1,2,3-oxadiazol-3-ium-5-yl)((3-(2-phenylacetamido)-5- (trifluoromethyl)phenyl)carbamoyl)amide | 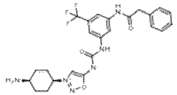 | 502.49 |
| 92 | (3-((1*R*,4*R*)-4-Aminocyclohexyl)-1,2,3-oxadiazol-3-ium-5-yl)((3-(2-phenylacetamido)-5-(trifluoromethyl)phenyl)carbamoyl)amide | 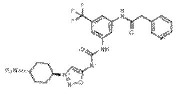 | 502.49 |
| 93 | (3-((1*R*,4*R*)-4-(Aminomethyl)cyclohexyl)-1,2,3-oxadiazol-3-ium-5-yl)((3-(2-phenyl-acetamido)-5-(trifluoromethyl)phenyl)-carbamoyl)amide | 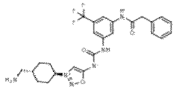 | 516.52 |
| 94 | (3-((1*R*,4*R*)-4-((Dimethylamino)methyl)-cyclohexyl)-1,2,3-oxadiazol-3-ium-5-yl)((3 -(2-phenylacetamido)-5-(trifluoromethyl)phenyl)-carbamoyl)amide | 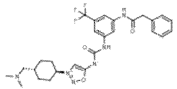 | 544.57 |
| 95 | (3-((1*S*,4*S*)-4-(Dimethylamino)cyclohexyl)-1,2,3-oxadiazol-3-ium-5-yl)((3-(2-phenylacetamido)-5-(trifluoromethyl)phenyl)-carbamoyl)amide | 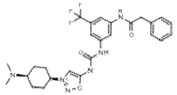 | 530.54 |
| 96 | (3-((1*R*,4*R*)-4-(dimethylamino)cyclohexyl)-1,2,3-oxadiazol-3-ium-5-yl)((3-(2-phenylacetamido)-5-(trifluoromethyl)phenyl)-carbamoyl)amide | 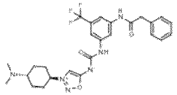 | 530.54 |
| 97 | (3-(2-(Allyloxy)-2-oxoethyl)-1,2,3-oxadiazol-3- ium-5-yl)((3-(2-phenylacetamido)-5-(trifluoromethyl)phenyl)carbamoyl)amide | 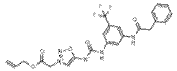 | 503.43 |
| 98 | (3-(4-(Methoxycarbonyl)benzyl)-1,2,3-oxadiazol-3-ium-5-yl)((3-(2-phenylacetamido)-5- (trifluoromethyl)phenyl)carbamoyl)amide | 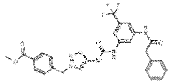 | 553.49 |
| 99 | ((3-(2-Phenylacetamido)-5-(trifluoromethyl)-phenyl)carbamoyl)(3-(piperidin-4-yl)-1,2,3-oxadiazol-3-ium-5-yl)amide | 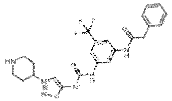 | 488.46 |
| 100 | (3-(1-Methylpiperidin-4-yl)-1,2,3-oxadiazol-3- ium-5-yl)((3-(2-phenylacetamido)-5-(trifluoromethyl)phenyl)carbamoyl)amide | 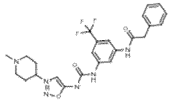 | 501.51 |
| 101 | (3-(4-Carboxybenzyl)-1,2,3-oxadiazol-3-ium-5-yl)((3-(2-phenylacetamido)-5-(trifluoromethyl)-phenyl)carbamoyl)amide | 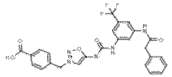 | 539.46 |
| 102 | (3-(4-(Hydroxymethyl)benzyl)-1,2,3-oxadiazol-3- ium-5-yl)((3-(2-phenylacetamido)-5-(trifluoromethyl)phenyl)carbamoyl)amide | 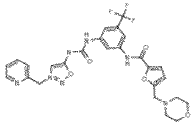 | 525.48 |
| 103 | (3-(4-(Aminomethyl)benzyl)-1,2,3-oxadiazol-3- ium-5-yl)((3-(2-phenylacetamido)-5-(trifluoromethyl)phenyl)carbamoyl)amide | 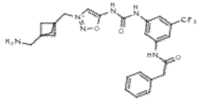 | 515.51 |
| 104 | (3-(4-Carbamoylbenzyl)-1,2,3-oxadiazol-3-ium-5-yl)((3-(2-phenylacetamido)-5- (trifluoromethyl)phenyl)carbamoyl)amide | 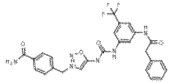 | 538.48 |
| 105 | (3-((1-Methylpiperidin-2-yl)methyl)-1,2,3-oxadiazol-3-ium-5-yl)((3-(2-phenylacetamido)-5-(trifluoromethyl)phenyl)carbamoyl)amide | 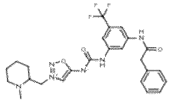 | 516.52 |
| 106 | (3-((1-Methylpiperidin-4-yl)methyl)-1,2,3-oxadiazol-3-ium-5-yl)((3-(2-phenylacetamido)-5-(trifluoromethyl)phenyl)carbamoyl)amide | 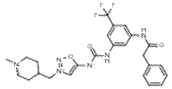 | 516.52 |
| 107 | (3-(3-((1,3-Dioxoisoindolin-2-yl)methyl)benzyl)-1,2,3-oxadiazol-3-ium-5-yl)((3-(2-phenylacetamido)-5-(trifluoromethyl)phenyl)carbamoyl)amide | 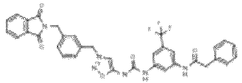 | 654.59 |
| 108 | (3-(3-(Aminomethyl)benzyl)-1,2,3-oxadiazol-3- ium-5-yl)((3-(2-phenylacetamido)-5-(trifluoromethyl)phenyl)carbamoyl)amide | 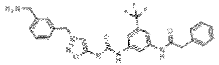 | 524.49 |
| 109 | (3-(4-(2-Methoxy-2-oxoethyl)benzyl)-1,2,3-oxadiazol-3-ium-5-yl)((3-(2-phenylacetamido)-5-(trifluoromethyl)phenyl)carbamoyl)amide | 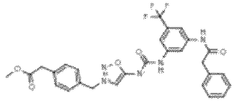 | 567.52 |
| 110 | ((3-(2-Phenylacetamido)-5-(trifluoromethyl)-phenyl)carbamoyl)(3-(piperidin-3-ylmethyl)-1,2,3-oxadiazol-3-ium-5-yl)amide | 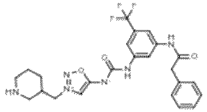 | 502.49 |
| 111 | (3-(4-(2-Hydroxyethyl)benzyl)-1,2,3-oxadiazol-3-ium-5-yl)((3-(2-phenylacetamido)-5-(trifluoromethyl)phenyl)carbamoyl)amide | 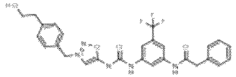 | 539.51 |
| 112 | (3-(9-Amino-3-oxabicyclo[3.3.1]nonan-7-yl)-1,2,3-oxadiazol-3-ium-5-yl)((3-(2-phenylacetamido)-5 -(trifluoromethyl)phenyl)-carbamoyl)amide | 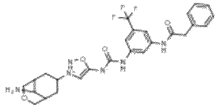 | 544.53 |
| 113 | (3-((5-(Methoxycarbonyl)pyridin-2-yl)methyl)-1,2,3-oxadiazol-3-ium-5-yl)((3-(2-phenylacetamido)-5-(trifluoromethyl)phenyl)carbamoyl)amide | 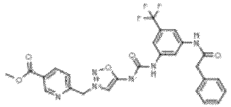 | 554.48 |
| 114 | (3 -(2-(Aminomethyl)benzyl)-1,2,3 -oxadiazol-3 - ium-5-yl)((3-(2-phenylacetamido)-5-(trifluoromethyl)phenyl)carbamoyl)amide | 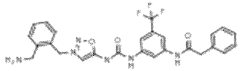 | 524.49 |
| 115 | (3-((1,4-Oxazepan-2-yl)methyl)-1,2,3-oxadiazol-3-ium-5-yl)((3-(2-phenylacetamido)-5-(trifluoromethyl)phenyl)carbamoyl)amide | 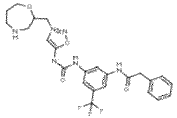 | 518.49 |
| 116 | (3-((5-(Hydroxymethyl)pyridin-2-yl)methyl)-1,2,3-oxadiazol-3-ium-5-yl)((3-(2-phenylacetamido)-5-(trifluoromethyl)phenyl)-carbamoyl)amide | 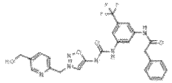 | 526.47 |
| 117 | (3-(((1-Methylpiperidin-3-yl)methyl)-1,2,3-oxadiazol-3-ium-5-yl)((3-(2-phenylacetamido)-5-(trifluoromethyl)phenyl)carbamoyl)amide | 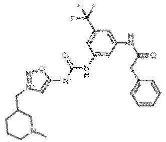 | 516.52 |
| 118 | (3-((3-Aminocyclobutyl)methyl)-1,2,3-oxadiazol-3-ium-5-yl)((3-(2-phenylacetamido)-5-(trifluoromethyl)phenyl)carbamoyl)amide | 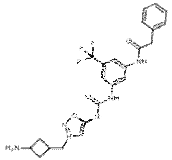 | 488.46 |
| 119 | (3-((1*S*,4*S*)-4-(Aminomethyl)cyclohexyl)-1,2,3-oxadiazol-3-ium-5-yl)((3-(2-oxo-3-phenylpyrrolidin-1-yl)-5-(trifluoromethyl)phenyl)-carbamoyl)amide | 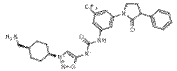 | 542.55 |
| 120 | ((3-(2-Phenylacetamido)-5-(trifluoromethyl)-phenyl)carbamoyl)(3-(piperidin-4-ylmethyl)-1,2,3-oxadiazol-3-ium-5-yl)amide | 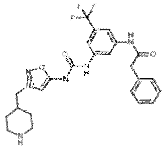 | 502.49 |
| 121 | (3-((5-(Isopropoxycarbonyl)pyridin-2-yl)methyl)-1,2,3-oxadiazol-3-ium-5-yl)((3-(2-phenylacetamido)-5-(trifluoromethyl)phenyl)-carbamoyl)amide | 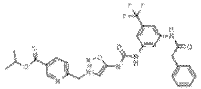 | 582.53 |
| 122 | ((3-(2-Oxo-4-phenylpyrrolidin-1-yl)-5-(trifluoromethyl)phenyl)carbamoyl)(3-(pyridin-2-ylmethyl)-1,2,3-oxadiazol-3-ium-5-yl)amide | 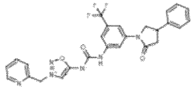 | 522.48 |
| 123 | ((3-(2-(6-Oxo-1,6-dihydropyridin-3-yl)acetamido)-5-(trifluoromethyl)phenyl)-carbamoyl)(3-(pyridin-2-ylmethyl)-1,2,3-oxadiazol-3-ium-5-yl)amide | 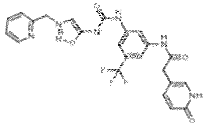 | 513.43 |
| 124 | ((3-(2-(1*H*-Benzo[d]imidazol-6-yl)acetamido)-5-(trifluoromethyl)phenyl)carbamoyl)(3-(pyridin-2-ylmethyl)-1,2,3-oxadiazol-3-ium-5-yl)amide | 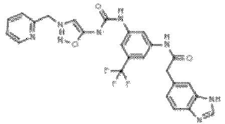 | 536.47 |
| 125 | ((3-(2-Phenylacetamido)-5-(trifluoromethyl)-phenyl)carbamoyl)(3-(piperidin-2-ylmethyl)-1,2,3-oxadiazol-3-ium-5-yl)amide | 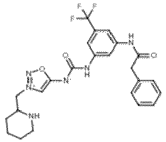 | 502.49 |
| 126 | ((3-(2-(1*H*-Indazol-7-yl)acetamido)-5-(trifluoromethyl)phenyl)carbamoyl)(3-(pyridin-2-ylmethyl)-1,2,3-oxadiazol-3-ium-5-yl)amide | 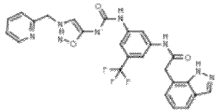 | 536.47 |
| 127 | ((3 -Cyclopropyl-5-(2-phenylacetamido)phenyl)-carbamoyl)(3-((1-methylpiperidin-2-yl)methyl)-1,2,3-oxadiazol-3-ium-5-yl)amide | 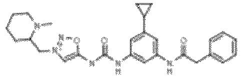 | 488.58 |
| 128 | ((3-Cyclopropyl-5-(2-phenylacetamido)phenyl)-carbamoyl)(3-((5-(methoxycarbonyl)pyridin-2-yl)methyl)-1,2,3-oxadiazol-3-ium-5-yl)amide | 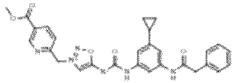 | 526.54 |
| 129 | ((3-Cyclopropyl-5-(2-phenylacetamido)phenyl)-carbamoyl)(3-((5-(hydroxymethyl)pyridin-2-yl)methyl)-1,2,3-oxadiazol-3-ium-5-yl)amide | 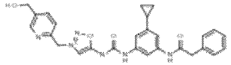 | 498.53 |
| 130 | ((3-((1*R*,2*R*)-2-Methylcyclopropyl)-5-(2-phenylacetamido)phenyl)carbamoyl)(3-(pyridin-2-ylmethyl)-1,2,3-oxadiazol-3-ium-5-yl)amide | 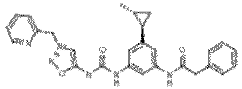 | 482.53 |
| 131 | (3-(((1*S*,4*S*)-4-Aminocyclohexyl)methyl)-1,2,3-oxadiazol-3-ium-5-yl)((3-(2-oxo-3-phenylpyrrolidin-1-yl)-5-(trifluoromethyl)-phenyl)carbamoyl)amide | 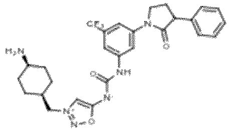 | 542.55 |
| 132 | (3-(((1*R*,4*R*)-4-Aminocyclohexyl)methyl)-1,2,3-oxadiazol-3-ium-5-yl)((3-(2-oxo-3-phenylpyrrolidin-1-yl)-5-(trifluoromethyl)-phenyl)carbamoyl)amide | 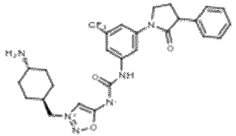 | 542.55 |
| 133 | (3-(((1*S*,4*S*)-4-(Aminomethyl)cyclohexyl)-methyl)-1,2,3-oxadiazol-3-ium-5-yl)((3-(2-oxo-3-phenylpyrrolidin-1-yl)-5 -(trifluoromethyl)-phenyl)carbamoyl)amide | 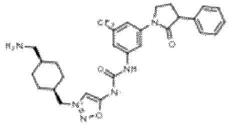 | 556.58 |
| 134 | (3-((3-(((5-Hydroxypyridin-2-yl)methyl)amino)-cyclobutyl)methyl)-1,2,3-oxadiazol-3-ium-5- yl)((3-(2-phenylacetamido)-5-(trifluoromethyl)-phenyl)carbamoyl)amide | 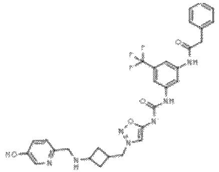 | 595.57 |
| 135 | ((3-(2-Oxo-3-phenylpyrrolidin-1-yl)-5-(trifluoromethyl)phenyl)carbamoyl)(3-((1*S*,4*S*)-4-(((2,2,2-trifluoroethyl)amino)methyl)-cyclohexyl)-1,2,3-oxadiazol-3-ium-5-yl)amide | 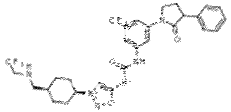 | 624.58 |
| 136 | ((3-(2-Oxo-3-phenylpyrrolidin-1-yl)-5-(trifluoromethyl)phenyl)carbamoyl)(3-(((1s,4s)-4-((2,2,2-trifluoroethyl)amino)cyclohexyl)methyl)-1,2,3-oxadiazol-3-ium-5-yl)amide | 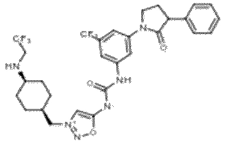 | 624.58 |
| 137 | ((3-(2-Oxo-3-phenylpyrrolidin-1-yl)-5-(trifluoromethyl)phenyl)carbamoyl)(3-(((1*R*,4*R*)-4-((2,2,2-trifluoroethyl)amino)-cyclohexyl)methyl)-1,2,3-oxadiazol-3-ium-5-yl)amide | 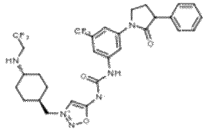 | 624.58 |
| 138 | (3-((1S,4S)-4-((Dimethylamino)methyl)-cyclohexyl)- 1,2,3 -oxadiazol-3 -ium-5-yl)((3 -(2-oxo-3-phenylpyrrolidin-1-yl)-5 -(trifluoromethyl)-phenyl)carbamoyl)amide | 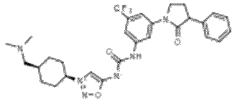 | 570.61 |
| 139 | ((3-(2-Oxo-3-phenylpyrrolidin-1-yl)-5-(trifluoromethyl)phenyl)carbamoyl)(3-(((1s,4s)-4-(((2,2,2-trifluoroethyl)amino)methyl)cyclohexyl)-methyl)-1,2,3-oxadiazol-3-ium-5-yl)amide | 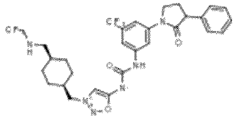 | 638.6 |
| 140 | (3-((1-(2-Ethoxy-2-oxoethyl)piperidin-4-yl)methyl)-1,2,3-oxadiazol-3-ium-5-yl)((3-(2-phenylacetamido)-5-(trifluoromethyl)phenyl)-carbamoyl)amide | 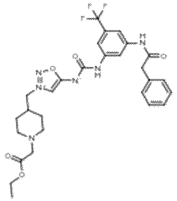 | 588.58 |
| 141 | (3-((1-(2-Hydroxyethyl)piperidin-4-yl)methyl)-1,2,3-oxadiazol-3-ium-5-yl)((3-(2-phenylacetamido)-5-(trifluoromethyl)phenyl)-carbamoyl)amide | 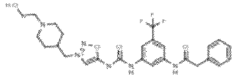 | 546.54 |
| 142 | ((3-(2-Phenylacetamido)-5-(trifluoromethyl)-phenyl)carbamoyl)(3-((1-(2,2,2-trifluoroethyl)-piperidin-2-yl)methyl)-1,2,3-oxadiazol-3-ium-5-yl)amide | 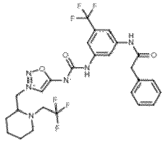 | 584.51 |
| 143 | (3-(((1*R*,4*R*)-4-Aminocyclohexyl)methyl)-1,2,3-oxadiazol-3-ium-5-yl)((3-((*R*)-2-oxo-3-phenylpyrrolidin-1-yl)-5-(trifluoromethyl)-phenyl)carbamoyl)amide | 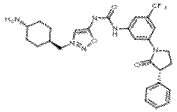 | 542.55 |
| 144 | ((3-((*R*)-2-Oxo-3-phenylpyrrolidin-1-yl)-5- (trifluoromethyl)phenyl)carbamoyl)(3-(((1r,4r)-4-((2,2,2-trifluoroethyl)amino)cyclohexyl)-methyl)-1,2,3-oxadiazol-3-ium-5-yl)amide | 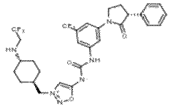 | 624.58 |
| 145 | ((3-((*R*)-2-Oxo-3-phenylpyrrolidin-1-yl)-5- (trifluoromethyl)phenyl)carbamoyl)(3-(((1*R*,4*R*)-4-(2,2,2-trifluoroacetamido)cyclohexyl)methyl)-1,2,3-oxadiazol-3-ium-5-yl)amide | 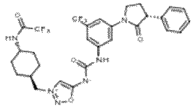 | 639.57 |
| 146 | (3-(((1*R*,4*R*)-4-(Methyl(2,2,2-trifluoroethyl)-amino)cyclohexyl)methyl)-1,2,3-oxadiazol-3-ium-5-yl)((3-((*R*)-2-oxo-3-phenylpyrrolidin-1-yl)-5-(trifluoromethyl)phenyl)carbamoyl)amide | 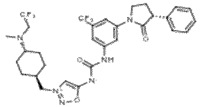 | 639.61 |
| 147 | (3-((1*R*,4*R*)-4-((Dimethylamino)methyl)-cyclohexyl)-1,2,3-oxadiazol-3-ium-5-yl)((3-(2-phenylacetamido)-5-(trifluoromethyl)phenyl)-carbamoyl)amide | 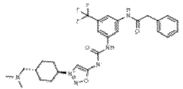 | 544.56865 |
| 148 | (3-((1*R*,4*R*)-4-((Dimethylamino)methyl)-cyclohexyl)-1,2,3-oxadiazol-3-ium-5-yl)((3-(2-oxo-3-phenylpyrrolidin-1-yl)-5-(trifluoromethyl)-phenyl)carbamoyl)amide | 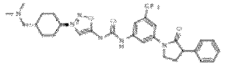 | 570.60593 |
| 149 | (3-((5-(4,6-Dimethylpyrimidin-5-yl)pyridin-2-yl)methyl)-1,2,3-oxadiazol-3-ium-5-yl)((3-(2-phenylacetamido)-5-(trifluoromethyl)phenyl)-carbamoyl)amide | 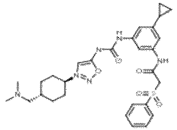 | 580.69834 |
| 150 | ((3-Cyclopropyl-5-(2-(phenylsulfonyl)-acetamido)phenyl)carbamoyl)(3-((1*R*,4*R*)-4-((dimethylamino)methyl)cyclohexyl)-1,2,3-oxadiazol-3-ium-5-yl)amide | 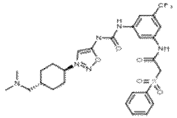 | 608.63245 |
| 151 | (3-((1*R*,4*R*)-4-((Dimethylamino)methyl)-cyclohexyl)-1,2,3-oxadiazol-3-ium-5-yl)((3-(2-(phenylsulfonyl)acetamido)-5- (trifluoromethyl)phenyl)carbamoyl)amide | 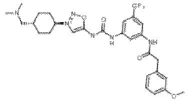 | 574.59463 |
| 152 | (3-((1*R*,4*R*)-4-((Dimethylamino)methyl)-cyclohexyl)-1,2,3-oxadiazol-3-ium-5-yl)((3-(2-(3- methoxyphenyl)acetamido)-5-(trifluoromethyl)- phenyl)carbamoyl)amide | 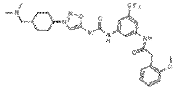 | 574.59463 |
| 153 | (3-((1*R*,4*R*)-4-((Dimethylamino)methyl)-cyclohexyl)-1,2,3-oxadiazol-3-ium-5-yl)((3-(2-(2-methoxyphenyl)acetamido)-5-(trifluoromethyl)- phenyl)carbamoyl)amide | 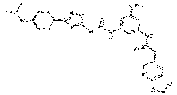 | 588.57815 |
| 154 | ((3-(2-(Benzo[d][1,3]dioxol-5-yl)acetamido)-5- (trifluoromethyl)phenyl)carbamoyl)(3-((1*R*,4*R*)-4-((dimethylamino)methyl)cyclohexyl)-1,2,3-oxadiazol-3-ium-5-yl)amide | 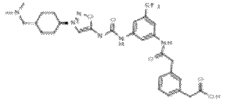 | 602.60473 |
| 155 | ((3-(2-(3-(Carboxymethyl)phenyl)acetamido)-5-(trifluoromethyl)phenyl)carbamoyl)(3-((1*R*,4*R*)-4-((dimethylamino)methyl)cyclohexyl)-1,2,3-oxadiazol-3-ium-5-yl)amide | 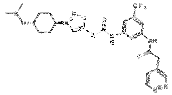 | 546.54477 |
| 156 | ((3-(2-(4-Carboxyphenyl)acetamido)-5-(trifluoromethyl)phenyl)carbamoyl)(3-((1*R*,4*R*)-4-((dimethylamino)methyl)cyclohexyl)-1,2,3-oxadiazol-3-ium-5-yl)amide | 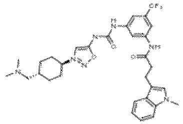 | 611.65785 |
| 157 | (3-((1*R*,4*R*)-4-((Dimethylamino)methyl)-cyclohexyl)-1,2,3-oxadiazol-3-ium-5-yl)((3-(3-(1-methyl-1*H*-indol-3-yl)propanamido)-5-(trifluoromethyl)phenyl)carbamoyl)amide | 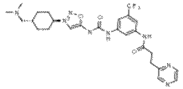 | 560.57135 |
| 158 | (3-((1R,4R)-4-((Dimethylamino)methyl)-cyclohexyl)-1,2,3-oxadiazol-3-ium-5-yl)((3-(3- (pyrazin-2-yl)propanamido)-5-(trifluoromethyl-)phenyl)carbamoyl)amide | 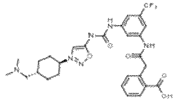 | 588.57815 |
| 159 | ((3-(2-(2-Carboxyphenyl)acetamido)-5-(trifluoromethyl)phenyl)carbamoyl)(3-((1*R*,4*R*)-4-((dimethylamino)methyl)cyclohexyl)-1,2,3-oxadiazol-3-ium-5-yl)amide | 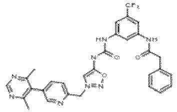 | 602.56651 |
| 160 | ((3-(2,2-Difluoro-3-(pyrrolidin-1-yl)propanamido)-5-(trifluoromethyl)phenyl)-carbamoyl)(3-((1*R*,4*R*)-4-((dimethylamino)-methyl)cyclohexyl)-1,2,3-oxadiazol-3-ium-5-yl)amide | 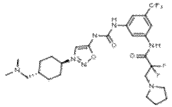 | 587.58528 |
| 161 | (3-((1*R*,4*R*)-4-((dimethylamino)methyl)-cyclohexyl)-1,2,3-oxadiazol-3-ium-5-yl)((3-(2-(1-methyl-1*H*-indol-3-yl)acetamido)-5-(trifluoromethyl)phenyl)carbamoyl)amide | 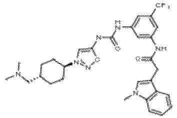 | 597.63127 |
| 162 | ((3-(2-(3-(2-Amino-2-oxoethyl)phenyl)-acetamido)-5-(trifluoromethyl)phenyl)-carbamoyl)(3-((1*R*,4*R*)-4-((dimethylamino)-methyl)cyclohexyl)-1,2,3-oxadiazol-3-ium-5-yl)amide | 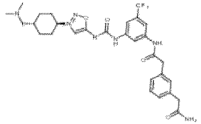 | 601.61997 |
| 163 | ((3-(2-(3-(Aminomethyl)phenyl)acetamido)-5- (trifluoromethyl)phenyl)carbamoyl)(3-((1*R*,4*R*)-4-((dimethylamino)methyl)cyclohexyl)-1,2,3-oxadiazol-3-ium-5-yl)amide | 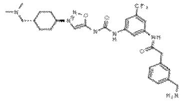 | 573.60987 |
| 164 | (3-((1*R*,4*R*)-4-((Dimethylamino)methyl)-cyclohexyl)-1,2,3-oxadiazol-3-ium-5-yl)((3-(2-phenylpropanamido)-5-(trifluoromethyl)phenyl)-carbamoyl)amide | 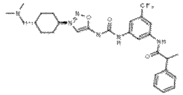 | 558.59523 |
| 165 | (3-((1*R*,4*R*)-4-((Dimethylamino)methyl)-cyclohexyl)-1,2,3-oxadiazol-3-ium-5-yl)((3-(2-phenylbutanamido)-5-(trifluoromethyl)phenyl)-carbamoyl)amide | 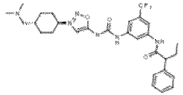 | 572.62181 |
| 166 | (3-((1*R*,4*R*)-4-((Dimethylamino)methyl)-cyclohexyl)-1,2,3-oxadiazol-3-ium-5-yl)((3- (trifluoromethyl)-5-(2-(2-(trifluoromethyl)-phenyl)acetamido)phenyl)carbamoyl)amide | 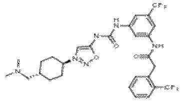 | 612.56662 |
| 167 | (3-((1R,4R)-4-((Dimethylamino)methyl)-cyclohexyl)-1,2,3-oxadiazol-3-ium-5-yl)((3-(*N-*methyl-2-phenylpropanamido)-5-(trifluoromethyl)phenyl)carbamoyl)amide | 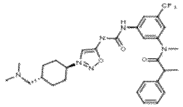 | 572.62181 |
| 168 | ((3-(2-(2-Chlorophenyl)acetamido)-5-(trifluoromethyl)phenyl)carbamoyl)(3-((1*R*,4*R*)-4-((dimethylamino)methyl)cyclohexyl)-1,2,3-oxadiazol-3-ium-5-yl)amide | 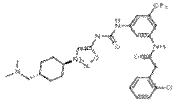 | 579.01371 |
| 169 | ((3-(2-(2,3-Difluorophenyl)acetamido)-5-(trifluoromethyl)phenyl)carbamoyl)(3-((1*R*,4*R*)-4-((dimethylamino)methyl)cyclohexyl)-1,2,3-oxadiazol-3-ium-5-yl)amide | 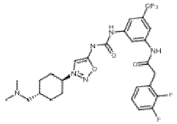 | 580.54958 |
| 170 | (3-((1*R*,4*S*)-4-((Dimethylamino)methyl)-cyclohexyl)-1,2,3-oxadiazol-3-ium-5-yl)((3-((*S*)-2-phenylpropanamido)-5-(trifluoromethyl)-phenyl)carbamoyl)amide | 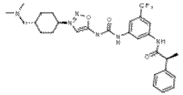 | 558.59523 |
| 171 | (3-((1*R*,4*R*)-4-((Dimethylamino)methyl)-cyclohexyl)-1,2,3-oxadiazol-3-ium-5-yl)((3-(2-(*o*-tolyl)acetamido)-5-(trifluoromethyl)phenyl)-carbamoyl)amide | 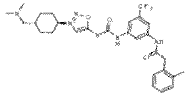 | 558.59523 |
| 172 | (3-((1*R*,4*R*)-4-((Dimethylamino)methyl)-cyclohexyl)-1,2,3-oxadiazol-3-ium-5-yl)((3-(*N-*methyl-2-phenylacetamido)-5-(trifluoromethyl)-phenyl)carbamoyl)amide | 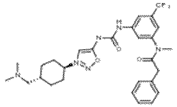 | 558.59523 |
| 173 | (3-((1*R*,3*R*)-3-(Dimethylamino)cyclohexyl)-1,2,3-oxadiazol-3-ium-5-yl)((3-(2-phenylacetamido)-5 -(trifluoromethyl)phenyl)-carbamoyl)amide | 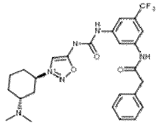 | 530.54207 |
| 174 | (3-((1*R*,3*S*)-3-(Dimethylamino)cyclohexyl)-1,2,3-oxadiazol-3-ium-5-yl)((3-(2-phenyl-acetamido)-5-(trifluoromethyl)phenyl)-carbamoyl)amide | 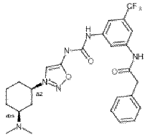 | 530.54207 |
| 175 | (3-((1*R*,4*R*)-4-((Dimethylamino)methyl)-cyclohexyl)-1,2,3-oxadiazol-3-ium-5-yl)((3-((*R*)-2-phenylpropanamido)-5-(trifluoromethyl)-phenyl)carbamoyl)amide | 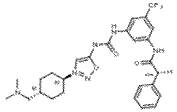 | 558.59523 |
| 176 | ((3-(2-(3-Chlorophenyl)acetamido)-5- (trifluoromethyl)phenyl)carbamoyl)(3-((1*R*,4*R*)-4-((dimethylamino)methyl)cyclohexyl)-1,2,3-oxadiazol-3-ium-5-yl)amide | 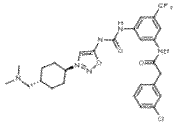 | 579.01371 |
| 177 | (3-((1*R*,4*R*)-4-((Dimethylamino)methyl)-cyclohexyl)-1,2,3-oxadiazol-3-ium-5-yl)((3- (1,2,3,4-tetrahydronaphthalene-1-carboxamido)-5-(trifluoromethyl)phenyl)carbamoyl)amide | 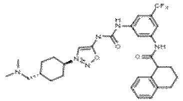 | 584.63251 |
| 178 | rac-(3-((1-Methyl-1,2,3,4-tetrahydroquinolin-2-yl)methyl)-1,2,3-oxadiazol-3-ium-5-yl)((2-(trifluoromethyl)pyridin-4-yl)carbamoyl)amide | 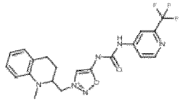 | 432.40 |
| 179 | (3-(((1S,4S)-4-(Pyrimidin-5-yl)cyclohexyl)-methyl)-1,2,3-oxadiazol-3-ium-5-yl)((3- (trifluoromethyl)phenyl)carbamoyl)amide | 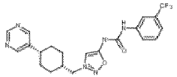 | 446.42 |
| 180 | rac-(((3*R*,5*S*)-1,1-Difluorospiro[2.4]heptan-5-yl)carbamoyl)(3-((5-(4,6-dimethylpyrimidin-5-yl)pyridin-2-yl)methyl)-1,2,3-oxadiazol-3-ium-5-yl)amide | 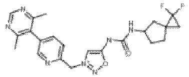 | 455.46 |
| 181 | rac-(3-((2-Methyl-1,2,3,4-tetrahydroisoquinolin- 1-yl)methyl)-1,2,3-oxadiazol-3-ium-5-yl)((2-(trifluoromethyl)pyridin-4-yl)carbamoyl)amide | 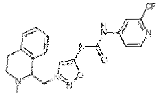 | 432.40 |
| 182 | (3-(((1*S*,4*S*)-4-(Pyrimidin-5-yl)cyclohexyl) methyl)-1,2,3-oxadiazol-3-ium-5-yl)((2-(trifluoro methyl)pyridin-4-yl)carbamoyl)amide | 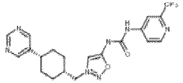 | 447.41 |
| 183 | (3-((3-(Pyrimidin-5-yl)bicyclo[1.1.1]pentan-1-yl)methyl)-1,2,3-oxadiazol-3-ium-5-yl)((3- (trifluoromethyl)phenyl)carbamoyl)amide | 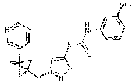 | 455.46 |
| 184 | (3-((6-(4,6-Dimethylpyrimidin-5-yl)pyridazin-3-yl)methyl)-1,2,3-oxadiazol-3-ium-5-yl)((3- (trifluoromethyl)phenyl)carbamoyl)amide | 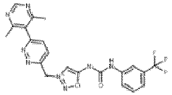 | 470.40 |
| 185 | (3-((4-(Pyrimidin-5-yl)bicyclo[2.2.1]heptan-1-yl)methyl)-1,2,3-oxadiazol-3-ium-5-yl)((2-(trifluoromethyl)pyridin-4-yl)carbamoyl)amide | 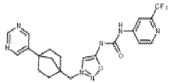 | 459.42 |
| 186 | (3-(((1*R*,4*R*)-4-(Pyrimidin-5-yl)cyclohexyl) methyl)-1,2,3-oxadiazol-3-ium-5-yl)((3-(trifluoro methyl)phenyl)carbamoyl)amide | 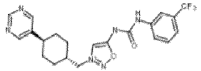 | 446.42 |
| 187 | (3-(((1*R*,4*R*)-4-(Pyrimidin-5-yl)cyclohexyl)-methyl)-1,2,3-oxadiazol-3-ium-5-yl)((2-(trifluoromethyl)pyridin-4-yl)carbamoyl)amide | 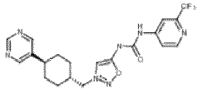 | 447.41 |
| 188 | (3-(4-(3-Methylpyridazin-4-yl)benzyl)-1,2,3-oxadiazol-3-ium-5-yl)((2-(trifluoromethyl)-pyridin-4-yl)carbamoyl)amide | 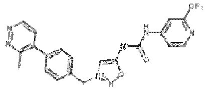 | 455.39 |
| 189 | ((2-(Trifluoromethyl)pyridin-4-yl)carbamoyl)(3-(4-(4-(trifluoromethyl)pyrimidin-5-yl)benzyl)-1,2,3-oxadiazol-3-ium-5-yl)amide | 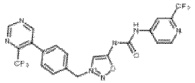 | 509.36 |
| 190 | (3-(4-(4-Methoxy-6-methylpyrimidin-5-yl)benzyl)-1,2,3-oxadiazol-3-ium-5-yl)((2-(trifluoromethyl)pyridin-4-yl)carbamoyl)amide | 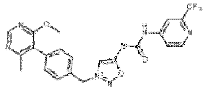 | 485.41 |
| 191 | (3-((*1R*,*4R*)-4-((Dimethylamino)methyl)-cyclohexyl)-1,2,3 -oxadiazol-3 -ium-5-yl)((3 -(2-(2-hydroxyphenyl)acetamido)-5-(trifluoromethyl)-phenyl)carbamoyl)amide | 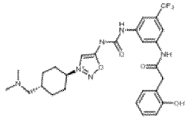 | 560.56 |
| 192 | rac-(3-((2-Methyl-1,2,3,4-tetrahydro isoquinolin-1-yl)methyl)-1,2,3-oxadiazol-3-ium-5-yl)((3-(2-phenylacetamido)-5-(trifluoromethyl) phenyl)carbamoyl)amide | 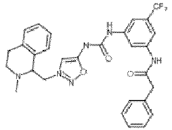 | 564.59 |
| 193 | ((2-(2-(2-Chlorophenyl)acetamido)-6-(trifluoromethyl)pyridin-4-yl)carbamoyl)(3-((1*R*,4*R*)-4-((dimethylamino)methyl)-cyclohexyl)-1,2,3-oxadiazol-3-ium-5-yl)amide | 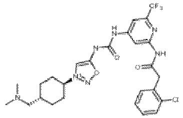 | 580.00 |
| 194 | (3-((1*R*,4*R*)-4-((Dimethylamino)methyl)-cyclohexyl)-1,2,3-oxadiazol-3-ium-5-yl)((3-(2-fluoro-2-(2-fluorophenyl)acetamido)-5-(trifluoromethyl)phenyl)carbamoyl)amide | 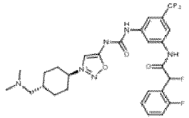 | 580.54 |
| 195 | ((3-(Bicyclo[4.2.0]octa-1(6),2,4-triene-7-carboxamido)-5-(trifluoromethyl)phenyl)-carbamoyl)(3-((1*R*,4*R*)-4-((dimethylamino-methyl)cyclohexyl)-1,2,3-oxadiazol-3-ium-5-yl)amide | 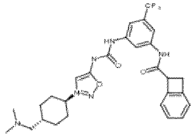 | 556.57 |
| 196 | (3-((1*R*,4*R*)-4-((Dimethylamino)methyl)-cyclohexyl)-1,2,3-oxadiazol-3-ium-5-yl)((3-(2-(6-hydroxypyridin-2-yl)acetamido)-5-(trifluoromethyl)phenyl)carbamoyl)amide | 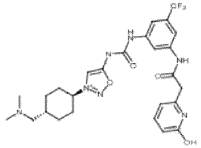 | 561.55 |
| 197 | (3-((1*R*,4*R*)-4-((Dimethylamino)methyl)-cyclohexyl)-1,2,3-oxadiazol-3-ium-5-yl)((3-(2-(3,5-dimethylisoxazol-4-yl)acetamido)-5-(trifluoromethyl)phenyl)carbamoyl)amide | 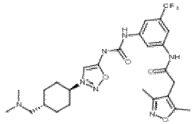 | 563.57 |
| 198 | ((3 -(2-(2-Chloro-6-fluorophenyl)acetamido)-5- (trifluoromethyl)phenyl)carbamoyl)(3-((1*R*,4*R*)-4-((dimethylamino)methyl)cyclohexyl)-1,2,3-oxadiazol-3-ium-5-yl)amide | 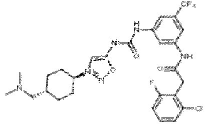 | 597.01 |
| 199 | ((3-(2-(2,6-Difluorophenyl)acetamido)-5-(trifluoromethyl)phenyl)carbamoyl)(3-((1*R*,4*R*)-4-((dimethylamino)methyl)cyclohexyl)-1,2,3-oxadiazol-3-ium-5-yl)amide | 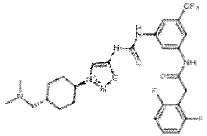 | 580.55 |
| 200 | Rac-(3-((1,2,3,4-Tetrahydronaphthalen-1-yl)methyl)-1,2,3-oxadiazol-3-ium-5-yl)((2-(trifluoromethyl)pyridin-4-yl)carbamoyl)amide | 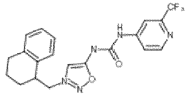 | 417.38 |
| 201 | (3-(4-(1,4-Dimethyl-1*H*-pyrazol-5-yl)benzyl)-1,2,3-oxadiazol-3-ium-5-yl)((2-(trifluoromethyl)-pyridin-4-yl)carbamoyl)amide | 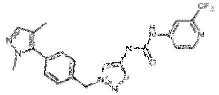 | 457.40 |
| 202 | (3-((1*R*,3*S*)-3-(Dimethylamino)cyclohexyl)-1,2,3-oxadiazol-3-ium-5-yl)((3-(2-(*o*-tolyl)acetamido)-5-(trifluoromethyl)phenyl)-carbamoyl)amide | 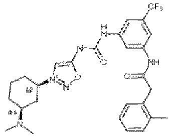 | 544.59 |
| 203 | (3-((1*R*,2*R*)-2-Hydroxycyclohexyl)-1,2,3-oxadiazol-3-ium-5-yl)((3-(2-(*o*-tolyl)acetamido)-5-(trifluoromethyl)phenyl)carbamoyl)amide | 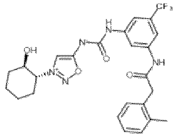 | 517.500 |
| 204 | (3-((1*R*,3*S*)-3-(Dimethylamino)cyclohexyl)-1,2,3-oxadiazol-3-ium-5-yl)((2-(trifluoromethyl)-pyridin-4-yl)carbamoyl)amide | 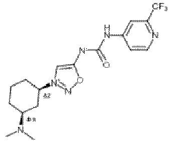 | 398.38 |
| 205 | ((3 -(2-(2-Chlorophenyl)propanamido)-5- (trifluoromethyl)phenyl)carbamoyl)(3-((1*R*,4*R*)-4-((dimethylamino)methyl)cyclohexyl)-1,2,3-oxadiazol-3-ium-5-yl)amide | 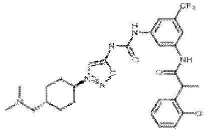 | 593.04 |
| 206 | (3-((1*R*,4*R*)-4-(2-Hydroxypropan-2-yl)cyclohexyl)-1,2,3-oxadiazol-3-ium-5-yl)((3-(2-(*o*-tolyl)acetamido)-5-(trifluoromethyl)phenyl)-carbamoyl)amide | 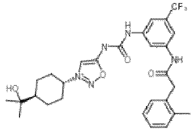 | 559.58 |
| 207 | ((4-Cyano-3-(trifluoromethyl)phenyl)-carbamoyl)(3-(4-(4,6-dimethylpyrimidin-5-yl)benzyl)-1,2,3-oxadiazol-3-ium-5 -yl)amide | 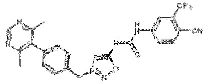 | 493.44 |
| 208 | (3-((1*R*,4*R*)-4-((Dimethylamino)methyl)-cyclohexyl)-1,2,3-oxadiazol-3-ium-5-yl)((3-(2-methyl-2-phenylpropanamido)-5-(trifluoromethyl)phenyl)carbamoyl)amide | 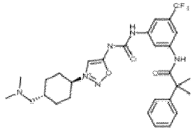 | 572.62 |
| 209 | (3-(4-(4,6-Dimethylpyrimidin-5-yl)benzyl)-1,2,3-oxadiazol-3-ium-5-yl)((2-methoxypyridin-4-yl)carbamoyl)amide | 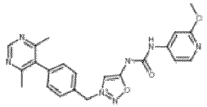 | 431.45 |
| 210 | (3-(4-(2-Methylthiazol-4-yl)benzyl)-1,2,3-oxadiazol-3-ium-5-yl)((2-(trifluoromethyl)pyridin -4-yl)carbamoyl)amide | 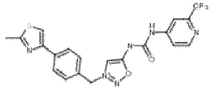 | 460.43 |
| 211 | (3-(4-(3,5-Dimethylisothiazol-4-yl)benzyl)-1,2,3-oxadiazol-3-ium-5-yl)((2-(trifluoromethyl)-pyridin-4-yl)carbamoyl)amide | 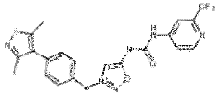 | 474.46 |
| 212 | (3-((1*R*,4*R*)-4-((Dimethylamino)methyl)-cyclohexyl)-1,2,3-oxadiazol-3-ium-5-yl)((3-(2-(2,3-dimethylphenyl)acetamido)-5-(trifluoromethyl)phenyl)carbamoyl)amide | 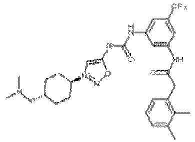 | 572.62 |
| 213 | (3-((1*S*,3*R*)-3-((Dimethylamino)methyl)-cyclohexyl)-1,2,3-oxadiazol-3-ium-5-yl)((3-(2-(o-tolyl)acetamido)-5-(trifluoromethyl)phenyl) carbamoyl)amide | 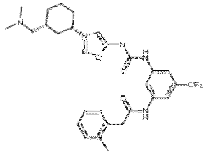 | 558.59 |
| 214 | (3-((1*R*,3*S*)-3-Hydroxycyclohexyl)-1,2,3-oxadiazol-3-ium-5-yl)((3-(2-(*o*-tolyl)acetamido)-5-(trifluoromethyl)phenyl)carbamoyl)amide | 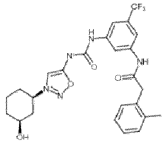 | 517.500 |
| 215 | (3-((1*R*,3*R*)-3-Hydroxycyclohexyl)-1,2,3-oxadiazol-3-ium-5-yl)((3-(2-(*o*-tolyl)acetamido)-5-(trifluoromethyl)phenyl)carbamoyl)amide | 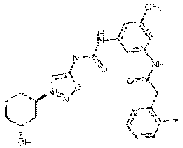 | 517.500 |
| 216 | (3-((4-(4,6-Dimethylpyrimidin-5-yl)cyclohex-3- en-1-yl)methyl)-1,2,3-oxadiazol-3-ium-5-yl)((2-(trifluoromethyl)pyridin-4-yl)carbamoyl)amide | 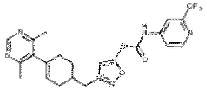 | 473.45 |
| 217 | (3-(((1*R*,2*R*)-2Hydroxycyclohexyl)methyl)-1,2,3-oxadiazol-3-ium-5-yl)((3-(2-(*o*-tolyl)acetamido)-5-(trifluoromethyl)phenyl)-carbamoyl)amide | 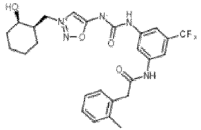 | 531.53 |
| 218 | (3-(((1*S*,2*R*)-2-Hydroxycyclohexyl)methyl)-1,2,3-oxadiazol-3-ium-5-yl)((3-(2-(*o*-tolyl)acetamido)-5-(trifluoromethyl)phenyl)-carbamoyl)amide | 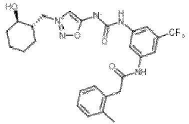 | 531.53 |
| 219 | (3-((1*S*,3*S*)-3-(Pyrimidin-5-yl)cyclohexyl)-1,2,3-oxadiazol-3-ium-5-yl)((2-(trifluoromethyl)pyridin -4-yl)carbamoyl)amide | 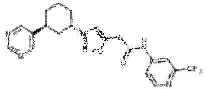 | 433.39 |
| 220 | (3-((1*S*,3*R*)-3-(Pyrimidin-5-yl)cyclohexyl)-1,2,3-oxadiazol-3-ium-5-yl)((2-(trifluoromethyl)-pyridin-4-yl)carbamoyl)amide | 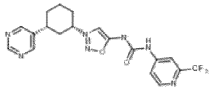 | 433.39 |
| 221 | (3-((1*S*,3*S*)-3-(Pyrimidin-5-yl)cyclohexyl)-1,2,3-oxadiazol-3-ium-5-yl)((3-(2-(*o*-tolyl)acetamido)-5-(trifluoromethyl)phenyl)carbamoyl)amide | 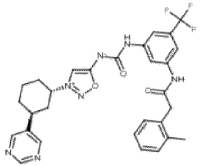 | 579.58 |
| 222 | (3-((1*S*,3*R*)-3-(Pyrimidin-5-yl)cyclohexyl)-1,2,3-oxadiazol-3-ium-5-yl)((3-(2-(*o*-tolyl)acetamido)-5-(trifluoromethyl)phenyl)-carbamoyl)amide | 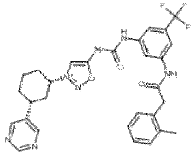 | 579.57 |
| 223 | (3-((1*R*,3*S*)-3-(Methoxycarbonyl)cyclohexyl)-1,2,3-oxadiazol-3-ium-5-yl)((3-(2-(*o-*tolyl)acetamido)-5-(trifluoromethyl)phenyl)-carbamoyl)amide | 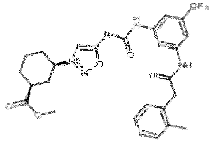 | 559.54 |
| 224 | (3-((1*R*,3*R*)-3-(Methoxycarbonyl)cyclohexyl)-1,2,3-oxadiazol-3-ium-5-yl)((3-(2-(*o*-tolyl)-acetamido)-5-(trifluoromethyl)phenyl)-carbamoyl)amide | 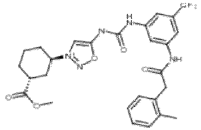 | 559.54 |
| 225 | (3-(4-(4-Methylpyrimidin-5-yl)benzyl)-1,2,3-oxadiazol-3-ium-5-yl)((2-(trifluoromethyl)pyridin -4-yl)carbamoyl)amide | 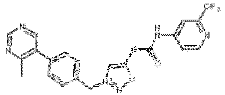 | 455.39 |
| 226 | ((2-(*tert*-Butyl)pyridin-4-yl)carbamoyl)(3-(4-(4,6-dimethylpyrimidin-5 -yl)benzyl)-1,2,3 -oxadiazol-3-ium-5-yl)amide | 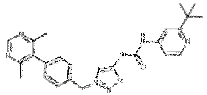 | 457.52 |
| 227 | (3-((1*R*,4*R*)-4-(Methoxycarbonyl)cyclohexyl)-1,2,3-oxadiazol-3-ium-5-yl)((3-(2-(*o*-tolyl)-acetamido)-5-(trifluoromethyl)phenyl)carbamoyl) amide | 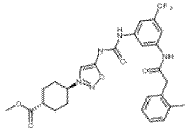 | 559.54 |
| 228 | (3-((1*R*,4*R*)-4-(Hydroxymethyl)cyclohexyl)-1,2,3-oxadiazol-3-ium-5-yl)((3-(2-(o-tolyl)acetamido)-5-(trifluoromethyl)phenyl) carbamoyl)amide | 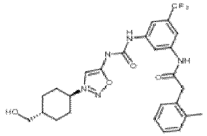 | 531.52 |
| 229 | (3-((1*R*,4*R*)-4-Carboxycyclohexyl)-1,2,3-oxadiazol-3-ium-5-yl)((3-(2-(*o*-tolyl)acetamido)-5-(trifluoromethyl)phenyl)carbamoyl)amide | 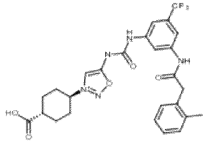 | 545.51 |
| 230 | (3-((1*R*)-3-(Hydroxymethyl)cyclohexyl)-1,2,3-oxadiazol-3-ium-5-yl)((3-(2-(*o*-tolyl)acetamido)-5-(trifluoromethyl)phenyl)carbamoyl)amide | 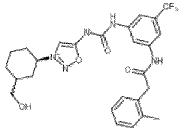 | 531.53 |
| 231 | (3-((1*R*,3*R*)-3-(Methylcarbamoyl)cyclohexyl)-1,2,3-oxadiazol-3-ium-5-yl)((3-(2-(*o-*tolyl)acetamido)-5-(trifluoromethyl)phenyl)-carbamoyl)amide | 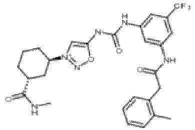 | 558.55 |
| 232 | (3-((1*R*,3*S*)-3-(Methylcarbamoyl)cyclohexyl)-1,2,3-oxadiazol-3-ium-5-yl)((3-(2-(*o-*tolyl)acetamido)-5-(trifluoromethyl)phenyl)-carbamoyl)amide | 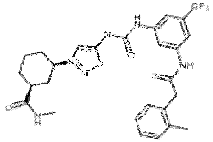 | 558.55 |
| 233 | (3-(4-(4,6-Dimethylpyrimidin-5 -yl)benzyl)-1,2,3-oxadiazol-3-ium-5-yl)(((1*S*,3*S*)-3-(trifluoromethyl)cyclopentyl)carbamoyl)amide | 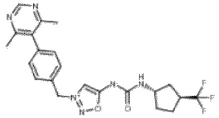 | 460.45 |
| 234 | (3-((1*R*,3*S*)-3-(Dimethylcarbamoyl)cyclohexyl)-1,2,3-oxadiazol-3-ium-5-yl)((3-(2-(*o*-tolyl)-acetamido)-5-(trifluoromethyl)phenyl)-carbamoyl)amide | 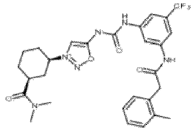 | 572.59 |
| 235 | (3-((1*R*,3*R*)-3-Carbamoylcyclohexyl)-1,2,3-oxadiazol-3-ium-5-yl)((3-(2-(*o*-tolyl)acetamido)-5-(trifluoromethyl)phenyl)carbamoyl)amide | 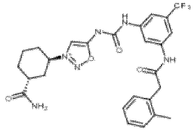 | 544.52 |
| 236 | (3-(4-(Pyrimidin-5-yl)benzyl)-1,2,3-oxadiazol-3-ium-5-yl)((2-(trifluoromethyl)pyridin-4-yl)-carbamoyl)amide | 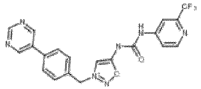 | 441.37 |
| 237 | (3-(4-(1,3-Dimethyl-1*H*-pyrazol-4-yl)benzyl)-1,2,3-oxadiazol-3-ium-5-yl)((2-(trifluoromethyl)-pyridin-4-yl)carbamoyl)amide | 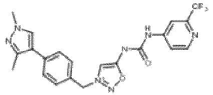 | 457.41 |
| 238 | ((4-Cyano-3-(trifluoromethyl)phenyl)-carbamoyl)(3-(4-(1,4-dimethyl-1*H*-pyrazol-5-yl)benzyl)-1,2,3-oxadiazol-3-ium-5-yl)amide | 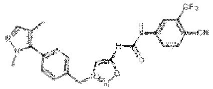 | 457.41 |
| 239 | (3-((4-(4-Methyl-2-(oxetan-3-yl)pyrazol-3-yl]phenyl)methyl)oxadiazol-3-ium-5-yl)-((2-(trifluoromethyl)pyridin-4-yl)carbamoyl)-azanide | 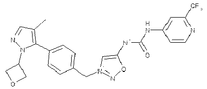 | 499.44 |
| 240 | (3-((1*S*,2*R*)-2-Hydroxycyclohexyl)-1,2,3-oxadiazol-3-ium-5-yl)((3-(2-(*o*-tolyl)acetamido)-5-(trifluoromethyl)phenyl)carbamoyl)amide | 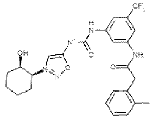 | 517.50 |
| 241 | (3-(((1*R*,4*S*)-4-(4-Methylpyrimidin-5-yl)cyclohexyl)methyl)-1,2,3-oxadiazol-3-ium-5-yl)((2-(trifluoromethyl)pyridin-4-yl)carbamoyl)-amide | 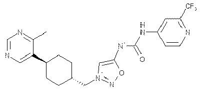 | 461.44 |
| 242 | (3-(4-(1,4-Dimethyl-1*H*-pyrazol-5-yl)benzyl)-1,2,3-oxadiazol-3-ium-5-yl)((2-(trifluoromethyl)-pyridin-4-yl)carbamoyl)amide | 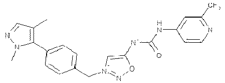 | 457.40 |
| 243 | (3-((1*R*,4*S*)-4-((Dimethylamino)methyl)-cyclohexyl)-1,2,3-oxadiazol-3-ium-5-yl)((3-((*S*)-2-fluoro-2-(2-fluorophenyl)acetamido)-5-(trifluoromethyl)phenyl)carbamoyl)amide | 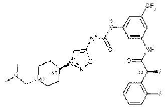 | 580.55 |
| 244 | rac-(3-((1*R*,2*S*)-2-(Dimethylamino)cyclohexyl)-1,2,3-oxadiazol-3-ium-5-yl)((3-(2-(*o-*tolyl)acetamido)-5-(trifluoromethyl)phenyl)-carbamoyl)amide | 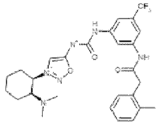 | 544.57 |
| 245 | rac-(3-((1*R*,3*S*)-3-(Methylcarbamoyl)cyclohexyl)-1,2,3-oxadiazol-3-ium-5-yl)((3-(2-(*o-*tolyl)acetamido)-5-(trifluoromethyl)phenyl)carbamoyl)amide | 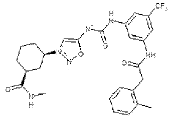 | 558.55 |
| 246 | rac-(3-((1*S*,3*R*)-3-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide | 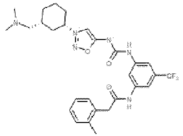 | 558.60 |
| 247 | rac-(3-((1*R*,3*S*)-3-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide | 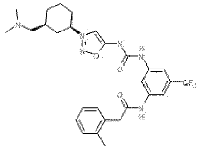 | 558.593 |
| 248 | (3-((1*R*,4*R*)-4-(Dimethylcarbamoyl)-cyclohexyl)-1,2,3-oxadiazol-3-ium-5-yl)((3-(2-(*o-*tolyl)acetamido)-5-(trifluoromethyl)phenyl)-carbamoyl)amide | 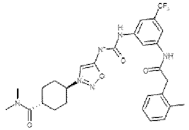 | 572.58 |
| 249 | (3-(4-(4,6-Dimethylpyrimidin-5-yl)benzyl)-1,2,3-oxadiazol-3-ium-5-yl)((4-methoxy-3-(trifluoromethyl)phenyl)carbamoyl)amide | 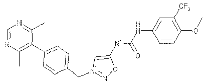 | 498.46 |
| 250 | (3-(4-(4-Methyl-1-(oxetan-3-yl)-1*H*-pyrazol-3-yl)benzyl)-1,2,3-oxadiazol-3-ium-5-yl)((2-(trifluoromethyl)pyridin-4-yl)carbamoyl)amide | 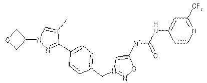 | 499.44 |
| 251 | rac-(3-((1*R*,4*R*)-4-(Methylcarbamoyl)-cyclohexyl)-1,2,3-oxadiazol-3-ium-5-yl)((3-(2-(*o-*tolyl)acetamido)-5-(trifluoromethyl)phenyl)-carbamoyl)amide | 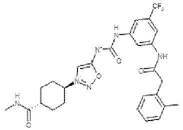 | 558.55 |
| 252 | rac-(3-((1*R*,3*R*)-3-(Methylcarbamoyl)-cyclohexyl)-1,2,3-oxadiazol-3-ium-5-yl)((3-(2-(*o-*tolyl)-acetamido)-5-(trifluoromethyl)phenyl)-carbamoyl)amide | 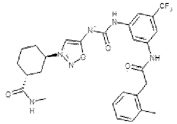 | 558.55 |
| 253 | rac-(3-((1*R*,3*S*)-3-(Dimethylcarbamoyl)-cyclohexyl)-1,2,3-oxadiazol-3-ium-5-yl)((3-(2-(*o-*tolyl)-acetamido)-5-(trifluoromethyl)phenyl)-carbamoyl)amide | 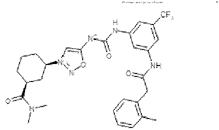 | 572.58 |
| 254 | (3-((1*R*,4*R*)-4-(Pyrrolidine-1-carbonyl)-cyclohexyl)-1,2,3-oxadiazol-3-ium-5-yl)((3-(2-(*o-*tolyl)acetamido)-5-(trifluoromethyl)phenyl)-carbamoyl)amide | 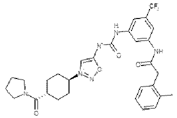 | 598.62 |
| 255 | (3-((1*R*,3*S*)-3-Carbamoylcyclohexyl)-1,2,3-oxadiazol-3-ium-5-yl)((3-(2-(*o*-tolyl)acetamido)-5-(trifluoromethyl)phenyl)carbamoyl)amide | 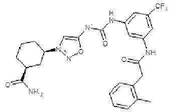 | 544.52 |
| 256 | (3-((4-(4,6-Dimethylpyrimidin-5- yl)phenyl)methyl)oxadiazol-3-ium-5-yl)-((rac-(1*R*,3*R*)-3-(trifluoromethyl)cyclopentyl)-carbamoyl)azanide | 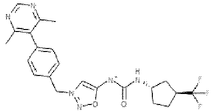 | 460.45 |
| 257 | (3-((1*R*,4*R*)-4-(Dimethylamino)cyclohexyl)-1,2,3-oxadiazol-3-ium-5-yl)((3-(2-(*o*-tolyl)-acetamido)-5-(trifluoromethyl)phenyl)-carbamoyl)amide | 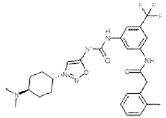 | 544.57 |
| 258 | (3-(4-(2-Methylpyridin-3-yl)benzyl)-1,2,3-oxadiazol-3-ium-5-yl)((2-(trifluoromethyl)pyridin -4-yl)carbamoyl)amide | 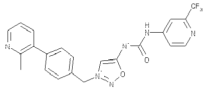 | 454.40 |
| 259 | (3-(4-(2,5-Dimethylthiazol-4-yl)benzyl)-1,2,3-oxadiazol-3-ium-5-yl)((2-(trifluoromethyl)pyridin -4-yl)carbamoyl)amide | 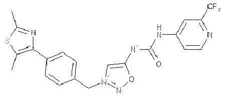 | 474.46 |
| 260 | (3-(4-(2-Amino-4-methylpyrimidin-5-yl)benzyl)-1,2,3-oxadiazol-3-ium-5-yl)((2-(trifluoromethyl)pyridin-4-yl)carbamoyl)amide | 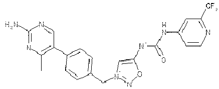 | 470.41 |
| 261 | (3-(4-(4,6-Dimethylpyrimidin-5-yl)benzyl)-1,2,3-oxadiazol-3-ium-5-yl)((4-hydroxy-3-(trifluoromethyl)phenyl)carbamoyl)amide | 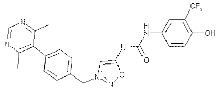 | 484.43 |
| 262 | (3-(4-(4,6-Dimethylpyrimidin-5-yl)benzyl)-1,2,3-oxadiazol-3-ium-5-yl)((4-(methoxy-carbonyl)-3-(trifluoromethyl)phenyl)carbamoyl) amide | 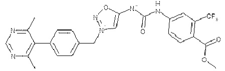 | 526.47 |
| 263 | (3-(4-(2,4-Dimethylpyridin-3-yl)benzyl)-1,2,3- oxadiazol-3-ium-5-yl)((2-(trifluoromethyl)pyridin -4-yl)carbamoyl)amide | 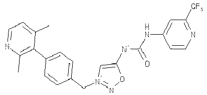 | 468.43 |
| 264 | ((4-Acetamido-3-(trifluoromethyl)phenyl)-carbamoyl)(3-(4-(4,6-dimethylpyrimidin-5-yl)benzyl)-1,2,3-oxadiazol-3-ium-5-yl)amide | 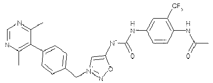 | 525.48 |
| 265 | (3-(3-(Hydroxymethyl)cyclohexyl)-1,2,3-oxadiazol-3-ium-5-yl)((3-(2-(*o*-tolyl)acetamido)-5-(trifluoromethyl)phenyl)carbamoyl)amide | 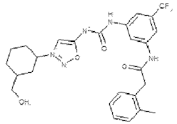 | 531.53 |
| 266 | (3-(4-(Pyrimidin-5-yl)benzyl)-1,2,3-oxadiazol-3-ium-5-yl)((2-(trifluoromethyl)pyridin-4-yl)carbamoyl)amide | 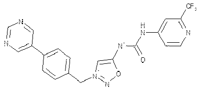 | 441.37 |
| 267 | (3-((1*R*,3*R*)-3-Carbamoylcyclohexyl)-1,2,3-oxadiazol-3-ium-5-yl)((3-(2-(*o*-tolyl)acetamido)-5-(trifluoromethyl)phenyl)carbamoyl)amide | 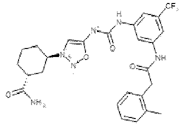 | 544.52 |
| 268 | ((2-(Trifluoromethyl)pyridin-4-yl)carbamoyl)(3-(4-(1,3,5-trimethyl-1*H*-pyrazol-4-yl)benzyl)-1,2,3 -oxadiazol-3 -ium-5 -yl)amide | 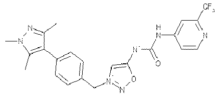 | 471.43 |
| 269 | (3-(4-(4-Methoxy-6-methylpyrimidin-5-yl)benzyl)-1,2,3-oxadiazol-3-ium-5-yl)((2-(trifluoromethyl)pyridin-4-yl)carbamoyl)amide | 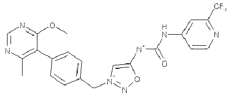 | 485.42 |
| 270 | ((2-(Trifluoromethyl)pyridin-4-yl)carbamoyl)(3-(4-(4-(trifluoromethyl)pyrimidin-5-yl)benzyl)-1,2,3-oxadiazol-3 -ium-5-yl)amide | 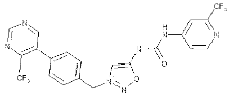 | 509.36 |
| 271 | (3-((1*R*,4*R*)-4-((Dimethylamino)methyl)-cyclohexyl)-1,2,3-oxadiazol-3-ium-5-yl)((3-((*R*)-2-fluoro-2-(2-fluorophenyl)acetamido)-5-(trifluoromethyl)phenyl)carbamoyl)amide | 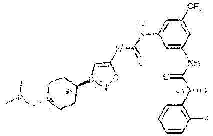 | 580.55 |
| 272 | (3 -(4-(4,6-Dimethylpyrimidin-5 -yl)benzyl)-1,2,3-oxadiazol-3-ium-5-yl)((8-(trifluoromethyl)-quinolin-6-yl)carbamoyl)amide | 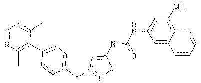 | 519.48 |
| 273 | (3-(4-(4,6-Dimethylpyrimidin-5-yl)benzyl)-1,2,3-oxadiazol-3-ium-5-yl)((4-(methyl-carbamoyl)-3-(trifluoromethyl)phenyl)carbamoyl) amide | 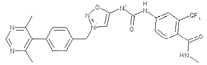 | 525.48 |

### Biology

### ZIKV protease expression and purification

The sequence coding for NS2B (aa 49-95, R95A) and NS3 (aa 1-170, R29G) were synthesized (GenScript, Piscataway, New Jersey, United States), cloned in pET15b vector downstream the sequence for 6 x His tag and fused by a not cleavable linker (G₄SG₄). The protease was expressed in *E. coli* BL21 (DE3) cells: bacteria were grown in LB medium at 37 °C up to 0.8 O.D. 600, then the temperature was lowered to 20 °C and protein expression induced with 0.5 mM IPTG (cat. N. I5502, Sigma Aldrich) for 18 h. Cells were collected by centrifugation (4000 *x g*, 4 °C for 30 min), then re-suspended (7 ml/g of cell paste) in lysis buffer (25 mM Tris pH 8.0, 100 mM NaCl, 5% glycerol) supplemented with 0.7 mg/ml of lysozyme (cat. N. 16876, Sigma Aldrich) and 25 U/ml of benzonase (cat. N. E1014, Sigma Aldrich). After incubating at 25 °C for 30 min, cells were homogenized at 800 bars by PANDA homogenizer (GEA) then cell extract was cleared by centrifugation (32914 *x* g, 4 °C, 1h), brought to 500 mM NaCl then loaded on 1 ml HisTrap HP column (cat. N. 17-52407-01, Cytiva, Sweden) equilibrated in 25 mM Tris pH 8.5, 500 mM NaCl, 5% glycerol. After three wash steps at 20, 30 and 50 mM imidazole, recombinant protein was eluted by a linear gradient up to 350 mM imidazole (cat. N. I202, Sigma Aldrich). Fractions containing ZIKV protease were pooled and treated with 2 mM TCEP (cat. N. C4706, Sigma Aldrich) at 4 °C for 40 min, then concentrated threefold on Vivaspin 20, 3 KDa cut off device (cat. N. Z614610-48EA, Sigma Aldrich) and loaded on HiLoad 16/60 Superdex 75 column (cat. N. 28989333, Cytiva, Sweden) using 25 mM Tris pH 8.5, 5% glycerol and 1 mM DTT (cat. N. A2948, PanReac AppliChem) as mobile phase. Monomer peak fractions were collected, divided in small aliquots and frozen in liquid nitrogen.

### DEN2V protease expression and purification

The expression of DEN2V serine protease was performed as previously described (Acta Cryst. (2006). F62, 157-162), with few changes. The sequence coding for NS2B (aa 1394-1440, Uniprot Q91H74) and NS3 (aa 1476-1660, Uniprot Q91H74) were synthesized (GenScript, Piscataway, New Jersey, United States), cloned in pET15b vector downstream the sequence for 6 x His tag and fused by a not cleavable linker (G4SG4). The protease was expressed in E. coli BL21 (DE3) cells: bacteria were grown in MDG minimal not inducing medium at 25 °C for 24 h, with shaking at 300 RPM. Then the bacterial starter culture was diluted 1:20 in ZYM-5052 auto-inducing medium and let grow for additional 24 h at 25 °C with 200 RPM. Cells were collected by centrifugation (4000 x g, 4 °C for 30 min), then re-suspended (7 ml/g of cell paste) in lysis buffer (20 mM Tris pH 8.5, 50 mM NaCl, 5% glycerol) supplemented with 0.7 mg/ml of lysozyme (cat. N. 16876, Sigma Aldrich) and 25 U/ml of benzonase (cat. N. E1014, Sigma Aldrich). After incubating at 25 °C for 30 min, cells were homogenized at 800 bar by PANDA homogenizer (GEA) then cell extract was cleared by centrifugation (32914 x g, 4 °C, 1 h), brought to 500 mM NaCl and loaded on 5 ml HisTrap HP column (cat. N. 17525501, Cytiva, Sweden) equilibrated in 20 mM Tris pH 8.5, 500 mM NaCl, 5% glycerol. After two wash steps at 10 and 20 mM imidazole, recombinant protein was eluted by a linear gradient up to 440 mM imidazole (cat. N. I202, Sigma Aldrich). Fractions containing DEN2V protease were pooled, concentrated threefold on Vivaspin 20, 3 KDa cut off device (cat. N. Z614610-48EA, Sigma Aldrich) and loaded on HiLoad 16/60 Superdex 75 column (cat. N. 28989333, Cytiva, Sweden) using 20 mM Tris pH 8.5, 50 mM NaCl, 5 % glycerol as mobile phase. Monomer peak fractions were collected, divided in small aliquots and frozen in liquid nitrogen. Similarly, the DEN1V, DEN3V and DEN4V NS2B-G4SG4-NS3 proteases were produced Viva Biotech Ltd^{®}.

### JEV protease expression and purification

The expression of JEV serine protease was performed as previously described (PLoS ONE 7(5): e36872), with few changes. The sequence coding for NS2B (aa 51-95, JEV genotype III strain JaOArS 982, Genebank accession number: M18370) and NS3 (aa 1-180, JEV genotype III strain JaOArS 982, Genebank accession number: M18370) were synthesized (GenScript, Piscataway, New Jersey, United States), cloned in pET15b vector downstream the sequence for 6 x His tag and fused by a cleavable linker derived from NS2B sequence (aa 121-131). The protease was expressed in *E. coli* BL21 (DE3) cells: bacteria were grown in MDG minimal not inducing medium at 25 °C for 24 h, with shaking at 300 RPM. Then the bacterial starter culture was diluted 1:20 in ZYM-5052 auto-inducing medium and let grow for additional 24 h at 25 °C with 200 RPM. Cells were collected by centrifugation (4000 *xg,* 4 °C for 30 min), then re-suspended (7 ml/g of cell paste) in lysis buffer (25 mM Tris pH 8.5, 100 mM NaCl, 5% glycerol) supplemented with 0.7 mg/ml of lysozyme (cat. N. 16876, Sigma Aldrich) and 25 U/ml of benzonase (cat. N. E1014, Sigma Aldrich). After incubating at 25 °C for 30 min, cells were homogenized at 800 bars by PANDA homogenizer (GEA) then cell extract was cleared by centrifugation (32914 x g, 4 °C, 1 h), brought to 500 mM NaCl then loaded on 5 ml HisTrap HP column (cat. N. 17525501, Cytiva, Sweden) equilibrated in 25 mM Tris pH 8.5, 500 mM NaCl, 5% glycerol. After two wash steps at 10 and 30 mM imidazole, recombinant protein was eluted by a linear gradient up to 500 mM imidazole (cat. N. I202, Sigma Aldrich). Fractions containing JEV protease were pooled then treated with 2 mM TCEP (cat. N. C4706, Sigma Aldrich) at 4 °C for 40 min, finally concentrated threefold on Vivaspin 20, 3 KDa cut off device (cat. N. Z614610-48EA, Sigma Aldrich) and loaded on HiLoad 16/60 Superdex 75 column (cat. N. 28989333, Cytiva, Sweden) using 25 mM Tris pH 9.0, 5% glycerol, 1 mM TCEP as mobile phase. Monomer peak fractions were collected, divided in small aliquots and frozen in liquid nitrogen.

### ZIKV protease inhibition assay

The enzyme inhibition assay was performed in 384-well flat bottom black polystyrene microplates (cat. N. 781900, Greiner,) in a reaction volume of 20 µl. ZIKV NS2B-G₄SG₄-NS3 serine protease (MGS SHHHHHHS SGLVPRGSHMVDMYIERAGDITWEKDAEVTGNSPRLDVALDESGDF SLVEDDGPPMAGGGGSGGGGSGALWDVPAPKEVKKGETTDGVYRVMTRGLLGSTQV GVGVMQEGVFHTMWHVTKGSALRSGEGRLDPYWGDVKQDLVSYCGPWKLDAAWD GHSEVQLLAVPPGERARNIQTLPGIFKTKDGDIGAVALDYPAGTSGSPILDKCGRVIGLY GNGVVIKNGSYVSAITQGRR; SEQ. ID. No. 1) (1.25 nM) was incubated with increasing inhibitor concentrations (0.097-100 µM or 0.0009 -1 µM for the most active compounds) in assay buffer (50 mM Tris-HCl, pH 8.5, 1% glycerol, 1 mM CHAPS, 1% DMSO) for 10 minutes at 25 °C. 10 µM of Bz-Nle-KRR-AMC substrate (cat. N. 4055312, Bachem) was added and the reaction mixture incubated for additional 30 minutes at 25 °C. Product formation was evaluated by fluorescence measurement (excitation 360 nm, emission 465 nM), using a SPARK TM10 Tecan instrument. Results were analysed using Prism (GraphPad, San Diego, CA) and Vortex software (Dotmatics, Bioshops Stortford, UK). Dose-response curves were fitted by four-parameter logistic regression.

### DEN2V protease inhibition assay

The enzyme inhibition assay was performed in 384-well flat bottom black polystyrene microplates (cat. N. 781900, Greiner,) in a reaction volume of 20 µl. DEN2V NS2B-G4SG4-NS3 serine protease (MGSSHHHHHHSSGLVPRGSHMADLELERAADVRWEEQAEISGSSPILSITISED GSMSIKNEEEEQTLGGGGSGGGGAGVLWDVPSPPPVGKAELEDGAYRIKQKGILGYSQI GAGVYKEGTFHTMWHVTRGAVLMHKGKRIEPSWADVKKDLISYGGGWKLEGEWKE GEEVQVLALEPGKNPRAVQTKPGLFKTNTGTIGAVSLDFSPGTSGSPIVDKKGKVVGLY GNGVVTRSGAYVSAIAQTEKSIEDNPEIEDDIFRK; SEQ ID No 2) (10 nM) was incubated with increasing inhibitor concentrations (0.097-100 µM or 0.0009-1 µM for the most active compounds) in assay buffer (50 mM Tris-HCl, pH 8.5, 1% glycerol, 1 mM CHAPS, 1% DMSO) for 10 minutes at 25 °C. 15 µM of Bz-Nle-KRR-AMC substrate (cat. N. 4055312, Bachem) was added and the reaction mixture incubated for additional 30 minutes at 25 °C. Product formation was evaluated by fluorescence measurement (excitation 360 nm, emission 465 nM), using a SPARK TM10 Tecan instrument. Results were analyzed using Prism (GraphPad, San Diego, CA) and Vortex software (Dotmatics, Bioshops Stortford, UK). Dose-response curves were fitted by four-parameter logistic regression.

### DEN1V protease inhibition assay

The enzyme inhibition assay was performed in a similar manner to the DEN2V assay using DEN1V NS2B-G4SG4NS3 serine protease

(ADLSLEKAAEVSWEEEAEHSGASHNILVEVQDDGTMKIKDEERDDTGGGGSGGGGSG
VLWDTPSPPEVERAVLDDGIYRIMQRGLLGRSQVGVGVFQDGVFHTMWHVTRGAVLM
YQGKRLEPSWASVKKDLISYGGGWRFQGSWNTGEEVQVIAVEPGKNPKNVQTAPGTF
KTPEGEVGAIALDFKPGTSGSPIVNREGKIVGLYGNGVVTTSGTYVSAIAQAKASQEGPL
PEIEDEVFRK; SEQ ID No. 3) (10 nM).

### DEN3V protease inhibition assay

The enzyme inhibition assay was performed in a similar manner to the DEN2V assay using DEN3V NS2B-G4SG4NS3 serine protease

(DLTVEKAADVTWEEEAEQTGVSHNLMITVDDDGTMRIKDDETENILGGGGSGGGGSG

VLWDVPSPPETQKAELEEGVYRIKQQGIFGKTQVGVGVQKEGVFHTMWHVTRGAVLT
HNGKRLEPNWASVKKDLISYGGGWRLSAQWQKGEEVQVIAVEPGKNPKNFQTMPGIF
QTTTGEIGAIALDFKPGTSGSPIINREGKVVGLYGNGVVTKNGGYVSGIAQTNAEPDGPT
PELEEE; SEQ ID No. 4) (10 nM).

### DEN4V protease inhibition assay

The enzyme inhibition assay was performed in a similar manner to the DEN2V assay using DEN4V NS2B-G4SG4NS3 serine protease

(ADLSLEKAANVQWDEMADITGSSPIIEVKQDEDGSFSIRDVEETNMIGGGGSGGGGSG
ALWDVPSPAAAQKATLTEGVYRIMQRGLFGKTQVGVGIHMEGVFHTMWHVTRGSVIC
HESGRLEPSWADVRNDMISYGGGWRLGDKWDKEEDVQVLAIEPGKNPKHVQTKPGLF
KTLTGEIGAVTLDFKPGTSGSPIINKKGKVIGLYGNGVVTKSGDYVSAITQAERIGEPDY
EVDEDIFRK; SEQ ID No. 5) (5 nM).

### WNV protease inhibition assay

The enzyme inhibition assay was performed in 384-well flat bottom black polystyrene microplates (cat. N. 781900, Greiner,) in a reaction volume of 20 µl. West Nile Virus (WNV) NS2B-NS3 protease, MHHHHHH (SEQ ID No 6; NS2B₁₄₂₃₋₁₄₇₀) - GGGGSGGGG (SEQ ID No 7; NS3₁₅₀₆₋₁₆₈₉) Accession # YP_001527877.1, R&D, cat. N. 2907-SE-020, (2 nM) was incubated with increasing inhibitor concentrations (0.097-100 µM or 0.0009-1 µM for the most active compounds) in assay buffer (50 mM Tris-HCl, pH 9.0, 30% glycerol, 1% DMSO) for 10 minutes at 25 °C. 10 µM of pERTKR-AMC substrate (R&D, cat. N. ES013) was added and the reaction mixture incubated for additional 30 minutes at 25 °C. Product formation was evaluated by fluorescence measurement (excitation 360 nm, emission 465 nM), using a SPARK TM10 Tecan instrument. Results were analyzed using Prism (GraphPad, San Diego, CA) and Vortex software (Dotmatics, Bioshops Stortford, UK). Dose-response curves were fitted by four-parameter logistic regression.

### JEV protease inhibition assay

The enzyme inhibition assay was performed in 384-well flat bottom black polystyrene microplates (cat. N. 781900, Greiner,) in a reaction volume of 20 µl. Japanese encephalitis virus (JEV)-NS2B-NS3 protease (600 nM) was incubated with increasing inhibitor concentrations (0.097-100 µM or 0.009-1 µM for the most active compounds) in assay buffer (50 mM Tris-HCl, pH 9.0, 0.1% BSA, 0.1% Triton X-100, 1% DMSO) for 10 minutes at 25 °C. 10 µM of Pyr-RTKR-AMC substrate (Bachem, cat. N.4018149) was added and the reaction mixture incubated for additional 120 minutes at 25 °C. Product formation was evaluated by fluorescence measurement (excitation 360 nm, emission 465 nM), using a SPARK TM10 Tecan instrument. Results were analyzed using Prism (GraphPad, San Diego, CA) and Vortex software (Dotmatics, Bioshops Stortford, UK). Dose-response curves were fitted by four-parameter logistic regression.

### ZIKV replicon generation

To construct the subgenomic replicon we used the Natal RGN isolate of Asian lineage (GenBank: KU527068.1). In the replicon, the coding sequences of structural genes were removed with the exception of 31 amino acids at the N-terminus of the capsid protein fused with 32 amino acids at the C-terminus of the E protein. These sequences were retained to preserve the correct processing and translocation of NS1and of the non-structural polyprotein across the endothelium reticulum membrane. An EMCV IRES was placed after the stop codon of the polyprotein. The IRES drives the translation of a downstream fusion protein consisting of a luciferase (Nano-Luc) gene followed by the ubiquitin gene (UBI), and the neomycin phosphotransferase resistance gene (NEO). This fusion protein is required for reporting purposes and to confer resistance to G-418. The subgenomic replicon was transcribed as RNA via an upstream T7 promoter and transfected in Vero cells. The stable cell line used for the ZIKV replication assay was generated via selection with G-418.

### ZIKV replication inhibition assay and cell viability assay

A green monkey cell line (Vero) with a stable ZIKV replicon was grown and maintained in Dulbecco's modified eagle's medium (DMEM) with high glucose and pyruvate (GIBCO #41966) completed with 10% fetal bovine serum (FBS) (Gibco #10270), 1% Penicillin Streptomycin (10 mg/ml) (Gibco #14140). 0.760 µg/mL G418 solution (Sigma-Aldrich, Cat 4727894001) were used to maintain the ZIKV replication in the cells. The day of the experiment, compounds previously dissolved in 100% dimethyl sulfoxide (DMSO, Sigma-Aldrich, D5879-1L) were transferred to a 384 black plate (Greiner #781086) using an acoustic system (ATS-100 EDC). Compounds were tested in dose response. 50 µM of mycophenolic acid (Sigma-Aldrich, M3536) was used in as positive control for the ZIKV replication assay while 32 µM of Gambogic Acid (Sigma-Aldrich, G8171) was used as positive control for the cell viability assay. In both cases, in the negative control wells were added with 0.5% DMSO. 8000 cells/well were plated on the compound containing plates in 40 µL growth medium. 72 h after the treatment at 37 °C, 5% CO₂ and 90% humidity, 20 µL Nano-Glo (Promega #N1150) were added to reveal the NanoLuc signal, while 20 µL CellTiter-Glo (Promega #G7573) were added to determine the cell viability. The luminescent signal for both assays was detected reading the plate on the Envision plate reading 10 minutes post detection reagent addition (luminescence was measured at 0.5 sec/well). Data were normalized between 0 and 100% inhibition (negative control and positive control respectively). For EC₅₀ determinations, the dose-response was fitted with a 4-p logistic regression approach using the Dotmatics Studies software.

### Results

The exemplified compounds described herein were tested in the assays described above. Tables 2 -5 report respectively results from IC₅₀ calculation for inhibition of ZIKV protease and replicon and for inhibition of JEV virus, West Nile Virus and Dengue Virus proteases. The following letter codes are used in the tables:
A: IC₅₀ or EC₅₀ lower than 0.1 µM; B: IC₅₀ or EC₅₀ from 0.1 µM to 1 µM; C: IC₅₀ or EC₅₀ from 1 µM to 20 µM ; D: IC₅₀ or EC₅₀ higher than 20 µM; n.a.: not available.

**Table 2**

| **Example N.** | **Zika - NS3 protease** | **ZIKA Cell_Nanoluc** |
|---|---|---|
| 1 | A | C |
| 2 | C | D |
| 3 | B | B |
| 4 | B | C |
| 5 | A | C |
| 6 | A | C |
| 7 | A | B |
| 8 | A | C |
| 9 | A | B |
| 10 | B | B |
| 11 | A | C |
| 12 | A | B |
| 13 | B | B |
| 14 | A | C |
| 15 | A | B |
| 16 | B | C |
| 17 | B | C |
| 18 | A | B |
| 19 | A | C |
| 20 | A | D |
| 21 | A | B |
| 22 | A | C |
| 23 | A | C |
| 24 | B | C |
| 25 | A | C |
| 26 | B | D |
| 27 | C | C |
| 28 | B | C |
| 29 | C | C |
| 30 | C | C |
| 31 | C | C |
| 32 | A | B |
| 33 | C | C |
| 34 | B | C |
| 35 | C | C |
| 36 | A | C |
| 37 | B | C |
| 38 | A | C |
| 39 | B | C |
| 40 | C | C |
| 41 | B | C |
| 42 | B | C |
| 43 | A | C |
| 44 | C | C |
| 45 | C | C |
| 46 | B | C |
| 47 | B | C |
| 48 | C | C |
| 49 | C | C |
| 50 | A | B |
| 51 | B | C |
| 52 | C | C |
| 53 | B | C |
| 54 | B | C |
| 55 | B | C |
| 56 | A | C |
| 57 | C | D |
| 58 | B | C |
| 59 | A | C |
| 60 | B | C |
| 61 | A | C |
| 62 | A | D |
| 63 | B | C |
| 64 | B | D |
| 65 | C | D |
| 66 | B | D |
| 67 | C | C |
| 68 | B | C |
| 69 | B | D |
| 70 | C | D |
| 71 | B | C |
| 72 | C | D |
| 73 | B | C |
| 74 | B | C |
| 75 | C | D |
| 76 | B | D |
| 77 | B | D |
| 78 | B | D |
| 79 | B | C |
| 80 | C | D |
| 81 | B | C |
| 82 | C | D |
| 83 | C | C |
| 84 | B | C |
| 85 | B | C |
| 86 | B | C |
| 87 | C | D |
| 88 | C | D |
| 89 | A | C |
| 90 | B | C |
| 91 | B | D |
| 92 | A | D |
| 93 | A | C |
| 94 | A | C |
| 95 | B | C |
| 96 | A | C |
| 97 | A | D |
| 98 | A | C |
| 99 | B | C |
| 100 | B | C |
| 101 | B | D |
| 102 | A | C |
| 103 | B | D |
| 104 | A | C |
| 105 | A | B |
| 106 | A | C |
| 107 | A | C |
| 108 | A | C |
| 109 | A | B |
| 110 | B | D |
| 111 | A | B |
| 112 | B | D |
| 113 | A | C |
| 114 | A | C |
| 115 | B | C |
| 116 | A | C |
| 117 | A | C |
| 118 | A | C |
| 119 | A | C |
| 120 | A | D |
| 121 | A | C |
| 122 | C | C |
| 123 | B | D |
| 124 | A | D |
| 125 | A | C |
| 126 | B | D |
| 127 | A | C |
| 128 | A | C |
| 129 | A | C |
| 130 | B | C |
| 131 | B | C |
| 132 | B | D |
| 133 | A | C |
| 134 | B | D |
| 135 | B | C |
| 136 | B | B |
| 137 | B | B |
| 138 | B | C |
| 139 | B | C |
| 140 | A | B |
| 141 | A | C |
| 142 | A | NA |
| 143 | B | D |
| 144 | B | B |
| 145 | B | C |
| 146 | A | B |
| 147 | A | C |
| 148 | B | C |
| 149 | B | D |
| 150 | B | C |
| 151 | B | C |
| 152 | B | C |
| 153 | A | C |
| 154 | C | D |
| 155 | B | C |
| 156 | C | C |
| 157 | A | C |
| 158 | D | D |
| 159 | A | C |
| 160 | C | D |
| 161 | C | C |
| 162 | B | C |
| 163 | B | C |
| 164 | A | B |
| 165 | A | B |
| 166 | B | C |
| 167 | C | C |
| 168 | A | B |
| 169 | A | B |
| 170 | A | B |
| 171 | A | B |
| 172 | C | C |
| 173 | A | B |
| 174 | B | C |
| 175 | B | C |
| 176 | B | C |
| 177 | B | C |
| 178 | B | B |
| 179 | C | C |
| 180 | C | C |
| 181 | B | B |
| 182 | C | C |
| 183 | B | B |
| 184 | B | B |
| 185 | C | C |
| 186 | B | C |
| 187 | B | B |
| 188 | A | A |
| 189 | A | A |
| 190 | A | A |
| 191 | A | A |
| 192 | A | B |
| 193 | B | B |
| 194 | A | A |
| 195 | B | B |
| 196 | C | B |
| 197 | B | A |
| 198 | A | A |
| 199 | A | A |
| 200 | A | B |
| 201 | A | A |
| 202 | B | C |
| 203 | B | B |
| 204 | B | C |
| 205 | B | C |
| 206 | A | B |
| 207 | B | B |
| 208 | B | C |
| 209 | C | C |
| 210 | C | C |
| 211 | B | C |
| 212 | B | C |
| 213 | C | C |
| 214 | B | C |
| 215 | A | A |
| 216 | B | C |
| 217 | A | A |
| 218 | A | B |
| 219 | B | C |
| 220 | A | B |
| 221 | C | C |
| 222 | A | B |
| 223 | A | A |
| 224 | A | A |
| 225 | B | B |
| 226 | B | C |
| 227 | B | C |
| 228 | B | B |
| 229 | B | C |
| 230 | B | C |
| 231 | B | C |
| 232 | C | C |
| 233 | C | B |
| 234 | A | C |
| 235 | A | B |
| 236 | A | B |
| 237 | B | C |
| 238 | B | C |
| 239 | A | A |
| 240 | B | C |
| 241 | B | B |
| 242 | A | A |
| 243 | A | B |
| 244 | B | C |
| 245 | B | C |
| 246 | B | C |
| 247 | B | C |
| 248 | B | C |
| 249 | A | A |
| 250 | A | B |
| 251 | B | C |
| 252 | B | C |
| 253 | B | C |
| 254 | B | C |
| 255 | B | C |
| 256 | C | C |
| 257 | B | C |
| 258 | A | A |
| 259 | A | A |
| 260 | A | A |
| 261 | A | A |
| 262 | B | B |
| 263 | A | A |
| 264 | A | A |
| 265 | B | C |
| 266 | A | A |
| 267 | B | C |
| 268 | A | A |
| 269 | A | A |
| 270 | A | A |
| 271 | A | B |
| 272 | C | B |
| 273 | C | C |

**Table 3**

| **Example N.** | **JEV - NS3 protease** |
|---|---|
| 1 | C |
| 2 | C |
| 3 | C |
| 4 | C |
| 5 | C |
| 6 | C |
| 7 | C |
| 8 | D |
| 9 | C |
| 10 | D |
| 11 | C |
| 12 | C |
| 13 | C |
| 14 | C |
| 15 | C |
| 16 | D |
| 17 | D |
| 18 | D |
| 19 | C |
| 20 | D |
| 21 | C |
| 22 | D |
| 25 | C |
| 26 | C |
| 55 | C |
| 56 | B |
| 57 | D |
| 58 | C |
| 59 | C |
| 60 | C |
| 61 | C |
| 62 | C |
| 76 | D |
| 89 | C |
| 91 | C |
| 92 | C |
| 93 | B |
| 94 | C |
| 95 | C |
| 96 | C |
| 101 | C |
| 102 | B |
| 103 | C |
| 104 | B |
| 105 | C |
| 106 | C |
| 108 | B |
| 109 | B |
| 111 | B |
| 112 | C |
| 113 | C |
| 114 | C |
| 115 | C |
| 116 | C |
| 117 | C |
| 118 | C |
| 119 | C |
| 122 | D |
| 124 | C |
| 127 | C |
| 129 | C |
| 130 | C |
| 135 | D |
| 138 | C |
| 139 | D |
| 143 | C |
| 144 | C |
| 147 | C |
| 150 | C |
| 154 | D |

**Table 4**

| **Example N.** | **WNV** - **NS3 protease** |
|---|---|
| 1 | C |
| 147 | A |
| 148 | B |
| 149 | A |
| 150 | A |
| 151 | B |
| 152 | B |
| 153 | B |
| 154 | C |
| 155 | B |
| 156 | C |
| 157 | A |
| 158 | C |
| 159 | A |
| 160 | C |
| 161 | B |
| 162 | B |
| 163 | B |
| 164 | A |
| 165 | B |
| 166 | B |
| 167 | B |
| 168 | A |
| 169 | A |
| 170 | A |
| 171 | A |
| 172 | B |
| 173 | A |
| 174 | B |
| 175 | A |
| 176 | B |
| 177 | B |
| 178 | B |
| 179 | C |
| 180 | C |
| 181 | A |
| 182 | B |
| 183 | B |
| 184 | B |
| 185 | B |
| 186 | B |
| 187 | B |
| 188 | A |
| 189 | A |
| 190 | A |
| 191 | C |
| 192 | B |
| 193 | C |
| 194 | B |
| 195 | C |
| 196 | C |
| 197 | C |
| 198 | C |
| 199 | B |
| 200 | B |
| 201 | A |
| 202 | B |
| 203 | B |
| 204 | B |
| 205 | B |
| 206 | A |
| 207 | B |
| 208 | B |
| 209 | B |
| 210 | B |
| 211 | B |
| 212 | B |
| 213 | B |
| 214 | B |
| 215 | A |
| 216 | B |
| 217 | A |
| 218 | B |
| 219 | B |
| 220 | B |
| 221 | B |
| 222 | A |
| 223 | A |
| 224 | A |
| 225 | B |
| 226 | A |
| 227 | A |
| 228 | B |
| 229 | B |
| 230 | A |
| 231 | A |
| 232 | B |
| 233 | B |
| 234 | B |
| 235 | A |
| 236 | B |
| 237 | B |
| 238 | B |
| 239 | A |
| 240 | B |
| 241 | A |
| 242 | A |
| 243 | A |
| 244 | B |
| 245 | B |
| 246 | A |
| 247 | B |
| 248 | A |
| 249 | B |
| 250 | B |
| 251 | B |
| 252 | B |
| 253 | B |
| 254 | A |
| 255 | B |
| 256 | B |
| 257 | A |
| 258 | A |
| 259 | B |
| 260 | A |
| 261 | A |
| 262 | B |
| 263 | A |
| 264 | A |
| 265 | B |
| 266 | A |
| 267 | B |
| 268 | A |
| 269 | A |
| 270 | A |
| 271 | A |
| 272 | C |
| 273 | B |

**Table 5**

| **Example N.** | **Dengue 1** - **NS3 protease** | **Dengue 2** - **NS3 protease** | **Dengue 3 - NS3 protease** | **Dengue 4** - **NS3 protease** |
|---|---|---|---|---|
| 1 | C | A | A | A |
| 2 | NA | B | NA | NA |
| 3 | D | C | C | C |
| 4 | D | B | C | C |
| 5 | C | A | NA | NA |
| 6 | NA | A | NA | NA |
| 7 | C | A | B | B |
| 8 | D | A | B | B |
| 9 | B | A | B | B |
| 10 | D | A | B | B |
| 11 | D | B | C | C |
| 12 | NA | A | NA | NA |
| 13 | B | A | A | A |
| 14 | C | A | A | A |
| 15 | NA | A | NA | NA |
| 16 | NA | B | NA | NA |
| 17 | NA | A | NA | NA |
| 18 | D | A | B | B |
| 19 | NA | A | NA | NA |
| 20 | NA | A | NA | NA |
| 21 | NA | A | NA | NA |
| 22 | NA | B | NA | NA |
| 23 | C | A | A | B |
| 24 | NA | A | NA | NA |
| 25 | B | A | B | C |
| 26 | D | B | C | C |
| 27 | NA | C | NA | NA |
| 28 | D | A | B | C |
| 29 | NA | C | NA | NA |
| 30 | NA | C | NA | NA |
| 31 | NA | C | NA | NA |
| 32 | C | A | NA | NA |
| 33 | NA | D | NA | NA |
| 34 | C | A | B | C |
| 35 | NA | C | NA | NA |
| 36 | C | A | A | B |
| 37 | NA | C | NA | NA |
| 38 | NA | B | NA | NA |
| 39 | NA | C | NA | NA |
| 40 | NA | B | NA | NA |
| 41 | NA | B | NA | NA |
| 42 | NA | B | NA | NA |
| 43 | NA | B | NA | NA |
| 44 | NA | C | NA | NA |
| 45 | NA | C | NA | NA |
| 46 | NA | B | NA | NA |
| 47 | NA | B | NA | NA |
| 48 | NA | C | NA | NA |
| 49 | NA | C | NA | NA |
| 50 | NA | B | NA | NA |
| 51 | NA | C | NA | NA |
| 52 | NA | C | NA | NA |
| 53 | NA | C | NA | NA |
| 54 | NA | B | NA | NA |
| 55 | D | B | C | D |
| 56 | C | A | A | B |
| 57 | D | B | D | D |
| 58 | D | B | C | D |
| 59 | C | A | B | C |
| 60 | D | B | C | D |
| 61 | D | A | B | C |
| 62 | D | A | C | C |
| 63 | NA | B | NA | NA |
| 64 | NA | C | NA | NA |
| 65 | NA | C | NA | NA |
| 66 | NA | C | NA | NA |
| 67 | NA | B | NA | NA |
| 68 | NA | B | NA | NA |
| 69 | NA | C | NA | NA |
| 70 | NA | C | NA | NA |
| 71 | NA | C | NA | NA |
| 72 | NA | C | NA | NA |
| 73 | NA | A | NA | NA |
| 74 | NA | B | NA | NA |
| 75 | NA | C | NA | NA |
| 76 | D | A | C | D |
| 77 | NA | C | NA | NA |
| 78 | NA | C | NA | NA |
| 79 | NA | C | NA | NA |
| 80 | NA | C | NA | NA |
| 81 | NA | C | NA | NA |
| 82 | NA | C | NA | NA |
| 83 | NA | B | NA | NA |
| 84 | NA | C | NA | NA |
| 85 | NA | B | NA | NA |
| 86 | NA | B | NA | NA |
| 87 | NA | D | NA | NA |
| 88 | NA | D | NA | NA |
| 89 | C | A | B | C |
| 90 | NA | B | NA | NA |
| 91 | C | A | B | C |
| 92 | C | A | B | C |
| 93 | C | A | B | B |
| 94 | C | A | B | B |
| 95 | C | A | B | C |
| 96 | C | A | B | C |
| 97 | NA | A | NA | NA |
| 98 | NA | A | NA | NA |
| 99 | NA | A | NA | NA |
| 100 | NA | B | NA | NA |
| 101 | C | A | C | C |
| 102 | C | A | A | B |
| 103 | D | A | C | C |
| 104 | C | A | A | B |
| 105 | D | A | B | C |
| 106 | C | A | B | C |
| 107 | NA | A | NA | NA |
| 108 | C | A | B | C |
| 109 | C | A | A | B |
| 110 | C | A | NA | NA |
| 111 | C | A | A | B |
| 112 | C | A | B | C |
| 113 | D | A | B | C |
| 114 | C | A | B | C |
| 115 | C | A | B | C |
| 116 | C | A | B | B |
| 117 | C | A | B | C |
| 118 | C | A | B | C |
| 119 | C | A | B | C |
| 120 | NA | A | NA | NA |
| 121 | NA | A | NA | NA |
| 122 | D | B | C | D |
| 123 | NA | C | NA | NA |
| 124 | C | A | B | C |
| 125 | NA | A | NA | NA |
| 126 | NA | B | NA | NA |
| 127 | C | A | B | C |
| 128 | NA | A | NA | NA |
| 129 | C | A | A | B |
| 130 | C | A | B | C |
| 131 | NA | A | NA | NA |
| 132 | NA | A | NA | NA |
| 133 | NA | A | NA | NA |
| 134 | NA | A | NA | NA |
| 135 | D | B | C | C |
| 136 | NA | A | NA | NA |
| 137 | NA | A | NA | NA |
| 138 | C | A | B | C |
| 139 | D | A | B | C |
| 140 | NA | A | NA | NA |
| 141 | NA | A | NA | NA |
| 142 | NA | B | NA | NA |
| 143 | C | A | B | C |
| 144 | D | A | B | B |
| 145 | NA | A | NA | NA |
| 146 | NA | A | NA | NA |
| 147 | C | A | B | B |
| 150 | NA | B | NA | NA |
| 154 | NA | C | NA | NA |

## Claims

1. A compound of general formula (I): 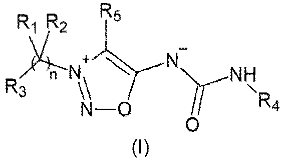 wherein:
n is 0, 1 or 2;
R₁ and R₂ are independently selected from Hand C₁₋₆alkyl optionally substituted with one or more substituents selected from: hydroxy, OC₁₋₃alkyl, halogen, NH₂, NHCOC₁₋₆alkyl, NHCOOC₁₋₆alkyl, NHC(=O)NHC₁₋₆alkyl, NHSO₂C₁₋₆alkyl, NHC₁₋₃alkyl, N(C₁₋₃alkyl)₂, or a cyclic amine selected from aziridine, azetidine, pyrrolidine, pyperidine, morpholine, piperazine, N-methylpyperazine;
or R₁ and R₂ are linked together to form a C₃₋₁₀-cycloalkyl ring or a C₄₋₁₂-heterocycloalkyl ring;
R₃ is selected from:
- saturated or unsaturated C₃₋₁₀cycloalkyl ring, saturated or unsaturated C₃₋₁₀cycloheteroalkyl ring, each of said ring being optionally substituted with one or more substituents independently selected from halogen, hydroxy, NH₂, NHC₁₋₃alkyl, N(C₁₋₃alkyl)₂, C₁₋₆alkylNH₂, C₁₋₆alkylNHC₁₋₃alkyl, C₁₋₆alkylN(C₁₋₃alkyl)₂, C₁₋₆alkylNHC₁₋₃haloalkyl, C₁₋₆alkylC(O)OC₁₋₃alkyl, NHCOC₁₋₆alkyl, NHCOOC₁₋₆alkyl, NHC(=O)NHC₁₋₆alkyl, NHSO₂C₁₋₆alkyl, C₁₋₆alkyl, C₁₋₆haloalkyl, C₁₋₆alkylOH, C(O)OH, C(O)OC₁₋₃alkyl, C(O)NH₂, C(O)NH(C₁₋₃alkyl), C(O)N(C₁₋₃alkyl)₂, optionally substituted aziridine, optionally substituted azetidine, optionally substituted pyrrolidine, optionally substituted pyperidine, optionally substituted morpholine, optionally substituted piperazine, optionally substituted *N*-methylpyperazine, optionally substituted aryl, optionally substituted heteroaryl; or
- aromatic or heteroaromatic ring optionally substituted with one or more substituents independently selected from halogen, hydroxy, NH₂, NHCOC₁₋₆alkyl, NHCOOC₁₋₆alkyl, NHC(=O)NHC₁₋₆alkyl, NHSO₂C₁₋₆alkyl, NHC₁₋₃alkyl, N(C₁₋₃alkyl)₂, C₁₋₆alkyl, C₁₋₆alkylC(O)OH, C₁₋₆alkylC(O)OC₁₋₃alkyl, hydroxyC₁₋₆alkyl C₃₋₆cycloalkyl, haloC₁₋₆alkyl, C₁₋₆alkoxy, cyano, aryl, heteroaryl wherein said aryl or heteroaryl ring are optionally substituted with one or more substituents each independently selected from halogen, hydroxy, cyano, NH₂, NHCOC₁₋₆alkyl, NHCOOC₁₋₆alkyl, NHC₁₋₃alkyl, N(C₁₋₃alkyl)₂, C₃₋₆cycloalkyl, aziridine, azetidine, pyrrolidine, pyperidine, morpholine, piperazine, *N-*methylpyperazine, C₁₋₆alkyl, C₁₋₆alkoxy, wherein said C₁₋₆alkyl, C₁₋₆alkoxy are optionally substituted with one or more halogen, hydroxy, NH₂, NHCOCH₃, NHCOOC₁₋₆alkyl, NHC₁₋₃alkyl, N(C₁₋₃alkyl)₂, aziridine, azetidine, pyrrolidine, pyperidine, morpholine, piperazine and *N*-methylpyperazine;
R₄ is selected from:
a) a ring of formula (IIa) or (IIb): 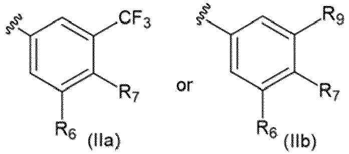 wherein
- R₆ is selected from NHC(O)Rs, N(C₁₋₆alkyl)C(O)R₈, NHSO₂R₈, NH(C₁₋₆alkyl), NH(C₁₋₆haloalkyl), hydroxy-C₁₋₆alkyl, CN, C(O)OC₁₋₆alkyl, C(O)NHRs, C₂₋₆alkynyl, C₁₋₆alkylNHC(O)R₈, C₁₋₆alkylN(C₁₋₆alkyl)C(O)R₈, and 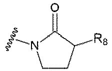 and R₇ is H; or
- R₆ is H and R₇ is selected from NHC(O)Rs, N(C₁₋₆alkyl)C(O)R₈, NHSO₂R₈, NH(C₁₋₆alkyl), NH(C₁₋₆haloalkyl), hydroxy-C₁₋₆alkyl, CN, C(O)OC₁₋₆alkyl, C(O)NHR₈ C₂₋₆alkynyl, C₁₋₆alkylNHC(O)R₈, C₁₋₆alkylN(C₁₋₆alkyl)C(O)R₈, and 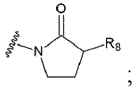 or
- R₆ and R₇ are H, with the proviso that when R₆ and R₇ are H, R₃ is selected from saturated or unsaturated C₃₋₁₀cycloalkyl ring, saturated or unsaturated C₃₋₁₀heterocycloalkyl ring, each of said ring being optionally substituted with halogen, C₁₋₆alkyl, haloC₁₋₆alkyl, halogen, CN, OH, C₁₋₆alkoxy, amine, optionally substituted aryl, optionally substituted heteroaryl; or when R₆ and R₇ are H, R₃ is (4,6-dimethylpyrimidin-5-yl)pyridazin-3-yl; or
- R₆ is OCH₃ or OH and R₇ is H, or R₆ is H and R₇ is OCH₃ or C(O)OCH₃, or R₆ and R₇ are joined to form together with the ring of formula (IIa) a 8-(trifluoromethyl)quinoline-6-yl ring, with the proviso that R₃ is 4-(4,6-dimethylpyrimidin-5-yl)phenyl;
- R₈ is C₁₋₆alkyl, C₁₋₆haloalkyl, C₁₋₆alkyl-aryl, C₁₋₆alkyl-heteroaryl, C₁₋₆alkyl-C₃₋₁₀cycloalkyl, C₁₋₆alkyl-C₃₋₁₀heterocycloalkyl, C₁₋₆haloalkyl-aryl, C₁₋₆haloalkyl-heteroaryl, C₁₋₆haloalkyl-C₃₋₁₀cycloalkyl, C₁₋₆haloalkyl-C₃₋₁₀heterocycloalkyl, C₁₋₆alkylSO₂aryl, C₁₋6alkylSO₂heterocycloalkyl, C₂₋₆alkynyl-aryl, aryl, heteroaryl, C₃₋₁₀cycloalkyl ring, C₃₋₁₀heterocycloalkyl ring, wherein each of said cycloalkyl and heterocycloalkyl ring is saturated or unsaturated and wherein each of said aryl, heteroaryl, cycloalkyl and herocycloalkyl ring is optionally substituted with one or more substituents independently selected from C₁₋₆alkyl, C₁₋₆alkoxy, C₃₋₁₀cycloalkyl, C₃₋₁₀heteroycloalkyl, amine, CN, C₁₋₃alkyl-aryl, C₁₋₃alkyl-heteroaryl, C₁₋₃alkyl-C₃₋₁₀cycloalkyl, C₁₋₃alkyl-C₃₋₁₀heterocycloalkyl, C₁₋₆alkylCOOH, C₁₋₆alkylCOOCH₃, C₁₋₆alkylCONH₂ COOH, C(O)OC₁₋₃alkyl, NHC(O)C₁₋₆alkyl, NHC(O)C₃₋₆cycloalkyl, C₁₋₆haloalkyl, halogen, C₁₋₆alkylamine, OH, optionally substituted C₁₋₃alkylaryl, optionally substituted aryl, optionally substituted heteroaryl, optionally substituted heteroaryloxy, optionally substituted aryloxy optionally substituted heteroaryl-alkoxy, heterocycloalkoxy, C₁₋₆alkyl-SO₂NH₂, C₁₋₆alkyl-OC₁₋₃alkylheteroaryl wherein said heteroaryl is optionally substituted with CH₃, halogen or OH;
- R₉ is C₁₋₆alkyl, halogen, C₃₋₆cycloalkyl or C₃₋₆heterocycloalkyl each of said cycloalkyl or heterocycloalkyl being optionally substituted with C₁₋₃alkyl, haloC₁₋₃alkyl, halogen;
b) saturated or unsaturated C₃₋₁₀cycloalkyl ring, saturated or unsaturated C₃₋₁₀cycloheteroalkyl ring, each of said ring being optionally substituted with one or more substituents each independently selected from C₁₋₆alkyl, haloC₁₋₆alkyl, halogen, CN, OH, C₁₋₆alkoxy, NHC(O)Rs, N(C₁₋₆alkyl)C(O)R₈, NHSO₂R₈, NH(C₁₋₆alkyl), NH(C₁₋₆haloalkyl), hydroxy-C₁₋₆alkyl, C(O)OC₁₋₆alkyl, C(O)NHR₈, C₂₋₆alkynyl, C₁₋₆alkylNHC(O)R₈, C₁₋₆alkylN(C₁₋₆alkyl)C(O)R₈, and;
c) a ring of formula (III): 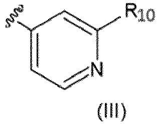
wherein R₁₀ is CF₃, OCH₃, t-butyl; and wherein when R₄ is a ring of formula (III), R₃ is selected from:
- C₃₋₁₀cycloalkyl ring, C₃₋₁₀cycloalkenyl ring, C₃₋₁₀cycloheteroalkyl ring, each of said ring being optionally substituted with halogen, C₁₋₆alkyl, haloC₁₋₆alkyl, halogen, CN, OH, C₁₋₆alkoxy, amine, optionally substituted aryl, optionally substituted heteroaryl; or
- (1-methyl-1,2,3,4-tetrahydroquinolin-2-yl), 2-methyl-1,2,3,4-tetrahydroisoquinolin-1-yl, (3-methylpyridazin-4-yl)phenyl, 4-(4-(trifluoromethyl)pyrimidin-5-yl)phenyl, 4-(4-methoxy-6-methylpyrimidin-5-yl)phenyl, 4-(1,4-dimethyl-1*H*-pyrazol-5-yl)phenyl, (2-methylthiazol-4-yl)phenyl, (3,5-dimethylisothiazol-4-yl)phenyl, (4-methylpyrimidin-5-yl)phenyl, (1,3-dimethyl-1*H*-pyrazol-4-yl)phenyl, 4-(4-methyl-1-(oxetan-3-yl)-1*H-*pyrazol-5yl)phenyl, 4-(4-methyl-1-(oxetan-3-yl)-1*H*-pyrazol-3-yl)phenyl, 4-(2-methylpyridin-3-yl)phenyl, 4-(2,5-dimethylthiazol-4-yl)phenyl, 4-(2-amino-4-methylpyrimidin-5-yl)phenyl, 4-(2,4-dimethylpyridin-3-yl)phenyl, 4-(pyrimidin-5-yl)phenyl, 4-(1,3,5-trimethyl-1*H*-pyrazol-4-yl)phenyl, 4-(4,6-dimethylpyrimidin-5-yl)phenyl, 4-(4-Methyl-2-(oxetan-3-yl)pyrazol-3-yl]phenyl);
and R₅ is H, C₁₋₆alkyl, C₁₋₆haloalkyl or halogen;
or a pharmaceutically acceptable salt, tautomer, solvate, or stereoisomer thereof.

2. The compound according to claim 1 wherein:
- n is 0 or 1;
- R₁ and R₂ are H; and/or
- R₅ is H; and/or
- R₉ is cyclopropyl, methyl-cyclopropyl, trifluoromethyl-cyclopropyl, fluorocyclopropyl, difluorocyclopropyl, oxetane.

3. The compound according to any one of previous claims wherein R₃ is phenyl, pyridinyl, pyrazynyl, trifluoromethylphenyl, cyclobutyl, cyclohexyl, pyperidine, oxabicyclo[3.3.1]nonanyl, oxazepanyl, bicyclo[1.1.1]pentanyl, tetrahydroquinolinyl, bicyclo[2.2.1]heptanyl, tetrahydronaphtalenyl, azabicyclo[3.2.1]octane, 2-azaspiro[3.5]nonane, 3-azaspiro[5.5]undecane, wherein each of said ring is optionally substituted with one or more substituents independently selected from optionally substituted heteroaryl, optionally substituted aryl, C₁₋₃alkyl, halogen, hydroxy, isoindoline, NH₂, NHCH₃, N(CH₃)₂, N(C₁₋₃alkyl)(haloC₁₋₃alkyl), C₁₋₃alkyl-NH₂, C₁₋₃alkyl-N(CH₃)₂, (1,3-dioxoisoindolin-2-yl)methyl, C₁₋₃alkyl-NHCH₃, C₁₋₃alkyl-N(CH₃)₂, C₁₋₃alkyl-NHC(O)CH₃, COOH, C(O)NH₂, C(O)NH(C₁₋₃alkyl), C(O)N(C₁₋₃alkyl)₂, cycloamino-C(O) C₁₋₃alkyl-C(O)O(C₁₋₃alkyl), C₁₋₃alkyl-OH, C(O)OC₁₋₃alkyl, NHCH₂CF₃, CH₂NHCH₂CF₃, C₁₋₃alkyl-OH, haloC₁₋₃alkyl, NH(CO)CF₃.

4. The compound according to any one of previous claims wherein R₄ is a ring of formula (IIa) or (IIb): 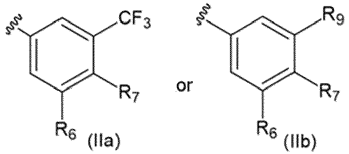 wherein
- R₆ is selected from NHC(O)Rs, N(C₁₋₃alkyl)C(O)R₈, NHSO₂R₈, NH(C₁₋₃alkyl), NH(C₁₋₃haloalkyl), hydroxy-C₁₋₃alkyl, CN, C(O)OC₁₋₃alkyl, C(O)NHRs, C₂₋₆alkynyl, C₁₋₃alkylNHC(O)R₈, C₁₋₃alkylN(C₁₋₃alkyl)C(O)R₈, and 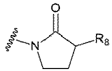 and R₇ is H; or
- R₆ is H and R₇ is selected from NHC(O)Rs, N(C₁₋₃alkyl)C(O)R₈, NHSO₂R₈, NH(C₁₋₃alkyl), NH(C₁₋₃haloalkyl), hydroxy-C₁₋₃alkyl, CN, C(O)OC₁₋₃alkyl, C(O)NHRs C₂₋₆alkynyl, C₁₋₃alkylNHC(O)R₈, C₁₋₃alkylN(C₁₋₃alkyl)C(O)R₈, and 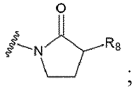 or
- R₆ and R₇ are H, with the proviso that when R₆ and R₇ are H, R₃ is selected from saturated or unsaturated C₃₋₁₀cycloalkyl ring, saturated or unsaturated C₃₋₁₀heterocycloalkyl ring, each of said ring being optionally substituted with halogen, C₁₋₆alkyl, haloC₁₋₆alkyl, halogen, CN, OH, C₁₋₆alkoxy, amine, optionally substituted aryl, optionally substituted heteroaryl; or when R₆ and R₇ are H, R₃ is (4,6-dimethylpyrimidin-5-yl)pyridazin-3-yl; or
- R₆ is OCH₃ or OH and R₇ is H, or R₆ is H and R₇ is OCH₃ or C(O)OCH₃, or R₆ and R₇ are joined to form together with the ring of formula (IIa) a 8-(trifluoromethyl)quinoline-6-yl ring, with the proviso that R₃ is 4-(4,6-dimethylpyrimidin-5-yl)phenyl;
- Rs is C₁₋₆alkyl, C₁₋₆haloalkyl, C₁₋₆alkyl-aryl, C₁₋₃alkyl-heteroaryl, C₁₋₃alkyl-C₃₋₆cycloalkyl, C₁₋₃alkyl-C₃₋₈heterocycloalkyl, C₁₋₃haloalkyl-aryl, C₁₋₃haloalkyl-heteroaryl, C₁₋₃haloalkyl-C₃₋₆cycloalkyl, C₁₋₃haloalkyl-C₃₋₈heterocycloalkyl, C₁₋₃alkylSO₂aryl, C₁₋₃alkylSO₂heterocycloalkyl, C₂₋₆alkynyl-aryl, aryl, heteroaryl, C₃₋₆cycloalkyl ring, C₃₋₁₀heterocycloalkyl ring, wherein each of said cycloalkyl and heterocycloalkyl ring is saturated or unsaturated and wherein each of said aryl, heteroaryl, cycloalkyl and herocycloalkyl ring is optionally substituted with one or more substituents independently selected from C₁₋₃alkyl, C₁₋₃alkoxy, C₃₋₆cycloalkyl, C₃₋₈heteroycloalkyl, amine, CN, C₁₋₃alkyl-aryl, C₁₋₃alkyl-heteroaryl, C₁₋₃alkyl-C₃₋₆cycloalkyl, C₁₋₃alkyl-C₃₋₈heterocycloalkyl, C₁₋₃alkylCOOH, C₁₋₃alkylCOOCH₃, C₁₋₃alkylCONH₂ COOH, C(O)OC₁₋₃alkyl, NHC(O)C₁₋₃alkyl, NHC(O)C₃₋₆cycloalkyl, C₁₋₃haloalkyl, halogen, C₁₋₃alkylamine, OH, optionally substituted C₁₋₃alkylaryl, optionally substituted aryl, optionally substituted heteroaryl, optionally substituted heteroaryloxy, optionally substituted aryloxy optionally substituted heteroaryl-alkoxy, heterocycloalkoxy, C₁₋₃alkyl-SO₂NH₂, C₁₋₃alkyl-OC₁₋₃alkylheteroaryl wherein said heteroaryl is optionally substituted with CH₃, halogen or OH;
or R₄ is saturated or unsaturated C₃₋₁₀cycloalkyl ring, saturated or unsaturated C₃₋₁₀cycloheteroalkyl ring, each of said ring being optionally substituted with one or more substituents each independently selected from C₁₋₆alkyl, haloC₁₋₆alkyl, halogen, CN, OH, C₁₋₆alkoxy, NHC(O)R₈, N(C₁₋₆alkyl)C(O)R₈, NHSO₂R₈, NH(C₁₋₆alkyl), NH(C₁₋₆haloalkyl), hydroxy-C₁₋₆alkyl, C(O)OC₁₋₆alkyl, C(O)NHRs, C₂₋₆alkynyl, C₁₋₆alkylNHC(O)R₈, C₁₋₆alkylN(C₁₋₆alkyl)C(O)R₈.

5. The compound according to any one of previous claims having formula (Ia), or formula (Ib) or formula (Ic) or formula (Id): 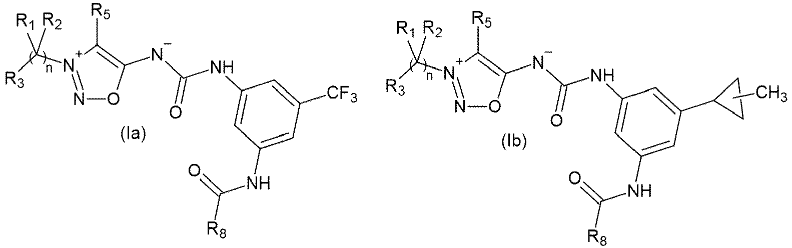 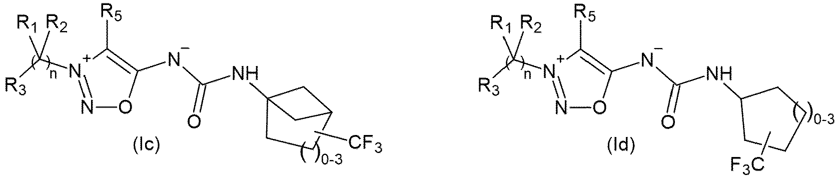 Wherein R₁, R₂, R₃, R₅, R₈ and n are as defined in any one of previous claims.

6. A compound of general formula (I) according to claim 1 selected from the following list:
- (3-(4-(2-Methoxy-4-methylpyrimidin-5-yl)benzyl)-1,2,3-oxadiazol-3-ium-5-yl)((3-(2-phenylacetamido)-5-(trifluoromethyl)phenyl)carbamoyl)amide
- ((3-((Cyclohexylmethyl)sulfonamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-Amino-5-(trifluoromethyl)phenyl)-carbamoyl)(3-(4-(2-methoxy-4-methylpyrimidin-5-yl)benzyl)-1,2,3-oxadiazol-3-ium-5-yl)amide
- (3-((5-(2-(Aminomethyl)phenyl)pyridin-2-yl)methyl)-1,2,3-oxadiazol-3-ium-5-yl)((3-(2-phenylacetamido)-5-(trifluoromethyl)phenyl-)carbamoyl)amide
- (3-((5-(1-Methyl-1H-imidazol-5-yl)pyridin-2-yl)methyl)-1,2,3-oxadiazol-3-ium-5-yl)((3-(2-phenylacetamido)-5-(trifluoromethyl)-phenyl)carbamoyl)amide
- ((3-(2-Phenylacetamido)-5-(trifluoromethyl)-phenyl)carbamoyl)(3-(pyridin-2-ylmethyl)-1,2,3-oxadiazol-3-ium-5-yl)amide
- ((3-(2-Cyclohexylacetamido)-5-(trifluoro-methyl)phenyl)carbamoyl)(3-(4-(2-methoxy-4-methylpyrimidin-5-yl)benzyl)-1,2,3-oxadiazol-3-ium-5-yl)amide
- (3-(4-(2-Methoxy-4-methylpyrimidin-5-yl)benzyl)-1,2,3-oxadiazol-3-ium-5-yl)((3-(3-phenylpropanamido)-5-(trifluoro-methyl)phenyl)carbamoyl)amide
- (3-(4-(2-Methoxy-4-methylpyrimidin-5-yl)benzyl)-1,2,3-oxadiazol-3-ium-5-yl)((3-((phenylmethyl)sulfonamido)-5-(trifluoro-methyl)phenyl)carbamoyl)amide
- ((3-Acetamido-5-(trifluoromethyl)phenyl)-carbamoyl)(3-(4-(2-methoxy-4-methylpyrimidin-5-yl)benzyl)-1,2,3-oxadiazol-3-ium-5-yl)amide
- (3-((5-Bromopyridin-2-yl)methyl)-1,2,3-oxadiazol-3-ium-5-yl)((3-(2-phenylacetamido)-5-(trifluoromethyl)phenyl)carbamoyl)amide
- (3-((5-(2-Methoxy-4-methylpyrimidin-5-yl)-pyridin-2-yl)methyl)-1,2,3-oxadiazol-3-ium-5-yl)((3-(2-phenylacetamido)-5-(trifluoromethyl)-phenyl)carbamoyl)amide
- (3-((5-(Isoindolin-4-yl)pyridin-2-yl)methyl)-1,2,3-oxadiazol-3-ium-5-yl)((3-(2-phenyla-cetamido)-5-(trifluoromethyl)phenyl)carbamoyl)-amide
- ((3-Benzamido-5-(trifluoromethyl)phenyl)-carbamoyl)(3-(4-(2-methoxy-4-methylpyrimidin-5-yl)benzyl)-1,2,3-oxadiazol-3-ium-5-yl)amide
- (3-(4-(2-Methoxy-4-methylpyrimidin-5-yl)benzyl)-1,2,3-oxadiazol-3-ium-5-yl)((3-(2-(4-methoxyphenyl)acetamido)-5-(trifluoromethyl)-phenyl)carbamoyl)amide
- ((3-(2-(4-Chlorophenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-(Cyclopentanecarboxamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- (3-(4-(2-Methoxy-4-methylpyrimidin-5-yl)benzyl)-1,2,3-oxadiazol-3-ium-5-yl)((3-(2-(pyridin-3-yl)acetamido)-5-(trifluoromethyl)phenyl)carbamoyl)amide
- ((3-(2-(Benzo[d]isoxazol-3-yl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-(2-(2-Chlorophenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- ((3-(2-(4-(Aminomethyl)phenyl)acetamido)-5-(trifluoromethyl)phenyl)carbamoyl)(3-(4-(2-methoxy-4-methylpyrimidin-5-yl)benzyl)-1,2,3-oxadiazol-3-ium-5-yl)amide
- (R)-(3-(2-Amino-1-(4-(3,5-dimethylisoxazol-4-yl)phenyl)ethyl)-1,2,3-oxadiazol-3-ium-5-yl)((3-(2-phenylacetamido)-5-(trifluoromethyl)phenyl)-carbamoyl)amide
- (S)-(3-(2-Amino-1-(4-(3,5-dimethylisoxazol-4-yl)phenyl)ethyl)-1,2,3-oxadiazol-3-ium-5-yl)((3-(2-phenylacetamido)-5-(trifluoromethyl)phenyl)carbamoyl)amide
- (3-((5-(2-Amino-4-methylpyrimidin-5-yl)pyridin-2-yl)methyl)-1,2,3-oxadiazol-3-ium-5-yl)((3-(2-phenylacetamido)-5-(trifluoromethyl)-phenyl)carbamoyl)amide
- (3-((1R,3R)-3-Aminocyclobutyl)-1,2,3-oxadiazol-3-ium-5-yl)((3-(2-phenylacetamido)-5-(trifluoromethyl)phenyl)carbamoyl)amide
- ((3-(2-(4-(Aminomethyl)phenyl)acetamido)-5-(trifluoromethyl)phenyl)carbamoyl)(3-(pyridin-2-ylmethyl)-1,2,3-oxadiazol-3-ium-5-yl)amide
- (3-Benzyl-1,2,3-oxadiazol-3-ium-5-yl)((3-(2-phenylacetamido)-5-(trifluoromethyl)phenyl)-carbamoyl)amide
- ((3-(1H-Benzo[d]imidazole-5-carboxamido)-5-(trifluoromethyl)phenyl)carbamoyl)(3-(pyridin-2-ylmethyl)-1,2,3-oxadiazol-3-ium-5-yl)amide
- 5-(3-(3-(5-(Morpholinomethyl)furan-2-carboxamido)-5-(trifluoromethyl)phenyl)ureido)-3-(pyridin-2-ylmethyl)-1,2,3-oxadiazol-3-ium
- ((3-(3-Phenoxybenzamido)-5-(trifluoromethyl)-phenyl)carbamoyl)(3-(pyridin-2-ylmethyl)-1,2,3-oxadiazol-3-ium-5-yl)amide
- ((3-(2-Phenylpropanamido)-5-(trifluoromethyl)-phenyl)carbamoyl)(3-(pyridin-2-ylmethyl)-1,2,3-oxadiazol-3-ium-5-yl)amide
- ((3-(2-(5-Methyl-2-phenyloxazol-4-yl)acetamido)-5-(trifluoromethyl)phenyl)-carbamoyl)(3-(pyridin-2-ylmethyl)-1,2,3-oxadiazol-3-ium-5-yl)amide
- ((3-(1-Phenyl-1H-pyrazole-5-carboxamido)-5-(trifluoromethyl)phenyl)carbamoyl)(3-(pyridin-2-ylmethyl)-1,2,3-oxadiazol-3-ium-5-yl)amide
- ((3-(1-Benzyl-1H-pyrazole-4-carboxamido)-5-(trifluoromethyl)phenyl)carbamoyl)(3-(pyridin-2-ylmethyl)-1,2,3-oxadiazol-3-ium-5-yl)amide
- ((3-(2-(1H-Pyrrolo[3,2-b]pyridin-3-yl)acetamido)-5-(trifluoromethyl)phenyl)-carbamoyl)(3-(pyridin-2-ylmethyl)-1,2,3-oxadiazol-3-ium-5-yl)amide
- ((3-(6-Cyanoimidazo[1,2-a]pyridine-2-carboxamido)-5-(trifluoromethyl)-phenyl)carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(4,5,6,7-tetrahydro-1H-indazole-7-carboxamido)-5-(trifluoromethyl)phenyl)-carbamoyl)amide
- ((3-(5-Methyl-5,6-dihydro-4H-thieno[2,3-c]pyrrole-2-carboxamido)-5-(trifluoromethyl)phenyl)carbamoyl)(3-(pyridin-2-ylmethyl)-1,2,3-oxadiazol-3-ium-5-yl)amide
- ((3-(2-(1-Methyl-1H-pyrazol-3-yl)acetamido)-5-(trifluoromethyl)phenyl)carbamoyl)(3-(pyridin-2-ylmethyl)-1,2,3-oxadiazol-3-ium-5-yl)amide
- ((3-(2-(2-Methylthiazol-4-yl)acetamido)-5-(trifluoromethyl)phenyl)-carbamoyl)(3-(pyridin-2-ylmethyl)-1,2,3-oxadiazol-3-ium-5-yl)amide
- ((3-(2-(6-Aminopyridin-3-yl)acetamido)-5-(trifluoromethyl)phenyl)carbamoyl)(3-(pyridin-2-ylmethyl)-1,2,3-oxadiazol-3-ium-5-yl)amide
- ((3-(2-(Phenylsulfonyl)acetamido)-5-(trifluoromethyl)phenyl)carbamoyl)(3-(pyridin-2-ylmethyl)-1,2,3-oxadiazol-3-ium-5-yl)amide
- ((3-(Isoxazole-3-carboxamido)-5-(trifluoromethyl)-phenyl)carbamoyl)(3-(pyridin-2-ylmethyl)-1,2,3-oxadiazol-3-ium-5-yl)amide
- ((3-(Pyrazine-2-carboxamido)-5-(trifluoromethyl)-phenyl)carbamoyl)(3-(pyridin-2-ylmethyl)-1,2,3-oxadiazol-3-ium-5-yl)amide
- ((3-(3-Phenylpropiolamido)-5-(trifluoromethyl)-phenyl)carbamoyl)(3-(pyridin-2-ylmethyl)-1,2,3-oxadiazol-3-ium-5-yl)amide
- ((3-(Bicyclo[4.2.0]octa-1(6),2,4-triene-7-carboxamido)-5-(trifluoromethyl)phenyl)-carbamoyl)(3-(pyridin-2-ylmethyl)-1,2,3-oxadiazol-3-ium-5-yl)amide
- ((3-(1-Isopropyl-1H-pyrazole-3-carboxamido)-5-(trifluoromethyl)phenyl)carbamoyl)(3-(pyridin-2-ylmethyl)-1,2,3-oxadiazol-3-ium-5-yl)amide
- ((3-(1-Methylazetidine-3-carboxamido)-5-(trifluoromethyl)phenyl)carbamoyl)(3-(pyridin-2-ylmethyl)-1,2,3-oxadiazol-3-ium-5-yl)amide
- ((3-(2-Phenylcyclopropane-1-carboxamido)-5-(trifluoromethyl)phenyl)carbamoyl)(3-(pyridin-2-ylmethyl)-1,2,3-oxadiazol-3-ium-5-yl)amide
- ((3-(3-(Methoxycarbonyl)bicyclo[1.1.1]pentane-1-carboxamido)-5-(trifluoromethyl)phenyl)-carbamoyl)(3-(pyridin-2-ylmethyl)-1,2,3-oxadiazol-3-ium-5-yl)amide
- ((3-(3-Carboxybicyclo[1.1.1]pentane-1-carboxamido)-5-(trifluoromethyl)phenyl)-carbamoyl)(3-(pyridin-2-ylmethyl)-1,2,3-oxadiazol-3-ium-5-yl)amide
- ((3-(Phenethylamino)-5-(trifluoromethyl)-phenyl)carbamoyl)(3-(pyridin-2-ylmethyl)-1,2,3-oxadiazol-3-ium-5-yl)amide
- ((3-(2-Cyclobutylacetamido)-5-(trifluoromethyl)phenyl)carbamoyl)(3-(pyridin-2-ylmethyl)-1,2,3-oxadiazol-3-ium-5-yl)amide
- (3-((1R3R)-3-(Dimethylamino)cyclobutyl)-1,2,3-oxadiazol-3-ium-5-yl)((3-(2-phenyl-acetamido)-5-(trifluoromethyl)phenyl)-carbamoyl)amide
- (3-(4-(Aminomethyl)benzyl)-1,2,3-oxadiazol-3-ium-5-yl)((3-(2-phenylacetamido)-5-(trifluoromethyl)phenyl)carbamoyl)amide
- ((3-Methyl-5-(2-phenylacetamido)phenyl)-carbamoyl)(3-(pyridin-2-ylmethyl)-1,2,3-oxadiazol-3-ium-5-yl)amide
- ((3-Chloro-5-(2-phenylacetamido)phenyl)-carbamoyl)(3-(pyridin-2-ylmethyl)-1,2,3-oxadiazol-3-ium-5-yl)amide
- (3-((1S,4S)-4-(Aminomethyl)cyclohexyl)-1,2,3-oxadiazol-3-ium-5-yl)((3-(2-phenyl-acetamido)-5-(trifluoromethyl)phenyl)-carbamoyl)amide
- (3-((1S,4S)- 4-((1,3-Dioxoisoindolin-2-yl)methyl)cyclohexyl)-1,2,3-oxadiazol-3-ium-5-yl)((3-(2-phenylacetamido)-5-(trifluoromethyl)-phenyl)carbamoyl)amide
- (3-((1S,4S)- -4-((Dimethylamino)methyl)cyclohexyl)-1,2,3-oxadiazol-3-ium-5-yl)((3-(2-phenylacetamido)-5-(trifluoromethyl)phenyl)carbamoyl)amide
- (3-((1S,4S)-4-(Acetamidomethyl)cyclohexyl)-1,2,3-oxadiazol-3-ium-5-yl)((3-(2-phenylacetamido)-5-(trifluoromethyl)phenyl)carbamoyl)amide
- ((3-(2-(Bicyclo[2.1.1]hexan-2-yl)acetamido)-5-(trifluoromethyl)phenyl)carbamoyl)(3-(pyridin-2-ylmethyl)-1,2,3-oxadiazol-3-ium-5-yl)amide
- ((3-(5,5-Dioxido-6,7-dihydro-4H-pyrazolo[5,1-c][1,4]thiazine-2-carboxamido)-5-(trifluoromethyl)phenyl)carbamoyl)(3-(pyridin-2-ylmethyl)-1,2,3-oxadiazol-3-ium-5-yl)amide
- ((3-(2-(2-(Difluoromethyl)-1H-benzo[d]imidazol-1-yl)acetamido)-5-(trifluoromethyl)phenyl)carbamoyl)(3-(pyridin-2-ylmethyl)-1,2,3-oxadiazol-3-ium-5-yl)amide
- ((3-(4-Hydroxybicyclo[2.2.1]heptane-1-carboxamido)-5-(trifluoromethyl)phenyl)-carbamoyl)(3-(pyridin-2-ylmethyl)-1,2,3-oxadiazol-3-ium-5-yl)amide
- ((3-(7-Methyl-2,3-dihydrobenzofuran-3-carboxamido)-5-(trifluoromethyl)phenyl)-carbamoyl)(3-(pyridin-2-ylmethyl)-1,2,3-oxadiazol-3-ium-5-yl)amide
- ((3-(3-Benzylcyclobutane-1-carboxamido)-5-(trifluoromethyl)phenyl)carbamoyl)(3-(pyridin-2-ylmethyl)-1,2,3-oxadiazol-3-ium-5-yl)amide
- ((3-((1S,3S)-3-hydroxy-3-(pyridin-2-yl)cyclobutane-1-carboxamido)-5-(trifluoromethyl)phenyl)carbamoyl)(3-(pyridin-2-ylmethyl)-1,2,3-oxadiazol-3-ium-5-yl)amide
- ((3-(1-((5-Methylfuran-2-yl)methyl)piperidine-4-carboxamido)-5-(trifluoromethyl)phenyl)-carbamoyl)(3-(pyridin-2-ylmethyl)-1,2,3-oxadiazol-3-ium-5-yl)amide
- ((3-(1-(3-Cyano-1H-pyrazol-1-yl)cyclopropane-1-carboxamido)-5-(trifluoromethyl)phenyl)carbamoyl)(3-(pyridin-2-ylmethyl)-1,2,3-oxadiazol-3-ium-5-yl)amide
- ((3-(2-((4-Methyl-4H-1,2,4-triazol-3-yl)methoxy)acetamido)-5-(trifluoromethyl)-phenyl)carbamoyl)(3-(pyridin-2-ylmethyl)-1,2,3-oxadiazol-3-ium-5-yl)amide
- ((3-(3-(Cyclopentyloxy)propanamido)-5-(trifluoromethyl)phenyl)carbamoyl)(3-(pyridin-2-ylmethyl)-1,2,3-oxadiazol-3-ium-5-yl)amide
- ((3-((1S,4R,5R)-4-(3-Methoxyphenyl)-2-oxabicyclo[2.1.1]hexane-5-carboxamido)-5-(trifluoromethyl)phenyl)carbamoyl)(3-(pyridin-2-ylmethyl)-1,2,3-oxadiazol-3-ium-5-yl)amide
- ((3-(1-Cyclopropyl-1H-imidazole-5-carboxamido)-5-(trifluoromethyl)phenyl)-carbamoyl)(3-(pyridin-2-ylmethyl)-1,2,3-oxadiazol-3-ium-5-yl)amide
- ((3-(2,2-Difluoro-3-(pyrrolidin-1-yl)propanamido)-5-(trifluoromethyl)phenyl)-carbamoyl)(3-(pyridin-2-ylmethyl)-1,2,3-oxadiazol-3-ium-5-yl)amide
- ((3-(1-Cyclopropylazetidine-3-carboxamido)-5-(trifluoromethyl)phenyl)carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(4-(1-sulfamoylethyl)benzamido)-5-(trifluoromethyl)phenyl)carbamoyl)amide
- ((3-(2,3-Dihydrofuro[3,2-b]pyridine-5-carboxamido)-5-(trifluoromethyl)phenyl)-carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(5,6,7,8-tetrahydroimidazo[1,5-a]pyridine-7-carboxamido)-5-(trifluoromethyl)
- phenyl)carbamoyl)amide
- ((3-(3,4-Dihydro-1H-pyrrolo[2,1-c][1,4]oxazine-8-carboxamido)-5-(trifluoromethyl)-phenyl)carbamoyl)(3-(pyridin-2-ylmethyl)-1,2,3-oxadiazol-3-ium-5-yl)amide
- ((3-(5-(Piperidin-1-ylsulfonyl)pentanamido)-5-(trifluoromethyl)phenyl)carbamoyl)(3-(pyridin-2-ylmethyl)-1,2,3-oxadiazol-3-ium-5-yl)amide
- ((3-(3-(4-Chlorophenoxy)oxetane-3-carboxamido)-5-(trifluoromethyl)phenyl)-carbamoyl)(3-(pyridin-2-ylmethyl)-1,2,3-oxadiazol-3-ium-5-yl)amide
- ((3-(2,6-Dioxaspiro[4.5]decane-7-carboxamido)-5-(trifluoromethyl)phenyl)carbamoyl)(3-(pyridin-2-ylmethyl)-1,2,3-oxadiazol-3-ium-5-yl)amide
- ((3-(3,3-Difluorospiro[3.3]heptane-1-carboxamido)-5-(trifluoromethyl)phenyl)-carbamoyl)(3-(pyridin-2-ylmethyl)-1,2,3-oxadiazol-3-ium-5-yl)amide
- (3-(Pyridin-2-ylmethyl)-1,2,3-oxadiazol-3-ium-5-yl)((3-(2-(3-(pyrrolidin-1-yl)oxetan-3-yl)acetamido)-5-(trifluoromethyl)phenyl)
- carbamoyl)amide
- (3-(Pyridin-2-ylmethyl)-1,2,3-oxadiazol-3-ium-5-yl)((3-(2,4,5,6-tetrahydrocyclopenta[c]pyrrole-1-carboxamido)-5-(trifluoromethyl)phenyl)-carbamoyl)amide
- ((3-(4,4-Dioxido-1,4-oxathiane-2-carboxamido)-5-(trifluoromethyl)phenyl)carbamoyl)(3-(pyridin-2-ylmethyl)-1,2,3-oxadiazol-3-ium-5-yl)amide
- ((3-Cyclopropyl-5-(2-phenylacetamido)phenyl)-carbamoyl)(3-(pyridin-2-ylmethyl)-1,2,3-oxadiazol-3-ium-5-yl)amide
- ((3-(2-(Benzylamino)-2-oxoethyl)-5-(trifluoromethyl)phenyl)carbamoyl)(3-(pyridin-2-ylmethyl)-1,2,3-oxadiazol-3-ium-5-yl)amide
- (3-((1S,4S)-4-Aminocyclohexyl)-1,2,3-oxadiazol-3-ium-5-yl)((3-(2-phenylacetamido)-5-(trifluoromethyl)phenyl)carbamoyl)amide
- (3-((1R,4R)-4-Aminocyclohexyl)-1,2,3-oxadiazol-3-ium-5-yl)((3-(2-phenylacetamido)-5-(trifluoromethyl)phenyl)carbamoyl)amide
- (3-((1R,4R)-4-(Aminomethyl)cyclohexyl)-1,2,3-oxadiazol-3-ium-5-yl)((3-(2-phenyl-acetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(2-phenylacetamido)-5-(trifluoromethyl)phenyl)-carbamoyl)amide
- (3-((1S,4S)-4-(Dimethylamino)cyclohexyl)-1,2,3-oxadiazol-3-ium-5-yl)((3-(2-phenylacetamido)-5-(trifluoromethyl)phenyl)-carbamoyl)amide
- (3-((1R,4R)-4-(dimethylamino)cyclohexyl)-1,2,3-oxadiazol-3-ium-5-yl)((3-(2-phenylacetamido)-5-(trifluoromethyl)phenyl)-carbamoyl)amide
- (3-(4-(Methoxycarbonyl)benzyl)-1,2,3-oxadiazol-3-ium-5-yl)((3-(2-phenylacetamido)-5-(trifluoromethyl)phenyl)carbamoyl)amide
- ((3-(2-Phenylacetamido)-5-(trifluoromethyl)-phenyl)carbamoyl)(3-(piperidin-4-yl)-1,2,3-oxadiazol-3-ium-5-yl)amide
- (3-(1-Methylpiperidin-4-yl)-1,2,3-oxadiazol-3-ium-5-yl)((3-(2-phenylacetamido)-5-(trifluoromethyl)phenyl)carbamoyl)amide
- (3-(4-Carboxybenzyl)-1,2,3-oxadiazol-3-ium-5-yl)((3-(2-phenylacetamido)-5-(trifluoromethyl)-phenyl)carbamoyl)amide
- (3-(4-(Hydroxymethyl)benzyl)-1,2,3-oxadiazol-3-ium-5-yl)((3-(2-phenylacetamido)-5-(trifluoromethyl)phenyl)carbamoyl)amide
- (3-(4-(Aminomethyl)benzyl)-1,2,3-oxadiazol-3-ium-5-yl)((3-(2-phenylacetamido)-5-(trifluoromethyl)phenyl)carbamoyl)amide
- (3-(4-Carbamoylbenzyl)-1,2,3-oxadiazol-3-ium-5-yl)((3-(2-phenylacetamido)-5-(trifluoromethyl)phenyl)carbamoyl)amide
- (3-((1-Methylpiperidin-2-yl)methyl)-1,2,3-oxadiazol-3-ium-5-yl)((3-(2-phenylacetamido)-5-(trifluoromethyl)phenyl)carbamoyl)amide
- (3-((1-Methylpiperidin-4-yl)methyl)-1,2,3-oxadiazol-3-ium-5-yl)((3-(2-phenylacetamido)-5-(trifluoromethyl)phenyl)carbamoyl)amide
- (3-(3-((1,3-Dioxoisoindolin-2-yl)methyl)benzyl)-1,2,3-oxadiazol-3-ium-5-yl)((3-(2-phenylacetamido)-5-(trifluoromethyl)phenyl)carbamoyl)amide
- (3-(3-(Aminomethyl)benzyl)-1,2,3-oxadiazol-3-ium-5-yl)((3-(2-phenylacetamido)-5-(trifluoromethyl)phenyl)carbamoyl)amide
- (3-(4-(2-Methoxy-2-oxoethyl)benzyl)-1,2,3-oxadiazol-3-ium-5-yl)((3-(2-phenylacetamido)-5-(trifluoromethyl)phenyl)carbamoyl)amide
- ((3-(2-Phenylacetamido)-5-(trifluoromethyl)-phenyl)carbamoyl)(3-(piperidin-3-ylmethyl)-1,2,3-oxadiazol-3-ium-5-yl)amide
- (3-(4-(2-Hydroxyethyl)benzyl)-1,2,3-oxadiazol-3-ium-5-yl)((3-(2-phenylacetamido)-5-(trifluoromethyl)phenyl)carbamoyl)amide
- (3-(9-Amino-3-oxabicyclo[3.3.1]nonan-7-yl)-1,2,3-oxadiazol-3-ium-5-yl)((3-(2-phenylacetamido)-5-(trifluoromethyl)phenyl)-carbamoyl)amide
- (3-((5-(Methoxycarbonyl)pyridin-2-yl)methyl)-1,2,3-oxadiazol-3-ium-5-yl)((3-(2-phenylacetamido)-5-(trifluoromethyl)phenyl)carbamoyl)amide
- (3-(2-(Aminomethyl)benzyl)-1,2,3-oxadiazol-3-ium-5-yl)((3-(2-phenylacetamido)-5-(trifluoromethyl)phenyl)carbamoyl)amide
- (3-((1,4-Oxazepan-2-yl)methyl)-1,2,3-oxadiazol-3-ium-5-yl)((3-(2-phenylacetamido)-5-(trifluoromethyl)phenyl)carbamoyl)amide
- (3-((5-(Hydroxymethyl)pyridin-2-yl)methyl)-1,2,3-oxadiazol-3-ium-5-yl)((3-(2-phenylacetamido)-5-(trifluoromethyl)phenyl)-carbamoyl)amide
- (3-((1-Methylpiperidin-3-yl)methyl)-1,2,3-oxadiazol-3-ium-5-yl)((3-(2-phenylacetamido)-5-(trifluoromethyl)phenyl)carbamoyl)amide
- (3-((3-Aminocyclobutyl)methyl)-1,2,3-oxadiazol-3-ium-5-yl)((3-(2-phenylacetamido)-5-(trifluoromethyl)phenyl)carbamoyl)amide
- (3-((1S,4S)-4-(Aminomethyl)cyclohexyl)-1,2,3-oxadiazol-3-ium-5-yl)((3-(2-oxo-3-phenyl-pyrrolidin-1-yl)-5-(trifluoromethyl)phenyl)-carbamoyl)amide
- ((3-(2-Phenylacetamido)-5-(trifluoromethyl)-phenyl)carbamoyl)(3-(piperidin-4-ylmethyl)-1,2,3-oxadiazol-3-ium-5-yl)amide
- (3-((5-(Isopropoxycarbonyl)pyridin-2-yl)methyl)-1,2,3-oxadiazol-3-ium-5-yl)((3-(2-phenylacetamido)-5-(trifluoromethyl)phenyl)-carbamoyl)amide
- ((3-(2-Oxo-4-phenylpyrrolidin-1-yl)-5-(trifluoromethyl)phenyl)carbamoyl)(3-(pyridin-2-ylmethyl)-1,2,3-oxadiazol-3-ium-5-yl)amide
- ((3-(2-(6-Oxo-1,6-dihydropyridin-3-yl)acetamido)-5-(trifluoromethyl)phenyl)-carbamoyl)(3-(pyridin-2-ylmethyl)-1,2,3-oxadiazol-3-ium-5-yl)amide
- ((3-(2-(1H-Benzo[d]imidazol-6-yl)acetamido)-5-(trifluoromethyl)phenyl)carbamoyl)(3-(pyridin-2-ylmethyl)-1,2,3-oxadiazol-3-ium-5-yl)amide
- ((3-(2-Phenylacetamido)-5-(trifluoromethyl)-phenyl)carbamoyl)(3-(piperidin-2-ylmethyl)-1,2,3-oxadiazol-3-ium-5-yl)amide
- ((3-(2-(1H-Indazol-7-yl)acetamido)-5-(trifluoromethyl)phenyl)carbamoyl)(3-(pyridin-2-ylmethyl)-1,2,3-oxadiazol-3-ium-5-yl)amide
- ((3-Cyclopropyl-5-(2-phenylacetamido)phenyl)-carbamoyl)(3-((1-methylpiperidin-2-yl)methyl)-1,2,3-oxadiazol-3-ium-5-yl)amide
- ((3-Cyclopropyl-5-(2-phenylacetamido)phenyl)-carbamoyl)(3-((5-(methoxycarbonyl)pyridin-2-yl)methyl)-1,2,3-oxadiazol-3-ium-5-yl)amide
- ((3-Cyclopropyl-5-(2-phenylacetamido)phenyl)-carbamoyl)(3-((5-(hydroxymethyl)pyridin-2-yl)methyl)-1,2,3-oxadiazol-3-ium-5-yl)amide
- ((3-((1R,2R)-2-Methylcyclopropyl)-5-(2-phenylacetamido)phenyl)carbamoyl)(3-(pyridin-2-ylmethyl)-1,2,3-oxadiazol-3-ium-5-yl)amide
- (3-(((1S,4S)-4-Aminocyclohexyl)methyl)-1,2,3-oxadiazol-3-ium-5-yl)((3-(2-oxo-3-phenylpyrrolidin-1-yl)-5-(trifluoromethyl)-phenyl)carbamoyl)amide
- (3-(((1R,4R)-4-Aminocyclohexyl)methyl)-1,2,3-oxadiazol-3-ium-5-yl)((3-(2-oxo-3-phenylpyrrolidin-1-yl)-5-(trifluoromethyl)-phenyl)carbamoyl)amide
- (3-(((1S,4S)-4-(Aminomethyl)cyclohexyl)-methyl)-1,2,3-oxadiazol-3-ium-5-yl)((3-(2-oxo-3-phenylpyrrolidin-1-yl)-5-(trifluoromethyl)-phenyl)carbamoyl)amide
- (3-((3-(((5-Hydroxypyridin-2-yl)methyl)amino)-cyclobutyl)methyl)-1,2,3-oxadiazol-3-ium-5-yl)((3-(2-phenylacetamido)-5-(trifluoromethyl)-phenyl)carbamoyl)amide
- ((3-(2-Oxo-3-phenylpyrrolidin-1-yl)-5-(trifluoromethyl)phenyl)carbamoyl)(3-((1S,4S)-4-(((2,2,2-trifluoroethyl)amino)methyl)-cyclohexyl)-1,2,3-oxadiazol-3-ium-5-yl)amide
- ((3-(2-Oxo-3-phenylpyrrolidin-1-yl)-5-(trifluoromethyl)phenyl)carbamoyl)(3-(((1s,4s)-4-((2,2,2-trifluoroethyl)amino)cyclohexyl)methyl)-1,2,3-oxadiazol-3-ium-5-yl)amide
- ((3-(2-Oxo-3-phenylpyrrolidin-1-yl)-5-(trifluoromethyl)phenyl)carbamoyl)(3-(((1R,4R)-4-((2,2,2-trifluoroethyl)amino)-cyclohexyl)methyl)-1,2,3-oxadiazol-3-ium-5-yl)amide
- (3-((1S,4S)-4-((Dimethylamino)methyl)-cyclohexyl)-1,2,3-oxadiazol-3-ium-5-yl)((3-(2-oxo-3-phenylpyrrolidin-1-yl)-5-(trifluoromethyl)-phenyl)carbamoyl)amide
- ((3-(2-Oxo-3-phenylpyrrolidin-1-yl)-5-(trifluoromethyl)phenyl)carbamoyl)(3-(((1s,4s)-4-(((2,2,2-trifluoroethyl)amino)methyl)cyclohexyl)-methyl)-1,2,3-oxadiazol-3-ium-5-yl)amide
- (3-((1-(2-Ethoxy-2-oxoethyl)piperidin-4-yl)methyl)-1,2,3-oxadiazol-3-ium-5-yl)((3-(2-phenylacetamido)-5-(trifluoromethyl)phenyl)-carbamoyl)amide
- (3-((1-(2-Hydroxyethyl)piperidin-4-yl)methyl)-1,2,3-oxadiazol-3-ium-5-yl)((3-(2-phenylacetamido)-5-(trifluoromethyl)phenyl)-carbamoyl)amide
- ((3-(2-Phenylacetamido)-5-(trifluoromethyl)-phenyl)carbamoyl)(3-((1-(2,2,2-trifluoroethyl)-piperidin-2-yl)methyl)-1,2,3-oxadiazol-3-ium-5-yl)amide
- (3-(((1R,4R)-4-Aminocyclohexyl)methyl)-1,2,3-oxadiazol-3-ium-5-yl)((3-((R)-2-oxo-3-phenylpyrrolidin-1-yl)-5-(trifluoromethyl)-phenyl)carbamoyl)amide
- ((3-((R)-2-Oxo-3-phenylpyrrolidin-1-yl)-5-(trifluoromethyl)phenyl)carbamoyl)(3-(((1r,4r)-4-((2,2,2-trifluoroethyl)amino)cyclohexyl)-methyl)-1,2,3-oxadiazol-3-ium-5-yl)amide
- ((3-((R)-2-Oxo-3-phenylpyrrolidin-1-yl)-5-(trifluoromethyl)phenyl)carbamoyl)(3-(((1R,4R)-4-(2,2,2-trifluoroacetamido)cyclohexyl)methyl)-1,2,3-oxadiazol-3-ium-5-yl)amide
- (3-(((1R,4R)-4-(Methyl(2,2,2-trifluoroethyl)-amino)cyclohexyl)methyl)-1,2,3-oxadiazol-3-ium-5-yl)((3-((R)-2-oxo-3-phenylpyrrolidin-1-yl)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(2-phenylacetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(2-oxo-3-phenylpyrrolidin-1-yl)-5-(trifluoromethyl)-phenyl)carbamoyl)amide
- (3-((5-(4,6-Dimethylpyrimidin-5-yl)pyridin-2-yl)methyl)-1,2,3-oxadiazol-3-ium-5-yl)((3-(2-phenylacetamido)-5-(trifluoromethyl)phenyl)-carbamoyl)amide
- ((3-Cyclopropyl-5-(2-(phenylsulfonyl)-acetamido)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-(phenylsulfonyl)acetamido)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(2-(3-methoxyphenyl)acetamido)-5-(trifluoromethyl)-phenyl)carbamoyl)amide
- (3-((1R,4R)-4-((Dimethylamino)methyl)-cyclohexyl)-1,2,3-oxadiazol-3-ium-5-yl)((3-(2-(2-methoxyphenyl)acetamido)-5-(trifluoromethyl)-phenyl)carbamoyl)amide
- ((3-(2-(Benzo[d][1,3]dioxol-5-yl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2-(3-(Carboxymethyl)phenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2-(4-Carboxyphenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(3-(1-methyl-1H-indol-3-yl)propanamido)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(3-(pyrazin-2-yl)propanamido)-5-(trifluoromethyl-)phenyl)carbamoyl)amide
- ((3-(2-(2-Carboxyphenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2,2-Difluoro-3-(pyrrolidin-1-yl)propanamido)-5-(trifluoromethyl)phenyl)-carbamoyl)(3-((1R,4R)-4-((dimethylamino)-methyl)cyclohexyl)-1,2,3-oxadiazol-3-ium-5-yl)amide
- (3-((1R,4R)-4-((dimethylamino)methyl)-cyclohexyl)-1,2,3-oxadiazol-3-ium-5-yl)((3-(2-(1-methyl-1H-indol-3-yl)acetamido)-5-(trifluoro-methyl)phenyl)carbamoyl)amide
- ((3-(2-(3-(2-Amino-2-oxoethyl)phenyl)-acetamido)-5-(trifluoromethyl)phenyl)-carbamoyl)(3-((1R,4R)-4-((dimethylamino)-methyl)cyclohexyl)-1,2,3-oxadiazol-3-ium-5-yl)amide
- ((3-(2-(3-(Aminomethyl)phenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-phenylpropanamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(2-phenylbutanamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(trifluoromethyl)-5-(2-(2-(trifluoromethyl)-phenyl)acetamido)phenyl)carbamoyl)amide
- (3-((1R,4R)-4-((Dimethylamino)methyl)-cyclohexyl)-1,2,3-oxadiazol-3-ium-5-yl)((3-(N-methyl-2-phenylpropanamido)-5-(trifluoro-methyl)phenyl)carbamoyl)amide
- ((3-(2-(2-Chlorophenyl)acetamido)-5-(trifluoro-methyl)phenyl)carbamoyl)(3-((1R,4R)-4-((dimethylamino)methyl)cyclohexyl)-1,2,3-oxadiazol-3-ium-5-yl)amide
- ((3-(2-(2,3-Difluorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4S)-4-((Dimethylamino)methyl)-cyclohexyl)-1,2,3-oxadiazol-3-ium-5-yl)((3-((S)-2-phenylpropanamido)-5-(trifluoromethyl)-phenyl)carbamoyl)amide
- (3-((1R,4R)-4-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-(N-methyl-2-phenylacetamido)-5-(trifluoromethyl)-phenyl)carbamoyl)amide
- (3-((1R,3R)-3-(Dimethylamino)cyclohexyl)-1,2,3-oxadiazol-3-ium-5-yl)((3-(2-phenylacetamido)-5-(trifluoromethyl)phenyl)-carbamoyl)amide
- (3-((1R,3S)-3-(Dimethylamino)cyclohexyl)-1,2,3-oxadiazol-3-ium-5-yl)((3-(2-phenyl-acetamido)-5-(trifluoromethyl)phenyl)-carbamoyl)amide
- (3-((1R,4R)-4-((Dimethylamino)methyl)-cyclohexyl)-1,2,3-oxadiazol-3-ium-5-yl)((3-((R)-2-phenylpropanamido)-5-(trifluoromethyl)-phenyl)carbamoyl)amide
- ((3-(2-(3-Chlorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(1,2,3,4-tetrahydronaphthalene-1-carboxamido)-5-(trifluoromethyl)phenyl)carbamoyl)amide
- rac-(3-((1-Methyl-1,2,3,4-tetrahydroquinolin-2-yl)methyl)-1,2,3-oxadiazol-3-ium-5-yl)((2-(trifluoromethyl)pyridin-4-yl)carbamoyl)amide
- (3-(((1S,4S)-4-(Pyrimidin-5-yl)cyclohexyl)-methyl)-1,2,3-oxadiazol-3-ium-5-yl)((3-(trifluoromethyl)phenyl)carbamoyl)amide
- rac-(((3R,5S)-1,1-Difluorospiro[2.4]heptan-5-yl)carbamoyl)(3-((5-(4,6-dimethylpyrimidin-5-yl)pyridin-2-yl)methyl)-1,2,3-oxadiazol-3-ium-5-yl)amide
- rac-(3-((2-Methyl-1,2,3,4-tetrahydroisoquinolin -1-yl)methyl)-1,2,3-oxadiazol-3-ium-5-yl)((2-(trifluoromethyl)pyridin-4-yl)carbamoyl)amide
- (3-(((1S,4S)-4-(Pyrimidin-5-yl)cyclohexyl) methyl)-1,2,3-oxadiazol-3-ium-5-yl)((2-(trifluoro methyl)pyridin-4-yl)carbamoyl)amide
- (3-((3-(Pyrimidin-5-yl)bicyclo[1.1.1]pentan-1-yl)methyl)-1,2,3-oxadiazol-3-ium-5-yl)((3-(trifluoromethyl)phenyl)carbamoyl)amide
- (3-((6-(4,6-Dimethylpyrimidin-5-yl)pyridazin-3-yl)methyl)-1,2,3-oxadiazol-3-ium-5-yl)((3-(trifluoromethyl)phenyl)carbamoyl)amide
- (3-((4-(Pyrimidin-5-yl)bicyclo[2.2.1]heptan-1-yl)methyl)-1,2,3-oxadiazol-3-ium-5-yl)((2-(trifluoromethyl)pyridin-4-yl)carbamoyl)amide
- (3-(((1R,4R)-4-(Pyrimidin-5-yl)cyclohexyl) methyl)-1,2,3-oxadiazol-3-ium-5-yl)((3-(trifluoro methyl)phenyl)carbamoyl)amide
- (3-(((1R,4R)-4-(Pyrimidin-5-yl)cyclohexyl)-methyl)-1,2,3-oxadiazol-3-ium-5-yl)((2-(trifluoromethyl)pyridin-4-yl)carbamoyl)amide
- (3-(4-(3-Methylpyridazin-4-yl)benzyl)-1,2,3-oxadiazol-3-ium-5-yl)((2-(trifluoromethyl)-pyridin-4-yl)carbamoyl)amide
- ((2-(Trifluoromethyl)pyridin-4-yl)carbamoyl)(3-(4-(4-(trifluoromethyl)pyrimidin-5-yl)benzyl)-1,2,3-oxadiazol-3-ium-5-yl)amide
- (3-(4-(4-Methoxy-6-methylpyrimidin-5-yl)benzyl)-1,2,3-oxadiazol-3-ium-5-yl)((2-(trifluoromethyl)pyridin-4-yl)carbamoyl)amide
- (3-((1R,4R)-4-((Dimethylamino)methyl)-cyclohexyl)-1,2,3-oxadiazol-3-ium-5-yl)((3-(2-(2-hydroxyphenyl)acetamido)-5-(trifluoromethyl)-phenyl)carbamoyl)amide
- rac-(3-((2-Methyl-1,2,3,4-tetrahydro isoquinolin-1-yl)methyl)-1,2,3-oxadiazol-3-ium-5-yl)((3-(2-phenylacetamido)-5-(trifluoromethyl) phenyl)carbamoyl)amide
- ((2-(2-(2-Chlorophenyl)acetamido)-6-(trifluoromethyl)pyridin-4-yl)carbamoyl)(3-((1R,4R)-4-((dimethylamino)methyl)-cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-fluoro-2-(2-fluorophenyl)acetamido)-5-(trifluoromethyl)phenyl)carbamoyl)amide
- ((3-(Bicyclo[4.2.0]octa-1(6),2,4-triene-7-carboxamido)-5-(trifluoromethyl)phenyl)-carbamoyl)(3-((1R,4R)-4-((dimethylamino-methyl)cyclohexyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-(6-hydroxypyridin-2-yl)acetamido)-5-(trifluoromethyl)phenyl)carbamoyl)amide
- (3-((1R,4R)-4-((Dimethylamino)methyl)-cyclohexyl)-1,2,3-oxadiazol-3-ium-5-yl)((3-(2-(3,5-dimethylisoxazol-4-yl)acetamido)-5-(trifluoromethyl)phenyl)carbamoyl)amide
- ((3-(2-(2-Chloro-6-fluorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- ((3-(2-(2,6-Difluorophenyl)acetamido)-5-(trifluoromethyl)phenyl)carbamoyl)(3-((1R,4R)-4-((dimethylamino)methyl)cyclohexyl)-1,2,3-oxadiazol-3-ium-5-yl)amide
- Rac-(3-((1,2,3,4-Tetrahydronaphthalen-1-yl)methyl)-1,2,3-oxadiazol-3-ium-5-yl)((2-(trifluoromethyl)pyridin-4-yl)carbamoyl)amide
- (3-(4-(1,4-Dimethyl-1H-pyrazol-5-yl)benzyl)-1,2,3-oxadiazol-3-ium-5-yl)((2-(trifluoromethyl)-pyridin-4-yl)carbamoyl)amide
- (3-((1R,3S)-3-(Dimethylamino)cyclohexyl)-1,2,3-oxadiazol-3-ium-5-yl)((3-(2-(o-tolyl)acetamido)-5-(trifluoromethyl)phenyl)-carbamoyl)amide
- (3-((1R,2R)-2-Hydroxycyclohexyl)-1,2,3-oxadiazol-3-ium-5-yl)((3-(2-(o-tolyl)acetamido)-5-(trifluoromethyl)phenyl)carbamoyl)amide
- (3-((1R,3S)-3-(Dimethylamino)cyclohexyl)-1,2,3-oxadiazol-3-ium-5-yl)((2-(trifluoromethyl)-pyridin-4-yl)carbamoyl)amide
- ((3-(2-(2-Chlorophenyl)propanamido)-5-(trifluoromethyl)phenyl)carbamoyl)(3-((1R,4R)-4-((dimethylamino)methyl)cyclohexyl)-1,2,3-oxadiazol-3-ium-5-yl)amide
- (3-((1R,4R)-4-(2-Hydroxypropan-2-yl)cyclohexyl)-1,2,3-oxadiazol-3-ium-5-yl)((3-(2-(o-tolyl)acetamido)-5-(trifluoromethyl)phenyl)-carbamoyl)amide
- ((4-Cyano-3-(trifluoromethyl)phenyl)-carbamoyl)(3-(4-(4,6-dimethylpyrimidin-5-yl)benzyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-(2-methyl-2-phenylpropanamido)-5-(trifluoro-methyl)phenyl)carbamoyl)amide
- (3-(4-(4,6-Dimethylpyrimidin-5-yl)benzyl)-1,2,3-oxadiazol-3-ium-5-yl)((2-methoxypyridin-4-yl)carbamoyl)amide
- (3-(4-(2-Methylthiazol-4-yl)benzyl)-1,2,3-oxadiazol-3-ium-5-yl)((2-(trifluoromethyl)pyridin -4-yl)carbamoyl)amide
- (3-(4-(3,5-Dimethylisothiazol-4-yl)benzyl)-1,2,3-oxadiazol-3-ium-5-yl)((2-(trifluoromethyl)-pyridin-4-yl)carbamoyl)amide
- (3-((1R,4R)-4-((Dimethylamino)methyl)-cyclohexyl)-1,2,3-oxadiazol-3-ium-5-yl)((3-(2-(2,3-dimethylphenyl)acetamido)-5-(trifluoromethyl)phenyl)carbamoyl)amide
- (3-((1S,3R)-3-((Dimethylamino)methyl)-cyclohexyl)-1,2,3-oxadiazol-3-ium-5-yl)((3-(2-(o-tolyl)acetamido)-5-(trifluoromethyl)phenyl) carbamoyl)amide
- (3-((1R,3S)-3-Hydroxycyclohexyl)-1,2,3-oxadiazol-3-ium-5-yl)((3-(2-(o-tolyl)acetamido)-5-(trifluoromethyl)phenyl)carbamoyl)amide
- (3-((1R,3R)-3-Hydroxycyclohexyl)-1,2,3-oxadiazol-3-ium-5-yl)((3-(2-(o-tolyl)acetamido)-5-(trifluoromethyl)phenyl)carbamoyl)amide
- (3-((4-(4,6-Dimethylpyrimidin-5-yl)cyclohex-3-en-1-yl)methyl)-1,2,3-oxadiazol-3-ium-5-yl)((2-(trifluoromethyl)pyridin-4-yl)carbamoyl)amide
- (3-(((1R,2R)-2Hydroxycyclohexyl)methyl)-1,2,3-oxadiazol-3-ium-5-yl)((3-(2-(o-tolyl)acetamido)-5-(trifluoromethyl)phenyl)-carbamoyl)amide
- (3-(((1S,2R)-2-Hydroxycyclohexyl)methyl)-1,2,3-oxadiazol-3-ium-5-yl)((3-(2-(o-tolyl)acetamido)-5-(trifluoromethyl)phenyl)-carbamoyl)amide
- (3-((1S,3S)-3-(Pyrimidin-5-yl)cyclohexyl)-1,2,3-oxadiazol-3-ium-5-yl)((2-(trifluoromethyl)pyridin -4-yl)carbamoyl)amide
- (3-((1S,3R)-3-(Pyrimidin-5-yl)cyclohexyl)-1,2,3-oxadiazol-3-ium-5-yl)((2-(trifluoromethyl)-pyridin-4-yl)carbamoyl)amide
- (3-((1S,3S)-3-(Pyrimidin-5-yl)cyclohexyl)-1,2,3-oxadiazol-3-ium-5-yl)((3-(2-(o-tolyl)acetamido)-5-(trifluoromethyl)phenyl)carbamoyl)amide
- (3-((1S,3R)-3-(Pyrimidin-5-yl)cyclohexyl)-1,2,3-oxadiazol-3-ium-5-yl)((3-(2-(o-tolyl)acetamido)-5-(trifluoromethyl)phenyl)-carbamoyl)amide
- (3-((1R,3S)-3-(Methoxycarbonyl)cyclohexyl)-1,2,3-oxadiazol-3-ium-5-yl)((3-(2-(o-tolyl)acetamido)-5-(trifluoromethyl)phenyl)-carbamoyl)amide
- (3-((1R,3R)-3-(Methoxycarbonyl)cyclohexyl)-1,2,3-oxadiazol-3-ium-5-yl)((3-(2-(o-tolyl)-acetamido)-5-(trifluoromethyl)phenyl)-carbamoyl)amide
- (3-(4-(4-Methylpyrimidin-5-yl)benzyl)-1,2,3-oxadiazol-3-ium-5-yl)((2-(trifluoromethyl)pyridin -4-yl)carbamoyl)amide
- ((2-(tert-Butyl)pyridin-4-yl)carbamoyl)(3-(4-(4,6-dimethylpyrimidin-5-yl)benzyl)-1,2,3-oxadiazol-3-ium-5-yl)amide
- (3-((1R,4R)-4-(Methoxycarbonyl)cyclohexyl)-1,2,3-oxadiazol-3-ium-5-yl)((3-(2-(o-tolyl)-acetamido)-5-(trifluoromethyl)phenyl)carbamoyl)
- amide
- (3-((1R,4R)-4-(Hydroxymethyl)cyclohexyl)-1,2,3-oxadiazol-3-ium-5-yl)((3-(2-(o-tolyl)acetamido)-5-(trifluoromethyl)phenyl) carbamoyl)amide
- (3-((1R,4R)-4-Carboxycyclohexyl)-1,2,3-oxadiazol-3-ium-5-yl)((3-(2-(o-tolyl)acetamido)-5-(trifluoromethyl)phenyl)carbamoyl)amide
- (3-((1R)-3-(Hydroxymethyl)cyclohexyl)-1,2,3-oxadiazol-3-ium-5-yl)((3-(2-(o-tolyl)acetamido)-5-(trifluoromethyl)phenyl)carbamoyl)amide
- (3-((1R,3R)-3-(Methylcarbamoyl)cyclohexyl)-1,2,3-oxadiazol-3-ium-5-yl)((3-(2-(o-tolyl)acetamido)-5-(trifluoromethyl)phenyl)-carbamoyl)amide
- (3-((1R,3S)-3-(Methylcarbamoyl)cyclohexyl)-1,2,3-oxadiazol-3-ium-5-yl)((3-(2-(o-tolyl)acetamido)-5-(trifluoromethyl)phenyl)-carbamoyl)amide
- (3-(4-(4,6-Dimethylpyrimidin-5-yl)benzyl)-1,2,3-oxadiazol-3-ium-5-yl)(((1S,3S)-3-(trifluoromethyl)cyclopentyl)carbamoyl)amide
- (3-((1R,3S)-3-(Dimethylcarbamoyl)cyclohexyl)-1,2,3-oxadiazol-3-ium-5-yl)((3-(2-(o-tolyl)-acetamido)-5-(trifluoromethyl)phenyl)-carbamoyl)amide
- (3-((1R,3R)-3-Carbamoylcyclohexyl)-1,2,3-oxadiazol-3-ium-5-yl)((3-(2-(o-tolyl)acetamido)-5-(trifluoromethyl)phenyl)carbamoyl)amide
- (3-(4-(Pyrimidin-5-yl)benzyl)-1,2,3-oxadiazol-3-ium-5-yl)((2-(trifluoromethyl)pyridin-4-yl)-carbamoyl)amide
- (3-(4-(1,3-Dimethyl-1H-pyrazol-4-yl)benzyl)-1,2,3-oxadiazol-3-ium-5-yl)((2-(trifluoromethyl)-pyridin-4-yl)carbamoyl)amide
- ((4-Cyano-3-(trifluoromethyl)phenyl)-carbamoyl)(3-(4-(1,4-dimethyl-1H-pyrazol-5-yl)benzyl)-1,2,3-oxadiazol-3-ium-5-yl)amide
- (3-((4-(4-Methyl-2-(oxetan-3-yl)pyrazol-3-yl]phenyl)methyl)oxadiazol-3-ium-5-yl)-((2-(trifluoromethyl)pyridin-4-yl)carbamoyl)-azanide
- (3-((1S,2R)-2-Hydroxycyclohexyl)-1,2,3-oxadiazol-3-ium-5-yl)((3-(2-(o-tolyl)acetamido)-5-(trifluoromethyl)phenyl)carbamoyl)amide
- (3-(((1R,4S)-4-(4-Methylpyrimidin-5-yl)cyclohexyl)methyl)-1,2,3-oxadiazol-3-ium-5-yl)((2-(trifluoromethyl)pyridin-4-yl)carbamoyl)-amide
- (3-(4-(1,4-Dimethyl-1H-pyrazol-5-yl)benzyl)-1,2,3-oxadiazol-3-ium-5-yl)((2-(trifluoromethyl)-pyridin-4-yl)carbamoyl)amide
- (3-((1R,4S)-4-((Dimethylamino)methyl)-cyclohexyl)-1,2,3-oxadiazol-3-ium-5-yl)((3-((S)-2-fluoro-2-(2-fluorophenyl)acetamido)-5-(trifluoromethyl)phenyl)carbamoyl)amide
- rac-(3-((1R,2S)-2-(Dimethylamino)cyclohexyl)-1,2,3-oxadiazol-3-ium-5-yl)((3-(2-(o-tolyl)acetamido)-5-(trifluoromethyl)phenyl)-carbamoyl)amide
- rac-(3-((1R,3S)-3-(Methylcarbamoyl)cyclohexyl)-1,2,3-oxadiazol-3-ium-5-yl)((3-(2-(o-tolyl)acetamido)-5-(trifluoromethyl)phenyl)carbamoyl)amide
- rac-(3-((1S,3R)-3-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide
- rac-(3-((1R,3 S)-3-((Dimethylamino)methyl)-cyclohexyl)-1,2,3-oxadiazol-3-ium-5-yl)((3-(2-(o-tolyl)acetamido)-5-(trifluoromethyl)phenyl)-carbamoyl)amide
- (3-((1R,4R)-4-(Dimethylcarbamoyl)-cyclohexyl)-1,2,3-oxadiazol-3-ium-5-yl)((3-(2-(o-tolyl)acetamido)-5-(trifluoromethyl)phenyl)-carbamoyl)amide
- (3-(4-(4,6-Dimethylpyrimidin-5-yl)benzyl)-1,2,3-oxadiazol-3-ium-5-yl)((4-methoxy-3-(trifluoromethyl)phenyl)carbamoyl)amide
- (3-(4-(4-Methyl-1-(oxetan-3-yl)-1H-pyrazol-3-yl)benzyl)-1,2,3-oxadiazol-3-ium-5-yl)((2-(trifluoromethyl)pyridin-4-yl)carbamoyl)amide
- rac-(3-((1R,4R)-4-(Methylcarbamoyl)-cyclohexyl)-1,2,3-oxadiazol-3-ium-5-yl)((3-(2-(o-tolyl)acetamido)-5-(trifluoromethyl)phenyl)-carbamoyl)amide
- rac-(3-((1R,3R)-3-(Methylcarbamoyl)-cyclohexyl)-1,2,3-oxadiazol-3-ium-5-yl)((3-(2-(o-tolyl)-acetamido)-5-(trifluoromethyl)phenyl)-carbamoyl)amide
- rac-(3-((1R,3S)-3-(Dimethylcarbamoyl)-cyclohexyl)-1,2,3-oxadiazol-3-ium-5-yl)((3-(2-(o-tolyl)-acetamido)-5-(trifluoromethyl)phenyl)-carbamoyl)amide
- (3-((1R,4R)-4-(Pyrrolidine-1-carbonyl)-cyclohexyl)-1,2,3-oxadiazol-3-ium-5-yl)((3-(2-(o-tolyl)acetamido)-5-(trifluoromethyl)phenyl)-carbamoyl)amide
- (3-((1R,3S)-3-Carbamoylcyclohexyl)-1,2,3-oxadiazol-3-ium-5-yl)((3-(2-(o-tolyl)acetamido)-5-(trifluoromethyl)phenyl)carbamoyl)amide
- (3-((4-(4,6-Dimethylpyrimidin-5-yl)phenyl)methyl)oxadiazol-3-ium-5-yl)-((rac-(1R,3R)-3-(trifluoromethyl)cyclopentyl)-carbamoyl)azanide
- (3-((1R,4R)-4-(Dimethylamino)cyclohexyl)-1,2,3-oxadiazol-3-ium-5-yl)((3-(2-(o-tolyl)-acetamido)-5-(trifluoromethyl)phenyl)-carbamoyl)amide
- (3-(4-(2-Methylpyridin-3-yl)benzyl)-1,2,3-oxadiazol-3-ium-5-yl)((2-(trifluoromethyl)pyridin -4-yl)carbamoyl)amide
- (3-(4-(2,5-Dimethylthiazol-4-yl)benzyl)-1,2,3-oxadiazol-3-ium-5-yl)((2-(trifluoromethyl)pyridin -4-yl)carbamoyl)amide
- (3-(4-(2-Amino-4-methylpyrimidin-5-yl)benzyl)-1,2,3-oxadiazol-3-ium-5-yl)((2-(trifluoromethyl)pyridin-4-yl)carbamoyl)amide
- (3-(4-(4,6-Dimethylpyrimidin-5-yl)benzyl)-1,2,3-oxadiazol-3-ium-5-yl)((4-hydroxy-3-(trifluoromethyl)phenyl)carbamoyl)amide
- (3-(4-(4,6-Dimethylpyrimidin-5-yl)benzyl)-1,2,3-oxadiazol-3-ium-5-yl)((4-(methoxycarbonyl)-3-(trifluoromethyl)phenyl)carbamoyl) amide
- (3-(4-(2,4-Dimethylpyridin-3-yl)benzyl)-1,2,3-oxadiazol-3-ium-5-yl)((2-(trifluoromethyl)pyridin -4-yl)carbamoyl)amide
- ((4-Acetamido-3-(trifluoromethyl)phenyl)-carbamoyl)(3-(4-(4,6-dimethylpyrimidin-5-yl)benzyl)-1,2,3-oxadiazol-3-ium-5-yl)amide
- (3-(3-(Hydroxymethyl)cyclohexyl)-1,2,3-oxadiazol-3-ium-5-yl)((3-(2-(o-tolyl)acetamido)-5-(trifluoromethyl)phenyl)carbamoyl)amide
- (3-(4-(Pyrimidin-5-yl)benzyl)-1,2,3-oxadiazol-3-ium-5-yl)((2-(trifluoromethyl)pyridin-4-yl)carbamoyl)amide
- (3-((1R,3R)-3-Carbamoylcyclohexyl)-1,2,3-oxadiazol-3-ium-5-yl)((3-(2-(o-tolyl)acetamido)-5-(trifluoromethyl)phenyl)carbamoyl)amide
- ((2-(Trifluoromethyl)pyridin-4-yl)carbamoyl)(3-(4-(1,3,5-trimethyl-1H-pyrazol-4-yl)benzyl)-1,2,3-oxadiazol-3-ium-5-yl)amide
- (3-(4-(4-Methoxy-6-methylpyrimidin-5-yl)benzyl)-1,2,3-oxadiazol-3-ium-5-yl)((2-(trifluoromethyl)pyridin-4-yl)carbamoyl)amide
- ((2-(Trifluoromethyl)pyridin-4-yl)carbamoyl)(3-(4-(4-(trifluoromethyl)pyrimidin-5-yl)benzyl)-1,2,3-oxadiazol-3-ium-5-yl)amide
- (3-((1R,4R)-4-((Dimethylamino)methyl)-cyclohexyl)-1,2,3-oxadiazol-3-ium-5-yl)((3-((R)-2-fluoro-2-(2-fluorophenyl)acetamido)-5-(trifluoromethyl)phenyl)carbamoyl)amide
- (3-(4-(4,6-Dimethylpyrimidin-5-yl)benzyl)-1,2,3-oxadiazol-3-ium-5-yl)((8-(trifluoromethyl)-quinolin-6-yl)carbamoyl)amide
- (3-(4-(4,6-Dimethylpyrimidin-5-yl)benzyl)-1,2,3-oxadiazol-3-ium-5-yl)((4-(methylcarbamoyl)-3-(trifluoromethyl)phenyl)carbamoyl) amide.

7. The compound according to any one of claims 1 to 6 being an inhibitor of NS2B-NS3 protease of a Flavivirus, preferably an inhibitor of the NS2B-NS3 protease of Zika and/or Dengue and/or West Nile and/or Japan Encephalitis virus.

8. The compound according to any one of previous claims for medical use.

9. The compound according to claim 8 for use in treatment and/or prevention of a Flavivirus infection, preferably wherein the Flavivirus is selected from the group consisting of Dengue, West Nile, Zika and Japan Encephalitis virus.

10. A pharmaceutical composition comprising the compound according to any one of claims 1 to 6, either alone or in combination with one further therapeutic agent, and at least one pharmaceutically acceptable excipient.

11. The pharmaceutical composition of claim 10 for use in the treatment and/or prevention of a Flavivirus infection, preferably wherein the Flavivirus is selected from the group consisting of Dengue, West Nile, Zika and Japan Encephalitis virus.
